# EUROPEAN PATENT SPECIFICATION

(11) **EP 4 355 426 B1**
(45) Date of publication and mention of the grant of the patent: **29.07.2026**
(21) Application number: 22735138.4
(22) Date of filing: 16.06.2022
(51) Int. Cl.: A61P 35/00, C07D 487/04, C07D 519/00, A61K 31/519

(54) **SUBSTITUTED PYRAZOLO[1,5-A]PYRIMIDINE-7-AMINE COMPOUNDS AS CDK INHIBITORS AND THEIR THERAPEUTIC USE**
SUBSTITUIERTE PYRAZOLO[1,5-A PYRIMIDIN-7-AMIN-VERBINDUNGEN ALS CDK INHIBITOREN UND IHRE THERAPEUTISCHE VERWENDUNG
COMPOSÉS DE PYRAZOLO[1,5-A]PYRIMIDINE-7-AMINE SUBSTITUÉS EN TANT QU'INHIBITEURS DE CDK ET LEUR UTILISATION THÉRAPEUTIQUE

(30) Priority: 16.06.2021 GB 202108572
(43) Date of publication of application: 24.04.2024
(60) Divisional application: 26187195.8
(73) Proprietor: Carrick Therapeutics Limited, Dublin 2, D02 T380 (IE)
(72) Inventor: AINSCOW, Edward, Dublin 2 (IE); BAHL, Ashwani, Dublin 2 (IE); SUNOSE, Mihiro, Nottingham NG1 1GR (GB); CREPIN, Damien Francis Philippe, Nottingham NG1 1GR (GB); CHOHAN, Kamaldeep Kaur, Nottingham NG1 1GR (GB); TOSCHI, Gianna, Nottingham NG1 1GR (GB)
(74) Representative: Mewburn Ellis LLP
(86) International application number: PCT/EP2022/066504
(87) International publication number: WO 2022/263604

(56) References cited:
- WO-A1-2004/022561
- WO-A1-2019/057825
- WO-A1-2019/197549
- SLABICKI MIKOLAJ ET AL: "The CDK inhibitor CR8 acts as a molecular glue degrader that depletes cyclin K", NATURE, NATURE PUBLISHING GROUP UK, LONDON, vol. 585, no. 7824, 3 June 2020 (2020-06-03), pages 293 - 297, XP037241512, ISSN: 0028-0836, [retrieved on 20200603], DOI: 10.1038/S41586-020-2374-X

## Description

### TECHNICAL FIELD

The present invention pertains generally to the field of therapeutic compounds.

More specifically the present invention pertains to certain substituted pyrazolo[1,5-a]pyrimidine-7-amine compounds (referred to herein as "PPA compounds") that, *inter alia,* inhibit cyclin-dependent protein kinases (CDKs), especially CDK12 and/or CDK13, and are selective, for example, for CDK12 and/or CDK13 as compared to CDK7. In addition to selectively inhibiting CDK12 and/or CDK13, the compounds also act as selective Cyclin K degraders thereby removing the key cofactor required for CDK12 and/or CDK13 activation; this confers additional cellular potency and selectivity. The present invention also pertains to pharmaceutical compositions comprising such compounds, and the use of such compounds and compositions, both *in vitro* and *in vivo,* to inhibit CDK, especially CDK12 and/or CDK13; and to treat disorders including: disorders that are associated with CDK, especially CDK12 and/or CDK13; disorders that result from an inappropriate activity of a CDK, especially CDK12 and/or CDK13; disorders that are associated with CDK mutation, especially CDK12 and/or CDK13mutation; disorders that are associated with CDK overexpression, especially CDK12 and/or CDK13 overexpression; disorders that are associated with upstream pathway activation of CDK, especially CDK12 and/or CDK13; disorders that are ameliorated by the inhibition of CDK, especially CDK12 and/or CDK13; proliferative disorders; cancer; viral infections (including HIV); neurodegenerative disorders (including Alzheimer's disease and Parkinson's disease); ischaemia; renal diseases; cardiovascular disorders (including atherosclerosis); autoimmune disorders (including rheumatoid arthritis); and disorders caused by dysfunction of translation in cells (including muscular dystrophy). Optionally, the treatment further comprises treatment (e.g., simultaneous or sequential treatment) with a further active agent which is, e.g., a DNA repair inhibitor, an immune checkpoint inhibitor, an agent stimulating the immune system, a cell cycle checkpoint inhibitor, a Her2 blocker, a transcriptional inhibitor, a cytotoxic chemotherapeutic agent, etc.

### BACKGROUND

A number of publications are cited herein in order to more fully describe and disclose the invention and the state of the art to which the invention pertains.

Throughout this specification, including the claims which follow, unless the context requires otherwise, the word "comprise," and variations such as "comprises" and "comprising," will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Thus, for example, reference to "a pharmaceutical carrier" includes mixtures of two or more such carriers, and the like.

Ranges are often expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment.

This disclosure includes information that may be useful in understanding the present invention. It is not an admission that any of the information provided herein is prior art or relevant to the presently claimed invention, or that any publication specifically or implicitly referenced is prior art.

### Cyclin-Dependent Protein Kinase (CDK)

Cyclin-dependent protein kinases (CDK) are the catalytic subunits of a family of 21 serine/threonine protein kinases (see, e.g., Malumbres *et al.,* 2009), some of which control progression of the cell through the stages of growth, DNA replication and mitosis (see, e.g., Pines, 1995; Morgan, 1995). Activation of specific CDKs is required for appropriate progression through the different stages of the cell cycle and entry into the next stage of the cell cycle.

Cyclin-dependent kinase 12 (CDK12) and its orthologue 13 (CDK13) belong to the cyclin-dependent kinase (CDK) family of serine/threonine protein kinases that regulate transcriptional and post-transcriptional processes, thereby modulating multiple cellular functions. Studies have characterised CDK12 and CDK13 as transcriptional CDKs that complexes with cyclin K to mediate gene transcription by phosphorylating RNA polymerase II (see, e.g., Li *et al.,* 2016; Greifenberg *et al.,* 2016). The CDK12/cyclin K and CDK13/cyclin K complexes phosphorylate RNA Pol II at Ser2 (Ser2p-RNA Pol II), which is thought to be a critical step in transition from transcriptional initiation to elongation. CDK12 has been demonstrated to specifically upregulate the expression of genes involved in response to DNA damage, stress and heat shock (see, e.g., Blazek *et al.,* 2011; Li *et al.,* 2016). Studies have also implicated CDK12 in regulating mRNA splicing, 3'-end processing, pre-replication complex assembly, and genomic stability (see, e.g., Choi *et al.,* 2020). Genomic alterations in CDK12 have been detected in oesophageal, stomach, breast, endometrial, uterine, ovarian, bladder, colorectal and pancreatic cancers (see, e.g., Gyl *et al.,* 2018). A number of studies point to CDK12 inhibition as an effective strategy to inhibit tumour growth, and synthetic lethal interactions have been described with a number of pathways relevant for cancer survival and progression (see, e.g., Johnson *et al.,* 2016; Choi *et al.,* 2019). CDK13 regulates a different set of genes to CDK12, with CDK13 activity mostly involved in growth signaling pathways, including tyrosine kinase signalling (Greifenberg *et al.,* 2016).

Cyclin K degradation is a property of some, but not all inhibitors of CDK 12 (see, e.g., S 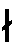 abicki *et al.,* 2020; Lv *et al.,* 2020). Upon binding of an inhibitor with a degrader activity, CDK12 acts as a surrogate substrate receptor for the CUL4-DDB1 ubiquitin ligase complex, presenting Cyclin K for ubiquitination by CRL4 and resulting in proteosomal degradation. Interaction between CDK12 and DDB1 is driven, in part, due to interactions of the inhibitor with DDB1. Therefore, only CDK12 inhibitors that simultaneously occupy the kinase active site and fill the hydrophobic pocket of DDB1 can promote Cyclin K degradation. For example, the pan-CDK inhibitor CR8 was found to cause Cyclin K degradation by this mechanism, whereas the CDK12 selective covalent inhibitor THZ-531 did not cause cyclin K degradation.

Cyclin K degradation can complement the direct inhibition of CDK12 and/or 13 in cells. This is advantageous for a number of reasons. Firstly, degradation can lead to enhanced potency over kinase inhibition alone, as shown by the increased potency of molecules in cell killing assays. Enhanced cellular potency can lead to reduced off-target interactions and effects between the inhibitor and other kinases than CDK12 and/or CDK13. Secondly, Cyclin K is the obligate partner for both CDK12 and CDK13 and is needed for their activity. Cyclin K degraders will therefore cause impaired activity of both kinases, even if the compound shows differential selectivity between CDK12 and CDK13. Finally, Cyclin K has been shown to have a half-life in cells in excess of 12 hours (see, e.g., Lei *et al.,* 2018). Hence degraders may have effects in cells and tumours that may extend beyond the duration of exposure to the compound.

### Known Compounds

Schering Corporation has describes certain pyrazolo[1,5-a]pyrimidine-7-amine compounds which allegedly act as CDK inhibitors. See, e.g., Guzi *et al.,* 2004a, 2004b, 2006, 2007, 2008a, 2008b, 2008c. It appears that the following compounds are shown therein:

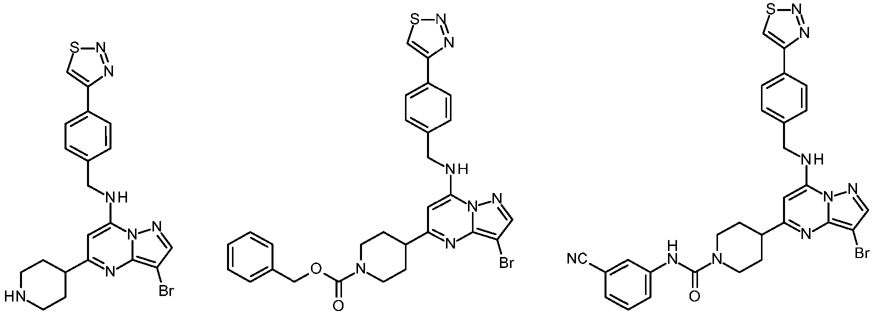

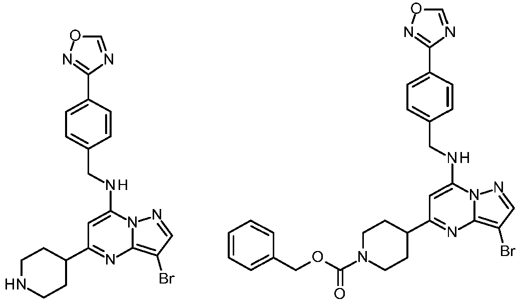

The 9H-purine-6-amine derivative known as CR8 (or more specifically, as (R)-CR8), shown below) is a CDK1 / 2 / 5 / 9 / 12 inhibitor, and causes proteosomal dependent degradation of Cyclin K. See, e.g., Slabicki *et al.,* 2020.

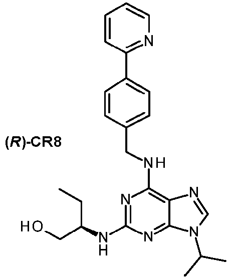

Nam *et al.,* 2019, describes certain compounds of the following formula (wherein X may be CH and R¹ may be hydrogen) which allegedly inhibit CDK (in particular CDK7) and are useful in the treatment of cell proliferative diseases, inflammatory diseases, etc.

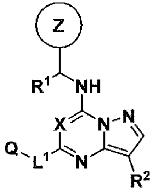

Among the examples provided therein (see, e.g., Table 11 at pages 139-183 therein) are Compounds 1, 47, and 62, shown below. None of the examples provided therein has a -Z group that is permitted by the -Ar¹-Ar² group of the compounds described herein.

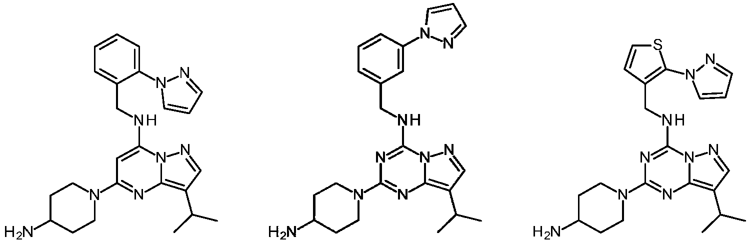

Parratt *et al.,* 2019, describes certain compounds of the following formula which allegedly are useful in the treatment of diseases mediated by excessive or in appropriate CDK2, PDK1, and/or CHK1 activity, including cancer, psoriasis, and restenosis. None of the examples provided therein has an -A-Q group that is permitted by the -Ar¹-Ar² group of the compounds described herein.

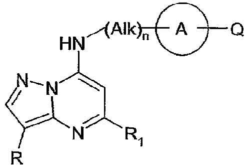

Samajdar *et al.,* 2016, describes certain compounds of the following formula (wherein X may be CH, m may be 1, R₆ may be hydrogen, and L₂ may be absent) which allegedly are selective transcription CDK inhibitors. None of the examples provided therein has an -A-L₂-B group that is permitted by the -Ar¹-Ar² group of the compounds described herein.

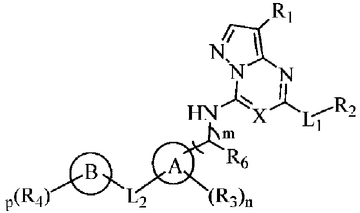

Vankayalapati *et al.,* 2020, describes certain compounds of the following formula which allegedly inhibit CDK7 are useful in the treatment of cancer. In these compounds, R² is defined as -C(=O)alkyl, optionally substituted aryl, or optionally substituted alkylaryl (see page 17 therein), including, e.g., benzyl (see pages 19-20 therein). None of the examples provided therein has an R² group that is permitted by the -Ar¹-Ar² group of the compounds described herein.

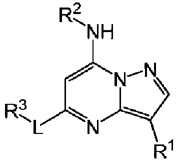

Bahl *et al.,* 2019, describes certain 4-[[(7-aminopyrazolo[1,5- a]pyrimidin-5-yl)amino methyl] piperidin-3-ol compounds (referred to therein as "AP-PAMP compounds") that, inter alia, inhibit CDK.

### Potency / Selectivity

The PPA compounds described herein are potent CDK12 and/or CDK13 inhibitors that are also selective for CDK12 and/or CDK13, for example, as compared to CDK7.

In addition to selectively inhibiting CDK12 and/or CDK13, the PPA compounds described herein may also act as selective Cyclin K degraders thereby removing the key signaling mechanism required for CDK12 and/or CDK13 activation; this confers additional cellular potency and selectivity.

### SUMMARY OF THE INVENTION

The invention is set out in the appended claims.

A first aspect of the invention is a compound of the following formula: 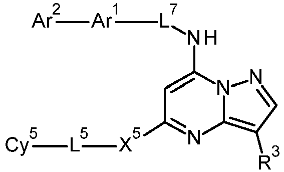
or a pharmaceutically acceptable salt or solvate thereof;
wherein:
   -L⁷- is independently -CH₂-, -CH(R^{L7})-, or -C(R^{L7)}₂-;
   each -R^{L7} is independently linear or branched saturated C₁₋₄alkyl;
   -Ar¹- is 1,4-phenylene;
   and is optionally substituted with one or more groups -R^{AR1C};
   -Ar² is pyridin-2-yl;
   and is optionally substituted *on carbon* with one or more groups -R^{AR2C};
   -X⁵- is independently -NH-, -NR^{X5}-, -O-, or a single bond;
   -R^{X5} is linear or branched saturated C₁₋₄alkyl;
   -L⁵- is independently -CH₂-, -CH(R^{L5})-, -C(R^{L5})₂-, or a single bond;
   each -R^{L5} is independently linear or branched saturated C₁₋₄alkyl;
   -Cy⁵ is independently -Cy^{5A} or -Cy^{5B};
   -Cy^{5A} is non-aromatic C₄₋₁₀heterocyclyl having at least one nitrogen ring atom; and is:
      optionally substituted *on carbon* with one or more groups -R^{Cy5AC}; and
      optionally substituted *on carbon* with =O; and
      optionally substituted *on secondary nitrogen, if present,* with a group -R^{Cy5AN}
      -Cy^{5B} is C₅₋₆heteroaryl having at least one nitrogen ring atom;
      and is:
         optionally substituted *on carbon* with one or more groups -R^{Cy5BC}; and
         optionally substituted *on secondary nitrogen, if present,* with a group -R^{Cy5BN};
         -R³ is -R^{3B};
         -R^{3B} is cyclopropyl;
         and wherein:
            each -R^{AR1C} and each -R^{AR2C} is independently selected from:
            -R^{TT},
            -F, -Cl, -Br, -I,
            -OH, -OR^{TT},
            -L^{T}-OH, -L^{T}-OR^{TT},
            -CF₃, -CHF₂, -OCF₃, -OCHF₂,
            -NH₂, -NHR^{TT}, -NR^{TT}₂, -R^{TM},
            -L^{T}-NH₂, -L^{T}-NHR^{TT}, -L^{T}-NR^{TT}₂, -L^{T}-R^{TM},
            -C(=O)OH, -C(=O)OR^{TT},
            -OC(=O)R^{TT},
            -C(=O)NH₂, -C(=O)NHR^{TT}, -C(=O)NR^{TT}₂, -C(=O)R^{™},
            -NHC(=O)R^{TT}, -NR^{TN}C(=O)R^{TT},
            -NHC(=O)NH₂, -NHC(=O)NHR^{TT}, -NHC(=O)NR^{TT}₂, -NHC(=O)R^{™},
            -NR^{TN}C(=O)NH₂, -NR ^{TN}C(=O)NHR^{TT}, -NR^{TN}C(=O)NR^{TT}2, -NR^{TN}(=O)R^{™},
            -NHC(=O)OR^{TT}, -NR^{TN}C(=O)OR^{TT},
            -OC(=O)NH₂, -OC(=O)NHR^{TT}, -OC(=O)NR^{TT}₂, -OC(=O)R^{™},
            -C(=O)R^{TT},
            -SR^{TT}, -S(=O)R^{TT}, -S(=O)₂R^{TT},
            -S(=O)NH₂, -S(=O)NHR^{TT}, -S(=O)NR^{TT}₂, -S(=O)R^{™},
            -S(=O)₂NH₂, -S(=O)₂NHR^{TT}, -S(=O)₂NR^{TT}2, -S(=O)₂R^{™},
            -NHS(=O)₂R^{TT}, -NR^{TN}S(=O)₂R^{TT},
            -CN, and -NO₂;
            wherein:
               each -L^{T}- is independently linear or branched saturated C₁₋₄alkylene;
               each -R^{TT} is independently -R^{TT1}, -R^{TT2}, -L^{TT}-R^{TT2}, -R^{TT3}, or -L^{TT}-R^{TT3};
               each -RTT¹ is independently linear or branched saturated C₁₋₆alkyl, and is optionally substituted with one or more groups selected from -F, -OH, and -OR^{TTT};
               each -R^{TT2} is saturated C₃₋₆cycloalkyl, and is optionally substituted with one or more groups selected from -F, -R^{TTT}, -OH, and -OR^{TTT};
               each -R^{TT3} is independently phenyl or naphthyl, and is optionally substituted with one or more groups selected from -F, -Cl, -Br, -I, -R^{TTT}, OH, -OR^{TTT}, -OCF₃, -NH₂, -NHR^{TTT}, and -NR^{TTT}₂;
               each -L^{TT}- is independently linear or branched saturated C₁₋₄alkylene;
               each -R^{TN} is linear or branched saturated C₁₋₄alkyl, phenyl, or benzyl;
               each -R^{™} is independently azetidino, pyrrolidino, piperidino, piperazino, morpholino, azepano, or diazepano, and is:
                  optionally substituted *on carbon* with one or more groups selected from: -R^{TMM} , -C(=O)R^{TMM} , -S(=O)₂R^{TMM}, -F, -NH₂, -NHR^{TMM}, -NR^{TMM}₂, -OH, and -OR^{TMM}; and
                  optionally substituted *on secondary nitrogen, if present,* with a group selected from: -R^{TMM}, -C(=O)R^{TMM}, -C(=O)OR^{TMM}, and -S(=O)₂R^{TMM}.
                  each -R^{TTT} is independently linear or branched saturated C₁₋₄alkyl, phenyl, or benzyl; and
                  each -R^{TMM} is independently linear or branched saturated C₁₋₄alkyl, phenyl, or benzyl;
            and wherein:
               each -R^{Cy5AC} and each -R^{Cy5BC} is independently selected from:
               -R^{JJ},
               -F, -Cl, -Br, -I,
               -OH, -OR^{JJ},
               -L^{J}-OH, -L^{J}-OR^{JJ},
               -CF₃, -CHF₂, -OCF₃, -OCHF₂,
               -NH₂, -NHR^{JJ}, -NR^{JJ}₂, -R^{JM},
               -L^{J}-NH₂, -L^{J}-NHR^{JJ}, -L^{J}-NR^{JJ}₂, -L^{J}-R^{JM},
               -C(=O)OH, -C(=O)OR^{JJ},
               -OC(=O)R^{JJ},
               -C(=O)NH₂, -C(=O)NHR^{JJ}, -C(=O)NR^{JJ}₂, -C(=O)R^{JM},
               -NHC(=O)R^{JJ}, -NR^{JN}C(=O)R^{JJ},
               -NHC(=O)NH₂, -NHC(=O)NHR^{JJ}, -NHC(=O)NR^{JJ}₂, -NHC(=O)R^{JM},
               -NR^{J}NC(=O)NH₂, -NR^{JN}C(=O)NHR^{JJ}, -NR^{JN}C(=O)NR^{JJ}₂, -NR^{JN}C(=O)R^{JM},
               -NHC(=O)OR^{JJ}, -NR^{JN}C(=O)OR^{JJ},
               -OC(=O)NH₂, -OC(=O)NHR^{JJ}, -OC(=O)NR^{JJ}₂, -OC(=O)R^{JM},
               -C(=O)R^{JJ},
               -SR^{JJ}, -S(=O)R^{JJ}, -S(=O)₂R^{JJ},
               -S(=O)NH₂, -S(=O)NHR^{JJ}, -S(=O)NR^{JJ}₂, -S(=O)R^{JM},
               -S(=O)₂NH₂, -S(=O)₂NHR^{JJ}, -S(=O)₂NR^{JJ}₂, -S(=O)₂R^{JM},
               -NHS(=O)₂R^{JJ}, -NR^{JN}S(=O)₂R^{JJ},
               -CN, and -NO₂;
               each -R^{Cy5AN} and each -R^{Cy5BN} is independently selected from:
                  -R^{JJ},
                  -L^{J}-OH, -L^{J}-OR^{JJ},
                  -L^{J}-NH₂, -L^{J}-NHR^{JJ}, -L^{J}-NR^{JJ}₂, -L^{J}-R^{JM},
                  -C(=O)R^{JJ},
                  -C(=O)OR^{JJ},
                  -C(=O)NH₂, -C(=O)NHR^{JJ}, -C(=O)NR^{JJ}₂, -C(=O)R^{JM}, and
                  -S(=O)₂R^{JJ};
                  wherein:
                     each -L^{J}- is independently linear or branched saturated C₁₋₄alkylene;
                     each -R^{JJ} is independently -R^{JJ1}, -R^{JJ2}, -L^{JJ}-R^{JJ2}, -R^{JJ3}, or -L^{JJ}-R^{JJ3};
                     each -R^{JJ1} is independently linear or branched saturated C₁₋₆alkyl, and is optionally substituted with one or more groups selected from -F, -OH, and -OR^{JJJ};
                     each -R^{JJ2} is saturated C₃₋₆cycloalkyl, and is optionally substituted with one or more groups selected from -F, -R^{JJJ}, -OH, and -OR^{JJJ};
                     each -R^{JJ3} is independently phenyl or naphthyl, and is optionally substituted with one or more groups selected from -F, -Cl, -Br, -I, -R^{JJJ}, OH, -OR^{JJJ}, -OCF₃, -NH₂, -NHR^{JJJ}, and -NR^{JJJ}₂;
                     each -L^{JJ}- is independently linear or branched saturated C₁₋₄alkylene;
                     each -R^{JN} is linear or branched saturated C₁₋₄alkyl, phenyl, or benzyl;
                     each -R^{JM} is independently azetidino, pyrrolidino, piperidino, piperazino, morpholino, azepano, or diazepano, and is:
                        optionally substituted *on carbon* with one or more groups selected from: -R^{JMM}, -C(=O)R^{JMM}, -S(=O)₂^{RJMM}, -F, -NH₂, -NHR^{JMM}, -NR^{JMM}₂, -OH, and -OR^{JMM}; and
                        optionally substituted *on secondary nitrogen, if present,* with a group selected from: -R^{JMM}, -C(=O)R^{JMM}, -C(=-O)OR^{JMM}, and -S(=O)₂R^{JMM};
                        each -R^{JJJ} is independently linear or branched saturated C₁₋₄alkyl, phenyl, or benzyl; and
                        each -R^{JMM} is independently linear or branched saturated C₁₋₄alkyl, phenyl, or benzyl.

A second aspect of the invention pertains to a composition (e.g., a pharmaceutical composition) comprising a compound of the first aspect and a pharmaceutically acceptable carrier or diluent.

A third aspect of the invention pertains to a method of preparing a composition (e.g., a pharmaceutical composition) of the second aspect comprising the step of mixing a compound of the first aspect and a pharmaceutically acceptable carrier or diluent.

A fourth aspect of the invention pertains to a method of inhibiting CDK12 and/or CDK13 function (e.g., in a cell), *in vitro,* comprising contacting the cell with an effective amount of a compound of the first aspect.

A fifth aspect of the invention pertains to a method of regulating (e.g., inhibiting) cell proliferation (e.g., proliferation of a cell), inhibiting cell cycle progression, promoting apoptosis, or a combination of one or more these, *in vitro,* comprising contacting a cell with an effective amount of a compound of the first aspect.

A sixth aspect of the invention pertains to a compound of the first aspect for use in a method of treatment of the human or animal body by therapy, for example, for use a method of treatment of a disorder (e.g., a disease) as described herein.

In one embodiment, the treatment further comprises treatment (e.g., simultaneous or sequential treatment) with a further active agent which is, e.g., a DNA repair inhibitor, an immune checkpoint inhibitor, an agent stimulating the immune system, a cell cycle checkpoint inhibitor, a Her2 blocker, a transcriptional inhibitor, a cytotoxic chemotherapeutic agent, etc., as described herein.

A seventh aspect of the invention pertains to a kit comprising (a) a compound of the first aspect, preferably provided as a pharmaceutical composition (of the second aspect) and in a suitable container and/or with suitable packaging; and (b) instructions for use, for example, written instructions on how to administer the compound.

An eighth aspect of the invention pertains to a compound of the first aspect *obtainable* by a method of synthesis as described herein, or a method comprising a method of synthesis as described herein.

A ninth aspect of the invention pertains to a compound of the first aspect *obtained* by a method of synthesis as described herein, or a method comprising a method of synthesis as described herein.

Also described herein are novel intermediates, as described herein, which are suitable for use in the methods of synthesis described herein.

Also described herein is use of such novel intermediates, as described herein, in the methods of synthesis described herein.

As will be appreciated by one of skill in the art, features and preferred embodiments of one aspect of the invention will also pertain to other aspects of the invention.

### BRIEF DESCRIPTION OF THE DRAWINGS

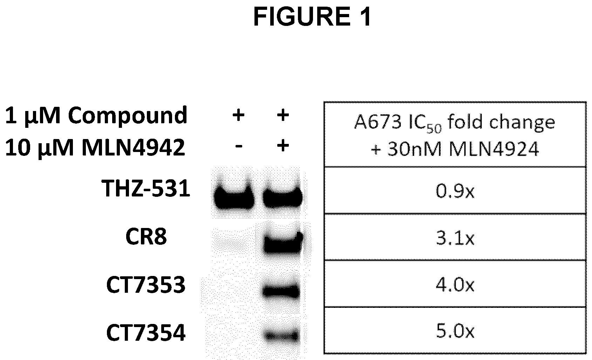

Figure 1 is shows the effect of THZ-531, CR8, PPA-005 and PPA-006 on cyclin K degradation in A673 cells, as assessed by Western blotting, as well as the effect of MLN4924 on cytotoxic potency in A673 cells.

### DETAILED DESCRIPTION OF THE INVENTION

Any references herein to methods of treatment by therapy or surgery or in vivo diagnosis are to be interpreted as references to compounds, pharmaceutical compositions and medicaments described herein for use in those methods.

### Compounds

The present invention relates to certain compounds which are structurally related to pyrazolo[1,5-a]pyrimidine-7-amine ("PPA"):

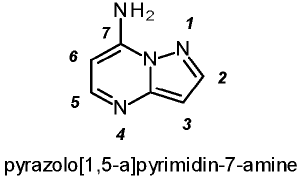

Thus, one aspect of the present invention is a compound of the following formula, or a pharmaceutically acceptable salt or solvate thereof, wherein -L⁷-, -Ar¹-, -Ar², -X⁵-, -L⁵-, -Cy⁵, and -R³ are as defined herein (for convenience, collectively referred to herein as "PPA" compounds"):

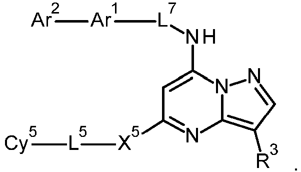

Note that it is intended that the -NH- group linking the -L⁷-Ar¹-Ar² group to the pyrazolo[1,5-a]pyrimidine ring is *unsubstituted.*

Note that it is intended that the pyrazolo[1,5-a]pyrimidine ring is *unsubstituted* at the 2- and 6-positions.

Some embodiments include the following:
(1) A compound of the following formula: 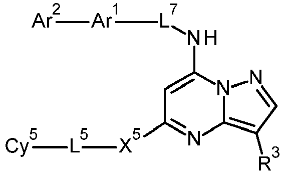
   or a pharmaceutically acceptable salt or solvate thereof;
   wherein:
      -L⁷- is independently -CH₂-, -CH(RL⁷)-, or -C(RL⁷)₂-;
      each -R^{L7} is independently linear or branched saturated C₁₋₄alkyl;
      -Ar¹- is 1,4-phenylene;
      and is optionally substituted with one or more groups -R^{AR1C};
      -Ar² is pyridin-2-yl;
      and is optionally substituted *on carbon* with one or more groups -R^{AR2C};
      -X⁵- is independently -NH-, -NR^{X5}-, -O-, or a single bond;
      -R^{X5} is linear or branched saturated C₁₋₄alkyl;
      -L⁵- is independently -CH₂-, -CH(R⁵)-, -C(RL⁵)₂-, or a single bond;
      each -R^{L5} is independently linear or branched saturated C₁₋₄alkyl;
      -Cy⁵ is independently -Cy^{5A} or -Cy^{5B};
      -Cy^{5A} is non-aromatic C₄₋₁₀heterocyclyl having at least one nitrogen ring atom;
      and is:
         optionally substituted *on carbon* with one or more groups -R^{Cy5AC}; and
         optionally substituted *on carbon* with =O; and
         optionally substituted *on secondary nitrogen, if present,* with a group -R^{Cy5AN}
         -Cy^{5B} is C₅₋₆ₕeteroaryl having at least one nitrogen ring atom;
         and is:
            optionally substituted *on carbon* with one or more groups -R^{Cy5BC}; and
            optionally substituted *on secondary nitrogen, if present,* with a group -R^{Cy5BN};
            -R³ is -R^{3B};
            -R^{3B} is cyclopropyl;
   and wherein:
      each -R^{AR1C} and each -R^{AR2C} is independently selected from:
      -R^{TT},
      -F, -Cl, -Br, -I,
      -OH, -OR^{TT},
      -L^{T}-OH, -L^{T}-OR^{TT},
      -CF₃, -CHF₂, -OCF₃, -OCHF₂,
      -NH₂, -NHR^{TT}, -NR^{TT}₂, -R^{TM},
      -L^{T}-NH₂, -L^{T}-NHR^{TT}, -L^{T}-NR^{TT}₂, -L^{T}-R^{TM},
      -C(=O)OH, -C(=O)OR^{TT},
      -OC(=O)R^{TT},
      -C(=O)NH₂, -C(=O)NHR^{TT}, -C(=O)NR^{TT}₂, -C(=O)R^{™},
      -NHC(=O)R^{TT}, -NR^{TN}C(=O)R^{TT},
      -NHC(=O)NH₂, -NHC(=O)NHR^{TT}, -NHC(=O)NR^{TT}₂, -NHC(=O)R^{™},
      -NR^{TN}C(=O)NH₂, -NR^{TN}C(=O)NHR^{TT}, -NR^{TN}C(=O)NR^{TT}2, -NR^{TN}C(=O)R^{™},
      -NHC(=O)OR^{TT}, -NR^{TN}C(=O)OR^{TT},
      -OC(=O)NH₂, -OC(=O)NHR^{TT}, -OC(=O)NR^{TT}₂, -OC(=O)R^{™},
      -C(=O)R^{TT},
      -SR^{TT}, -S(=O)R^{TT}, -S(=O)₂R^{TT},
      -S(=O)NH₂, -S(=O)NHR^{TT}, -S(=O)NR^{TT}₂, -S(=O)R^{™},
      -S(=O)₂NH₂, -S(=O)₂NHR^{TT}, -S(=O)₂NR^{TT}2, -S(=O)₂R^{™},
      -NHS(=O)₂R^{TT}, -NR^{TN}S(=O)₂R^{TT},
      -CN, and -NO₂;
   wherein:
      each -L^{T}- is independently linear or branched saturated C₁₋₄alkylene;
      each -R^{TT} is independently -R^{TT1}, -R^{TT2}, -L^{TT}-R^{TT2}, -R^{TT3}, or -L^{TT}-R^{TT3};
      each -R^{TT1} is independently linear or branched saturated C₁₋₆alkyl, and is optionally substituted with one or more groups selected from -F, -OH, and -OR^{TTT};
      each -R^{TT2} is saturated C₃₋₆cycloalkyl, and is optionally substituted with one or more groups selected from -F, -R^{TTT}, -OH, and -OR^{TTT};
      each -R^{TT3} is independently phenyl or naphthyl, and is optionally substituted with one or more groups selected from -F, -Cl, -Br, -I, -R^{TTT}, OH, -OR^{TTT}, -OCF₃, -NH₂, -NHR^{TTT}, and -NR^{TTT}₂;
      each -L^{TT}- is independently linear or branched saturated C₁₋₄alkylene;
      each -R^{TN} is linear or branched saturated C₁₋₄alkyl, phenyl, or benzyl;
      each -R^{™} is independently azetidino, pyrrolidino, piperidino, piperazino, morpholino, azepano, or diazepano, and is:
         optionally substituted *on carbon* with one or more groups selected from: -R^{TMM}, -C(=O)R^{TMM}, -S(=O)₂R^{TMM}, -F, -NH₂, -NHR^{TMM} -NR^{TMM}₂, -OH, and -OR^{TMM}; and
         optionally substituted *on secondary nitrogen, if present,* with a group selected from: -R^{TMM}, -C(=O)R^{TMM}, -C(=O)OR^{TMM}, and -S(=O)₂R^{TMM};
         each -R^{TTT} is independently linear or branched saturated C₁₋₄alkyl, phenyl, or benzyl; and
         each -R^{TMM} is independently linear or branched saturated C₁₋₄alkyl, phenyl, or benzyl;
   and wherein:
      each -R^{Cy5AC} and each -R^{Cy5BC} is independently selected from:
         -R^{JJ},
         -F, -Cl, -Br, -I,
         -OH, -OR^{JJ},
         -L^{J}-OH, -L^{J}-OR^{JJ},
         -CF₃, -CHF₂, -OCF₃, -OCHF₂,
         -NH₂, -NHR^{JJ}, -NR^{JJ}₂, -R^{JM},
         -L^{J}-NH₂, -L^{J}-NHR^{JJ}, -L^{J}-NR^{JJ}₂, -L^{J}-R^{JM},
         -C(=O)OH, -C(=O)OR^{JJ},
         -OC(=O)R^{JJ},
         -C(=O)NH₂, -C(=O)NHR^{JJ}, -C(=O)NR^{JJ}₂, -C(=O)R^{JM},
         -NHC(=O)R^{JJ}, -NR^{JN}C(=O)R^{JJ},
         -NHC(=O)NH₂, -NHC(=O)NHR^{JJ}, -NHC(=O)NR^{JJ}₂, -NHC(=O)R^{JM},
         -NR^{JN}C(=O)NH₂, -NR^{JN}C(=O)NHR^{JJ}, -NR^{JN}C(=O)NR^{JJ}₂, -NR^{JN}C(=O)R^{JM},
         -NHC(=O)OR^{JJ}, -NR^{JN}C(=O)OR^{JJ},
         -OC(=O)NH₂, -OC(=O)NHR^{JJ}, -OC(=O)NR^{JJ}₂, -OC(=O)R^{JM},
         -C(=O)R^{JJ},
         -SR^{JJ}, -S(=O)R^{JJ}, -S(=O)₂R^{JJ},
         -S(=O)NH₂, -S(=O)NHR^{JJ}, -S(=O)NR^{JJ}₂, -S(=O)R^{JM},
         -S(=O)₂NH₂, -S(=O)₂NHR^{JJ}, -S(=O)₂NR^{JJ}₂, -S(=O)₂R^{JM},
         -NHS(=O)₂R^{JJ}, -NR^{JN}S(=O)₂R^{JJ},
         -CN, and -NO₂;
      each -R^{Cy5AN} and each -R^{Cy5BN} is independently selected from:
         -R^{JJ},
         -L^{J}-OH, -L^{J}-OR^{JJ},
         -L^{J}-NH₂, -L^{J}-NHR^{JJ}, -L^{J}-NR^{JJ}₂, -L^{J}-R^{JM},
         -C(=O)R^{JJ},
         -C(=O)OR^{JJ},
         -C(=O)NH₂, -C(=O)NHR^{JJ}, -C(=O)NR^{JJ}₂, -C(=O)R^{JM}, and
         -S(=O)₂R^{JJ};
   wherein:
      each -L^{J}- is independently linear or branched saturated C₁₋₄alkylene;
      each -R^{JJ} is independently -R^{JJ1}, -R^{JJ2}, -L^{JJ}-R^{JJ2}, -R^{JJ3}, or -L^{JJ}-R^{JJ3};
      each -R^{JJ1} is independently linear or branched saturated C₁₋₆alkyl, and is optionally substituted with one or more groups selected from -F, -OH, and -OR^{JJJ};
      each -R^{JJ2} is saturated C₃₋₆cycloalkyl, and is optionally substituted with one or more groups selected from -F, -R^{JJJ}, -OH, and -OR^{JJJ};
      each -R^{JJ3} is independently phenyl or naphthyl, and is optionally substituted with one or more groups selected from -F, -Cl, -Br, -I, -R^{JJJ}, OH, -OR^{JJJ}, -OCF₃, -NH₂, -NHR^{JJJ}, and -NR^{JJJ}₂;
      each -L^{JJ}- is independently linear or branched saturated C₁₋₄alkylene;
      each -R^{JN} is linear or branched saturated C₁₋₄alkyl, phenyl, or benzyl;
      each -R^{JM} is independently azetidino, pyrrolidino, piperidino, piperazino, morpholino, azepano, or diazepano, and is:
         optionally substituted *on carbon* with one or more groups selected from: -R^{JMM}, -C(=O)R^{JMM}, -S(=O)₂R^{JMM}, -F, -NH₂, -NHR^{JMM}, -NR^{JMM}₂, -OH, and -OR^{JMM}; and
         optionally substituted *on secondary nitrogen, if present,* with a group selected from: -R^{JMM}, -C(=O)OR^{JMM}, and -S(=O)₂R^{JMM};
         each -R^{JJJ} is independently linear or branched saturated C₁₋₄alkyl, phenyl, or benzyl; and
         each -R^{JMM} is independently linear or branched saturated C₁₋₄alkyl, phenyl, or benzyl.

For the avoidance of doubt:
The index "C_{x-y}" in terms such as "C₉₋₁₀heteroaryl", "C₃₋₇heterocyclyl", and the like, refers to the number of ring atoms, which may be carbon atoms or heteroatoms (e.g., N, O, S, as the case may be). For example, pyridyl is an example of a C₆heteroaryl group, and piperidino is an example of a C₆heterocyclyl group.

The term "heteroaryl" refers to a group that is attached to the rest of the molecule by an atom that is part of an aromatic ring, wherein the aromatic ring is part of an aromatic ring system, and the aromatic ring system has one or more heteroatoms (e.g., N, O, S, as the case may be). For example, pyridyl is an example of a C₆heteroaryl group, and quinolyl is an example of a C₁₀heteroaryl group.

The term "heterocyclyl" refers to a group that is attached to the rest of the molecule by an atom that is part of a non-aromatic ring, wherein the non-aromatic ring is part of a non-aromatic ring system, and the non-aromatic ring system has one or more heteroatoms (e.g., N, O, S, as the case may be). Unless otherwise specified, "heterocyclyl" includes monocyclic heterocyclyl (e.g., piperidinyl, an example of a monocyclic C₆heterocyclyl), fused heterocyclyl (e.g., decahydroquinolinyl, an example of a fused C₁₀heterocyclyl), bridged heterocyclyl (e.g., 6-azabicyclo[3.1.1]heptanyl, an example of a bridged C₇heterocyclyl), and spiro heterocyclyl (7-azaspiro[3.5]nonyl, an example of a spiro C₉heterocyclyl).

The phrase "one or more groups" in the context of optional substituents (e.g., "one or more groups -R^{AR1C"}, etc.) is necessarily constrained by the parent moiety and the number of positions on it that are suitable for substitution. In some parent moieties (e.g., tetrazolyl) there is only one position available for substitution. However, for other parent moieties, there may be several (e.g., phenyl has five). Except when constrained by the parent moiety, the "one or more groups" may be, e.g., 1, 2, 3, 4, etc., though more preferably is 1, 2, or 3, yet more preferably 1 or 2, still more preferably 1.

The phrase "substituent on carbon" is intended to refer to a substituent which is attached to a carbon ring atom. Similarly, the phrase "substituent on secondary nitrogen" is intended to refer to a substituent which is attached to a nitrogen ring atom which, in the absence of the substituent, would be a secondary nitrogen ring atom (i.e., -NH-). Consequently, a pyridyl group may only have "substituents on carbon", whereas 1H-pyrrole may have both "substituents on carbon" and a "substituent on secondary nitrogen", as illustrated below.

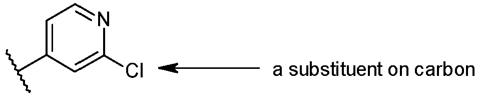

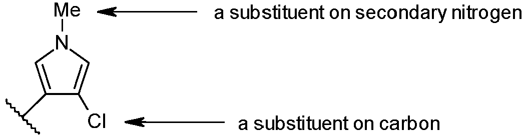

Similarly, a piperidino group may only have "substituents on carbon", whereas piperizino may have both "substituents on carbon" and a "substituent on secondary nitrogen", as illustrated below.

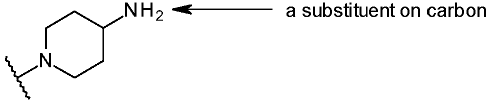

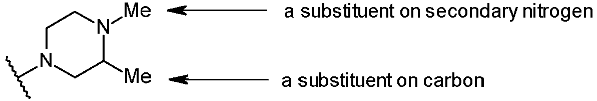

Certain groups despite having carbon ring atoms may not have any carbon ring atoms available for substitution. For example, a tetrazolyl group may only permit a "substituent on carbon" or may only permit a "substituent on secondary nitrogen", as illustrated below.

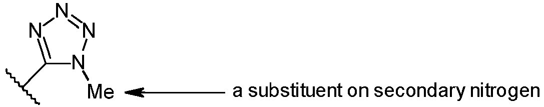

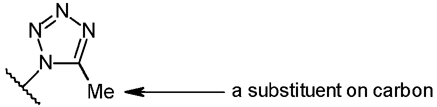

Unless otherwise indicated, where a compound is shown or described which has one or more chiral centres, and two or more stereoisomers are possible, all such stereoisomers are disclosed and encompassed, both individually (e.g., as isolated from the other stereoisomer(s)) and as mixtures (e.g., as equimolar or non-equimolar mixtures of two or more stereoisomers). For example, unless otherwise indicated, where a compound has one chiral centre, each of the (R) and (S) enantiomers are disclosed and encompassed, both individually (e.g., as isolated from the other enantiomer) and as a mixture (e.g., as equimolar or non-equimolar mixtures of the two enantiomers). For example, the initial carbon atom of a pendant sec-butyl group, -CH(CH₃)CH₂CH₃ is usually chiral, and so gives rise to stereoisomers, e.g., (R) and (S) enantiomers if it is the only chiral centre, each of which is disclosed and encompassed.

### The Group -L⁷-

(2) A compound according to (1), wherein -L⁷- is independently -CH₂- or -CH(RL⁷)-.

(3) A compound according to (1), wherein -L⁷- is -CH₂-.

### The Group -R^{L7}

(4) A compound according to any one of (1) to (3), wherein each -R^{L7}, if present, is independently -Me, -Et, -nPr, or -iPr.

(5) A compound according to any one of (1) to (3), wherein each -R^{L7}, if present, is independently -Me or -Et.

(6) A compound according to any one of (1) to (3), wherein each -R^{L7}, if present, is -Me.

### The Group -X⁵-

(7) A compound according to any one of (1) to (6), wherein -X⁵- is independently -NH-, -NR^{X5}-, or a single bond.

(8) A compound according to any one of (1) to (6), wherein -X⁵- is independently -NH- or -NR^{X5}-.

(9) A compound according to any one of (1) to (6), wherein -X⁵- is -NH-.

(10) A compound according to any one of (1) to (6), wherein -X⁵- is -NR^{X5}-.

(11) A compound according to any one of (1) to (6), wherein -X⁵- is -O-.

(12) A compound according to any one of (1) to (6), wherein -X⁵- is a single bond.

### The Group -L⁵-

(13) A compound according to any one of (1) to (12), wherein -L⁵- is independently -CH₂- or a single bond.

(14) A compound according to any one of (1) to (12), wherein -L⁵- is -CH₂-.

(15) A compound according to any one of (1) to (12), wherein -L⁵- is a single bond.

### Some Preferred Combinations of -X⁵- and -L⁵-

(16) A compound according to any one of (1) to (6), wherein Cy⁵-L⁵-X⁵- is independently:
Cy⁵-CH₂-NH- (i.e., -L⁵- is -CH₂- and -X⁵- is -NH-);
Cy⁵-CH₂-NR^{X5}- (i.e., -L⁵- is -CH₂- and -X⁵- is -NR^{X5}-);
Cy⁵-CH₂-O- (i.e., -L⁵- is -CH₂- and -X⁵- is -O-);
Cy⁵-NH- (i.e., -L⁵- is a single bond and -X⁵- is -NH-);
Cy⁵-NR^{X5}- (i.e., -L⁵- is a single bond and -X⁵- is -NR^{X5}-);
Cy⁵-O- (i.e., -L⁵- is a single bond and -X⁵- is -O-); or
Cy⁵- (i.e., each of -L⁵- and -X⁵- is a single bond).

(17) A compound according to any one of (1) to (6), wherein Cy⁵-L⁵-X⁵- is independently:
Cy⁵-CH₂-NH- (i.e., -L⁵- is -CH₂- and -X⁵- is -NH-);
Cy⁵-NH- (i.e., -L⁵- is a single bond and -X⁵- is -NH-);
Cy⁵-O- (i.e., -L⁵- is a single bond and -X⁵- is -O-); or
Cy⁵- (i.e., each of -L⁵- and -X⁵- is a single bond).

(18) A compound according to any one of (1) to (6), wherein Cy⁵-L⁵-X⁵- is independently:
Cy⁵-CH₂-NH- (i.e., -L⁵- is -CH₂- and -X⁵- is -NH-);
Cy⁵-NH- (i.e., -L⁵- is a single bond and -X⁵- is -NH-); or
Cy⁵-O- (i.e., -L⁵- is a single bond and -X⁵- is -O-).

(19) A compound according to any one of (1) to (6), wherein Cy⁵-L⁵-X⁵- is independently:
Cy⁵-CH₂-NH- (i.e., -L⁵- is -CH₂- and -X⁵- is -NH-); or
Cy⁵-NH- (i.e., -L⁵- is a single bond and -X⁵- is -NH-).

(20) A compound according to any one of (1) to (6), wherein Cy⁵-L⁵-X⁵- is:
Cy⁵-CH₂-NH- (i.e., -L⁵- is -CH₂- and -X⁵- is -NH-).

(21) A compound according to any one of (1) to (6), wherein Cy⁵-L⁵-X⁵- is:
Cy⁵-NH- (i.e., -L⁵- is a single bond and -X⁵- is -NH-).

### The Group -R^{X5}

(22) A compound according to any one of (1) to (21), wherein -R^{X5}, if present, is independently -Me, -Et, -nPr, or -iPr.

(23) A compound according to any one of (1) to (21), wherein -R^{X5}, if present, is independently -Me or -Et.

(24) A compound according to any one of (1) to (21), wherein -R^{X5}, if present, is -Me.

### The Group -R^{L5}

(25) A compound according to any one of (1) to (24), wherein each -R^{L5}, if present, is independently -Me, -Et, -nPr, or -iPr.

(26) A compound according to any one of (1) to (24), wherein each -R^{L5}, if present, is independently -Me or -Et.

(27) A compound according to any one of (1) to (24), wherein each -R^{L5}, if present, is -Me.

### The Group -Ar¹- :

(28) A compound according to any one of (1) to (27), wherein -Ar¹- is 1,4-phenylene.

### The Group -Ar²:

(29) A compound according to any one of (1) to (28), wherein -Ar² is pyridin-2-yl.

### Some Preferred Combinations of Ar¹ and Ar²

(30) A compound according to any one of (1) to (27), wherein:
-Ar¹- is 1,4-phenylene;
and is optionally substituted *on carbon* with one or more groups -R^{AR1C}; and
-Ar² is pyridin-2-yl.

(31) A compound according to any one of (1) to (27), wherein:
-Ar¹- is 1,4-phenylene; and
-Ar² is pyridin-2-yl;
and is optionally substituted *on carbon* with one or more groups -R^{AR2C}

(32) A compound according to any one of (1) to (27), wherein:
-Ar¹- is 1,4-phenylene; and
-Ar² is pyridin-2-yl.

### Substituents -R^{AR1C} and -R^{AR2C}

(33) A compound according to any one of (1) to (32), wherein each -R^{AR1C}, if present, and each -R^{AR2C}, if present, is independently selected from:
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{™},
-C(=O)OH, -C(=O)OR^{TT},
-OC(=O)R^{TT},
-C(=O)NH₂, -C(=O)NHR^{TT}, -C(=O)NR^{TT}₂, -C(=O)R^{™},
-NHC(=O)R^{TT}, -NR^{TN}C(=O)R^{TT},
-C(=O)R^{TT},
-S(=O)₂R^{TT},
-S(=O)NH₂, -S(=O)NHR^{TT}, -S(=O)NR^{TT}₂, -S(=O)R^{™},
-S(=O)₂NH₂, -S(=O)₂NHR^{TT}, -S(=O)₂NR^{TT}2, -S(=O)₂R^{™},
-NHS(=O)₂R^{TT}, -NR^{TN}S(=O)₂R^{TT},
-CN, and -NO₂.

(34) A compound according to any one of (1) to (32), wherein each -R^{AR1C}, if present, and each -R^{AR2C}, if present, is independently selected from:
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{™},
-C(=O)OH, -C(=O)OR^{TT},
-OC(=O)R^{TT},
-C(=O)NH₂, -C(=O)NHR^{TT}, -C(=O)NR^{TT}₂, -C(=O)R^{™},
-NHC(=O)R^{TT}, -NR^{TN}C(=O)R^{TT},
-C(=O)R^{TT},
-CN, and -NO₂.

(35) A compound according to any one of (1) to (32), wherein each -R^{AR1C}, if present, and each -R^{AR2C}, if present, is independently selected from:
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{TM},
-CN, and -NO₂.

(36) A compound according to any one of (1) to (32), wherein each -R^{AR1C}, if present, and each -R^{AR2C}, if present, is independently selected from:
-R^{TT},
-F, -Cl, -Br, -I,
-OH, -OR^{TT},
-CF₃, -CHF₂, -OCF₃, -OCHF₂,
-NH₂, -NHR^{TT}, -NR^{TT}₂, -R^{™}
-CN, and -NO₂.

(37) A compound according to any one of (1) to (32), wherein each -R^{AR1C}, if present, and each -R^{AR2C}, if present, is independently selected from:
-R^{TT},
-F, -Cl, -Br, -I,
-OH, -OR^{TT},
-CF₃, -CHF₂, -OCF₃, and -OCHF₂.

(38) A compound according to any one of (1) to (32), wherein each -R^{AR1C}, if present, and each -R^{AR2C}, if present, is independently selected from:
-F, -Me, -OMe, -CF₃, and -OCF₃.

(39) A compound according to any one of (1) to (32), wherein each -R^{AR1C}, if present, is independently selected from:
-F, -Me, and -OMe.

### The Group -R^{TT}

(40) A compound according to any one of (1) to (39), wherein each -R^{TT}, if present, is independently -R^{TT1}, -R^{TT2}, -R^{TT3}, or -L^{TT}-R^{TT3}.

(41) A compound according to any one of (1) to (39), wherein each -R^{TT}, if present, is independently -R^{TT1}, -R^{TT3}, or -L^{TT}-R^{TT3}.

(42) A compound according to any one of (1) to (39), wherein each -R^{TT}, if present, is -R^{TT1}.

### The Group -R^{TT1}

(43) A compound according to any one of (1) to (42), wherein each -R^{TT1}, if present, is independently linear or branched saturated C₁₋₆alkyl.

(44) A compound according to any one of (1) to (42), wherein each -R^{TT1}, if present, is independently linear or branched saturated C₁₋₄alkyl, and is optionally substituted with one or more groups selected from -F, -OH, and -OR^{TTT}.

(45) A compound according to any one of (1) to (42), wherein each -R^{TT1}, if present, is independently linear or branched saturated C₁₋₄alkyl.

(46) A compound according to any one of (1) to (42), wherein each -R^{TT1}, if present, is independently -Me, -Et, -nPr, -iPr, -nBu, -sBu, -iBu, or -tBu.

(47) A compound according to any one of (1) to (42), wherein each -R^{TT1}, if present, is independently -Me, -Et, -nPr, or -iPr.

(48) A compound according to any one of (1) to (42), wherein each -R^{TT1}, if present, is -Me.

### The Group -R^{TT2}

(49) A compound according to any one of (1) to (48), wherein each -R^{TT2}, if present, is saturated C₃₋₆cycloalkyl, and is optionally substituted with one or more groups selected from -F, -OH, and -OR^{TTT}.

(50) A compound according to any one of (1) to (48), wherein each -R^{TT2}, if present, is saturated C₃₋₆cycloalkyl.

(51) A compound according to any one of (1) to (48), wherein each -R^{TT2}, if present, is independently cyclopropyl, cyclobutyl, cyclopentyl, or cyclohexyl.

(52) A compound according to any one of (1) to (48), wherein each -R^{TT2}, if present, is cyclopropyl.

### The Group -R^{TT3}

(53) A compound according to any one of (1) to (52), wherein each -R^{TT3}, if present, is phenyl, and is optionally substituted with one or more groups selected from -F, -Cl, -Br, -I, -R^{TTT}, OH, -OR^{TTT}, -OCF₃, -NH₂, -NHR^{TTT}, and -NRT^{TT}₂.

(54) A compound according to any one of (1) to (52), wherein each -R^{TT3}, if present, is phenyl, and is optionally substituted with one or more groups selected from -F, -Cl, -Br, -I, -R^{TTT}, OH, -OR^{TTT}, and -OCF₃.

(55) A compound according to any one of (1) to (52), wherein each -R^{TT3}, if present, is phenyl, and is optionally substituted with one or more groups selected from -F, -Cl, -Br, -I, and -R^{TTT}.

(56) A compound according to any one of (1) to (52), wherein each -R^{TT3}, if present, is phenyl.

### The Group -L^{T}-

(57) A compound according to any one of (1) to (56), wherein each -L^{T}-, if present, is independently linear or branched saturated C₁₋₃alkylene.

(58) A compound according to any one of (1) to (56), wherein each -L^{T}-, if present, is independently -CH₂-, -CH₂CH₂-, -CH₂CH₂CH₂-, -CH(CH₃)-, -C(CH₃)₂-, -CH(CH₂CH₃)-, -CH(CH₃)CH₂-, or -CH₂CH(CH₃)-.

(59) A compound according to any one of (1) to (56), wherein each -L^{T}-, if present, is independently -CH₂-, -CH₂CH₂-, or -CH₂CH₂CH₂-.

(60)A compound according to any one of (1) to (56), wherein each -L^{T}-, if present, is -CH₂-.

### The Group -L^{TT}-

(61) A compound according to any one of (1) to (60), wherein each -L^{TT}-, if present, is independently linear or branched saturated C₁₋₃alkylene.

(62) A compound according to any one of (1) to (60), wherein each -L^{TT}-, if present, is independently -CH₂-, -CH₂CH₂-, -CH₂CH₂CH₂-, -CH(CH₃)-, -C(CH₃)₂-, -CH(CH₂CH₃)-, -CH(CH₃)CH₂-, or -CH₂CH(CH₃)-.

(63) A compound according to any one of (1) to (60), wherein each -L^{TT}-, if present, is independently -CH₂-, -CH₂CH₂-, or -CH₂CH₂CH₂-.

(64) A compound according to any one of (1) to (60), wherein each -L^{TT}-, if present, is -CH₂-.

### The Group -R^{TN}

(65) A compound according to any one of (1) to (64), wherein each -R^{TN}, if present, is independently linear or branched saturated C₁₋₄alkyl.

(66) A compound according to any one of (1) to (64), wherein each -R^{TN}, if present, is -Me.

### The Group -R^{™}

(67) A compound according to any one of (1) to (66), wherein each -R^{TM}, if present, is independently azetidino, pyrrolidino, piperidino, piperazino, morpholino, azepano, or diazepano, and is:
optionally substituted *on carbon* with one or more groups selected from: -R^{TMM}, -F, -OH, and -OR^{TMM}; and
optionally substituted *on secondary nitrogen, if present,* with a group selected from: -R^{TMM} , -C(=O)R^{TMM}, and -C(=O)OR^{TMM}.

(68) A compound according to any one of (1) to (66), wherein each -R^{TM}, if present, is independently pyrrolidino, piperidino, piperazino, or morpholino, and is:
optionally substituted *on carbon* with one or more groups selected from: -R^{TMM}, -F, -OH, and -OR^{TMM}; and
optionally substituted *on secondary nitrogen, if present,* with a group selected from: -R^{TMM}, -C(=O)R^{TMM}, and -C(=O)OR^{TMM}.

(69) A compound according to any one of (1) to (66), wherein each -R^{TM}, if present, is independently pyrrolidino, piperidino, piperazino, or morpholino, and is:
optionally substituted *on carbon* with one or more groups selected from: -R^{TMM}; and
optionally substituted *on secondary nitrogen, if present,* with a group selected from: -R^{TMM}, -C(=O)R^{TMM}, and -C(=O)OR^{TMM}.

(70) A compound according to any one of (1) to (66), wherein each -R^{TM}, if present, is independently pyrrolidino, piperidino, piperazino, or morpholino, and is:
optionally substituted *on carbon* with one or more groups selected from: -R^{TMM}; and
optionally substituted *on secondary nitrogen, if present,* with a group selected from: -R^{TMM}.

### The Group -R^{TTT}

(71) A compound according to any one of (1) to (70), wherein each -R^{TTT}, if present, is independently linear or branched saturated C₁₋₄alkyl.

(72) A compound according to any one of (1) to (70), wherein each -R^{TTT}, if present, is -Me.

### The Group -R^{TMM}

(73) A compound according to any one of (1) to (70), wherein each -R^{TMM}, if present, is independently linear or branched saturated C₁₋₄alkyl.

(74) A compound according to any one of (1) to (70), wherein each -R^{TMM}, if present, is -Me.

### The Group -Cy⁵

(75) A compound according to any one of (1) to (74), wherein -Cy⁵ is -Cy^{5A}.

(76) A compound according to any one of (1) to (74), wherein -Cy⁵ is -Cy^{5B}.

### The Group -Cy^{5A}

(77) A compound according to any one of (1) to (76), wherein -Cy^{5A}, if present, is independently: azetidinyl, pyrrolidinyl, piperidinyl, piperazinyl, morpholinyl, thiomorpholinyl, azepanyl, diazepanyl, tetrahydropyridinyl, dihydropyridinyl, 3,6-diazabicyclo[3.1.1]heptanyl, 3-azabicyclo[3.1.1]heptanyl, 3,8-diazabicyclo[3.2.1]octanyl, 3-azabicyclo[3.2.1]octanyl, 2,6-diazaspiro[3.3]heptanyl, 2,7-diazaspiro[3.4]octanyl, 2,7-diazaspiro[3.5]nonanyl, 2,7-diazaspiro[4.4]nonanyl, or 2,8-diazaspiro[4.5]decanyl;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

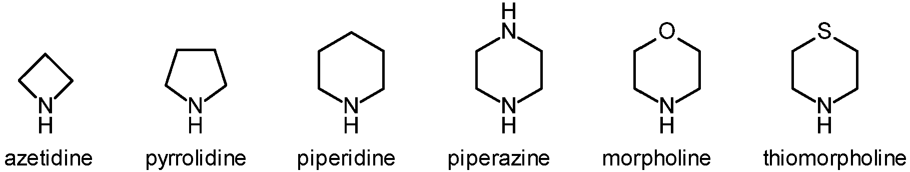

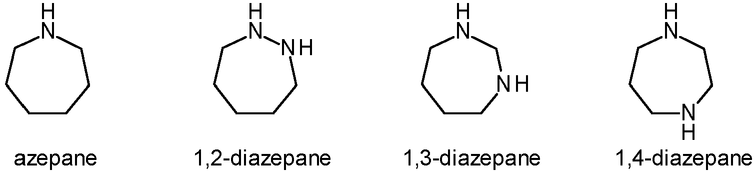

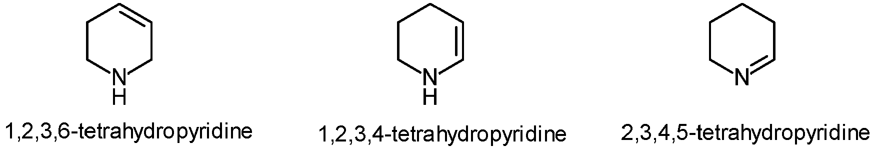

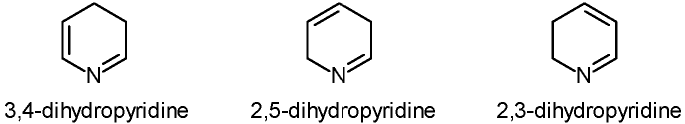

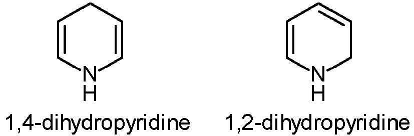

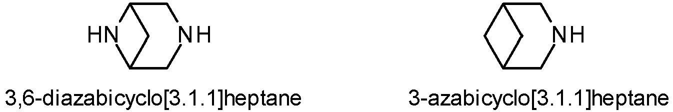

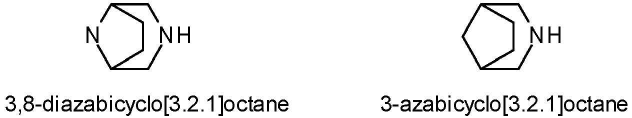

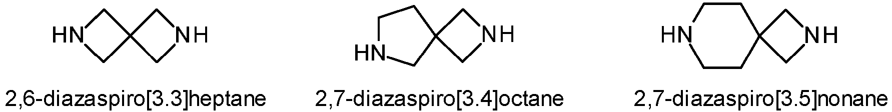

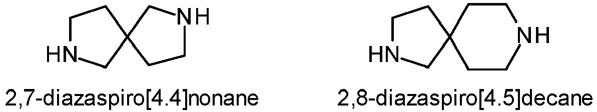

(78) A compound according to any one of (1) to (76), wherein -Cy^{5A}, if present, is non-aromatic C₄₋₇heterocyclyl having at least one nitrogen ring atom;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

(79) A compound according to any one of (1) to (76), wherein -Cy^{5A}, if present, is independently azetidinyl, pyrrolidinyl, piperidinyl, piperazinyl, morpholinyl, thiomorpholinyl, tetrahydropyridinyl, dihydropyridinyl, azepanyl, diazepanyl, or 6-diazabicyclo[3.1.1]heptanyl; and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

(80) A compound according to any one of (1) to (76), wherein -Cy^{5A}, if present, is non-aromatic C₄₋₆heterocyclyl having at least one nitrogen ring atom;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

(81) A compound according to any one of (1) to (76), wherein -Cy^{5A}, if present, is independently azetidinyl, pyrrolidinyl, piperidinyl, piperazinyl, morpholinyl, or thiomorpholinyl; and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

(82) A compound according to any one of (1) to (76), wherein -Cy^{5A}, if present, is independently azetidinyl, pyrrolidinyl, piperidinyl, piperazinyl, or morpholinyl;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

### Azetidinyl:

(83) A compound according to any one of (1) to (76), wherein -Cy^{5A}, if present, is azetidinyl (e.g., azetidin-1-yl, azetidin-2-yl, azetidin-3-yl);
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

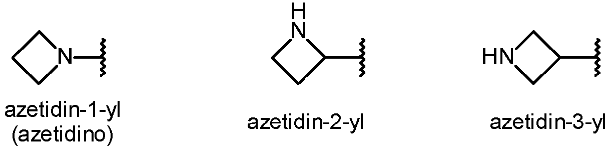

(84) A compound according to any one of (1) to (76), wherein -Cy^{5A}, if present, is azetidin-1-yl;
and is:
optionally substituted *on carbon* with one or more groups -R^{Cy5AC}; and
optionally substituted *on carbon* with =O.

(85) A compound according to any one of (1) to (76), wherein -Cy^{5A}, if present, is azetidin-2-yl;
and is:
optionally substituted *on carbon* with one or more groups -R^{Cy5AC}; and
optionally substituted *on carbon* with =O; and
optionally substituted *on secondary nitrogen,* with a group -R^{Cy5AN}.

(86) A compound according to any one of (1) to (76), wherein -Cy^{5A}, if present, is azetidin-3-yl;
and is:
optionally substituted *on carbon* with one or more groups -R^{Cy5AC}; and
optionally substituted *on carbon* with =O; and
optionally substituted *on secondary nitrogen,* with a group -R^{Cy5AN}.

### Pyrrolidinyl:

(87) A compound according to any one of (1) to (76), wherein -Cy^{5A}, if present, is pyrrolidinyl (e.g., pyrrolidin-1-yl, pyrrolidin-2-yl, pyrrolidin-3-yl);
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

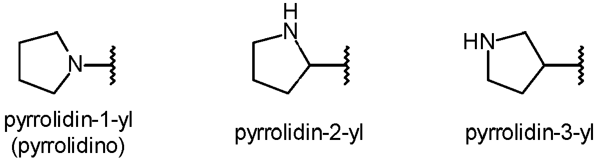

(88) A compound according to any one of (1) to (76), wherein -Cy^{5A}, if present, is pyrrolidin-1-yl;
and is:
optionally substituted *on carbon* with one or more groups -R^{Cy5AC}; and
optionally substituted *on carbon* with =O.

(89) A compound according to any one of (1) to (76), wherein -Cy^{5A}, if present, is pyrrolidin-2-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(90) A compound according to any one of (1) to (76), wherein -Cy^{5A}, if present, is pyrrolidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(91) A compound according to any one of (1) to (76), wherein -Cy^{5A}, if present, is pyrrolidin-3-yl.

(92) A compound according to any one of (1) to (76), wherein -Cy^{5A}, if present, is (3S)-pyrrolidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

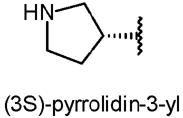

(93) A compound according to any one of (1) to (76), wherein -Cy^{5A}, if present, is (3S)-pyrrolidin-3-yl.

### Piperidinyl:

(94) A compound according to any one of (1) to (76), wherein -Cy^{5A}, if present, is piperidinyl (e.g., piperidin-1-yl, piperidin-2-yl, piperidin-3-yl, piperidin-4-yl);
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

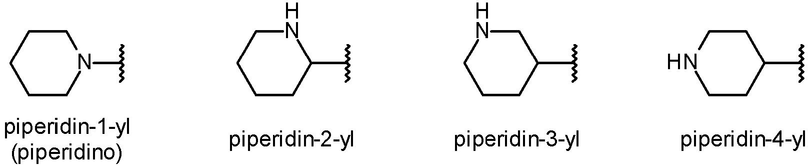

(95) A compound according to any one of (1) to (76), wherein -Cy^{5A}, if present, is piperidin-1-yl;
and is:
optionally substituted *on carbon* with one or more groups -R^{Cy5AC}; and
optionally substituted *on carbon* with =O.

(96) A compound according to any one of (1) to (76), wherein -Cy^{5A}, if present, is piperidin-2-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(97) A compound according to any one of (1) to (76), wherein -Cy^{5A}, if present, is piperidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(98) A compound according to any one of (1) to (76), wherein -Cy^{5A}, if present, is piperidin-3-yl.

(99) A compound according to any one of (1) to (76), wherein -Cy^{5A}, if present, is (3S)-piperidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

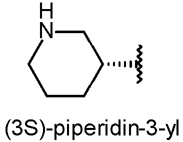

(100) A compound according to any one of (1) to (76), wherein -Cy^{5A}, if present, is (3S)-piperidin-3-yl.

(101) A compound according to any one of (1) to (76), wherein -Cy^{5A}, if present, is piperidin-4-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(102) A compound according to any one of (1) to (76), wherein -Cy^{5A}, if present, is piperidin-4-yl.

(103) A compound according to any one of (1) to (76), wherein -Cy^{5A}, if present, is 3-hydroxy-piperidin-4-yl.

(104) A compound according to any one of (1) to (76), wherein -Cy^{5A}, if present, is (3R,4R)-3-hydroxy-piperidin-4-yl.

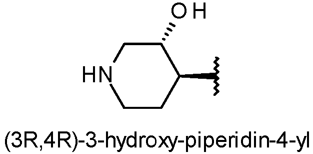

### Piperazinyl:

(105) A compound according to any one of (1) to (76), wherein -Cy^{5A}, if present, is piperazinyl (e.g., piperazin-1-yl, piperazin-2-yl);
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

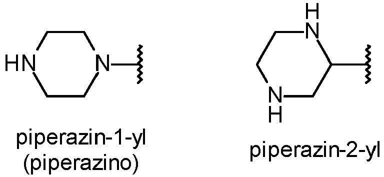

(106) A compound according to any one of (1) to (76), wherein -Cy^{5A}, if present, is piperazin-1-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(107) A compound according to any one of (1) to (76), wherein -Cy^{5A}, if present, is piperazin-2-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

### Morpholinyl:

(108) A compound according to any one of (1) to (76), wherein -Cy^{5A}, if present, is morpholinyl (e.g., morpholiny-2-yl, morpholiny-3-yl, morpholiny-4-yl);
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

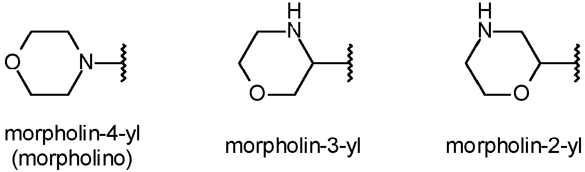

(109) A compound according to any one of (1) to (76), wherein -Cy^{5A}, if present, is morpholinyl-4-yl;
and is:
optionally substituted *on carbon* with one or more groups -R^{Cy5AC}; and
optionally substituted *on carbon* with =O.

(110) A compound according to any one of (1) to (76), wherein -Cy^{5A}, if present, is morpholinyl-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

(111) A compound according to any one of (1) to (76), wherein -Cy^{5A}, if present, is morpholinyl-2-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

### The Group -Cy^{5B}

(112) A compound according to any one of (1) to (111), wherein -Cy^{5B}, if present, is independently pyrrolyl, pyrazolyl, imidazolyl, triazolyl, tetrazolyl, oxazolyl, isoxazolyl thiazolyl, isothiazolyl, oxadiazolyl, thiadiazolyl, pyridinyl, pyrimidinyl, pyridazinyl, or pyrazinyl;
and is:
optionally substituted *on carbon* with one or more groups -R^{Cy5BC}; and
optionally substituted *on secondary nitrogen, if present,* with a group -R^{Cy5BN}.

### C₅Heteroaryl:

(113) A compound according to any one of (1) to (111), wherein -Cy^{5B}, if present, is C₅heteroaryl having at least one nitrogen ring atom;
and is:
optionally substituted *on carbon* with one or more groups -R^{Cy5BC}; and
optionally substituted *on secondary nitrogen, if present,* with a group -R^{Cy5BN}.

(114) A compound according to any one of (1) to (111), wherein -Cy^{5B}, if present, is independently pyrrolyl, pyrazolyl, imidazolyl, triazolyl, tetrazolyl, oxazole, isoxazole, thiazole, isothiazole, oxadiazolyl, or thiadiazolyl;
and is:
optionally substituted *on carbon* with one or more groups -R^{Cy5BC}; and
optionally substituted *on secondary nitrogen, if present,* with a group -R^{Cy5BN}.

(115) A compound according to any one of (1) to (111), wherein -Cy^{5B}, if present, is independently pyrrolyl, pyrazolyl, imidazolyl, oxazole, isoxazole, thiazole, or isothiazole; and is:
optionally substituted *on carbon* with one or more groups -R^{Cy5BC}; and
optionally substituted *on secondary nitrogen, if present,* with a group -R^{Cy5BN}.

### Pyrazolyl:

(116) A compound according to any one of (1) to (111), wherein -Cy^{5B}, if present, is pyrazolyl (e.g., pyrazol-1-yl, 1H-pyrazol-3-yl, 1H-pyrazol-4-yl, or 1H-pyrazol-5-yl);
and is:
optionally substituted *on carbon* with one or more groups -R^{Cy5BC}; and
optionally substituted *on secondary nitrogen, if present,* with a group -R^{Cy5BN}.

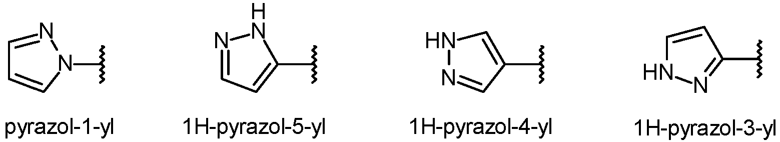

(117) A compound according to any one of (1) to (111), wherein -Cy^{5B}, if present, is 1H-pyrazol-4-yl;
and is:
optionally substituted *on carbon* with one or more groups -R^{Cy5BC}; and
optionally substituted *on secondary nitrogen* with a group -R^{Cy5BN}.

(118) A compound according to any one of (1) to (111), wherein -Cy^{5B}, if present, is 1H-pyrazol-5-yl;
and is:
optionally substituted *on carbon* with one or more groups -R^{Cy5BC}; and
optionally substituted *on secondary nitrogen, if present,* with a group -R^{Cy5BN}.

### C₆Heteroaryl:

(119) A compound according to any one of (1) to (111), wherein -Cy^{5B}, if present, is C₆heteroaryl having at least one nitrogen ring atom;
and is optionally substituted *on carbon* with one or more groups -R^{Cy5BC}

(120) A compound according to any one of (1) to (111), wherein -Cy^{5B}, if present, is independently pyridinyl, pyrimidinyl, pyridazinyl, or pyrazinyl;
and is optionally substituted *on carbon* with one or more groups -R^{Cy5BC}.

### Pyridinyl:

(121) A compound according to any one of (1) to (111), wherein -Cy^{5B}, if present, is pyridinyl (e.g., pyridin-2-yl, pyridin-3-yl, or pyridin-4-yl);
and is optionally substituted *on carbon* with one or more groups -R^{Cy5BC}.

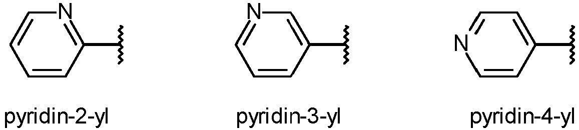

(122) A compound according to any one of (1) to (111), wherein -Cy^{5B}, if present, is pyridin-3-yl;
and is optionally substituted *on carbon* with one or more groups -R^{Cy5BC}.

### Substituents -R^{Cy5AC} and -R^{Cy5BC}

(123) A compound according to any one of (1) to (122), wherein each -R^{Cy5AC}, if present, and each -R^{Cy5BC}, if present, is independently selected from:
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-C(=O)OH, -C(=O)OR^{JJ},
-OC(=O)R^{JJ},
-C(=O)NH₂, -C(=O)NHR^{JJ}, -C(=O)NR^{JJ}₂, -C(=O)R^{JM},
-NHC(=O)R^{JJ}, -NR^{JN}C(=O)R^{JJ},
-C(=O)R^{JJ},
-S(=O)₂R^{JJ},
-S(=O)NH₂, -S(=O)NHR^{JJ}, -S(=O)NR^{JJ}₂, -S(=O)R^{JM},
-S(=O)₂NH₂, -S(=O)₂NHR^{JJ}, -S(=O)₂NR^{JJ}₂, -S(=O)₂R^{JM},
-NHS(=O)₂R^{JJ}, -NR^{JN}S(=O)₂R^{JJ},
-CN, and -NO₂.

(124) A compound according to any one of (1) to (122), wherein each -R^{Cy5AC}, if present, and each -R^{Cy5BC}, if present, is independently selected from:
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-C(=O)OH, -C(=O)OR^{JJ},
-OC(=O)R^{JJ},
-C(=O)NH₂, -C(=O)NHR^{JJ}, -C(=O)NR^{JJ}₂, -C(=O)R^{JM},
-NHC(=O)R^{JJ}, -NR^{JN}C(=O)R^{JJ},
-C(=O)R^{JJ},
-CN, and -NO₂.

(125) A compound according to any one of (1) to (122), wherein each -R^{Cy5AC}, if present, and each -R^{Cy5BC}, if present, is independently selected from:
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-CN, and -NO₂.

(126) A compound according to any one of (1) to (122), wherein each -R^{Cy5AC}, if present, and each -R^{Cy5BC}, if present, is independently selected from:
-R^{JJ},
-F, -Cl, -Br, -I,
-OH, -OR^{JJ},
-NH₂, -NHR^{JJ}, -NR^{JJ}₂, -R^{JM},
-CN, and -NO₂.

(127) A compound according to any one of (1) to (122), wherein each -R^{Cy5AC}, if present, and each -R^{Cy5BC}, if present, is independently selected from:
-R^{JJ},
-F, -Cl, -Br, -I,
-OH, -OR^{JJ},
-NH₂, -NHR^{JJ}, -NR^{JJ}₂, and -R^{JM}.

(128) A compound according to any one of (1) to (122), wherein each -R^{Cy5AC}, if present, and each -R^{Cy5BC}, if present, is independently selected from:
-F, -Me, -OH, -OMe, and -NH₂.

(129) A compound according to any one of (1) to (122), wherein each -R^{Cy5AC}, if present, is -OH.

(130) A compound according to any one of (1) to (122), wherein each -R^{Cy5BC}, if present, is -Me.

### Substituents -R^{Cy5AN} and -R^{Cy5BN}

(131) A compound according to any one of (1) to (130), wherein each -R^{Cy5AN}, if present, and each -R^{Cy5BN}, if present, is independently selected from:
-R^{JJ}, -C(=O)R^{JJ}, -C(=O)OR^{JJ}, and -S(=O)₂R^{JJ}.

(132) A compound according to any one of (1) to (130), wherein each -R^{Cy5AN}, if present, and each -R^{Cy5BN}, if present, is independently selected from:
-R^{JJ}, -C(=O)R^{JJ}, and -C(=O)OR^{JJ}.

(133) A compound according to any one of (1) to (130), wherein each -R^{Cy5AN}, if present, and each -R^{Cy5BN}, if present, is: -R^{JJ}.

### The Group -R^{JJ}

(134) A compound according to any one of (1) to (133), wherein each -R^{JJ}, if present, is independently -R^{JJ1}, -R^{JJ2}, -R^{JJ3}, or -L^{JJ}-R^{JJ3}.

(135) A compound according to any one of (1) to (133), wherein each -R^{JJ}, if present, is independently -R^{JJ1}, -R^{JJ3}, or -L^{JJ}-R^{JJ3}.

(136) A compound according to any one of (1) to (133), wherein each -R^{JJ}, if present, is -R^{JJ1}.

### The Group -R^{JJ1}

(137) A compound according to any one of (1) to (136), wherein each -R^{JJ1}, if present, is independently linear or branched saturated C₁₋₆alkyl.

(138) A compound according to any one of (1) to (136), wherein each -R^{JJ1}, if present, is independently linear or branched saturated C₁₋₄alkyl, and is optionally substituted with one or more groups selected from -F, -OH, and -OR^{JJJ}.

(139) A compound according to any one of (1) to (136), wherein each -R^{JJ1}, if present, is independently linear or branched saturated C₁₋₄alkyl.

(140) A compound according to any one of (1) to (136), wherein each -R^{JJ1}, if present, is independently -Me, -Et, -nPr, -iPr, -nBu, -sBu, -iBu, or -tBu.

(141) A compound according to any one of (1) to (136), wherein each -R^{JJ1}, if present, is independently -Me, -Et, -nPr, or -iPr.

(142) A compound according to any one of (1) to (136), wherein each -R^{JJ1}, if present, is -Me.

### The Group -R^{JJ2}

(143) A compound according to any one of (1) to (142), wherein each -R^{JJ2}, if present, is saturated C₃₋₆cycloalkyl, and is optionally substituted with one or more groups selected from -F, -OH, and -OR^{JJJ}.

(144) A compound according to any one of (1) to (142), wherein each -R^{JJ2}, if present, is saturated C₃₋₆cycloalkyl.

(145) A compound according to any one of (1) to (142), wherein each -R^{JJ2}, if present, is independently cyclopropyl, cyclobutyl, cyclopentyl, or cyclohexyl.

(146) A compound according to any one of (1) to (142), wherein each -R^{JJ2}, if present, is cyclopropyl.

### The Group -R^{JJ3}

(147) A compound according to any one of (1) to (146), wherein each -R^{JJ3}, if present, is phenyl, and is optionally substituted with one or more groups selected from -F, -Cl, -Br, -I, -R^{JJJ}, OH, -OR^{JJJ}, -OCF₃, -NH₂, -NHR^{JJJ}, and -NR^{JJJ}₂.

(148) A compound according to any one of (1) to (146), wherein each -R^{JJ3}, if present, is phenyl, and is optionally substituted with one or more groups selected from -F, -Cl, -Br, -I, -R^{JJJ}, OH, -OR^{JJJ}, and -OCF₃.

(149) A compound according to any one of (1) to (146), wherein each -R^{JJ3}, if present, is phenyl, and is optionally substituted with one or more groups selected from -F, -Cl, -Br, -I, and -R^{JJJ}.

(150) A compound according to any one of (1) to (146), wherein each -R^{JJ3}, if present, is phenyl.

### The Group -L^{J}-

(151) A compound according to any one of (1) to (150), wherein each -L^{J}-, if present, is independently linear or branched saturated C₁₋₃alkylene.

(152) A compound according to any one of (1) to (150), wherein each -L^{J}-, if present, is independently -CH₂-, -CH₂CH₂-, -CH₂CH₂CH₂-, -CH(CH₃)-, -C(CH₃)₂-, -CH(CH₂CH₃)-, -CH(CH₃)CH₂-, or -CH₂CH(CH₃)-.

(153) A compound according to any one of (1) to (150), wherein each -L^{J}-, if present, is independently -CH₂-, -CH₂CH₂-, or -CH₂CH₂CH₂-.

(154) A compound according to any one of (1) to (150), wherein each -L^{J}-, if present, is -CH₂-.

### The Group -L^{JJ}-

(155) A compound according to any one of (1) to (154), wherein each -L^{JJ}-, if present, is independently linear or branched saturated C₁₋₃alkylene.

(156) A compound according to any one of (1) to (154), wherein each -L^{JJ}-, if present, is independently -CH₂-, -CH₂CH₂-, -CH₂CH₂CH₂-, -CH(CH₃)-, -C(CH₃)₂-, -CH(CH₂CH₃)-, -CH(CH₃)CH₂-, or -CH₂CH(CH₃)-.

(157) A compound according to any one of (1) to (154), wherein each -L^{JJ}-, if present, is independently -CH₂-, -CH₂CH₂-, or -CH₂CH₂CH₂-.

(158) A compound according to any one of (1) to (154), wherein each -L^{JJ}-, if present, is -CH₂-.

### The Group -R^{JN}

(159) A compound according to any one of (1) to (158), wherein each -R^{JN}, if present, is independently linear or branched saturated C₁₋₄alkyl.

(160) A compound according to any one of (1) to (158), wherein each -R^{JN}, if present, is -Me.

### The Group -R^{JM}

(161) A compound according to any one of (1) to (160), wherein each -R^{JM}, if present, is independently azetidino, pyrrolidino, piperidino, piperazino, morpholino, azepano, or diazepano, and is:
optionally substituted *on carbon* with one or more groups selected from: -R^{JMM}, -F, -OH, and -OR^{JMM}; and
optionally substituted *on secondary nitrogen, if present,* with a group selected from: -R^{JMM}, -C(=O)R^{JMM}, and -C(=O)OR^{JMM}.

(162) A compound according to any one of (1) to (160), wherein each -R^{JM}, if present, is independently pyrrolidino, piperidino, piperazino, or morpholino, and is:
optionally substituted *on carbon* with one or more groups selected from: -R^{JMM}, -F, -OH, and -OR^{JMM}; and
optionally substituted *on secondary nitrogen, if present,* with a group selected from: -R^{JMM}, -C(=O)R^{JMM}, and -C(=O)OR^{JMM}.

(163) A compound according to any one of (1) to (160), wherein each -R^{JM}, if present, is independently pyrrolidino, piperidino, piperazino, or morpholino, and is:
optionally substituted *on carbon* with one or more groups selected from: -R^{JMM}; and
optionally substituted *on secondary nitrogen, if present,* with a group selected from: -R^{JMM}, -C(=O)R^{JMM}, and -C(=O)OR^{JMM}.

(164) A compound according to any one of (1) to (160), wherein each -R^{JM}, if present, is independently pyrrolidino, piperidino, piperazino, or morpholino, and is:
optionally substituted *on carbon* with one or more groups selected from: -R^{JMM}; and
optionally substituted *on secondary nitrogen, if present,* with a group selected from: -R^{JMM}.

### The Group -R^{JJJ}

(165) A compound according to any one of (1) to (164), wherein each -R^{JJJ}, if present, is independently linear or branched saturated C₁₋₄alkyl.

(166) A compound according to any one of (1) to (164), wherein each -R^{JJJ}, if present, is -Me.

### The Group -R^{JMM}

(167) A compound according to any one of (1) to (164), wherein each -R^{JMM}, if present, is independently linear or branched saturated C₁₋₄alkyl.

(168) A compound according to any one of (1) to (164), wherein each -R^{JMM}, if present, is -Me.

### Some Preferred Combinations

(169) A compound according to (1), wherein:
-L⁷- is -CH₂-;
-Cy⁵ is -Cy^{5A};
-Cy^{5A} is non-aromatic C₄₋₆heterocyclyl having at least one nitrogen ring atom;
and is:
   optionally substituted *on carbon* with one or more groups -R^{Cy5AC}; and
   optionally substituted *on carbon* with =O; and
   optionally substituted *on secondary nitrogen, if present,* with a group -R^{Cy5AN}.

(170) A compound according to (1), wherein:
-L⁷- is -CH₂-;
Cy⁵-L⁵-X⁵- is independently:
   Cy⁵-CH₂-NH- (i.e., -L⁵- is -CH₂- and -X⁵- is -NH-);
   Cy⁵-NH- (i.e., -L⁵- is a single bond and -X⁵- is -NH-);
   Cy⁵-O- (i.e., -L⁵- is a single bond and -X⁵- is -O-); or
   Cy⁵- (i.e., each of -L⁵- and -X⁵- is a single bond);
-Cy⁵ is -Cy^{5A};
-Cy^{5A} is independently azetidinyl, pyrrolidinyl, or piperidinyl;
and is:
   optionally substituted *on carbon* with one or more groups -R^{Cy5AC}; and
   optionally substituted *on carbon* with =O; and
   optionally substituted *on secondary nitrogen, if present,* with a group -R^{Cy5AN}.

### Specific Compounds

(171) A compound according to (1), selected from compounds of the following formulae and pharmaceutically acceptable salts and solvates thereof:

| Compound No. | Chemical Structure |
|---|---|
| PPA-006 | 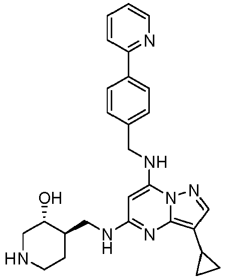 |
| PPA-015 | 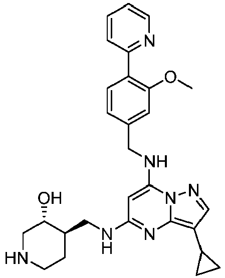 |
| PPA-019 | 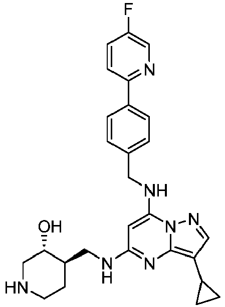 |
| PPA-020 | 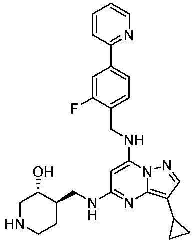 |
| PPA-022 | 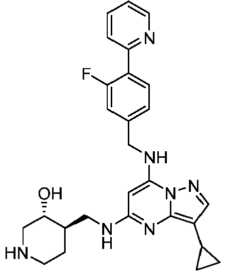 |
| PPA-027 | 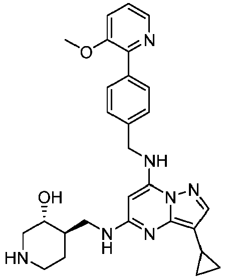 |
| PPA-028 | 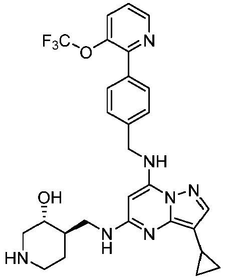 |
| PPA-030 | 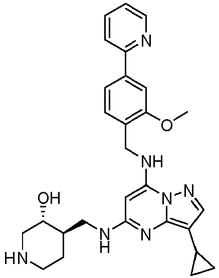 |
| PPA-032 | 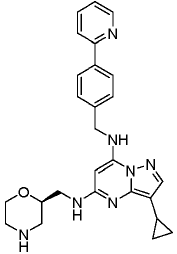 |
| PPA-033 | 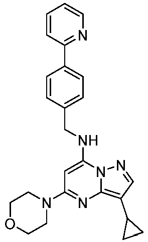 |
| PPA-036 | 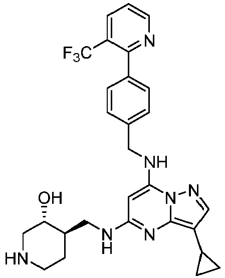 |
| PPA-037 | 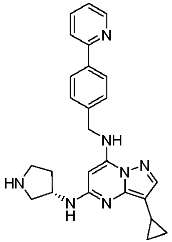 |
| PPA-038 | 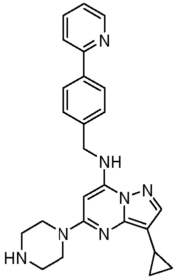 |
| PPA-039 | 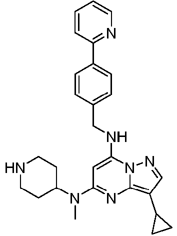 |
| PPA-040 | 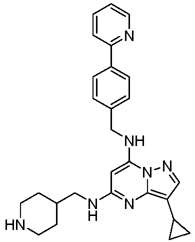 |
| PPA-041 | 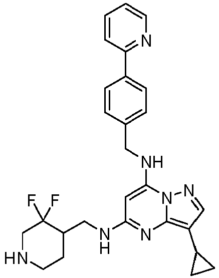 |
| PPA-042 | 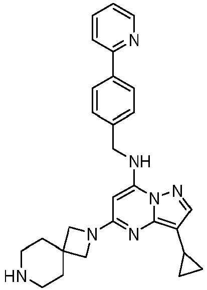 |
| PPA-043 | 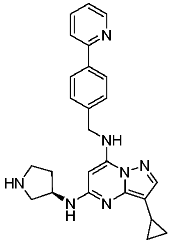 |
| PPA-044 | 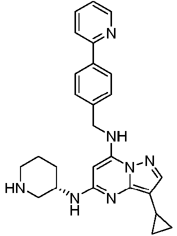 |
| PPA-045 | 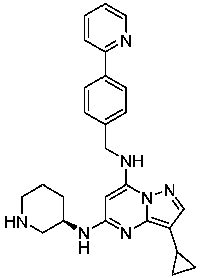 |
| PPA-046 | 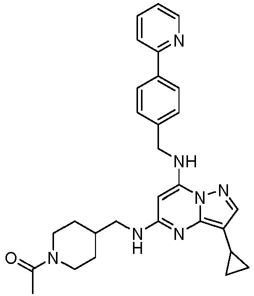 |
| PPA-047 | 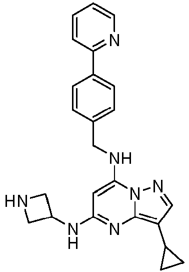 |
| PPA-048 | 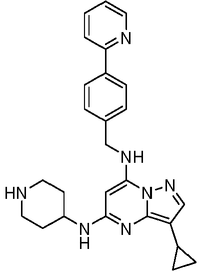 |
| PPA-049 | 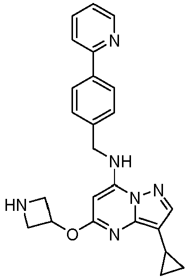 |
| PPA-050 | 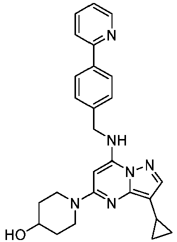 |
| PPA-051 | 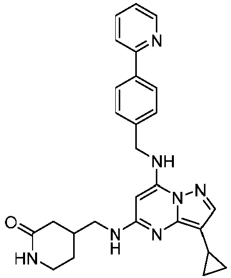 |
| PPA-052 | 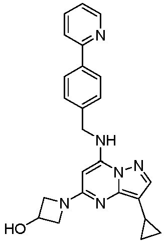 |
| PPA-053 | 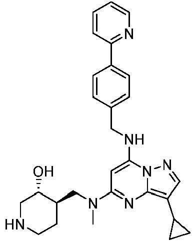 |
| PPA-055 | 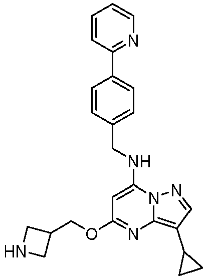 |
| PPA-056 | 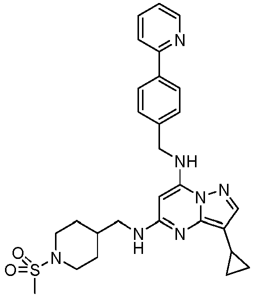 |
| PPA-057 | 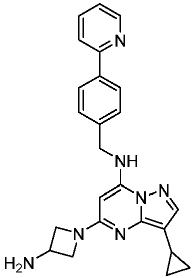 |
| PPA-058 | 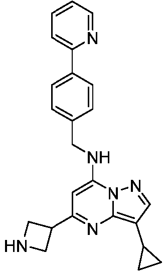 |
| PPA-060 | 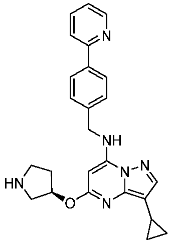 |
| PPA-061 | 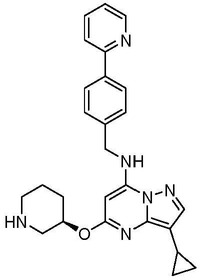 |
| PPA-062 | 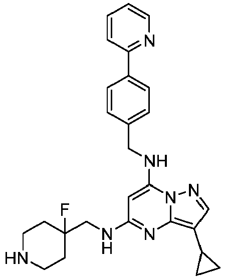 |
| PPA-063 | 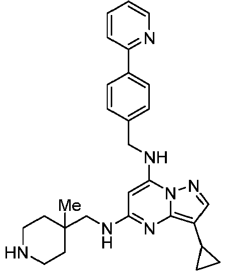 |
| PPA-064 | 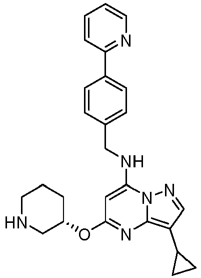 |
| PPA-065 | 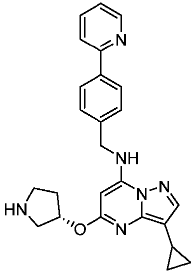 |
| PPA-072 | 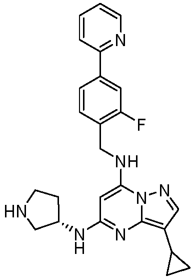 |
| PPA-073 | 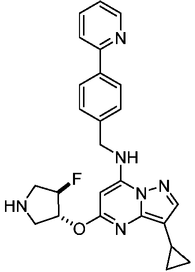 |
| PPA-075 | 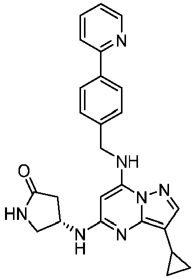 |
| PPA-077 | 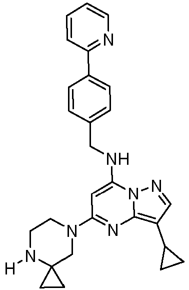 |
| PPA-078 | 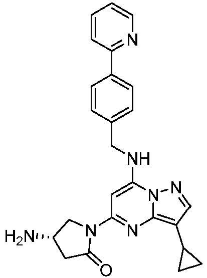 |
| PPA-079 | 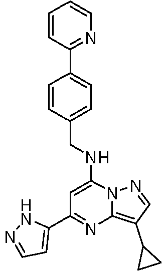 |
| PPA-080 | 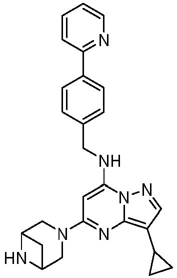 |
| PPA-084 | 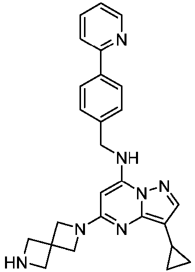 |
| PPA-087 | 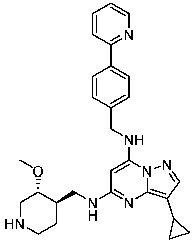 |
| PPA-088 | 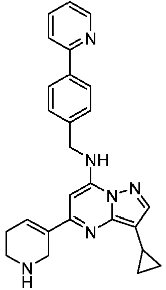 |
| PPA-095 | 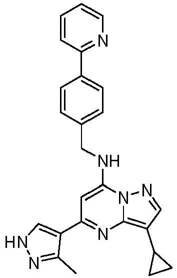 |
| PPA-096 | 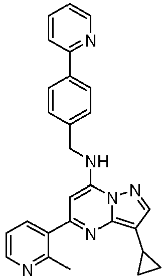 |
| PPA-100 | 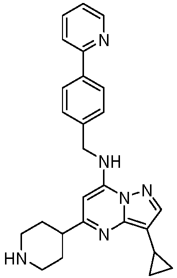 |

The following compound and its pharmaceutically acceptable salts and solvates, described herein, is a reference compound:

| Compound No. | Chemical Structure |
|---|---|
| PPA-005 | 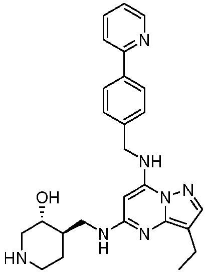 |

### Combinations

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. All combinations of the embodiments pertaining to the chemical groups represented by the variables (e.g., -L⁷-, -Ar¹-, -Ar², -X⁵-, -L⁵-, -Cy⁵, -R³, etc.) are specifically embraced by the present invention and are disclosed herein just as if each and every combination was individually and explicitly disclosed, to the extent that such combinations embrace compounds that are stable compounds (i.e., compounds that can be isolated, characterised, and tested for biological activity). In this context, the skilled person will readily appreciate that certain combinations of groups (e.g., substituents) may give rise to compounds which may not be readily synthesized and/or are chemically unstable.

In addition, all sub-combinations of the chemical groups listed in the embodiments describing such variables are also specifically embraced by the present invention and are disclosed herein just as if each and every such sub-combination of chemical groups was individually and explicitly disclosed herein.

### Substantially Purified Forms

One aspect of the present invention pertains to PPA compounds, as described herein, in substantially purified form and/or in a form substantially free from contaminants.

In one embodiment, the substantially purified form is at least 50% by weight, e.g., at least 60% by weight, e.g., at least 70% by weight, e.g., at least 80% by weight, e.g., at least 90% by weight, e.g., at least 95% by weight, e.g., at least 97% by weight, e.g., at least 98% by weight, e.g., at least 99% by weight.

Unless otherwise specified, the substantially purified form refers to the compound in any stereoisomeric or enantiomeric form. For example, in one embodiment, the substantially purified form refers to a mixture of stereoisomers, i.e., purified with respect to other compounds. In one embodiment, the substantially purified form refers to one stereoisomer, e.g., optically pure stereoisomer. In one embodiment, the substantially purified form refers to a mixture of enantiomers. In one embodiment, the substantially purified form refers to an equimolar mixture of enantiomers (i.e., a racemic mixture, a racemate). In one embodiment, the substantially purified form refers to one enantiomer, e.g., optically pure enantiomer.

In one embodiment, the contaminants represent no more than 50% by weight, e.g., no more than 40% by weight, e.g., no more than 30% by weight, e.g., no more than 20% by weight, e.g., no more than 10% by weight, e.g., no more than 5% by weight, e.g., no more than 3% by weight, e.g., no more than 2% by weight, e.g., no more than 1% by weight.

Unless specified, the contaminants refer to other compounds, that is, other than stereoisomers or enantiomers. In one embodiment, the contaminants refer to other compounds and other stereoisomers. In one embodiment, the contaminants refer to other compounds and the other enantiomer.

In one embodiment, the substantially purified form is at least 60% optically pure (i.e., 60% of the compound, on a molar basis, is the desired stereoisomer or enantiomer, and 40% is the undesired stereoisomer or enantiomer), e.g., at least 70% optically pure, e.g., at least 80% optically pure, e.g., at least 90% optically pure, e.g., at least 95% optically pure, e.g., at least 97% optically pure, e.g., at least 98% optically pure, e.g., at least 99% optically pure.

### Isomers

Certain compounds may exist in one or more particular geometric, optical, enantiomeric, diastereoisomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

A reference to a class of structures may well include structurally isomeric forms falling within that class (e.g., C₁₋₇alkyl includes n-propyl and iso-propyl; butyl includes n-, iso-, sec-, and tert-butyl; methoxyphenyl includes ortho-, meta-, and para-methoxyphenyl).

However, reference to a specific group or substitution pattern is not intended to include other structural (or constitutional isomers) which differ with respect to the connections between atoms rather than by positions in space. For example, a reference to a methoxy group, -OCH₃, is not to be construed as a reference to its structural isomer, a hydroxymethyl group, -CH₂OH. Similarly, a reference specifically to ortho-chlorophenyl is not to be construed as a reference to its structural isomer, meta-chlorophenyl.

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/amidine, nitroso/oxime, thioketone/enethiol, N-nitroso/hydroxyazo, and nitro/aci-nitro. A reference herein to one tautomer is intended to encompass both tautomers.

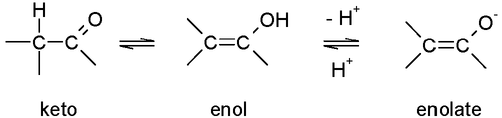

For example, 1H-pyridin-2-one-5-yl and 2-hydroxyl-pyridin-5-yl (shown below) are tautomers of one another. A reference herein to one is intended to encompass both.

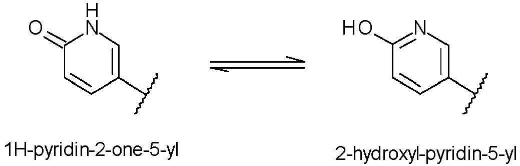

Note that specifically included in the term "isomer" are compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Unless otherwise specified, a reference to a particular compound includes all such isomeric forms, including mixtures (e.g., racemic mixtures) thereof. Methods for the preparation (e.g., asymmetric synthesis) and separation (e.g., fractional crystallisation and chromatographic means) of such isomeric forms are either known in the art or are readily obtained by adapting the methods taught herein, or known methods, in a known manner.

### Salts

It may be convenient or desirable to prepare, purify, and/or handle a corresponding salt of the compound, for example, a pharmaceutically-acceptable salt. Examples of pharmaceutically acceptable salts are discussed in Berge et al., 1977, "Pharmaceutically Acceptable Salts," J. Pharm. Sci., Vol. 66, pp. 1-19.

For example, if the compound is anionic, or has a functional group, which may be anionic (e.g., -COOH may be -COO⁻), then a salt may be formed with a suitable cation. Examples of suitable inorganic cations include, but are not limited to, alkali metal ions such as Na⁺ and K⁺, alkaline earth cations such as Ca²⁺ and Mg²⁺, and other cations such as Al³⁺ as well as the ammonium ion (i.e., NH₄⁺). Examples of suitable organic cations include, but are not limited to substituted ammonium ions (e.g., NH₃R⁺, NH₂R₂⁺, NHR₃⁺, NR₄⁺), for example, where each R is independently linear or branched saturated C₁₋₁₈alkyl, C₃₋₈cycloalkyl, C₃₋₈cycloalkyl-C₁₋₆alkyl, and phenyl-C₁₋₆alkyl, wherein the phenyl group is optionally substituted. Examples of some suitable substituted ammonium ions are those derived from: ethylamine, diethylamine, dicyclohexylamine, triethylamine, butylamine, ethylenediamine, ethanolamine, diethanolamine, piperazine, benzylamine, phenylbenzylamine, choline, meglumine, and tromethamine, as well as amino acids, such as lysine and arginine. An example of a common quaternary ammonium ion is N(CH₃)₄⁺.

If the compound is cationic, or has a functional group, which upon protonation may become cationic (e.g., -NH₂ may become -NH₃⁺), then a salt may be formed with a suitable anion.

For example, if a parent structure contains a cationic group (e.g., -NMe₂⁺), or has a functional group, which upon protonation may become cationic (e.g., -NH₂ may become -NH₃⁺), then a salt may be formed with a suitable anion. In the case of a quaternary ammonium compound a counter-anion is generally always present in order to balance the positive charge. If, in addition to a cationic group (e.g., -NMe₂⁺, -NH₃⁺), the compound also contains a group capable of forming an anion (e.g., -COOH), then an inner salt (also referred to as a zwitterion) may be formed.

Examples of suitable inorganic anions include, but are not limited to, those derived from the following inorganic acids: hydrochloric, hydrobromic, hydroiodic, sulfuric, sulfurous, nitric, nitrous, phosphoric, and phosphorous.

Examples of suitable organic anions include, but are not limited to, those derived from the following organic acids: 2-acetyloxybenzoic, acetic, trifluoroacetic, ascorbic, aspartic, benzoic, camphorsulfonic, cinnamic, citric, edetic, 1,2-ethanedisulfonic, ethanesulfonic, fumaric, glucoheptonic, gluconic, glutamic, glycolic, hydroxymaleic, hydroxynaphthalene carboxylic, isethionic, lactic, lactobionic, lauric, maleic, malic, methanesulfonic, mucic, oleic, oxalic, palmitic, pamoic, pantothenic, phenylacetic, phenylsulfonic, propionic, pyruvic, salicylic, stearic, succinic, sulfanilic, tartaric, toluenesulfonic, and valeric. Examples of suitable polymeric organic anions include, but are not limited to, those derived from the following polymeric acids: tannic acid, carboxymethyl cellulose.

Examples of suitable counter-ions which are especially suitable for quaternary ammonium compounds (e.g., those with a -NMe₂⁺ group) include 1-adamantanesulfonate, benzenesulfonate, bisulfate, bromide, chloride, iodide, methanesulfonate, methylsulfate, 1,5-napthalene-bis-sulfonate, 4-nitrobenzenesulfonate, formate, tartrate, tosylate, trifluoroacetate, trifluoromethylsulfonate, sulphate. Again, if the compound also contains a group capable of forming an anion (e.g., -COOH), then an inner salt may be formed.

Unless otherwise specified, a reference to a particular compound also includes salt forms thereof.

### Solvates and Hydrates

It may be convenient or desirable to prepare, purify, and/or handle a corresponding solvate of the compound. The term "solvate" is used herein in the conventional sense to refer to a complex of solute (e.g., compound, salt of compound) and solvent. If the solvent is water, the solvate may be conveniently referred to as a hydrate, for example, a mono-hydrate, a di-hydrate, a tri-hydrate, etc.

Unless otherwise specified, a reference to a particular compound also includes solvate and hydrate forms thereof.

### Chemically Protected Forms

It may be convenient or desirable to prepare, purify, and/or handle the compound in a chemically protected form. The term "chemically protected form" is used herein in the conventional chemical sense and pertains to a compound in which one or more reactive functional groups are protected from undesirable chemical reactions under specified conditions (e.g., pH, temperature, radiation, solvent, and the like). In practice, well-known chemical methods are employed to reversibly render unreactive a functional group, which otherwise would be reactive, under specified conditions. In a chemically protected form, one or more reactive functional groups are in the form of a protected or protecting group (alternatively as a masked or masking group or a blocked or blocking group). By protecting a reactive functional group, reactions involving other unprotected reactive functional groups can be performed, without affecting the protected group; the protecting group may be removed or the masking group transformed, usually in a subsequent step, without substantially affecting the remainder of the molecule. See, for example, Protective Groups in Organic Synthesis (T. Green and P. Wuts; 4th Edition; John Wiley and Sons, 2006).

A wide variety of such "protecting," "blocking," or "masking" methods are widely used and well known in organic synthesis. For example, a compound which has two nonequivalent reactive functional groups, both of which would be reactive under specified conditions, may be derivatized to render one of the functional groups "protected," and therefore unreactive, under the specified conditions; so protected, the compound may be used as a reactant which has effectively only one reactive functional group. After the desired reaction (involving the other functional group) is complete, the protected group may be "deprotected" to return it to its original functionality.

For example, a hydroxy group may be protected as an ether (-OR) or an ester (-OC(=O)R), for example, as: a t-butyl ether; a benzyl, benzhydryl (diphenylmethyl), or trityl (triphenylmethyl) ether; a trimethylsilyl or t-butyldimethylsilyl ether; or an acetyl ester (-OC(=O)CH₃, -OAc).

For example, an aldehyde or ketone group may be protected as an acetal (R-CH(OR)₂) or ketal (R₂C(OR)₂), respectively, in which the carbonyl group (>C=O) is converted to a 1,1-diether (>C(OR)₂), by reaction with, for example, a primary alcohol in the presence of an acid. The aldehyde or ketone group is readily regenerated, for example, by hydrolysis using water in the presence of acid.

For example, an amine group may be protected, for example, as an amide (-NRCO-R) or a urethane (-NRCO-OR), for example, as: an acetamide (-NHCO-CH₃); a benzyloxy amide (-NHCO-OCH₂C₆H₅, -NH-Cbz); as a t-butoxy amide (-NHCO-OC(CH₃)₃, -NH-Boc); a 2-biphenyl-2-propoxy amide (-NHCO-OC(CH₃)₂C₆H₄C₆H₅, -NH-Bpoc), as a 9-fluorenylmethoxy amide (-NH-Fmoc), as a 6-nitroveratryloxy amide (-NH-Nvoc), as a 2-trimethylsilylethyloxy amide (-NH-Teoc), as a 2,2,2-trichloroethyloxy amide (-NH-Troc), as an allyloxy amide (-NH-Alloc), as a 2(-phenylsulfonyl)ethyloxy amide (-NH-Psec); or, in suitable cases (e.g., cyclic amines), as a nitroxide radical (>N-O•).

For example, a carboxylic acid group may be protected as an ester for example, as: an C₁₋₇alkyl ester (e.g., a methyl ester; a t-butyl ester); a C₁₋₇haloalkyl ester (e.g., a 2,2,2-trihaloethyl ester); a 2-tri(C₁₋₇alkyl)silyl-ethyl ester; or a C₅₋₂₀aryl-C₁₋₇alkyl ester (e.g., a benzyl ester; a nitrobenzyl ester); or as an amide or hydrazide, for example, as acetamide or a *N,N,N'*-trimethylhydrazide.

For example, a thiol group may be protected as a thioether (-SR), for example, as: a benzyl thioether; an acetamidomethyl ether (-S-CH₂NHC(=O)CH₃).

### Prodrugs

It may be convenient or desirable to prepare, purify, and/or handle the compound in the form of a prodrug. The term "prodrug," as used herein, pertains to a compound, which yields the desired active compound *in vivo.* Typically, the prodrug is inactive, or less active than the desired active compound, but may provide advantageous handling, administration, or metabolic properties.

For example, some prodrugs are esters of the active compound (e.g., a physiologically acceptable metabolically labile ester). During metabolism, the ester group (-C(=O)OR) is cleaved to yield the active drug. Such esters may be formed by esterification, for example, of any of the carboxylic acid groups (-C(=O)OH) in the parent compound, with, where appropriate, prior protection of any other reactive groups present in the parent compound, followed by deprotection if required.

Also, some prodrugs are activated enzymatically to yield the active compound, or a compound, which, upon further chemical reaction, yields the active compound (for example, as in antibody directed enzyme prodrug therapy (ADEPT), gene directed enzyme prodrug therapy (GDEPT), lipid directed enzyme prodrug therapy (LIDEPT), etc.). For example, the prodrug may be a sugar derivative or other glycoside conjugate, or may be an amino acid ester derivative.

### Compositions

One aspect of the present invention pertains to a composition (e.g., a pharmaceutical composition) comprising a PPA compound, as described herein, and a pharmaceutically acceptable carrier, diluent, or excipient.

Another aspect of the present invention pertains to a method of preparing a composition (e.g., a pharmaceutical composition) comprising mixing a PPA compound, as described herein, and a pharmaceutically acceptable carrier, diluent, or excipient.

### Uses

The PPA compounds described herein are useful in the treatment of, for example, proliferative disorders (as "anti-proliferative agents"), cancer (as "anti-cancer agents"), viral infections (as "anti-viral agents"), neurodegenerative diseases (as "anti-neurodegenerative agents"), etc.

### Use in Methods of Inhibiting CDK

One aspect of the present invention pertains to a PPA compound described herein for use in a method of inhibiting CDK (e.g., CDK12 and/or CDK13) function (e.g., in a cell), *in vitro,* comprising contacting the cell with an effective amount of a PPA compound, as described herein.

One of ordinary skill in the art is readily able to determine whether or not a candidate compound inhibits CDK (e.g., CDK12 and/or CDK13). For example, suitable assays are described herein or are known in the art.

In one embodiment, the PPA compound is provided in the form of a pharmaceutically acceptable composition.

Any type of cell may be treated, including adipose, lung, gastrointestinal (including, e.g., bowel, colon), breast (mammary), ovarian, prostate, liver (hepatic), kidney (renal), bladder, pancreas, brain, and skin.

For example, a sample of cells may be grown *in vitro* and a compound brought into contact with said cells, and the effect of the compound on those cells observed. As an example of "effect," the morphological status of the cells (e.g., alive or dead, *etc.)* may be determined. Where the compound is found to exert an influence on the cells, this may be used as a prognostic or diagnostic marker of the efficacy of the compound in methods of treating a patient carrying cells of the same cellular type.

### Use in Methods of Inhibiting Cell Proliferation, etc.

The PPA compounds described herein, e.g., (a) regulate (e.g., inhibit) cell proliferation; (b) inhibit cell cycle progression; (c) promote apoptosis; or (d) a combination of one or more of these.

One aspect of the present invention pertains to a PPA compound described herein for use in a method of regulating (e.g., inhibiting) cell proliferation (e.g., proliferation of a cell), inhibiting cell cycle progression, promoting apoptosis, or a combination of one or more these, *in vitro* or *in vivo,* comprising contacting a cell with an effective amount of a PPA compound, as described herein.

In one embodiment, the method is a method of inhibiting cell proliferation (e.g., proliferation of a cell), *in vitro,* comprising contacting a cell with an effective amount of a PPA compound, as described herein.

In one embodiment, the PPA compound is provided in the form of a pharmaceutically acceptable composition.

Any type of cell may be treated, including lung, gastrointestinal (including, e.g., bowel, colon), breast (mammary), ovarian, prostate, liver (hepatic), kidney (renal), bladder, pancreas, brain, and skin.

One of ordinary skill in the art is readily able to determine whether or not a candidate compound regulates (e.g., inhibits) cell proliferation, *etc.* For example, assays, which may conveniently be used to assess the activity offered by a particular compound are described herein.

For example, a sample of cells (e.g., from a tumour) may be grown *in vitro* and a compound brought into contact with said cells, and the effect of the compound on those cells observed. As an example of "effect," the morphological status of the cells (e.g., alive or dead, *etc.)* may be determined. Where the compound is found to exert an influence on the cells, this may be used as a prognostic or diagnostic marker of the efficacy of the compound in methods of treating a patient carrying cells of the same cellular type.

### Use in Methods of Therapy

Another aspect of the present invention pertains to a PPA compound, as described herein, for use in a method of treatment of the human or animal body by therapy, for example, for use a method of treatment of a disorder (e.g., a disease) as described herein.

### Disorders Treated - Disorders Associated with CDK

In one embodiment, the treatment is treatment of: a disorder (e.g., a disease) that is associated with CDK, especially CDK12 and/or CDK13; a disorder (e.g., a disease) resulting from an inappropriate activity of a CDK, especially CDK12 and/or CDK13; a disorder (e.g., a disease) that is associated with CDK mutation, especially CDK12 and/or CDK13 mutation; a disorder (e.g., a disease) that is associated with CDK overexpression, especially CDK12 and/or CDK13 overexpression; a disorder (e.g., a disease) that is associated with upstream pathway activation of CDK, especially CDK12 and/or CDK13; a disorder (e.g., a disease) that is ameliorated by the inhibition (e.g., selective inhibition) of CDK, especially CDK12 and/or CDK13.

In one embodiment, the treatment is treatment of a disorder (e.g., a disease) that is associated with CDK, especially CDK12 and/or CDK13.

In one embodiment, the treatment is treatment of: a disorder (e.g., a disease) resulting from an inappropriate activity of CDK, especially CDK12 and/or CDK13.

In one embodiment, the treatment is treatment of: a disorder (e.g., a disease) that is associated with CDK mutation, especially CDK12 mutation; CDK overexpression, especially CDK12 and/or CDK13 overexpression (e.g., as compared to corresponding normal cells; e.g., wherein the overexpression is by a factor of 1.5, 2, 3, 5, 10, 20 or 50); or upstream pathway activation of CDK, especially CDK12 and/or CDK13.

In one embodiment, the treatment is treatment of a disorder (e.g., a disease) that is ameliorated by the inhibition (e.g., selective inhibition) of CDK, especially CDK12 and/or CDK13.

### Disorders Treated

In one embodiment, the treatment is treatment of: a proliferative disorder; cancer; a viral infection (e.g., HIV); a neurodegenerative disorder (e.g., Alzheimer's disease, Parkinson's disease); ischaemia; a renal disease; a cardiovascular disorder (e.g., atherosclerosis); or an autoimmune disorder (e.g., rheumatoid arthritis).

In one embodiment, the treatment is treatment of: a disorder (e.g., a disease) caused by dysfunction of translation in cells, for example, muscular dystrophy, amyotrophic lateral sclerosis, spinal muscular atrophy, and Fragile X syndrome.

In one embodiment, the treatment is treatment of: a disorder (e.g., a disease) in a patient who has received prior therapeutic treatments, but who receives little or no further clinical benefit from those treatments. This includes, for example, patients who have received prior therapeutic treatments with PARP inhibitors, CDK inhibitors, and/or HER2 directed therapies (see, e.g., Johnson *et al.,* 2016; Choi *et al.,* 2019).

### Disorders Treated - Proliferative Disorders

In one embodiment, the treatment is treatment of a proliferative disorder.

The term "proliferative disorder," as used herein, pertains to an unwanted or uncontrolled cellular proliferation of excessive or abnormal cells which is undesired, such as neoplastic or hyperplastic growth.

In one embodiment, the treatment is treatment of: a proliferative disorder characterised by benign, pre-malignant, or malignant cellular proliferation.

In one embodiment, the treatment is treatment of: hyperplasia; a neoplasm; a tumour (e.g., a histocytoma, a glioma, an astrocyoma, an osteoma); cancer; psoriasis; a bone disease; a fibroproliferative disorder (e.g., of connective tissues); pulmonary fibrosis; atherosclerosis; or smooth muscle cell proliferation in the blood vessels (e.g., stenosis or restenosis following angioplasty).

### Disorders Treated - Cancer

In one embodiment, the treatment is treatment of cancer.

In one embodiment, the treatment is treatment of cancer metastasis.

Included among cancers are:
(1) Carcinomas, including tumours derived from stratified squamous epithelia (squamous cell carcinomas) and tumours arising within organs or glands (adenocarcinomas). Examples include breast, colon, lung, prostate, ovary.
(2) Sarcomas, including: osteosarcoma and osteogenic sarcoma (bone); chondrosarcoma (cartilage); leiomyosarcoma (smooth muscle); rhabdomyosarcoma (skeletal muscle); mesothelial sarcoma and mesothelioma (membranous lining of body cavities); fibrosarcoma (fibrous tissue); angiosarcoma and haemangioendothelioma (blood vessels); liposarcoma (adipose tissue); glioma and astrocytoma (neurogenic connective tissue found in the brain); myxosarcoma (primitive embryonic connective tissue); mesenchymous and mixed mesodermal tumour (mixed connective tissue types).
(3) Myeloma.
(4) Haematopoietic tumours, including: myelogenous and granulocytic leukaemia (malignancy of the myeloid and granulocytic white blood cell series), e.g., chronic myeloid leukemia (CML), acute myeloid leukemia (AML); lymphatic, lymphocytic, and lymphoblastic leukaemia (malignancy of the lymphoid and lymphocytic blood cell series), e.g., acute lymphoblastic leukemia (ALL), chronic lymphocytic leukemia (CLL); polycythaemia vera (malignancy of various blood cell products, but with red cells predominating).
(5) Lymphomas, including: Hodgkin and Non-Hodgkin lymphomas.
(6) Mixed Types, including, e.g., adenosquamous carcinoma; mixed mesodermal tumour; carcinosarcoma; teratocarcinoma.

For example, in one embodiment, the treatment is treatment of breast cancer.

In one embodiment, the cancer is associated with CDK, especially CDK12 and/or CDK13.

In one embodiment, the cancer is characterised by, or further characterised by, inappropriate activity of CDK, especially CDK12 and/or CDK13.

In one embodiment, the cancer is characterised by, or further characterised by, overexpression of CDK, especially CDK12 and/or CDK13.

In one embodiment, the cancer is characterised by, or further characterised by, an amplification of the CDK12 and/or CDK13 gene, including, for example, cancers overexpressing the protein HER2 where the 17q12-q21 locus is amplified (see, e.g., Choi *et al.,* 2019).

In one embodiment, the cancer is characterised by, or further characterised by, a fusion of genes that cause cancers to appear, including, for example, cancers that have gene fusions of EWS-FLI (see, e.g., Inigues *et al.,* 2018), BCR-ABL, EML4-ALK, FGFR3-TACC3, KIF5B-RET, ETV6-RUNX1, or TMPRSS2-ERG.

The anti-cancer effect may arise through one or more mechanisms, including but not limited to, the regulation of cell proliferation, the inhibition of cell cycle progression, the inhibition of angiogenesis (the formation of new blood vessels), the inhibition of metastasis (the spread of a tumour from its origin), the inhibition of cell migration (the spread of cancer cells to other parts of the body), the inhibition of invasion (the spread of tumour cells into neighbouring normal structures), the promotion of apoptosis (programmed cell death), death by necrosis, or induction of death by autophagy. The compounds described herein may be used in the treatment of the cancers described herein, independent of the mechanisms discussed herein.

### Disorders Treated - DNA Repair

In one embodiment, the treatment is treatment of a disorder (e.g., a disease) in a patient having under-expression, defects, and/or mutations in the genes of proteins that are involved in DNA repair, including, e.g., BRCA1, BRCA2, ATM, ATR, BAP1, CDK12, CDK13, CHK1, CHK2, FANCA, FANCC, FANCD2, FANCE, FANCF, FANCI, PALB2, NBS1, WRN, RAD51B, RAD51C, RAD51D, MRE11A, BLM, BRIP1. This includes, for example, cancer patients whose tumours display "BRCAness" (see, e.g., Lord *et al.,* 2016).

In one embodiment, the treatment is treatment of a disorder (e.g., a disease) in a patient having under-expression, defects, and/or mutations in the genes of proteins that are involved in non-homologous DNA repair, including, e.g. XLF, RAD50, NBS1, MRE11, LIG4, XRCC4, POLL, POLM.

### Disorders Treated - Viral Infections

In one embodiment, the treatment is treatment of a viral infection.

In one embodiment, the treatment is treatment of a viral infection by:
(Group I:) a dsDNA virus, e.g., an adenovirus, a herpesvirus, a poxvirus;
(Group II:) a ssDNA virus, e.g., a parvovirus;
(Group III:) a dsRNA virus, e.g., a reovirus;
(Group IV:) a (+)ssRNA virus, e.g., a picornavirus, a togavirus;
(Group V:) a (-)ssRNA virus, e.g., an orthomyxovirus, a rhabdovirus;
(Group VI:) a ssRNA-RT virus, e.g., a retrovirus; or
(Group VII:) a dsDNA-RT virus, e.g., a hepadnavirus.

As used above: ds: double strand; ss: +strand; (+)ssRNA: +strand RNA; (-)ssRNA: -strand RNA; ssRNA-RT: (+ strand)RNA with DNA intermediate in life-cycle.

In one embodiment, the treatment is treatment of: human immunodeficiency virus (HIV); hepatitis B virus (HBV); hepatitis C virus (HCV); human papilloma virus (HPV); cytomegalovirus (CMV); or Epstein-Barr virus (EBV); human herpesvirus 8 (HHV) associated with Kaposi sarcoma; Coxsackievirus B3; Borna virus; influenza virus.

### Disorders Treated - Autoimmune Disorders

In one embodiment, the treatment is treatment of an autoimmune disorder.

In one embodiment, the treatment is treatment of: an autoimmune disorder associated with connective tissue, joints, skin, or the eye.

In one embodiment, the treatment is treatment of: rheumatoid arthritis, systemic lupus erythematosus, psoriasis, or Sjogren's syndrome.

### Disorders Treated - Disorders caused by Dysfunction of Translation in Cells

In one embodiment, the treatment is treatment of a disorder caused by dysfunction of translation in cells.

In one embodiment, the treatment is treatment of: muscular dystrophy, myotonic dystrophy, amyotrophic lateral sclerosis, spinal muscular atrophy, or Fragile X syndrome.

### Treatment

The term "treatment," as used herein in the context of treating a disorder, pertains generally to treatment of a human or an animal (e.g., in veterinary applications), in which some desired therapeutic effect is achieved, for example, the inhibition of the progress of the disorder, and includes a reduction in the rate of progress, a halt in the rate of progress, alleviation of symptoms of the disorder, amelioration of the disorder, and cure of the disorder. Treatment as a prophylactic measure (i.e., prophylaxis) is also included. For example, use with patients who have not yet developed the disorder, but who are at risk of developing the disorder, is encompassed by the term "treatment."

For example, treatment includes the prophylaxis of cancer, reducing the incidence of cancer, alleviating the symptoms of cancer, *etc.*

The term "therapeutically-effective amount," as used herein, pertains to that amount of a compound, or a material, composition or dosage form comprising a compound, which is effective for producing some desired therapeutic effect, commensurate with a reasonable benefit/risk ratio, when administered in accordance with a desired treatment regimen.

### Combination Therapies

The term "treatment" includes combination treatments and therapies, in which two or more treatments or therapies are combined, for example, sequentially or simultaneously. For example, the compounds described herein may also be used in combination therapies, e.g., in conjunction with other agents. Examples of treatments and therapies include chemotherapy (the administration of active agents, including, e.g., drugs, antibodies (e.g., as in immunotherapy), prodrugs (including, e.g., as in photodynamic therapy, GDEPT, ADEPT, *etc.));* surgery; radiation therapy; photodynamic therapy; gene therapy; and controlled diets.

One aspect of the present invention pertains to a compound as described herein, in combination with one or more (e.g., 1, 2, 3, 4, *etc.)* additional therapeutic agents, as described below.

The particular combination would be at the discretion of the physician who would select dosages using his common general knowledge and dosing regimens known to a skilled practitioner.

The agents (i.e., the compound described herein, plus one or more other agents) may be administered simultaneously or sequentially, and may be administered in individually varying dose schedules and via different routes. For example, when administered sequentially, the agents can be administered at closely spaced intervals (e.g., over a period of 5-10 minutes) or at longer intervals (e.g., 1, 2, 3, 4 or more hours apart, or even longer periods apart where required), the precise dosage regimen being commensurate with the properties of the therapeutic agent(s).

The agents (i.e., the compound described here, plus one or more other agents) may be formulated together in a single dosage form, or alternatively, the individual agents may be formulated separately and presented together in the form of a kit, optionally with instructions for their use.

Examples of additional agents/therapies that may be co-administered/combined with treatment with the PPA compounds described herein include the following:
a DNA repair inhibitor; for example, a PARP inhibitor, for example, Olaparib, Niraparib, etc.;
an immune checkpoint inhibitor; for example, an inhibitor of PD1, PD1L, CTLA4, etc., for example, pembrolizumab, atezolizumab, ipilimumab, etc.;
an agent stimulating the immune system; for example, a Toll-like receptor (TLR1-13) agonist, a Stimulator of Interferon Genes (STING) agonist, etc.;
a cell cycle checkpoint inhibitor; for example, an inhibitor of ATM, ATR, Wee1, etc., for example, AZD0156, AZD1390, AZD6738, adavosertib, etc.;
a Her2 blocker; for example, herceptin, pertuzumab, lapatinib, etc.;
a transcriptional inhibitor; for example, an inhibitor of BRD4, BRD2, CDK9, etc., for example, JQ-1, OTX015, AZD4573, etc.; and
a cytotoxic chemotherapeutic agent; for example, a taxane (e.g., paclitaxel also known as Taxol; docetaxel also known as Taxotere), cyclophosphamide, an antimetabolite (e.g., carboplatin, capecitabine, gemcitabine, doxorubicin, epirubicin, 5-fluorouracil, etc.).

Thus, in one embodiment, the treatment further comprises treatment (e.g., simultaneous or sequential treatment) with a further active agent which is, e.g., a DNA repair inhibitor, an immune checkpoint inhibitor, an agent stimulating the immune system, a cell cycle checkpoint inhibitor, a Her2 blocker, a transcriptional inhibitor, a cytotoxic chemotherapeutic agent, etc.

Further examples of additional agents/therapies that may be co-administered/combined with treatment with the PPA compounds described herein include the following:
an aromatase inhibitor; for example, exemestane (also known as Aromasin), letrozole (also known as Femara), anastrozole (also known as Arimidex), etc.;
an anti-estrogen; for example, faslodex (also known as Fulvestrant and ICI182780), tamoxifen (also known as Nolvadex), hydroxytamoxifen, etc.; and
an anti-androgen; for example, an anti-androgen used in the treatment of prostate cancer, for example, flutamide, enzalutamide, apalutamide, bicalutamide, nilutamide, etc.

### Other Uses

The PPA compounds described herein may also be used as cell culture additives to inhibit CDK (e.g., CDK12 and/or CDK13).

The PPA compounds described herein may also be used as part of an *in vitro* assay, for example, in order to determine whether a candidate host is likely to benefit from treatment with the compound in question.

The PPA compounds described herein may also be used as a standard, for example, in an assay, in order to identify other active compounds, other CDK12 and/or CDK13 inhibitors, etc.

### Kits

One aspect of the invention pertains to a kit comprising (a) a PPA compound as described herein, or a composition comprising a PPA compound as described herein, e.g., preferably provided in a suitable container and/or with suitable packaging; and (b) instructions for use, e.g., written instructions on how to administer the compound or composition.

The written instructions may also include a list of indications for which the active ingredient is a suitable treatment.

### Routes of Administration

The PPA compound or pharmaceutical composition comprising the PPA compound may be administered to a subject by any convenient route of administration, whether systemically/peripherally or topically (i.e., at the site of desired action).

Examples of routes of administration include oral (e.g., by ingestion); buccal; sublingual; transdermal (including, e.g., by a patch, plaster, *etc.*); transmucosal (including, e.g., by a patch, plaster, *etc*.); intranasal (e.g., by nasal spray); ocular (e.g., by eyedrops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intraarterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly.

### The Subject/Patient

The subject/patient may be a chordate, a vertebrate, a mammal, a placental mammal, a marsupial (e.g., kangaroo, wombat), a rodent (e.g., a guinea pig, a hamster, a rat, a mouse), murine (e.g., a mouse), a lagomorph (e.g., a rabbit), avian (e.g., a bird), canine (e.g., a dog), feline (e.g., a cat), equine (e.g., a horse), porcine (e.g., a pig), ovine (e.g., a sheep), bovine (e.g., a cow), a primate, simian (e.g., a monkey or ape), a monkey (e.g., marmoset, baboon), an ape (e.g., gorilla, chimpanzee, orangutang, gibbon), or a human.

Furthermore, the subject/patient may be any of its forms of development, for example, a foetus.

In one preferred embodiment, the subject/patient is a human.

### Formulations

While it is possible for a PPA compound to be administered alone, it is preferable to present it as a pharmaceutical formulation (e.g., composition, preparation, medicament) comprising at least one PPA compound, as described herein, together with one or more other pharmaceutically acceptable ingredients well known to those skilled in the art, including pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents. The formulation may further comprise other active agents, for example, other therapeutic or prophylactic agents.

Thus, the present invention further provides pharmaceutical compositions, as defined above, and methods of making a pharmaceutical composition comprising mixing at least one PPA compound, as described herein, together with one or more other pharmaceutically acceptable ingredients well known to those skilled in the art, e.g., carriers, diluents, excipients, *etc.* If formulated as discrete units (e.g., tablets, *etc.),* each unit contains a predetermined amount (dosage) of the compound.

The term "pharmaceutically acceptable," as used herein, pertains to compounds, ingredients, materials, compositions, dosage forms, *etc.,* which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, *etc.* must also be "acceptable" in the sense of being compatible with the other ingredients of the formulation.

Suitable carriers, diluents, excipients, *etc.* can be found in standard pharmaceutical texts, for example, Remington's Pharmaceutical Sciences, 18th edition, Mack Publishing Company, Easton, Pa., 1990; and Handbook of Pharmaceutical Excipients, 5th edition, 2005.

The formulations may be prepared by any methods well known in the art of pharmacy. Such methods include the step of bringing into association the compound with a carrier, which constitutes one or more accessory ingredients. In general, the formulations are prepared by uniformly and intimately bringing into association the compound with carriers (e.g., liquid carriers, finely divided solid carrier, *etc.),* and then shaping the product, if necessary.

The formulation may be prepared to provide for rapid or slow release; immediate, delayed, timed, or sustained release; or a combination thereof.

Formulations may suitably be in the form of liquids, solutions (e.g., aqueous, non-aqueous), suspensions (e.g., aqueous, non-aqueous), emulsions (e.g., oil-in-water, water-in-oil), elixirs, syrups, electuaries, mouthwashes, drops, tablets (including, e.g., coated tablets), granules, powders, losenges, pastilles, capsules (including, e.g., hard and soft gelatin capsules), cachets, pills, ampoules, boluses, suppositories, pessaries, tinctures, gels, pastes, ointments, creams, lotions, oils, foams, sprays, mists, or aerosols.

Formulations may suitably be provided as a patch, adhesive plaster, bandage, dressing, or the like which is impregnated with one or more compounds and optionally one or more other pharmaceutically acceptable ingredients, including, for example, penetration, permeation, and absorption enhancers. Formulations may also suitably be provided in the form of a depot or reservoir.

The compound may be dissolved in, suspended in, or mixed with one or more other pharmaceutically acceptable ingredients. The compound may be presented in a liposome or other microparticulate which is designed to target the compound, for example, to blood components or one or more organs.

Formulations suitable for oral administration (e.g., by ingestion) include liquids, solutions (e.g., aqueous, non-aqueous), suspensions (e.g., aqueous, non-aqueous), emulsions (e.g., oil-in-water, water-in-oil), elixirs, syrups, electuaries, tablets, granules, powders, capsules, cachets, pills, ampoules, boluses.

Formulations suitable for buccal administration include mouthwashes, losenges, pastilles, as well as patches, adhesive plasters, depots, and reservoirs. Losenges typically comprise the compound in a flavored basis, usually sucrose and acacia or tragacanth. Pastilles typically comprise the compound in an inert matrix, such as gelatin and glycerin, or sucrose and acacia. Mouthwashes typically comprise the compound in a suitable liquid carrier.

Formulations suitable for sublingual administration include tablets, losenges, pastilles, capsules, and pills.

Formulations suitable for oral transmucosal administration include liquids, solutions (e.g., aqueous, non-aqueous), suspensions (e.g., aqueous, non-aqueous), emulsions (e.g., oil-in-water, water-in-oil), mouthwashes, losenges, pastilles, as well as patches, adhesive plasters, depots, and reservoirs.

Formulations suitable for non-oral transmucosal administration include liquids, solutions (e.g., aqueous, non-aqueous), suspensions (e.g., aqueous, non-aqueous), emulsions (e.g., oil-in-water, water-in-oil), suppositories, pessaries, gels, pastes, ointments, creams, lotions, oils, as well as patches, adhesive plasters, depots, and reservoirs.

Formulations suitable for transdermal administration include gels, pastes, ointments, creams, lotions, and oils, as well as patches, adhesive plasters, bandages, dressings, depots, and reservoirs.

Tablets may be made by conventional means, e.g., compression or moulding, optionally with one or more accessory ingredients. Compressed tablets may be prepared by compressing in a suitable machine the compound in a free-flowing form such as a powder or granules, optionally mixed with one or more binders (e.g., povidone, gelatin, acacia, sorbitol, tragacanth, hydroxypropylmethyl cellulose); fillers or diluents (e.g., lactose, microcrystalline cellulose, calcium hydrogen phosphate); lubricants (e.g., magnesium stearate, talc, silica); disintegrants (e.g., sodium starch glycolate, cross-linked povidone, cross-linked sodium carboxymethyl cellulose); surface-active or dispersing or wetting agents (e.g., sodium lauryl sulfate); preservatives (e.g., methyl p-hydroxybenzoate, propyl p-hydroxybenzoate, sorbic acid); flavours, flavour enhancing agents, and sweeteners. Tablets may be made by moulding in a suitable machine a mixture of the powdered compound moistened with an inert liquid diluent. The tablets may optionally be coated or scored and may be formulated so as to provide slow or controlled release of the compound therein using, for example, hydroxypropylmethyl cellulose in varying proportions to provide the desired release profile. Tablets may optionally be provided with a coating, for example, to affect release, for example an enteric coating, to provide release in parts of the gut other than the stomach.

Ointments are typically prepared from the compound and a paraffinic or a water-miscible ointment base.

Creams are typically prepared from the compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the compound through the skin or other affected areas. Examples of such dermal penetration enhancers include dimethylsulfoxide and related analogues.

Emulsions are typically prepared from the compound and an oily phase, which may optionally comprise merely an emulsifier (otherwise known as an emulgent), or it may comprise a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties, since the solubility of the compound in most oils likely to be used in pharmaceutical emulsion formulations may be very low. Thus the cream should preferably be a non-greasy, non-staining and washable product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for intranasal administration, where the carrier is a liquid, include, for example, nasal spray, nasal drops, or by aerosol administration by nebuliser, include aqueous or oily solutions of the compound.

Formulations suitable for intranasal administration, where the carrier is a solid, include, for example, those presented as a coarse powder having a particle size, for example, in the range of about 20 to about 500 microns which is administered in the manner in which snuff is taken, i.e., by rapid inhalation through the nasal passage from a container of the powder held close up to the nose.

Formulations suitable for pulmonary administration (e.g., by inhalation or insufflation therapy) include those presented as an aerosol spray from a pressurised pack, with the use of a suitable propellant, such as dichlorodifluoromethane, trichlorofluoromethane, dichoro-tetrafluoroethane, carbon dioxide, or other suitable gases.

Formulations suitable for ocular administration include eye drops wherein the compound is dissolved or suspended in a suitable carrier, especially an aqueous solvent for the compound.

Formulations suitable for rectal administration may be presented as a suppository with a suitable base comprising, for example, natural or hardened oils, waxes, fats, semi-liquid or liquid polyols, for example, cocoa butter or a salicylate; or as a solution or suspension for treatment by enema.

Formulations suitable for vaginal administration may be presented as pessaries, tampons, creams, gels, pastes, foams or spray formulations containing in addition to the compound, such carriers as are known in the art to be appropriate.

Formulations suitable for parenteral administration (e.g., by injection) include aqueous or non-aqueous, isotonic, pyrogen-free, sterile liquids (e.g., solutions, suspensions), in which the compound is dissolved, suspended, or otherwise provided (e.g., in a liposome or other microparticulate). Such liquids may additionally contain other pharmaceutically acceptable ingredients, such as anti-oxidants, buffers, preservatives, stabilisers, bacteriostats, suspending agents, thickening agents, and solutes, which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Typically, the concentration of the compound in the liquid is from about 1 ng/mL to about 10 µg/mL, for example from about 10 ng/mL to about 1 µg/mL. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

### Dosage

It will be appreciated by one of skill in the art that appropriate dosages of the PPA compounds, and compositions comprising the PPA compounds, can vary from patient to patient. Determining the optimal dosage will generally involve the balancing of the level of therapeutic benefit against any risk or deleterious side effects. The selected dosage level will depend on a variety of factors including the activity of the particular PPA compound, the route of administration, the time of administration, the rate of excretion of the PPA compound, the duration of the treatment, other drugs, compounds, and/or materials used in combination, the severity of the disorder, and the species, sex, age, weight, condition, general health, and prior medical history of the patient. The amount of PPA compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

In general, a suitable dose of the PPA compound is in the range of about 10 µg to about 250 mg (more typically about 100 µg to about 25 mg) per kilogram body weight of the subject per day. Where the compound is a salt, an ester, an amide, a prodrug, or the like, the amount administered is calculated on the basis of the parent compound and so the actual weight to be used is increased proportionately.

### Chemical Synthesis

### Abbreviations

aq: aqueous;
BOC: tert-butoxycarbonyl;
Boc₂O: di-*tert*-butyl dicarbonate;
br: broad;
*ca.*: *circa*;
d: doublet;
^{t}BuBrettPhos Pd G3: [(2-Di-tert-butylphosphino-3,6-dimethoxy-2',4',6'-triisopropyl-1,1'-biphenyl)-2-(2'-amino-1,1'-biphenyl)]palladium(II) methanesulfonate;
DCM: dichloromethane;
DCE: 1,2-dichloroethane;
dioxane: 1,4-dioxane;
DIPEA: diisopropylethylamine;
DMAP: 4-(Dimethylamino)pyridine;
EtOAc: ethyl acetate;
EtOH: ethanol;
h: hours;
HPLC: high performance liquid chromatography;
IPA: 2-isopropanol;
LCMS: liquid chromatography - mass spectrometry;
LiHMDS: lithium hexamethyldisilazide;
m: multiplet;
M: molar, molecular ion;
mCPBA: 3-chloroperbenzoic acid;
MeCN: actetonitrile;
MeOH: methanol;
min: minutes;
MS: mass spectrometry;
NCS: N-chlorosuccinimide;
NIS: N-iodosuccinimide;
NMR: nuclear magnetic resonance;
Pd (dppf)Cl₂-DCM: [1,1'-Bis(diphenylphosphino)ferrocene]dichloropalladium(II), complex with dichloromethane;
Pd-162: Chloro(crotyl)(tri-tert-butylphosphine) palladium (II);
Pd-171: Chloro(crotyl)(2-Dicyclohexylphosphino-2',6'-diisopropoxy-1,1'-biphenyl) palladium(II)
q: quartet;
RT: room temperature (ca. 20 °C);
R_{T}: retention time;
Ruphos: 2-Dicyclohexylphosphino-2',6'-diisopropoxybiphenyl;
s: singlet, solid;
SCX: strong cation exchange;
t: triplet;
TBME: tert-butyl methyl ether;
TFA: trifluoroacetic acid;
THF: tetrahydrofuran;
UPLC/MS: ultra performance liquid chromatography - mass spectrometry.

Other abbreviations are intended to convey their generally accepted meaning.

Nomenclature of structures was generated using 'Structure to Name' conversion from ChemDraw^{®} Professional 20 (PerkinElmer).

### General Synthetic Methods

Methods for the chemical synthesis of the PPA compounds are described herein. These and/or other well-known methods may be modified and/or adapted in known ways in order to provide alternative or improved methods of synthesis of the PPA compounds.

In a first method, the synthesis starts with the 5,7-dichloro-3-pyrazolo[1,5-a]pyrimidine derivative I-1. Nucleophilic aromatic substitution with amine affords the corresponding 5-amino-pyrazolopyrimidine I-2. Boc-protection of the amino group affords the intermediate I-3. The latter is used in a Buchwald-Hartwig cross-coupling with a primary or secondary amine to yield the intermediate I-4. Final global Boc-deprotection yields the target compounds I-5.

This method is illustrated in the following chemical scheme.

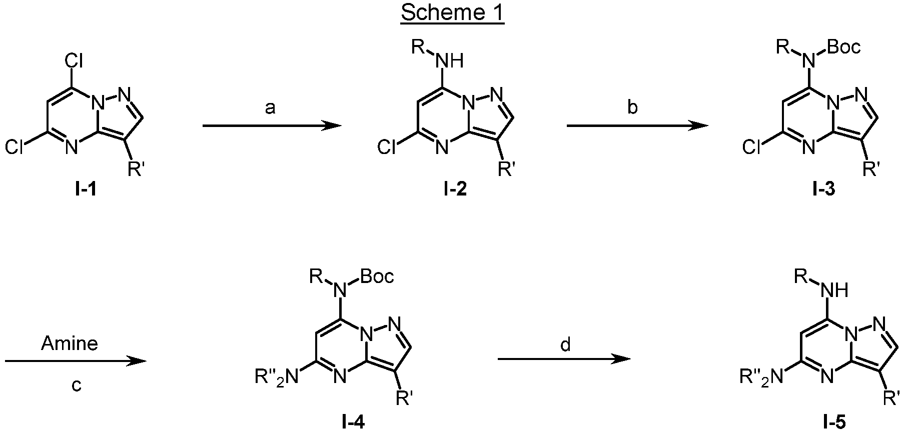

Representative reactions conditions for the above scheme are as follows: (a) RNH₂, DIPEA, EtOH or IPA, 50 °C to 90 °C; (b) Boc₂O, DMAP, THF, RT to 60 °C; (c) ^{t}BuBrettPhos Pd G3, LiHMDS, THF, 60 °C; or Pd-171, Cs₂CO₃, dioxane, 90 °C; (d) TFA, DCM, RT; or HCl, dioxane, RT to 40 °C.

In a second method, intermediate I-3 is used in a Buchwald-Hartwig cross-coupling with an alcohol to yield the intermediate I-6. Final global Boc-deprotection yields the target compounds I-7.

This method is illustrated in the following chemical scheme.

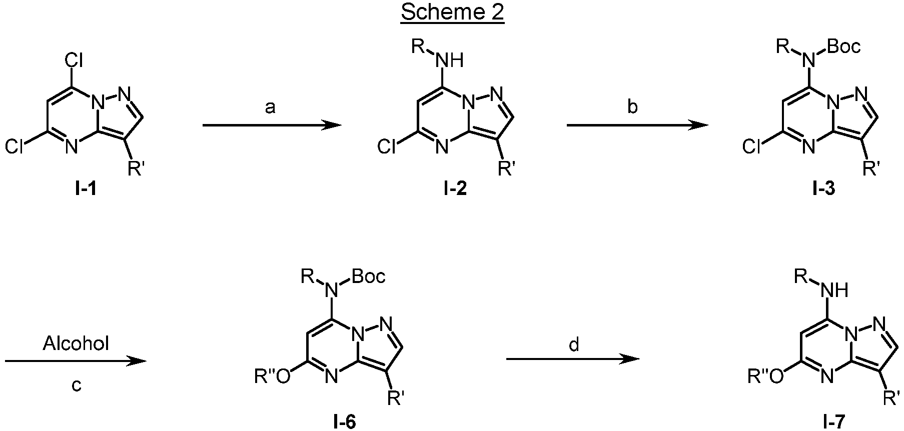

Representative reactions conditions for the above scheme are as follows: (a) RNH₂, DIPEA, EtOH or IPA, 50 °C to 90 °C; (b) Boc₂O, DMAP, THF, RT to 60 °C; (c) Pd-171, Cs₂CO₃, dioxane, 90 °C; (d) TFA, DCM, RT; or HCl, dioxane, RT to 40 °C.

In a third method, intermediate I-2 is used in a Buchwald-Hartwig cross-coupling with a primary or secondary amine to yield the intermediate I-8. Final global Boc-deprotection yields the target compounds I-5.

This method is illustrated in the following chemical scheme.

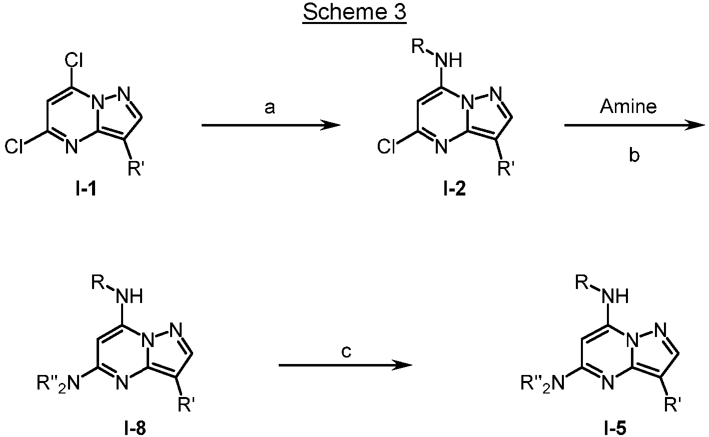

Representative reactions conditions for the above scheme are as follows: (a) RNH₂, DIPEA, EtOH or IPA, 50 °C to 90 °C; (b) ^{t}BuBrettPhos Pd G3, LiHMDS, dioxane, 90 °C; (c) TFA, DCM, RT; or HCl, dioxane, RT to 40 °C.

In a fourth method, intermediate I-2 is used in a nucleophilic aromatic substitution with an amine to yield I-8. Final global Boc-deprotection yields the target compounds I-5.

This method is illustrated in the following chemical scheme.

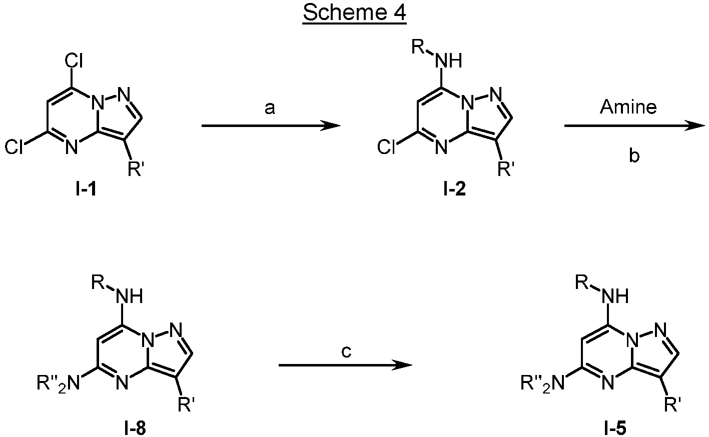

Representative reactions conditions for the above scheme are as follows: (a) RNH₂, DIPEA, EtOH or IPA, 50 °C to 90 °C; (b) DIPEA, NMP, 120 °C, microwave; (c) TFA, DCM, RT; or HCl, dioxane, RT to 40 °C.

In a fifth method, intermediate I-3 is used in a Negishi cross-coupling to yield I-12. Final global Boc-deprotection yields the target compounds I-13.

This method is illustrated in the following chemical scheme.

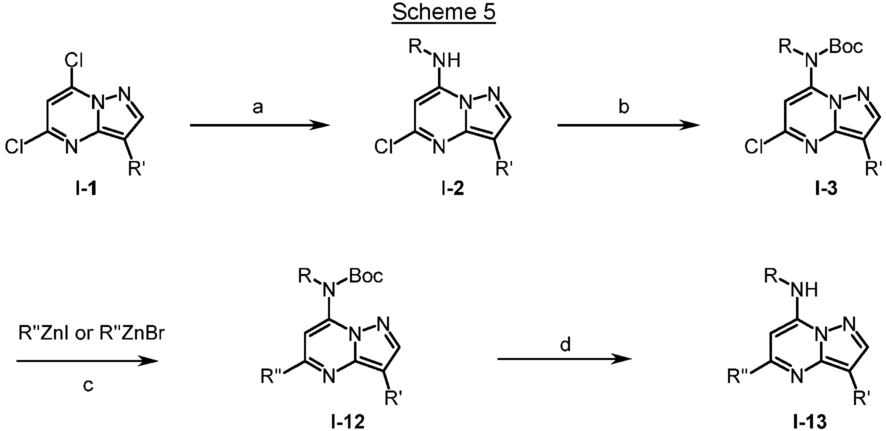

Representative reactions conditions for the above scheme are as follows: (a) RNH₂, DIPEA, EtOH or IPA, 50 °C to 90 °C; (b) Boc₂O, DMAP, THF, RT to 60 °C; (c) Pd(dppf)Cl₂, THF, 50 °C; (d) TFA, DCM, RT; or HCl, dioxane, RT to 40 °C.

In a sixth method, intermediate I-3 is used in a Suzuki cross-coupling to yield I-12. Final global Boc-deprotection yields the target compounds I-13.

This method is illustrated in the following chemical scheme.

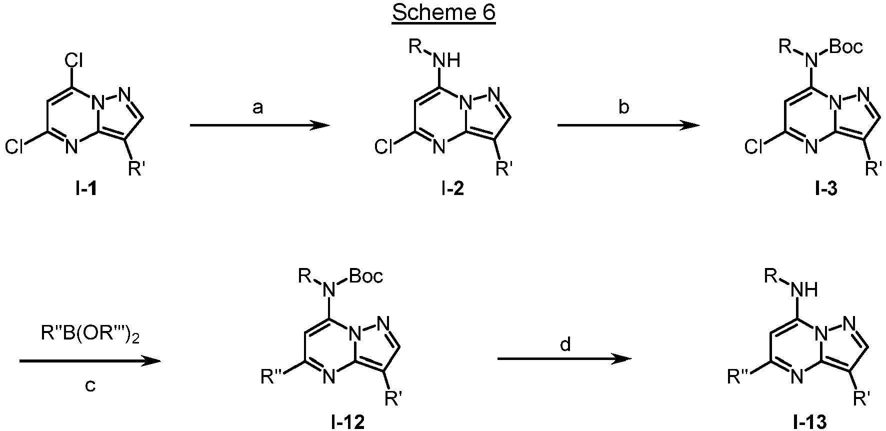

Representative reactions conditions for the above scheme are as follows: (a) RNH₂, DIPEA, EtOH , 50 °C to 90 °C; (b) Boc₂O, DMAP, THF, RT to 60 °C; (c) Pd(dppf)Cl₂, potassium phosphate tribasic, dioxane, water, 90 °C; (d) TFA, DCM, RT; or HCl, dioxane, RT to 40 °C.

### Chemical Synthesis Examples

The following examples are provided solely to illustrate the present invention and are not intended to limit the scope of the invention, as described herein.

### General Experimental Conditions

All starting materials and solvents were obtained either from commercial sources or prepared according to the literature citation. Reaction mixtures were magnetically stirred unless otherwise indicated.

Column chromatography was performed on an automated flash chromatography system, such as a CombiFlash Rf system, using Grace^{™} GraceResolv^{™} pre-packed silica (40 µm) cartridges, unless otherwise indicated.

¹H NMR spectra were recorded using a Bruker Avance III spectrometer (400 MHz) r Bruker (500 MHz). Chemical shifts are expressed in parts per million using either the central peaks of the residual protic solvent or an internal standard of tetramethylsilane as references. The spectra were recorded at ambient temperature unless otherwise stated.

Analytical LCMS experiments to determine retention times and associated mass ions were performed using an Agilent or Waters HPLC/UPLC system coupled to a mass spectrometer running Methods 1-8 described below.

Preparative HPLC purifications were performed using a Waters X-Bridge BEH C18, 5 µm, 19x50 mm column using a gradient of MeCN and 10 mM ammonium bicarbonate (aq). Fractions were collected following detection by UV at a single wavelength measured by a variable wavelength detector.

SCX resin was purchased from Sigma Aldrich or Silicycle and washed with MeOH prior to use.

### Analytical Methods

### Method 1:

LCMS_Acidic, Apparatus: Agilent 1260; Quaternary Pump, HiP Sampler, Column Compartment, DAD:260+/- 90nm, G6150 MSD: ESI; Column: Waters Cortecs C18, 30 x 2.1 mm, 2.7µm, Temp: 40°C, Flow: 1.35 mL/min, Gradient: t0 = 5% B, t2.5min = 100% B, t3.0min = 100% B, Eluent A: 0.1% Formic in water, Eluent B: acetonitrile.

### Method 2:

UPLC_Acidic, Apparatus: Waters HClass; Quaternary Solvent Pump, SM-FTN, CMA, PDA:210-400 nm, QDa: ACQ-QDa ESI; Column: Waters CSH C18, 30 x 2.1 mm, 1.7µm, Temp: 40°C, Flow: 0.77 mL/min, Gradient: t0 = 5% B, t3.0min = 95% B, Eluent A: 0.1% Formic acid in water, Eluent B: acetonitrile.

### Method 3:

UPLC_Basic, Apparatus: Waters HClass; Binary Solvent Pump, SM-FTN, CMA, PDA:210-400 nm, QDa: ACQ-QDa ESI; Column: Waters BEH C18, 30 x 2.1 mm, 1.7µm, Temp: 40°C, Flow: 0.77 mL/min, Gradient: t0 = 5% B, t3.0min = 95% B, Eluent A: 0.1% NH₃ in water or 10 nM ammonium bicarbonate, Eluent B: acetonitrile.

### Method 4:

UPLC_Acid, Apparatus: Waters HClass; Quaternary Solvent Pump, SM-FTN, CMA, PDA:210-400 nm, QDa: ACQ-QDa ESI; Column: Waters CSH C18, 30 x 2.1 mm, 1.7µm, Temp: 40°C, Flow: 0.77 mL/min, Gradient: t0 = 2% B, t2.5min = 100% B, t3.0min = 100% B, Eluent A: 0.1% Formic acid in water, Eluent B: acetonitrile.

### Method 5:

UPLC_Basic, Apparatus: Waters HClass; Binary Solvent Pump, SM-FTN, CMA, PDA:210-400 nm, QDa: ACQ-QDa ESI; Column: Waters BEH C18, 30 x 2.1 mm, 1.7µm, Temp: 40°C, Flow: 0.77 mL/min, Gradient: t0 = 2% B, t2.5min = 100% B, t3.0min = 100% B, Eluent A: 0.1% NH₃ in water or 10 nM ammonium bicarbonate, Eluent B: acetonitrile.

### Method 6:

UPLC_Basic, Apparatus: Waters HClass; Binary Solvent Pump, SM-FTN, CMA, PDA:210-400 nm, QDa: ACQ-QDa ESI; Column: Waters BEH C18, 30 x 2.1 mm, 1.7µm, Temp: 40°C, Flow: 0.77 mL/min, Gradient: t0 = 2% B, t9.5min = 100% B, t10.0min = 100% B, Eluent A: 0.1% NH₃ in water, Eluent B: acetonitrile.

### Synthesis 006

### (3R,4R)-4-(((3-cyclopropyl-7-((4-(pyridin-2-yl)benzyl)amino)pyrazolo[1,5-a]pyrimidin-5-yl)amino)methyl)piperidin-3-ol

### (PPA-006)

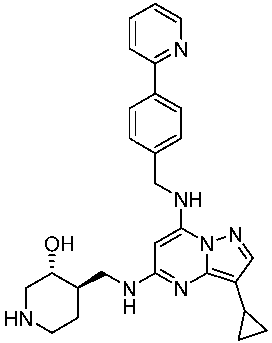

### Step 1:

### 5-chloro-3-cyclopropyl-N-(4-(pyridin-2-yl)benzyl)pyrazolo[1,5-a]pyrimidin-7-amine

DIPEA (0.99 mL, 5.70 mmol) was added to a solution of 5,7-dichloro-3-cyclopropylpyrazolo[1,5-a]pyrimidine (186 mg, 814 µmol) and (4-(pyridin-2-yl)phenyl)methanamine (150 mg, 814 µmol) in EtOH (4.0 mL). The reaction mixture was heated to 50 °C overnight. The reaction mixture was concentrated *in vacuo.* Purification by column chromatography (12 g cartridge, 0-10% MeOH/DCM) gave the title compound (239 mg, 0.52 mmol, 64% yield, 82% purity) as a yellow oil.

UPLC/MS (Method 3): m/z 376 (M+H)⁺, R_{T} 1.59 min.

### Step 2:

### tert-butyl (5-chloro-3-cyclopropylpyrazolo[1,5-a]pyrimidin-7-yl)(4-(pyridin-2-yl)benzyl)carbamate

DMAP (15 mg, 0.12 mmol) was added to a solution of 5-chloro-3-cyclopropyl-N-(4-(pyridin-2-yl)benzyl)pyrazolo[1,5-a]pyrimidin-7-amine (239 mg, 521 µmol) and BOC-anhydride (167 mg, 765 µmol) in anhydrous THF (6.0 mL). The reaction mixture was stirred at RT overnight then concentrated *in vacuo.* Purification by column chromatography (12 g cartridge, 0-5% MeOH/DCM) gave the title compound (164 mg, 0.32 mmol, 62% yield, 94% purity) as a yellow oil.

UPLC/MS (Method 3): m/z 476 (M+H)⁺, R_{T} 1.94 min.

### Step 3:

### tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(4-(pyridin-2-yl)benzyl)carbamate (164 mg, 345 µmol), tert-butyl (3R,4R)-4-(aminomethyl)-3-hydroxypiperidine-1-carboxylate (95 mg, 413 µmol) in THF (4.0 mL) was degassed in N₂ for 5 min before adding ^{t}BuBrettPhos Pd G3 (29 mg, 34.5 µmol) and LiHMDS (1M in THF) (448 µL, 448 µmol). The reaction was degassed for another 5 min before being heated to 60 °C for 3 h. The reaction mixture was cooled to RT and concentrated *in vacuo.* Purification by column chromatography (12 g cartridge, 0-10% MeOH/DCM) followed by further purification on RP Flash C18 (12 g cartridge, 0-100% MeCN/10 mM ammonium bicarbonate) gave the title compound (124 mg, 0.17 mmol, 50% yield, 93% purity) a white solid.

UPLC/MS (Method 3): m/z 670 (M+H)⁺, R_{T} 1.80 min.

### Step 4:

### (3R,4R)-4-(((3-cyclopropyl-7-((4-(pyridin-2-yl)benzyl)amino)pyrazolo[1,5-a]pyrimidin-5-yl)amino)methyl)piperidin-3-ol

TFA (0.3 mL) was added to a solution of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-hydroxypiperidine-1-carboxylate (124 mg, 185 µmol) in DCM (1.2 mL). The reaction mixture was stirred at RT overnight and concentrated *in vacuo.* The solid was loaded onto a column of SCX. The solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (44 mg, 93 µmol, 50% yield, 99% purity) as an off-white solid.

UPLC/MS (Method 3): m/z 470 (M+H)⁺, RT 1.22 min.

¹H NMR (400 MHz, DMSO-d₆) δ 8.65 (ddd, *J* = 4.8, 1.9, 1.0 Hz, 1H), 8.05 (d, *J* = 8.4 Hz, 2H), 7.96 - 7.91 (m, 1H), 7.90 - 7.83 (m, 2H), 7.52 (s, 1H), 7.46 (d, *J* = 8.1 Hz, 2H), 7.33 (ddd, *J* = 7.4, 4.8, 1.2 Hz, 1H), 6.71 (t, *J =* 5.8 Hz, 1H), 5.33 - 5.26 (m, 1H), 5.18 (s, 1H), 4.50 (d, *J* = 6.2 Hz, 2H), 3.56 - 3.44 (m, 1H), 3.24 - 3.13 (m, 1H), 3.06 - 2.97 (m, 1H), 2.90 (dd, *J* = 11.5, 4.5 Hz, 1H), 2.81 - 2.73 (m, 1H), 2.33 - 2.24 (m, 1H), 2.21 - 2.08 (m, 1H), 1.76 - 1.66 (m, 1H), 1.58 - 1.51 (m, 1H), 1.38 - 1.26 (m, 1H), 1.19 - 1.09 (m, 1H), 0.79 - 0.72 (m, 2H), 0.70 - 0.60 (m, 2H). 1H under water.

### Synthesis 020

### (3R,4R)-4-(((3-cyclopropyl-7-((3-methoxy-4-(pyridin-2-yl)benzyl)amino)pyrazolo[1,5-a]pyrimidin-5-yl)amino)methyl)piperidin-3-ol

### (PPA-015)

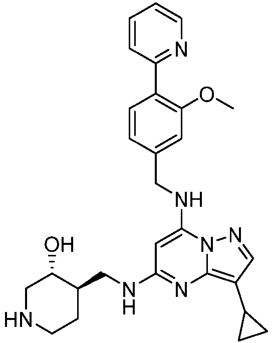

### Step 1: 5-chloro-3-cyclopropyl-N-(3-methoxy-4-(pyridin-2-yl)benzyl)pyrazolo[1,5-a]pyrimidin-7-amine

DIPEA (0.73 mL, 4.16 mmol) was added to a solution of (3-methoxy-4-(pyridin-2-yl)phenyl)methanamine (140 mg, 653 µmol) and 5,7-dichloro-3-cyclopropylpyrazolo[1,5-a]pyrimidine (135 mg, 594 µmol) in EtOH (6.0 mL). The reaction mixture was heated at 50 °C for 3 h. The reaction mixture was concentrated *in vacuo.* Purification by column chromatography (12 g cartridge, 0-100% EtOAc/isohexane) gave the title compound (105 mg, 0.25 mmol, 41% yield, 95% purity) as a white crystalline solid.

UPLC/MS (Method 3): m/z 406 (M+H)⁺, R_{T} 1.59 min.

### Step 2: tert-butyl (5-chloro-3-cyclopropylpyrazolo[1,5-a]pyrimidin-7-yl)(3-methoxy-4-(pyridin-2-yl)benzyl)carbamate

BOC-anhydride (68 mg, 310 µmol) was added to a solution of 5-chloro-3-cyclopropyl-N-(3-methoxy-4-(pyridin-2-yl)benzyl)pyrazolo[1,5-a]pyrimidin-7-amine (105 mg, 259 µmol) and DMAP (6.3 mg, 52 µmol) in THF (3.0 mL). The reaction mixture was stirred at RT for 1.5 h. The reaction mixture was concentrated under reduced pressure. Purification by column chromatography (12 g cartridge, 0-100% EtOAc/isohexane) gave the title compound (112 mg, 0.21 mmol, 81% yield, 95% purity) as a green oil.

UPLC/MS (Method 3): m/z 506 (M+H)⁺, R_{T} 1.91 min.

### Step 3: tert-butyl (3R,4R)-4-(((7-((tert-butoxycarbonyl)(3-methoxy-4-(pyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(3-methoxy-4-(pyridin-2-yl)benzyl)carbamate (112 mg, 221 µmol) and tert-butyl (3R,4R)-4-(aminomethyl)-3-hydroxypiperidine-1-carboxylate (61 mg, 266 µmol) and in THF (3.0 mL) was degassed with N₂ for 10 min. before adding ^{t}BuBrettPhos Pd G3 (19 mg, 22 µmol) and LiHMDS (1 M in THF) (288 µL, 288 µmol). The reaction was degassed for another 5 min before being heated to 60 °C for 2.5 h. The reaction mixture was concentrated under reduced pressure. Purification by column chromatography (12 g cartridge, 0-10% MeOH/DCM) gave the title compound (147 mg, 0.18 mmol, 81% yield, 85% purity) as a brown glass.

UPLC/MS (Method 3): m/z 663 (M+H)⁺, R_{T} 1.61 min.

### Step 4: (3R,4R)-4-(((3-cyclopropyl-7-((3-methoxy-4-(pyridin-2-yl)benzyl)amino)pyrazolo[1,5-a]pyrimidin-5-yl)amino)methyl)piperidin-3-ol

TFA (1.0 mL) was added to a solution of tert-butyl (3R,4R)-4-(((7-((tert-butoxycarbonyl)(3-methoxy-4-(pyridin-2-yl)benzyl)amino)-3-cyclopropylpyrazolo[1,5-a]pyrimidin-5-yl)amino)methyl)-3-hydroxypiperidine-1-carboxylate (147 mg, 210 µmol) in DCM (3.0 mL). The reaction mixture was stirred at RT for 3 h and concentrated *in vacuo.* The residue was loaded onto a column of SCX. The column was washed with MeOH (30 mL) and the product eluted with 0.7 M NH₃ in MeOH (30 mL). The ammoniacal methanol solution was concentrated *in vacuo* to give the title compound (73 mg, 0.14 mmol, 66 % yield, 95% purity) as a pink solid.

UPLC/MS (Method 3): m/z 500 (M+H)+, R_{T} 1.59 min.

¹H NMR (400 MHz, DMSO-d₆) δ 8.65 - 8.60 (m, 1H), 7.88 (t, *J* = 6.4 Hz, 1H), 7.85 - 7.74 (m, 2H), 7.69 (d, *J* = 7.9, 2.1 Hz, 1H), 7.53 (s, 1H), 7.32 - 7.26 (m, 1H), 7.19 (s, 1H), 7.03 (d, *J* = 7.6 Hz, 1H), 6.76 - 6.69 (m, 1H), 5.36 - 5.27 (m, 1H), 5.21 (s, 1H), 4.49 (d, *J* = 6.2 Hz, 2H), 3.82 (s, 3H), 3.58 - 3.45 (m, 1H), 3.25 - 3.17 (m, 1H), 3.08 - 2.98 (m, 1H), 2.95 - 2.87 (m, 1H), 2.83 - 2.75 (m, 1H), 2.35 - 2.26 (m, 1H), 2.20 - 2.12 (m, 1H), 1.77 - 1.67 (m, 1H), 1.60 - 1.52 (m, 1H), 1.39 - 1.28 (m, 1H), 1.20 - 1.11 (m, 1H), 0.79 - 0.74 (m, 2H), 0.70 - 0.59 (m, 2H). 1H under water.

### Synthesis 021

### (3-methoxy-4-(pyridin-2-yl)phenyl)methanamine

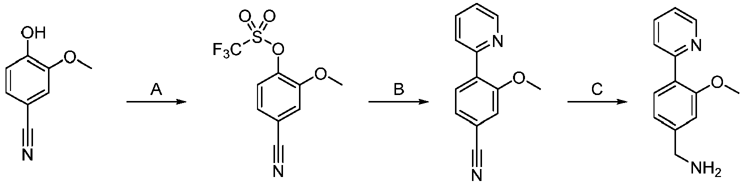

### Step A:

### 4-cyano-2-methoxyphenyl trifluoromethanesulfonate

Trifluoromethanesulfonic anhydride (0.73 mL, 4.36 mmol) was added to a solution of 3,4-hydroxy-3-methoxybenzonitrile (500 mg, 3.35 mmol) and triethylamine (509 mg, 701 µL, 1.5 Eq, 5.03 mmol) in DCM (15 mL) at 0 °C. The reaction mixture was stirred at RT for 2 h. The reaction mixture was diluted with aq. sat. NaHCO₃ (20 mL) and extracted with DCM (3 x 10 mL). The combined organic fractions were washed with brine (50 mL), dried over MgSO₄, filtered, then concentrated *in vacuo* to give the title compound (938 mg, 3.20 mmol, 95% yield, 95% purity) as a white solid.

¹H NMR (400 MHz, DMSO-d₆) δ 7.91 (d, J = 1.9 Hz, 1H), 7.71 (d, J = 8.4 Hz, 1H), 7.60 (dd, J = 8.4, 1.9 Hz, 1H), 3.97 (s, 3H).

### Step B:

### 3-methoxy-4-(pyridin-2-yl)benzonitrile

A solution of 2-(tributylstannyl)pyridine (1.47 g, 4.00 mmol), 4-cyano-2-methoxyphenyl trifluoromethanesulfonate (938 mg, 3.34 mmol), Pd(Ph₃P)₄ (385 mg, 334 µmol) and lithium chloride (141 mg, 3.34 mmol) in DMF (15.0 mL) was degassed with N₂ for 15 min. Bubbling was ceased and the reaction mixture heated to 90 °C for 16 h. The reaction mixture was concentrated under reduced pressure. The mixture was cooled to RT then diluted with water (50 mL) and brine (50 mL) and extracted into MTBE (3 x 50 mL). The combined organic layers were washed with brine (50 mL) then dried over MgSO₄, filtered and concentrated *in vacuo.* Purification by column chromatography (24 g cartridge, 0-100% EtOAc/isohexane) gave the title compound (233 mg, 0.94 mmol, 28% yield, 85% prity) as a white solid.

UPLC/MS (Method 3): m/z 211 (M+H)⁺, R_{T} 1.16 min.

### Step C:

### (3-methoxy-4-(pyridin-2-yl)phenyl)methanamine

LiAlH₄ (4 M in Et2O) (180 µL, 720 µmol) was added to a solution of 3-methoxy-4-(pyridin-2-yl)benzonitrile (155 mg, 627 µmol) in THF (8.0 mL) at 0 °C. The mixture was stirred for 15 min at 0 °C then allowed to warm to RT and stirred for a further 15 min. The mixture was quenched with methanol until effervescence ceased then the mixture was diluted with water (100 mL) and extracted with DCM (3 x 50 mL). The combined organic layers were washed with brine (50 mL), dried over MgSO₄, filtered and concentrated *in vacuo* to afford the title compound (140 mg, 0.52 mmol, 83% yield, 80% purity) as a brown solid.

UPLC/MS (Method 3): m/z 215 (M+H)⁺, R_{T} 0.93 min.

### Synthesis 025

### (3R,4R)-4-(((3-cyclopropyl-7-((4-(5-fluoropyridin-2-yl)benzyl)amino)pyrazolo[1,5-a]pyrimidin-5-yl)amino)methyl)piperidin-3-ol

### (PPA-019)

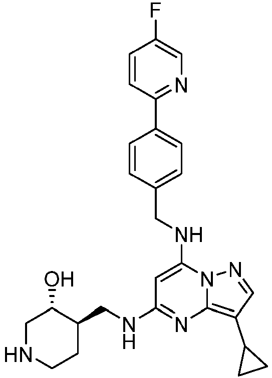

### Step 1/2:

### tert-butyl (5-chloro-3-cyclopropylpyrazolo[1,5-a]pyrimidin-7-yl)(4-(5-fluoropyridin-2-yl)benzyl)carbamate

DIPEA (0.46 mL, 2.63 mmol) was added to a solution of 5,7-dichloro-3-cyclopropylpyrazolo[1,5-a]pyrimidine (100 mg, 438 µmol) and (4-(5-fluoropyridin-2-yl)phenyl)methanamine (102 mg, 504 µmol) in EtOH (3.0 mL). The reaction mixture was heated at 60 °C overnight. The reaction mixture was concentrated under reduced pressure. Purification by column chromatography silica gel (12 g cartridge, 0-10% MeOH/DCM) gave the intermediate 5-chloro-3-cyclopropyl-N-(4-(5-fluoropyridin-2-yl)benzyl)pyrazolo[1,5-a]pyrimidin-7-amine.

5-chloro-3-cyclopropyl-N-(4-(5-fluoropyridin-2-yl)benzyl)pyrazolo[1,5-a]pyrimidin-7-amine was dissolved in THF (2.5 mL) then BOC-anhydride (87.3 mg, 400 µmol) and DMAP (3.26 mg, 26.7 µmol) were added. The reaction mixture was concentrated under reduced pressure. Purification by column chromatography silica gel (12 g cartridge, 0-10% MeOH (containing 0.7 M NH₃)/DCM) gave the title compound (115 mg, 0.22 mmol, 50% yield, 95% purity) as a yellow foam.

¹H NMR (400 MHz, DMSO-d₆) δ 8.62 (d, *J* = 2.9 Hz, 1H), 8.10 (s, 1H), 8.00 (dd, *J* = 8.9, 4.4 Hz, 1H), 7.99 - 7.92 (m, 2H), 7.79 (td, *J =* 8.8, 3.0 Hz, 1H), 7.45 - 7.39 (m, 2H), 7.18 (s, 1H), 5.05 (s, 2H), 1.96 (tt, *J* = 8.3, 5.1 Hz, 1H), 1.28 (s, 9H), 0.97 - 0.86 (m, 2H), 0.82 - 0.72 (m, 2H).

### Step 3:

### tert-butyl (3R,4R)-4-(((7-((tert-butoxycarbonyl)(4-(5-fluoropyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(4-(5-fluoropyridin-2-yl)benzyl)carbamate (116 mg, 222 µmol) and tert-butyl (3R,4R)-4-(aminomethyl)-3-hydroxypiperidine-1-carboxylate (61 mg, 266 µmol) in THF (3.8 mL) was degassed with N₂ for 5 min before adding ^{t}BuBrettPhos Pd G3 (19 mg, 22 µmol) and LiHMDS (1 M in THF) (267 µL, 267 µmol) were added under bubbling N₂. The reaction was degassed for another 5 min before heating to 60-65 °C for 2 h. The reaction mixture was concentrated under reduced pressure. Purification by column chromatography (12 g cartridge, 0-100% (25% EtOH in EtOAc/ isohexane) gave the title compound (97 mg, 0.14 mmol, 61% yield, 96% purity) as a brown oil.

UPLC/MS (Method 3): m/z 688 (M+H)⁺, R_{T} 1.88 min.

### Step 4:

### (3R,4R)-4-(((3-cyclopropyl-7-((4-(5-fluoropyridin-2-yl)benzyl)amino)pyrazolo[1,5-a]pyrimidin-5-yl)amino)methyl)piperidin-3-ol

HCl (4 M in dioxane) (0.67 mL, 2.7 mmol) was added to a solution of tert-butyl (3R,4R)-4-(((7-((tert-butoxycarbonyl)(4-(5-fluoropyridin-2-yl)benzyl)amino)-3-cyclopropylpyrazolo[1,5-a]pyrimidin-5-yl)amino)methyl)-3-hydroxypiperidine-1-carboxylate (96 mg, 0.13 mmol) in dioxane (1.8 mL). The suspension was stirred at 40 °C for 1.5 h before adding HCl (1.25 M in MeOH) (1.00 mL, 1.25 mmol) and further stirring at 40 °C for 30 min. The reaction mixture was concentrated *in vacuo.* The residue was loaded onto a column of SCX. The column was washed with DCM/MeOH (1:2; 120 mL) and the product eluted with DCM/0.7 M NH3 in MeOH (1:2; 150 mL). The ammoniacal methanol solution was concentrated *in vacuo* to give the title compound (61.5 mg, 0.12 mmol, 91% yield, 97% purity) as a beige solid.

UPLC/MS (Method 3: m/z 488 (M+H)⁺, R_{T} 1.49 min.

¹H NMR (400 MHz, DMSO-d₆) δ 8.63 (d, J = 3.0 Hz, 1H), 8.05 - 7.97 (m, 3H), 7.90 (t, J = 6.5 Hz, 1H), 7.84 - 7.77 (m, 1H), 7.52 (s, 1H), 7.45 (d, J = 8.1 Hz, 2H), 6.74 - 6.68 (m, 1H), 5.36 - 5.26 (m, 1H), 5.17 (s, 1H), 4.50 (d, J = 6.2 Hz, 2H), 3.56 - 3.44 (m, 1H), 3.23 - 3.16 (m, 1H), 3.07 - 2.98 (m, 1H), 2.94 - 2.87 (m, 1H), 2.81 - 2.74 (m, 1H), 2.32 - 2.24 (m, 1H), 2.19 - 2.12 (m, 1H), 1.77 - 1.67 (m, 1H), 1.59 - 1.50 (m, 1H), 1.39 - 1.27 (m, 1H), 1.19 - 1.11 (m, 1H), 0.79 - 0.73 (m, 2H), 0.69 - 0.60 (m, 2H). 1H under water.

### Synthesis 026

### (3R,4R)-4-(((3-cyclopropyl-7-((2-fluoro-4-(pyridin-2-yl)benzyl)amino)pyrazolo[1,5-a]pyrimidin-5-yl)amino)methyl)piperidin-3-ol

### (PPA-020)

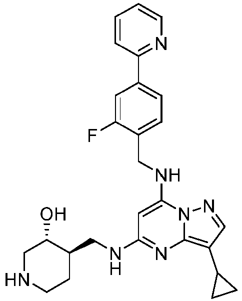

### Step 1:

### 5-chloro-3-cyclopropyl-N-(2-fluoro-4-(pyridin-2-yl)benzyl)pyrazolo[1,5-a]pyrimidin-7-amine

DIPEA (0.15 mL, 0.88 mmol) was added to a solution of 5,7-dichloro-3-cyclopropylpyrazolo[1,5-a]pyrimidine (38 mg, 0.15 mmol) and (2-fluoro-4-(pyridin-2-yl)phenyl)methanamine (30 mg, 0.15 mmol)in EtOH (3.0 mL). The reaction mixture was heated at 60 °C overnight. The reaction mixture was concentrated under reduced pressure. Purification by column chromatography (12 g cartridge, 12 g cartridge, 0-10% MeOH (containing 0.7 M NH₃)/DCM) gave the title compound (40 mg, 96 µmol, 66% yield, 95% purity) as a colourless oil.

UPLC/MS (Method 3): m/z 394 (M+H)⁺, R_{T} 1.68 min.

### Step 2:

### tert-butyl (5-amino-3-cyclopropylpyrazolo[1,5-a]pyrimidin-7-yl)(2-fluoro-4-(pyridin-2-yl)benzyl)carbamate

BOC-anhydride (27 mg, 0.12 mmol) was added to a solution of 5-chloro-3-cyclopropyl-N-(2-fluoro-4-(pyridin-2-yl)benzyl)pyrazolo[1,5-a]pyrimidin-7-amine (40 mg, 0.10 mmol) and DMAP (2.5 mg, 20 µmol) in THF (3.0 mL). The reaction mixture was stirred at RT for 1.5 h. The reaction mixture was concentrated under reduced pressure. Purification by column chromatography (12 g cartridge, 0-100% EtOAc/isohexane) gave the title compound (43 mg, 86 µmol, 85% yield, 99% purity) as a thick green oil.

UPLC/MS (Method 3): m/z 494 (M+H)⁺, R_{T} 1.99 min.

### Step 3:

### tert-butyl (3R,4R)-4-(((7-((tert-butoxycarbonyl)(2-fluoro-4-(pyridin-2-yl)benzyl)amino)-3-cyclopropylpyrazolo[1,5-a]pyrimidin-5-yl)amino)methyl)-3-hydroxypiperidine-1-carboxylate

A suspension of tert-butyl (5-chloro-3-cyclopropylpyrazolo[1,5-a]pyrimidin-7-yl)(2-fluoro-4-(pyridin-2-yl)benzyl)carbamate (40.0 mg, 81.0 µmol), tert-butyl (3R,4R)-4-(aminomethyl)-3-hydroxypiperidine-1-carboxylate (28.0 mg, 121 µmol) and cesium carbonate (79.2 mg, 243 µmol) in dioxane (1.5 mL) was degassed with N₂ for 5 min. Pd-171 (1.6 mg, 2.4 µmol) and Ruphos phosphane (1.3 mg, 2.8 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (39 mg, 50 µmol, 62% yield, 89% purity) as a yellow glass solid.

UPLC/MS (Method 3): m/z 688 (M+H)⁺, R_{T} 1.92 min.

### Step 4:

### ((3R,4R)-4-(((3-cyclopropyl-7-((2-fluoro-4-(pyridin-2-yl)benzyl)amino)pyrazolo[1,5-a]pyrimidin-5-yl)amino)methyl)piperidin-3-ol

HCl (4 M in dioxane) (126 µL, 505 µmol) was added to a solution of tert-butyl (3R,4R)-4-(((7-((tert-butoxycarbonyl)(2-fluoro-4-(pyridin-2-yl)benzyl)amino)-3-cyclopropylpyrazolo[1,5-a]pyrimidin-5-yl)amino)methyl)-3-hydroxypiperidine-1-carboxylate (39 mg, 50 µmol) in dioxane (2.0 mL). The suspension was stirred at 35 °C for 4 h. The reaction mixture was concentrated *in vacuo.* The residue was loaded onto a column of SCX. The column was washed with MeOH (140 mL) and the product eluted with 0.7 M NH₃ in MeOH (10 mL). The ammoniacal methanol solution was concentrated *in vacuo* to give the title compound (16 mg, 32 µmol, 64% yield, 98% purity). as a yellow solid.

UPLC/MS (Method 3): m/z 488 (M+H)⁺, R_{T} 1.53 min.

¹H NMR (400 MHz, DMSO-d₆) δ 7.95 - 7.88 (m, 4H), 7.77 (d, J = 3.2 Hz, 1H), 7.53 (s, 1H), 7.46 (d, J = 8.2 Hz, 2H), 6.70 (t, J = 6.0 Hz, 1H), 5.32 - 5.26 (m, 1H), 5.15 (s, 1H), 4.50 (d, J = 6.1 Hz, 2H), 3.56 - 3.43 (m, 1H), 3.23 - 3.14 (m, 1H), 3.07 - 2.97 (m, 1H), 2.93 - 2.86 (m, 1H), 2.81 - 2.73 (m, 1H), 2.34 - 2.23 (m, 1H), 2.18 - 2.10 (m, 1H), 1.75 - 1.66 (m, 1H), 1.59 - 1.50 (m, 1H), 1.38 - 1.26 (m, 1H), 1.19 - 1.05 (m, 1H), 0.81 - 0.71 (m, 2H), 0.70 - 0.60 (m, 2H). 1H under water.

### Synthesis 027

### (2-fluoro-4-(pyridin-2-yl)phenyl)methanamine

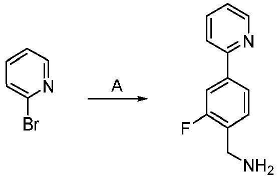

### Step A:

### (2-fluoro-4-(pyridin-2-yl)phenyl)methanamine

A suspension of 2-bromopyridine (60 µL, 633 µmol), (4-(aminomethyl)-3-fluorophenyl)boronic acid (118 mg, 696 µmol), Pd(dppf)Cl₂.DCM (52 mg, 63 µmol) and cesium carbonate (206 mg, 633 µmol) in dioxane (9.0 mL) and water (1.0 mL) was degassed with N₂ for 5 min. The reaction mixture was stirred at 90 °C for 2 h. At RT, the reaction mixture was diluted with water (50 mL) and extracted with EtOAc (2 x 25 mL) and filtered through celite, rinsing with EtOAc (20 mL). The combined organic layers were dried over MgSO₄, filtered and concentrated *in vacuo.* Purification by column chromatography (24 g cartridge, 0-10% MeOH (containing 0.7 M NH₃)/DCM) gave the title compound (38 mg, 0.17 mmol, 26% yield, 88% purity) as an orange oil.

UPLC/MS (Method 6): m/z 203 (M+H)⁺, R_{T} 1.98 min.

### Synthesis 030

### (3R,4R)-4-(((3-cyclopropyl-7-((3-fluoro-4-(pyridin-2-yl)benzyl)amino)pyrazolo[1,5-a]pyrimidin-5-yl)amino)methyl)piperidin-3-ol

### (PPA-022)

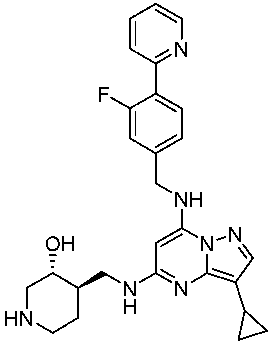

### Step 1:

### 5-chloro-3-cyclopropyl-N-(3-fluoro-4-(pyridin-2-yl)benzyl)pyrazolo[1,5-a]pyrimidin-7-amine

DIPEA (367 µL, 2.10 mmol) was added to a solution of 5,7-dichloro-3-cyclopropylpyrazolo[1,5-a]pyrimidine (80 mg, 351 µmol) and (3-fluoro-4-(pyridin-2-yl)phenyl)methanamine (82 mg, 389 µmol), in EtOH (1.4 mL). The reaction mixture was heated at 65 °C for 2.5 h. The reaction mixture was concentrated under reduced pressure. Purification by column chromatography (12 g cartridge, 0-100% (25% EtOH in EtOAc)/isohexane) gave the title compound (93 mg, 0.21 mmol, 59% yield, 88% purity) as a yellow oil.

UPLC/MS (Method 2): m/z 394 (M+H)⁺, R_{T} 1.60 min.

### Step 2:

### tert-butyl (5-chloro-3-cyclopropylpyrazolo[1,5-a]pyrimidin-7-yl)(3-fluoro-4-(pyridin-2-yl)benzyl)carbamate

DMAP (5.1 mg, 42 µmol) was added to a solution of 5-chloro-3-cyclopropyl-N-(3-fluoro-4-(pyridin-2-yl)benzyl)pyrazolo[1,5-a]pyrimidin-7-amine (93 mg, 208 µmol), and BOC-anhydride (59 mg, 270 µmol) in THF (4.6 mL). The resulting reaction mixture was heated at 65 °C for 4 h. The reaction mixture was concentrated under reduced pressure. Purification by column chromatography (12 g cartridge, 0-100% (25% EtOH in EtOAc)/isohexane) gave the title compound (94 mg, 0.16 mmol, 79% yield, 87% purity) as a yellow oil.

UPLC/MS (Method 2): m/z 494 (M+H)⁺, R_{T} 1.98 min.

### Step 3:

### tert-butyl (3R,4R)-4-(((7-((tert-butoxycarbonyl)(3-fluoro-4-(pyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(3-fluoro-4-(pyridin-2-yl)benzyl)carbamate (93 mg, 185 µmol) and tert-butyl (3R,4R)-4-(aminomethyl)-3-hydroxypiperidine-1-carboxylate (51 mg, 221 µmol) in THF (2.0 mL) was degassed with N₂ for 5 min, before adding ^{t}BuBrettPhos Pd G3 (15.8 mg, 18.5 µmol) and LiHMDS (1 M in THF) (221 µL, 221 µmol). The reaction was degassed for another 5 min before heating to 60-65 °C for 4 h. The reaction mixture was concentrated under reduced pressure. Purification by column chromatography (12 g cartridge, 0-100% (25% EtOH in EtOAc)/isohexane) gave the title compound (36 mg, 50 µmol, 27% yield, 95% purity) as a yellow oil.

UPLC/MS (Method 2): m/z 688 (M+H)⁺, R_{T} 1.84 min.

### Step 4:

### (3R,4R)-4-(((3-cyclopropyl-7-((3-fluoro-4-(pyridin-2-yl)benzyl)amino)pyrazolo[1,5-a]pyrimidin-5-yl)amino)methyl)piperidin-3-ol

HCl (4 M in dioxane) (244 µL, 974 µmol) was added to a solution of tert-butyl (3R,4R)-4-(((7-((tert-butoxycarbonyl)(3-fluoro-4-(pyridin-2-yl)benzyl)amino)-3-cyclopropylpyrazolo[1,5-a]pyrimidin-5-yl)amino)methyl)-3-hydroxypiperidine-1-carboxylate (33.5 mg, 48.7 µmol) in dioxane (1.0 mL). The suspension was stirred at 40 °C for 2 h before adding HCl in methanol (1.25 M in MeOH) (1.00 mL, 1.25 mmol) and stirring for further 30 min. The reaction mixture was concentrated *in vacuo.* The residue was loaded onto a column of SCX. The column was washed with MeOH (100 mL) and the product eluted with 0.7 M NH₃ in MeOH (100 mL). The ammoniacal methanol solution was concentrated *in vacuo* to give the title compound (20 mg, 40 µmol, 82% yield, 97% purity). as a grey solid.

UPLC/MS (Method 3): m/z 488 (M+H)+, R_{T} 1.31 min.

¹H NMR (400 MHz, DMSO-d₆) δ 8.73 - 8.65 (m, 1H), 7.99 - 7.85 (m, 3H), 7.79 - 7.73 (m, 1H), 7.53 (s, 1H), 7.43 - 7.36 (m, 1H), 7.34 - 7.25 (m, 2H), 6.72 (t, J = 6.0 Hz, 1H), 5.31 - 5.24 (m, 1H), 5.18 (s, 1H), 4.51 (d, J = 6.3 Hz, 2H), 3.57 - 3.41 (m, 1H), 3.25 - 3.17 (m, 1H), 3.08 - 2.98 (m, 1H), 2.94 - 2.86 (m, 1H), 2.82 - 2.74 (m, 1H), 2.35 - 2.24 (m, 1H), 2.18 - 2.11 (m, 1H), 1.76 - 1.67 (m, 1H), 1.59 - 1.51 (m, 1H), 1.38 - 1.26 (m, 1H), 1.18 - 1.06 (m, 1H), 0.79 - 0.73 (m, 2H), 0.70 - 0.60 (m, 2H). 1H under water.

### Synthesis 031

### (3-fluoro-4-(pyridin-2-yl)phenyl)methanamine

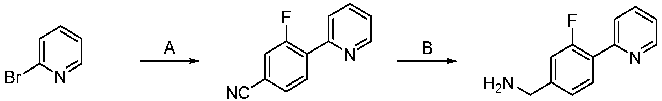

### Step A:

### 3-fluoro-4-(pyridin-2-yl)benzonitrile

2-Bromopyridine (250 mg, 151 µL, 1.58 mmol), (4-cyano-2-fluorophenyl)boronic acid (346 mg, 2.06 mmol), and Pd(dppf)Cl2 (116 mg, 158 µmol) were taken up in dioxane (7.0 mL). The mixture was sparged with N₂ for 10 min then solution of potassium carbonate (656 mg, 4.75 mmol) in water (778 µL) was added and N2 was bubbled through the reaction mixture for further 5 min. The mixture was heated to 90 °C ON then allowed to cool to RT. The reaction mixture was diluted in EtOAc (20 mL) and filtered on celite. The filtrated was further diluted with water (10 mL) and extracted with EtOAc (2x 15 mL). The combined organics were washed with brine (2 x 15 mL), dried with MgSO₄, filtered and concentrated under reduced pressure. Purification by column chromatography (24 g cartridge, 0-100% 25% EtOH in EtOAc/isohexane) gave the title compound (297 mg, 1.4 mmol, 89% yield, 94% purity) as a white solid.

UPLC/MS (Method 2): m/z 199 (M+H)+, R_{T} 1.16 min.

### Step B:

### (3-fluoro-4-(pyridin-2-yl)phenyl)methanamine

3-Fluoro-4-(pyridin-2-yl)benzonitrile (142 mg, 573 µmol) and acetic acid (1.00 mL, 17.4 mmol) in MeOH (19 mL) was subjected to hydrogenation (hydrogen pressure set to 30 bar) in the H-cube for 2.5 h at 45 °C using a 10% palladium on carbon cartridge, recirculating the mixture through the cartridge (flow rate: 1 mL/min). The reaction mixture was concentrated under reduced pressure (co-evaporation with MeCN). The crude was precipitated and triturated in MeCN in an ice bath. The suspension was filtrated and the solid was dried under reduced pressure to afford the title compound (82 mg, 0.33 mmol, 48% yield, 80% purity) as a yellow solid.

UPLC/MS (Method 3): m/z 203 (M+H)⁺, R_{T} 1.12 min.

### Synthesis 037

### (3R,4R)-4-(((3-cyclopropyl-7-((4-(3-methoxypyridin-2-yl)benzyl)amino)pyrazolo[1,5-a]pyrimidin-5-yl)amino)methyl)piperidin-3-ol

### (PPA-027)

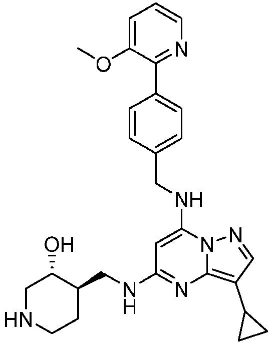

### Step 1:

### 5-chloro-3-cyclopropyl-N-(4-(3-methoxypyridin-2-yl)benzyl)pyrazolo[1,5-a]pyrimidin-7-amine

DIPEA (0.26 mL, 1.50 mmol) was added to a solution of 5,7-dichloro-3-cyclopropylpyrazolo[1,5-a]pyrimidine (57 mg, 250 µmol), and (4-(3-methoxy)pyridin-2-yl)phenyl)methanamine (57 mg, 250 µmol) in EtOH (1.0 mL). The reaction mixture was heated to 65 °C for 4 h. The reaction mixture was concentrated *in vacuo* then the residue redissolved in EtOH (1.0 mL) and more DIPEA (0.17 mL, 1.00 mmol) and 5,7-dichloro-3-cyclopropylpyrazolo[1,5-a]pyrimidine (20 mg, 88 µmol) were added. The reaction mixture was heated to 65 °C for 2 h. The reaction mixture was concentrated *in vacuo.* Purification by column chromatography (12 g cartridge, 0-100% EtOH in EtOAc 25:75/isohexane) gave the title compound (126 mg, 0.30 mmol, 90% yield, 98% purity) as a yellow solid.

UPLC/MS (Method 2): m/z 406 (M+H)⁺, R_{T} 1.60 min.

### Step 2:

### tert-butyl (5-chloro-3-cyclopropylpyrazolo[1,5-a]pyrimidin-7-yl)(4-(3-methoxypyridin-2-yl)benzyl)carbamate

DMAP (7.4 mg, 61 µmol) was added to a solution of 5-chloro-3-cyclopropyl-N-(4-(3-methoxypyridin-2-yl)benzyl)pyrazolo[1,5-a]pyrimidin-7-amine (124 mg, 304 µmol) and BOC-anhydride (100 mg, 456 µmol) in anhydrous THF (6.0 mL). The reaction mixture was heated at 65 °C for 4 h then concentrated *in vacuo.* Purification by column chromatography (12 g cartridge, 0-100% EtOH in EtOAc 25:75/isohexane) gave the title compound (151 mg, 0.29 mmol, 96% yield, 98% purity) as a yellow oil.

UPLC/MS (Method 3): m/z 506 (M+H)⁺, R_{T} 1.92 min.

### Step 3:

### tert-butyl (3R,4R)-4-(((7-((tert-butoxycarbonyl)(4-(3-methoxypyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(4-(3-methoxypyridin-2-yl)benzyl)carbamate (149 mg, 295 µmol), tert-butyl (3R,4R)-4-(aminomethyl)-3-hydroxypiperidine-1-carboxylate (81 mg, 353 µmol) in THF (3.3 mL) was degassed in N₂ for 5 min before adding ^{t}BuBrettPhos Pd G3 (25.2 mg, 29.5 µmol) and LiHMDS (1M in THF) (353 µL, 353 µmol). The reaction was degassed for another 5 min before being heated to 60 °C for 4 h. The reaction mixture concentrated *in vacuo.* Purification by column chromatography (12 g cartridge, 0-100% EtOH in EtOAc 25:75/isohexane) gave the title compound (114 mg, 0.15 mmol, 52% yield, 95% purity) as a brown oil.

UPLC/MS (Method 3): m/z 700 (M+H)⁺, R_{T} 1.89 min.

### Step 4:

### (3R,4R)-4-(((3-cyclopropyl-7-((4-(3-methoxypyridin-2-yl)benzyl)amino)pyrazolo[1,5-a]pyrimidin-5-yl)amino)methyl)piperidin-3-ol

Hydrogen chloride (4 M in dioxane) (0.77 mL, 3.07 mmol) was added to a solution of tert-butyl (3R,4R)-4-(((7-((tert-butoxycarbonyl)(4-(3-methoxypyridin-2-yl)benzyl)amino)-3-cyclopropylpyrazolo[1,5-a]pyrimidin-5-yl)amino)methyl)-3-hydroxypiperidine-1-carboxylate (113 mg, 153 µmol) in dioxane (2.4 mL). The reaction mixture was stirred at 40 °C for 2 h then Hydrogen chloride (1.25 M in MeOH) (1.0 mL, 1.3 mmol) was added, and the reaction mixture was stirred at 40 °C for 30 min then concentrated *in vacuo.* The solid was triturated in MeCN (2 x 2 mL) then was loaded onto a column of SCX. The column was washed with MeOH (80 mL) and the product was eluted with 0.7 M NH₃ in MeOH (100 mL). The ammoniacal methanol solution was concentrated *in vacuo* and the solid was triturated in Et₂O (2 x 1 mL) to give the title compound (68 mg, 0.13 mmol, 87% yield, 97% purity) as an orange solid.

LCMS (Method 2): m/z 500 (M+H)⁺, RT 0.65 min.

¹H NMR (400 MHz, DMSO-d₆) δ 8.23 (dd, *J* = 4.6, 1.3 Hz, 1H), 7.89 (t, *J* = 6.5 Hz, 1H), 7.83 (d, *J* = 8.4 Hz, 2H), 7.54 (dd, *J* = 8.4, 1.4 Hz, 1H), 7.53 (s, 1H), 7.40 (d, *J* = 8.3 Hz, 2H), 7.34 (dd, *J* = 8.3, 4.6 Hz, 1H), 6.73 (t, *J* = 6.0 Hz, 1H), 5.42 - 5.29 (m, 1H), 5.19 (s, 1H), 4.49 (d, *J* = 6.4 Hz, 2H), 3.83 (s, 3H), 3.57 - 3.44 (m, 1H), 3.24 - 3.16 (m, 1H), 3.09 - 2.99 (m, 1H), 2.91 (dd, *J* = 11.6, 4.6 Hz, 1H), 2.84 - 2.73 (m, 1H), 2.36 - 2.27 (m, 1H), 2.17 (dd, *J* = 11.6, 9.9 Hz, 1H), 1.78 - 1.65 (m, 1H), 1.61 - 1.53 (m, 1H), 1.39 - 1.29 (m, 1H), 1.21 - 1.08 (m, 1H), 0.83 - 0.71 (m, 2H), 0.70 - 0.59 (m, 2H). 1H under water.

### Synthesis 039

### (3R,4R)-4-(((3-cyclopropyl-7-((4-(3-(trifluoromethoxy)pyridin-2-yl)benzyl)amino)pyrazolo[1,5-a]pyrimidin-5-yl)amino)methyl)piperidin-3-ol

### (PPA-028)

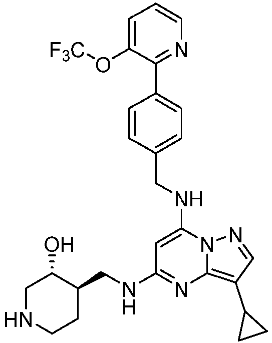

### Step 1:

### 5-chloro-3-cyclopropyl-N-(4-(3-(trifluoromethoxy)pyridin-2-yl)benzyl)pyrazolo[1,5-a]pyrimidin-7-amine

DIPEA (0.28 mL, 1.58 mmol) was added to a solution of 5,7-dichloro-3-cyclopropylpyrazolo[1,5-a]pyrimidine (60 mg, 263 µmol), and (4-(3-trifluoromethoxy)pyridin-2-yl)phenyl)methanamine (71 mg, 263 µmol) in EtOH (3.0 mL). The reaction mixture was heated to 60 °C for 3 h. The reaction mixture was concentrated *in vacuo.* Purification by column chromatography (12 g cartridge, 0-10% MeOH (containing 0.7 M NH₃)/DCM) gave the title compound (92 mg, 0.19 mmol, 73% yield, 96% purity) as a colourless oil.

UPLC/MS (Method 3): m/z 460 (M+H)⁺, R_{T} 1.78 min.

### Step 2:

### tert-butyl (5-chloro-3-cyclopropylpyrazolo[1,5-a]pyrimidin-7-yl)(4-(3-(trifluoromethoxy)pyridin-2-yl)benzyl)carbamate

DMAP (4.9 mg, 40 µmol) was added to a solution of 5-chloro-3-cyclopropyl-N-(4-(3-(trifluoromethoxy)pyridin-2-yl)benzyl)pyrazolo[1,5-a]pyrimidin-7-amine (92 mg, 200 µmol) and BOC-anhydride (52 mg, 240 µmol) in anhydrous THF (3.0 mL). The reaction mixture was heated at RT for 1.5 h then concentrated *in vacuo.* Purification by column chromatography (12 g cartridge, 0-10% MeOH (containing 0.7 M NH₃)/DCM) gave the title compound (110 mg, 0.19 mmol, 95% yield, 97% purity) as a yellow oil.

UPLC/MS (Method 3): m/z 560 (M+H)⁺, R_{T} 2.05 min.

### Step 3:

### tert-butyl (3R,4R)-4-(((7-((tert-butoxycarbonyl)(4-(3-(trifluoromethoxy)pyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(4-(3-(trifluoromethoxy)pyridin-2-yl)benzyl)carbamate (106 mg, 189 µmol), tert-butyl (3R,4R)-4-(aminomethyl)-3-hydroxypiperidine-1-carboxylate (52 mg, 227 µmol) in THF (3.2 mL) was degassed in N₂ for 5 min before adding ^{t}BuBrettPhos Pd G3 (16.2 mg, 18.9 µmol) and LiHMDS (1M in THF) (246 µL, 246 µmol). The reaction was degassed for another 5 min before being heated to 60 °C for 3 h. The reaction mixture concentrated *in vacuo.* Purification by column chromatography (12 g cartridge, 0-10% MeOH/DCM) gave the title compound (135 mg, 0.16 mmol, 85% yield, 90% purity) as a yellow solid.

UPLC/MS (Method 3): m/z 754 (M+H)⁺, R_{T} 2.01 min.

### Step 4:

### (3R,4R)-4-(((3-cyclopropyl-7-((4-(3-(trifluoromethoxy)pyridin-2-yl)benzyl)amino)pyrazolo[1,5-a]pyrimidin-5-yl)amino)methyl)piperidin-3-ol

Hydrogen chloride (4 M in dioxane) (0.84 mL, 3.34 mmol) was added to a solution of tert-butyl (3R,4R)-4-(((7-((tert-butoxycarbonyl)(4-(3-(trifluoromethoxy)pyridin-2-yl)benzyl)amino)-3-cyclopropylpyrazolo[1,5-a]pyrimidin-5-yl)amino)methyl)-3-hydroxypiperidine-1-carboxylate (134 mg, 167 µmol) in dioxane (2.4 mL). The reaction mixture was stirred at 40 °C for 2 h then hydrogen chloride (1.25 M in MeOH) (1.0 mL, 1.3 mmol) was added and the reaction mixture was stirred at 40 °C for 30 min then concentrated *in vacuo.* The solid was triturated in MeCN (2 x 2 mL) then was loaded onto a column of SCX. The column was washed with MeOH (80 mL) and the product was eluted with 0.7 M NH₃ in MeOH (100 mL). The ammoniacal methanol solution was concentrated *in vacuo* and the solid was triturated in Et₂O (2 x 1 mL) to give the title compound (67 mg, 0.11 mmol, 69% yield, 95% purity) as a beige solid.

LCMS (Method 2): m/z 554 (M+H)⁺, RT 0.88 min.

¹H NMR (400 MHz, DMSO-d₆) δ 8.69 (dd, *J* = 4.6, 1.4 Hz, 1H), 7.98 (dt, *J* = 8.4, 1.5 Hz, 1H), 7.90 (t, *J* = 6.5 Hz, 1H), 7.75 (d, *J* = 8.3 Hz, 2H), 7.55 (dd, *J* = 8.4, 4.6 Hz, 1H), 7.53 (s, 1H), 7.48 (d, *J =* 8.2 Hz, 2H), 6.76 - 6.68 (m, 1H), 5.37 - 5.28 (m, 1H), 5.21 (s, 1H), 4.52 (d, *J =* 6.4 Hz, 2H), 3.60 - 3.42 (m, 1H), 3.25 - 3.16 (m, 1H), 3.09 - 2.98 (m, 1H), 2.91 (dd, *J =* 11.5, 4.6 Hz, 1H), 2.83 - 2.75 (m, 1H), 2.33 - 2.25 (m, 1H), 2.17 (t, *J* = 10.8 Hz, 1H), 1.75 - 1.67 (m, 1H), 1.61 - 1.51 (m, 1H), 1.38 - 1.28 (m, 1H), 1.18 - 1.11 (m, 1H), 0.80 - 0.71 (m, 2H), 0.69 - 0.59 (m, 2H). 1H under water.

### Synthesis 040

### (4-(3-(trifluoromethoxy)pyridin-2-yl)phenyl)methanamine

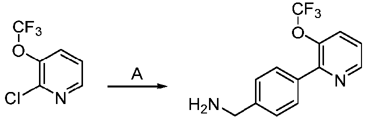

### Step A:

### (4-(3-(trifluoromethoxy)pyridin-2-yl)phenyl)methanamine

A mixture of 2-Chloro-3-(trifluoromethoxy)pyridine (150 mg, 759 µmol), (4-(aminomethyl)phenyl)boronic acid (206 mg, 1.37 mmol) and Pd(dppf)Cl₂.DCM (62 mg, 76 µmol) in dioxane (9.0 mL) was sparged with N₂ for 5 min. A solution of cesium carbonate (990 mg, 3.04 mmol) in water (1.0 mL) was added and the reaction mixture was sparged with N₂ for 5 min. The mixture was heated to 90 °C for 2 h. At RT, water (50 mL) was added, and the mixture was extracted with EtOAc (2 x 25 mL). The combined organic layers were dried over MgSO₄, filtered and concentrated *in vacuo.* Purification by column chromatography (24 g cartridge, 0-10% MeOH/DCM) gave the title compound (160 mg, 0.55 mmol, 72% yield, 92% purity) as an orange oil.

UPLC/MS (Method 1): m/z 269 (M+H)⁺, R_{T} 1.15 min.

### Synthesis 042

### (3R,4R)-4-(((3-cyclopropyl-7-((2-methoxy-4-(pyridin-2-yl)benzyl)amino)pyrazolo[1,5-a]pyrimidin-5-yl)amino)methyl)piperidin-3-ol

### (PPA-030)

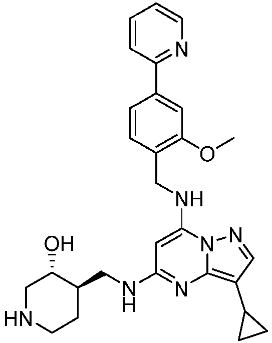

### Step 1:

### 5-chloro-3-cyclopropyl-N-(2-methoxy-4-(pyridin-2-yl)benzyl)pyrazolo[1,5-a]pyrimidin-7-amine

DIPEA (0.41 mL, 2.4 mmol) was added to a solution of 5,7-dichloro-3-cyclopropylpyrazolo[1,5-a]pyrimidine (90 mg, 0.39 mmol), and (2-methoxy-4-(pyridin-2-yl)phenyl)methanamine (110 mg, 0.51 mmol) in EtOH (3.0 mL). The reaction mixture was heated to 60 °C for 3 h. The reaction mixture was concentrated *in vacuo.* Purification by column chromatography (12 g cartridge, 0-10% MeOH (containing 0.7 M NH₃)/DCM) gave the title compound (150 mg, 0.33 mmol, 84% yield, 90% purity) as a white solid.

¹H NMR (400 MHz, DMSO-d₆) δ 8.74 - 8.62 (m, 2H), 7.97 (dt, *J* = 8.1, 1.1 Hz, 1H), 7.94 (s, 1H), 7.87 (td, *J =* 7.7, 1.8 Hz, 1H), 7.75 (d, *J* = 1.6 Hz, 1H), 7.61 (dd, *J* = 7.9, 1.6 Hz, 1H), 7.35 (ddd, *J* = 7.4, 4.8, 1.1 Hz, 1H), 7.28 (d, *J* = 7.9 Hz, 1H), 6.05 (s, 1H), 4.61 (d, *J* = 6.4 Hz, 2H), 3.98 (s, 3H), 1.95 - 1.84 (m, 1H), 0.92 - 0.83 (m, 2H), 0.80 - 0.70 (m, 2H).

### Step 2:

### tert-butyl (3R,4R)-4-(((7-((tert-butoxycarbonyl)(2-methoxy-4-(pyridin-2-yl)benzyl)amino)-3-cyclopropylpyrazolo[1,5-a]pyrimidin-5-yl)amino)methyl)-3-hydroxypiperidine-1-carboxylate

A solution of 5-chloro-3-cyclopropyl-N-(2-methoxy-4-(pyridin-2-yl)benzyl)pyrazolo[1,5-a]pyrimidin-7-amine (130 mg, 320 µmol), tert-butyl (3R,4R)-4-(aminomethyl)-3-hydroxypiperidine-1-carboxylate (81 mg, 352 µmol) in dioxane (2.5 mL) was degassed in N₂ for 5 min before adding ^{t}BuBrettPhos Pd G3 (27 mg, 32 µmol) and LiHMDS (1M in THF) (416 µL, 416 µmol). The reaction was degassed for another 5 min before being heated to 90 °C overnight. At RT, the reaction mixture was concentrated *in vacuo.* Purification by column chromatography (12 g cartridge, 0-10% MeOH (containing 0.7 M NH₃)/DCM) followed by further purification on RP Flash C18 (12 g cartridge, 15-75% MeCN/10 mM ammonium bicarbonate) gave the title compound (94 mg, 150 µmol, 46% yield, 95% purity) as a white solid.

UPLC/MS (Method 3): m/z 600 (M+H)⁺, R_{T} 1.19 min.

### Step 3:

### (3R,4R)-4-(((3-cyclopropyl-7-((2-methoxy-4-(pyridin-2-yl)benzyl)amino)pyrazolo[1,5-a]pyrimidin-5-yl)amino)methyl)piperidin-3-ol

Hydrogen chloride (4 M in dioxane) (0.59 mL, 2.4 mmol) was added to a solution of tert-butyl (3R,4R)-4-(((7-((tert-butoxycarbonyl)(2-methoxy-4-(pyridin-2-yl)benzyl)amino)-3-cyclopropylpyrazolo[1,5-a]pyrimidin-5-yl)amino)methyl)-3-hydroxypiperidine-1-carboxylate (94 mg, 160 µmol) in dioxane (1.0 mL). The reaction mixture was stirred at 35 °C for 2 h then concentrated *in vacuo.* The solid was loaded onto a column of SCX. The column was washed with MeOH (20 mL) and the product was eluted with 0.7 M NH₃ in MeOH (20 mL). The ammoniacal methanol solution was concentrated *in vacuo* to give the title compound (61 mg, 0.11 mmol, 73% yield, 94% purity) as a white solid.

LCMS (Method 2): m/z 500 (M+H)⁺, RT 0.71 min.

¹H NMR (400 MHz, DMSO-d₆) δ 8.66 (ddd, *J* = 4.7, 1.9, 0.9 Hz, 1H), 8.00 - 7.93 (m, 1H), 7.87 (td, *J* = 7.7, 1.8 Hz, 1H), 7.74 (d, *J =* 1.7 Hz, 1H), 7.71 (t, *J* = 6.4 Hz, 1H), 7.61 (dd, *J* = 8.0, 1.6 Hz, 1H), 7.55 (s, 1H), 7.35 (ddd, *J* = 7.4, 4.8, 1.1 Hz, 1H), 7.22 (d, *J* = 7.9 Hz, 1H), 6.82 (t, *J =* 6.1 Hz, 1H), 5.89 - 5.79 (m, 1H), 5.10 (s, 1H), 4.46 (d, *J* = 6.4 Hz, 2H), 3.97 (s, 3H), 3.59 - 3.48 (m, 1H), 3.29 - 3.22 (m, 2H), 3.12 (dd, *J* = 11.8, 4.3 Hz, 1H), 3.08 - 3.00 (m, 1H), 2.66 - 2.58 (m, 1H), 2.49 - 2.44 (m, 1H), 1.77 - 1.67 (m, 2H), 1.56 - 1.45 (m, 1H), 1.39 - 1.28 (m, 1H), 0.82 - 0.72 (m, 2H), 0.72 - 0.61 (m, 2H). 1H under water.

### Synthesis 043

### (2-methoxy-4-(pyridin-2-yl)phenyl)methanamine

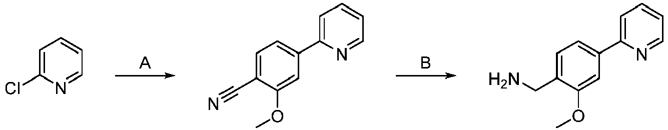

### Step A:

### 2-methoxy-4-(pyridin-2-yl)benzonitrile

2-Chloropyridine (250 mg, 2.20 mmol), (4-cyano-3-methoxyphenyl)boronic acid (423 mg, 1.98 mmol) Pd(dppf)Cl₂ (161 mg, 220 µmol) and potassium phosphate, tribasic (1.87 g, 8.81 mmol) were taken up in dioxane (16 mL) and water (4.0 mL). The reaction mixture was sparged with N₂ for 15 min then heated to 60 °C for 4 h then allowed to cool to RT. The mixture was diluted with EtOAc (30 mL) and water (10 mL). The layers were separated and the aq layer extracted with EtOAc (2 x 15 mL). The combined organic layer was dried over Na₂SO₄, filtered then concentrated *in vacuo.* Purification by column chromatography (24 g cartridge, 0-10% MeOH (containing 0.7 M NH₃)/DCM) gave the title compound (320 mg, 1.3 mmol, 63% yield, 85% purity) as a brown oil.

¹H NMR (400 MHz, DMSO-d₆) δ 8.74 (ddd, *J* = 4.8, 1.8, 0.9 Hz, 1H), 8.20 - 8.10 (m, 1H), 7.96 (td, *J* = 7.8, 1.8 Hz, 1H), 7.90 (d, *J* = 1.4 Hz, 1H), 7.88 - 7.78 (m, 2H), 7.46 (ddd, *J* = 7.5, 4.8, 1.1 Hz, 1H), 4.03 (s, 3H).

### Step B:

### (2-methoxy-4-(pyridin-2-yl)phenyl)methanamine

2-Methoxy-4-(pyridin-2-yl)benzonitrile (200 mg, 951 µmol) was taken up in THF (8.0 mL) and the solution was cooled to 0 °C. LiAlH₄ (4M in Et₂O) (238 µL, 951 µmol) was added and the mixture was stirred for 15 min at 0 °C then at RT for 30 min. The reaction mixture was heated at 50 °C for 1 h before being cooled to 0 °C and quenched with a few drops of aq. sat. Na₂SO₄. The mixture was filtered through a short pad of celite, rinsing with THF (30 mL). The filtrate was concentrated to give the title compound (217 mg, 0.91 mmol, 96% yield, 90% purity) as a brown solid.

¹H NMR (400 MHz, DMSO-d₆) δ 8.65 (ddd, *J* = 4.8, 1.8, 0.9 Hz, 1H), 8.03 - 7.93 (m, 1H), 7.86 (td, *J* = 7.7, 1.8 Hz, 1H), 7.67 (d, *J* = 1.6 Hz, 1H), 7.62 (dd, *J* = 7.8, 1.6 Hz, 1H), 7.43 (d, *J = 7.8* Hz, 1H), 7.33 (ddd, *J* = 7.4, 4.8, 1.1 Hz, 1H), 3.89 (s, 3H), 3.71 (s, 2H), 1.72 (s (br), 2H).

### Synthesis 045

### (R)-3-cyclopropyl-N⁵-(morpholin-2-ylmethyl)-N⁷-(4-(pyridin-2-yl)benzyl)pyrazolo[1,5-a]pyrimidine-5,7-diamine

### (PPA-032)

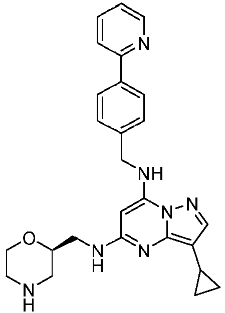

### Step 3:

### tert-butyl (S)-2-(((7-((tert-butoxycarbonyl)(4-(pyridin-2-yl)benzyl)amino)-3-cyclopropylpyrazolo[1,5-a]pyrimidin-5-yl)amino)methyl)morpholine-4-carboxylate

A suspension of tert-butyl (5-chloro-3-cyclopropylpyrazolo[1,5-a]pyrimidin-7-yl)(4-(pyridin-2-yl)benzyl)carbamate (90 mg, 189 µmol) (prepared as described herein), tert-butyl (S)-2-(aminomethyl)morpholine-4-carboxylate (61 mg, 284 µmol) and cesium carbonate (185 mg, 567 µmol) in dioxane (4.0 mL) was degassed with N₂ for 5 min. Pd-171 (3.8 mg, 5.7 µmol) and Ruphos (3.1 mg, 6.62 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (66 mg, 96 µmol, 51% yield, 95% purity) as a yellow glass solid.

UPLC/MS (Method 3): m/z 656 (M+H)⁺, RT 2.17 min.

### Step 4:

### (R)-3-cyclopropyl-N⁵-(morpholin-2-ylmethyl)-N⁷-(4-(pyridin-2-yl)benzyl)pyrazolo[1,5-a]pyrimidine-5,7-diamine

Hydrogen chloride (4 M in dioxane) (252 µL, 1.01 mmol) was added to a solution of tert-butyl (S)-2-(((7-((tert-butoxycarbonyl)(4-(pyridin-2-yl)benzyl)amino)-3-cyclopropylpyrazolo[1,5-a]pyrimidin-5-yl)amino)methyl)morpholine-4-carboxylate (66 mg, 101 µmol) in dioxane (2.0 mL). The reaction mixture was stirred at 35 °C for 4 h then concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (22 mg, 47 µmol, 47% yield, 98% purity) as a white solid.

LCMS (Method 2): m/z 456 (M+H)⁺, RT 0.89 min.

¹H NMR (400 MHz, DMSO-d₆) δ 8.68 - 8.61 (m, 1H), 8.05 (d, *J* = 8.3 Hz, 2H), 7.96 - 7.90 (m, 1H), 7.90 - 7.81 (m, 2H), 7.55 (s, 1H), 7.45 (d, *J* = 8.3 Hz, 2H), 7.33 (ddd, *J* = 7.3, 4.8, 1.2 Hz, 1H), 6.65 (t, *J* = 5.7 Hz, 1H), 5.19 (s, 1H), 4.49 (d, *J* = 6.3 Hz, 2H), 3.76 - 3.69 (m, 1H), 3.54 - 3.46 (m, 1H), 3.46 - 3.36 (m, 1H), 3.29 - 3.15 (m, 1H), 2.87 - 2.79 (m, 1H), 2.73 - 2.59 (m, 2H), 2.45 - 2.35 (m, 1H), 1.82 - 1.71 (m, 1H), 0.80 - 0.73 (m, 4H). 2H under water.

### Synthesis 046

### 3-cyclopropyl-5-morpholino-N-(4-(pyridin-2-yl)benzyl)pyrazolo[1,5-a]pyrimidin-7-amine

### (PPA-033)

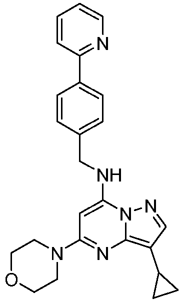

### Step 3:

### tert-butyl (3-cyclopropyl-5-morpholinopyrazolo[1,5-a]pyrimidin-7-yl)(4-(pyridin-2-yl)benzyl)carbamate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), morpholine (92 mg, 1.05 mmol) and cesium carbonate (205 mg, 630 µmol) in dioxane (4.0 mL) was degassed with N₂ for 5 min. Pd-171 (4.2 mg, 6.30 µmol) and Ruphos (3.4 mg, 7.35 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (105 mg, 0.16 mmol, 76% yield, 80% purity) as a yellow glass solid.

UPLC/MS (Method 2): m/z 527 (M+H)⁺, RT 2.00 min.

### Step 4:

### 3-cyclopropyl-5-morpholino-N-(4-(pyridin-2-yl)benzyl)pyrazolo[1,5-a]pyrimidin-7-amine

Hydrogen chloride (4 M in dioxane) (404 µL, 1.61 mmol) was added to a solution of tert-butyl (3-cyclopropyl-5-morpholinopyrazolo[1,5-a]pyrimidin-7-yl)(4-(pyridin-2-yl)benzyl)carbamate (105 mg, 161 µmol) in dioxane (2.0 mL). The reaction mixture was stirred at 35 °C for 4 h then concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (24 mg, 53 µmol, 33% yield, 95% purity) as a yellow solid.

UPLC/MS (Method 2): m/z 427 (M+H)⁺, RT 1.41 min.

¹H NMR (400 MHz, DMSO-d₆) δ 8.67 - 8.61 (m, 1H), 8.07 - 7.98 (m, 3H), 7.96 - 7.89 (m, 1H), 7.86 (td, *J =* 7.7, 1.8 Hz, 1H), 7.63 (s, 1H), 7.50 (d, *J =* 8.2 Hz, 2H), 7.33 (ddd, *J =* 7.4, 4.8, 1.2 Hz, 1H), 5.56 (s, 1H), 4.62 (d, *J =* 6.6 Hz, 2H), 3.64 (t, *J =* 4.8 Hz, 4H), 3.45 (t, *J =* 4.8 Hz, 4H), 1.84 - 1.73 (m, 1H), 0.82 - 0.70 (m, 4H).

### Synthesis 050

### (3R,4R)-4-(((3-cyclopropyl-7-((4-(3-(trifluoromethyl)pyridin-2-yl)benzyl)amino)pyrazolo[1,5-a]pyrimidin-5-yl)amino)methyl)piperidin-3-ol

### (PPA-036)

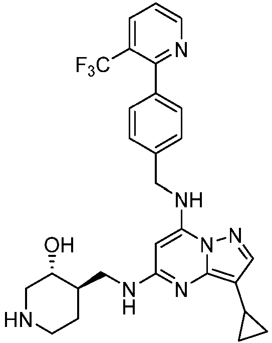

### Step 1:

### 5-chloro-3-cyclopropyl-N-(4-(3-(trifluoromethyl)pyridin-2-yl)benzyl)pyrazolo[1,5-a]pyrimidin-7-amine

DIPEA (1.04 mL, 5.99 mmol) was added to a solution of 5,7-dichloro-3-cyclopropylpyrazolo[1,5-a]pyrimidine (195 mg, 0.86 mmol), and (4-(3-(trifluoromethyl)pyridin-2-yl)phenyl)methanamine (240 mg, 0.86 mmol) in EtOH (5.0 mL). The reaction mixture was heated to 60 °C for 3 h then was concentrated *in vacuo.* Purification by column chromatography (24 g cartridge, 0-100% EtOAc/isohexane) gave the title compound (270 mg, 0.58 mmol, 68% yield, 96% purity) as a white solid.

UPLC/MS (Method 2): m/z 444 (M+H)⁺, R_{T} 2.12 min.

### Step 2:

### tert-butyl (5-chloro-3-cyclopropylpyrazolo[1,5-a]pyrimidin-7-yl)(4-(3-(trifluoromethyl)pyridin-2-yl)benzyl)carbamate

DMAP (5.5 mg, 45 µmol) was added to a solution of 5-chloro-3-cyclopropyl-N-(4-(3-(trifluoromethyl)pyridin-2-yl)benzyl)pyrazolo[1,5-a]pyrimidin-7-amine (100 mg, 225 µmol) and BOC-anhydride (59 mg, 270 µmol) in anhydrous THF (3.0 mL). The reaction mixture was heated at RT for 4 h then concentrated *in vacuo.* Purification by column chromatography (12 g cartridge, 0-10% MeOH/DCM) gave the title compound (128 mg, 0.21 mmol, 93% yield, 89% purity) as a green oil.

UPLC/MS (Method 3): m/z 444 (M-Boc+H)⁺, R_{T} 1.97 min.

### Step 3:

tert-butyl (3R,4R)-4-(((7-((tert-butoxycarbonyl)(4-(3-(trifluoromethyl)pyridin-2-yl)benzyl)amino)-3-cyclopropylpyrazolo[1,5-a]pyrimidin-5-yl)amino)methyl)-3-hydroxypiperidine-1-carboxylate

A solution of tert-butyl (5-chloro-3-cyclopropylpyrazolo[1,5-a]pyrimidin-7-yl)(4-(3-(trifluoromethyl)pyridin-2-yl)benzyl)carbamate (128 mg, 209 µmol), tert-butyl (3R,4R)-4-(aminomethyl)-3-hydroxypiperidine-1-carboxylate (58 mg, 251 µmol) in THF (2.0 mL) was degassed in N₂ for 5 min before adding ^{t}BuBrettPhos Pd G3 (17.9 mg, 20.9 µmol) and LiHMDS (1M in THF) (272 µL, 272 µmol). The reaction was degassed for another 5 min before being heated to 60 °C for 4 h. The reaction mixture was concentrated *in vacuo.* Purification by column chromatography (12 g cartridge, 0-10% MeOH/DCM) gave the title compound (180 mg, 0.21 mmol, 99% yield, 85% purity) as a brown oil.

UPLC/MS (Method 3): m/z 738 (M+H)⁺, R_{T} 1.88 min.

### Step 4:

### (3R,4R)-4-(((3-cyclopropyl-7-((4-(3-(trifluoromethyl)pyridin-2-yl)benzyl)amino)pyrazolo[1,5-a]pyrimidin-5-yl)amino)methyl)piperidin-3-ol

TFA (0.5 mL) was added to a solution of tert-butyl (3R,4R)-4-(((7-((tert-butoxycarbonyl)(4-(3-(trifluoromethyl)pyridin-2-yl)benzyl)amino)-3-cyclopropylpyrazolo[1,5-a]pyrimidin-5-yl)amino)methyl)-3-hydroxypiperidine-1-carboxylate (180 mg, 207 µmol) in DCM (1.5 mL). The reaction mixture was stirred at RT for 16 h and concentrated *in vacuo.* The solid was loaded onto a column of SCX. The column was washed with MeOH (30 mL) and the product was eluted with 0.7 M NH₃ in MeOH (30 mL). The ammoniacal methanol solution was concentrated *in vacuo* and the solid was triturated in Et₂O (2 x 1 mL) to give the title compound (96 mg, 0.17 mmol, 83% yield, 96% purity) as a tan solid.

UPLC/MS (Method 3): m/z 538 (M+H)⁺, RT 1.38 min.

¹H NMR (400 MHz, DMSO-d₆) δ 8.91 - 8.87 (m, 1H), 8.29 (dd, *J* = 8.1, 1.6 Hz, 1H), 7.90 (t, *J* = 6.5 Hz, 1H), 7.68 - 7.61 (m, 1H), 7.53 (s, 1H), 7.47 - 7.40 (m, 4H), 6.78 - 6.68 (m, 1H), 5.38 - 5.26 (m, 1H), 5.22 (s, 1H), 4.53 (d, *J* = 6.4 Hz, 2H), 3.59 - 3.43 (m, 1H), 3.26 - 3.17 (m, 1H), 3.09 - 2.99 (m, 1H), 2.95 - 2.87 (m, 1H), 2.85 - 2.74 (m, 1H), 2.36 - 2.26 (m, 1H), 2.21 - 2.12 (m, 1H), 1.78 - 1.67 (m, 1H), 1.61 - 1.52 (m, 1H), 1.40 - 1.28 (m, 1H), 1.22 - 1.11 (m, 1H), 0.80 - 0.71 (m, 2H), 0.71 - 0.60 (m, 2H). 1H under water.

### Synthesis 051

### (4-(3-(trifluoromethyl)pyridin-2-yl)phenyl)methanamine

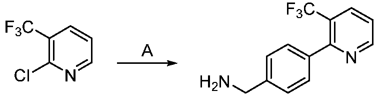

### Step A:

### (4-(3-(trifluoromethyl)pyridin-2-yl)phenyl)methanamine

A mixture of 2-chloro-3-(trifluoromethyl)pyridine (200 mg, 1.10 mmol), (4-(aminomethyl)phenyl)boronic acid, HCl (227 mg, 1.21 mmol) and Pd(dppf)Cl₂ (81 mg, 110 µmol) in dioxane (9.0 mL) was sparged with N₂ for 5 min. A solution of potassium phosphate, tribasic (935 mg, 4.41 mmol) in water (1.0 mL) was added and the reaction mixture was sparged with N₂ for 5 min. The mixture was heated to 70 °C for 2 h. At RT, water (100 mL) was added and the mixture was extracted with DCM (3 x 25 mL). The combined organic layers were dried over MgSO₄, filtered and concentrated *in vacuo.* The oil was loaded onto a column of SCX. The column was washed with MeOH (30 mL) and the product was eluted with 0.7 M NH₃ in MeOH (30 mL) to give the title compound (244 mg, 0.87 mmol, 79% yield, 90% purity) as a red solid.

UPLC/MS (Method 3): m/z 253 (M+H)⁺, R_{T} 1.06 min.

### Synthesis 052

### (S)-3-cyclopropyl-N⁷-(4-(pyridin-2-yl)benzyl)-N⁵-(pyrrolidin-3-yl)pyrazolo[1,5-a]pyrimidine-5,7-diamine

### (PPA-037)

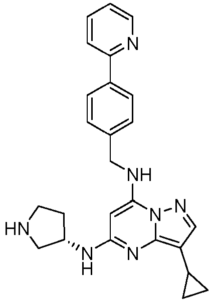

### Step 3:

### tert-butyl (S)-3-((7-((tert-butoxycarbonyl)(4-(pyridin-2-yl)benzyl)amino)-3-cyclopropylpyrazolo[1,5-a]pyrimidin-5-yl)amino)pyrrolidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl (S)-3-aminopyrrolidine-1-carboxylate (117 mg, 630 µmol) and cesium carbonate (205 mg, 630 µmol) in dioxane (4.0 mL) was degassed with N₂ for 5 min. Pd-171 (4.2 mg, 6.30 µmol) and Ruphos (3.4 mg, 7.35 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (90 mg, 0.13 mmol, 62% yield, 90% purity) as a yellow glass solid.

UPLC/MS (Method 3): m/z 626 (M+H)⁺, RT 1.94 min.

### Step 4:

### (S)-3-cyclopropyl-N⁷-(4-(pyridin-2-yl)benzyl)-N⁵-(pyrrolidin-3-yl)pyrazolo[1,5-a]pyrimidine-5,7-diamine

Hydrogen chloride (4 M in dioxane) (0.36 mL, 1.4 mmol) was added to a solution of tert-butyl (S)-3-((7-((tert-butoxycarbonyl)(4-(pyridin-2-yl)benzyl)amino)-3-cyclopropylpyrazolo[1,5-a]pyrimidin-5-yl)amino)pyrrolidine-1-carboxylate (90 mg, 0.14 mmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (10 mg, 23 µmol, 16% yield, 96% purity) as a white solid.

UPLC/MS (Method 2): m/z 426 (M+H)⁺, RT 1.68 min.

¹H NMR (400 MHz, DMSO-d₆) δ 8.68 - 8.61 (m, 1H), 8.08 - 8.01 (m, 2H), 7.92 (d, J = 8.0 Hz, 1H), 7.91 - 7.80 (m, 2H), 7.55 (s, 1H), 7.44 (d, J = 8.2 Hz, 2H), 7.33 (ddd, J = 7.3, 4.7, 1.2 Hz, 1H), 6.64 (d, J = 6.5 Hz, 1H), 5.08 (s, 1H), 4.50 (d, J = 6.3 Hz, 2H), 4.18 - 4.14 (m, 1H), 2.98 (dd, J = 11.1, 6.5 Hz, 1H), 2.87 - 2.77 (m, 1H), 2.75 - 2.69 (m, 1H), 2.00 - 1.83 (m, 1H), 1.81 - 1.70 (m, 1H), 1.55 - 1.44 (m, 1H), 0.81 - 0.71 (m, 4H). 2H under water.

### Synthesis 053

### 3-cyclopropyl-5-(piperazin-1-yl)-N-(4-(pyridin-2-yl)benzyl)pyrazolo[1,5-a]pyrimidin-7-amine

### (PPA-038)

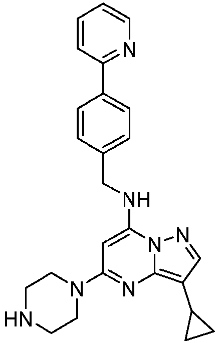

### Step 3:

### tert-butyl 4-(7-((tert-butoxycarbonyl)(4-(pyridin-2-yl)benzyl)amino)-3-cyclopropylpyrazolo[1,5-a]pyrimidin-5-yl)piperazine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl piperazine-1-carboxylate (43 mg, 231 µmol) and cesium carbonate (205 mg, 630 µmol) in dioxane (2.0 mL) was degassed with N₂ for 5 min. Pd-171 (7.0 mg, 10.5 µmol) and Ruphos (5.4 mg, 11.6 µmol) were added and N₂ was bubbled through the reaction mixture for another 5 min. The reaction mixture was stirred at 90 °C overnight. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH3)/DCM) followed by further purification on RP Flash C18 (12 g cartridge, 15-75% MeCN/10 mM ammonium bicarbonate) gave the title compound (59 mg, 91 µmol, 44% yield, 97% purity) as an off-white solid.

UPLC/MS (Method 3): m/z 626 (M+H)⁺, RT 2.02 min.

### Step 4:

### 3-cyclopropyl-5-(piperazin-1-yl)-N-(4-(pyridin-2-yl)benzyl)pyrazolo[1,5-a]pyrimidin-7-amine

Hydrogen chloride (4 M in dioxane) (0.34 mL, 1.3 mmol) was added to a solution of tert-butyl 4-(7-((tert-butoxycarbonyl)(4-(pyridin-2-yl)benzyl)amino)-3-cyclopropylpyrazolo[1,5-a]pyrimidin-5-yl)piperazine-1-carboxylate (56 mg, 89 µmol) in dioxane (0.5 mL). The reaction mixture was stirred at 35 °C for 2 h and concentrated *in vacuo.* The solid was loaded onto a column of SCX. The column was washed with MeOH (20 mL) and the product was eluted with 0.7 M NH₃ in MeOH (20 mL). The ammoniacal methanol solution was concentrated *in vacuo.* Purification on RP Flash C18 (12 g cartridge, 15-75% MeCN/10 mM ammonium bicarbonate) gave the title compound (15 mg, 33 µmol, 37 yield, 95% purity) as a white solid.

LCMS (Method 2): m/z 426 (M+H)⁺, RT 0.79 min.

¹H NMR (400 MHz, DMSO-d₆) δ 8.66 - 8.62 (m, 1H), 8.04 (d, *J* = 8.3 Hz, 2H), 7.98 - 7.90 (m, 2H), 7.86 (td, *J* = 7.7, 1.9 Hz, 1H), 7.60 (s, 1H), 7.50 (d, *J* = 8.3 Hz, 2H), 7.33 (ddd, *J =* 7.4, 4.8, 1.2 Hz, 1H), 5.50 (s, 1H), 4.61 (d, *J* = 6.5 Hz, 2H), 3.40 (t, *J* = 5.1 Hz, 4H), 2.75 - 2.67 (m, 4H), 1.83 - 1.72 (m, 1H), 0.80 - 0.71 (m, 4H). 1H under water.

### Synthesis 054

### 3-cyclopropyl-N⁵-methyl-N⁵-(piperidin-4-yl)-N⁷-(4-(pyridin-2-yl)benzyl)pyrazolo[1,5-a]pyrimidine-5,7-diamine

### (PPA-039)

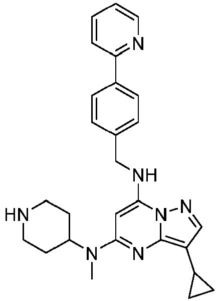

### Step 3:

### tert-butyl 4-((7-((tert-butoxycarbonyl)(4-(pyridin-2-yl)benzyl)amino)-3-cyclopropylpyrazolo[1,5-a]pyrimidin-5-yl)(methyl)amino)piperidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 4-(methylamino)piperidine-1-carboxylate (180 mg, 840 µmol) and cesium carbonate (205 mg, 630 µmol) in dioxane (4.0 mL) was degassed with N₂ for 5 min. Pd-171 (4.2 mg, 6.3 µmol) and Ruphos (3.4 mg, 7.4 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (100 mg, 0.13 mmol, 64% yield, 88% purity) as a yellow glass solid.

UPLC/MS (Method 2): m/z 654 (M+H)⁺, RT 2.09 min.

### Step 4:

### 3-cyclopropyl-N⁵-methyl-N⁵-(piperidin-4-yl)-N⁷-(4-(pyridin-2-yl)benzyl)pyrazolo[1,5-a]pyrimidine-5,7-diamine

Hydrogen chloride (4 M in dioxane) (0.38 mL, 1.53 mmol) was added to a solution of tert-butyl 4-((7-((tert-butoxycarbonyl)(4-(pyridin-2-yl)benzyl)amino)-3-cyclopropylpyrazolo[1,5-a]pyrimidin-5-yl)(methyl)amino)piperidine-1-carboxylate (100 mg, 153 µmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo.* Purification by RP preparative HPLC (35-100% MeCN/0.3% NH₃ in water) gave the title compound (11 mg, 24 µmol, 15% yield, 97% purity) as a white solid.

UPLC/MS (Method 4): m/z 454 (M+H)⁺, RT 1.70 min.

¹H NMR (400 MHz, DMSO-d₆) δ 8.67 - 8.61 (m, 1H), 8.04 (d, *J* = 8.3 Hz, 2H), 7.95 (t, *J* = 6.4 Hz, 1H), 7.93 - 7.90 (m, 1H), 7.85 (td, *J =* 7.7, 1.9 Hz, 1H), 7.59 (s, 1H), 7.51 (d, *J =* 8.3 Hz, 2H), 7.33 (ddd, *J* = 7.4, 4.8, 1.2 Hz, 1H), 5.25 (s, 1H), 4.60 (d, *J* = 6.2 Hz, 2H), 4.25 - 4.12 (m, 1H), 2.98 - 2.90 (m, 2H), 2.79 (s, 3H), 2.56 - 2.52 (m, 1H), 2.48 - 2.44 (m, 1H), 1.82 - 1.70 (m, 1H), 1.55 - 1.35 (m, 4H), 0.83 - 0.70 (m, 4H). 1H under water.

### Synthesis 055

### 3-cyclopropyl-N⁵-(piperidin-4-ylmethyl)-N⁷-(4-(pyridin-2-yl)benzyl)pyrazolo[1,5-a]pyrimidine-5,7-diamine

### (PPA-040)

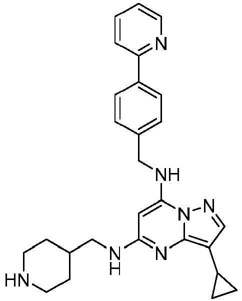

### Step 3:

### tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)piperidine-1-carboxylate

A solution tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 4-(aminomethyl)piperidine-1-carboxylate (180 mg, 840 µmol) in THF (2.0 mL) was degassed in N₂ for 5 min before adding tBuBrettPhos Pd G3 (18.0 mg, 21.0 µmol) and LiHMDS (1M in THF) (273 µL, 273 µmol). The reaction was degassed for another 5 min before being heated to 60 °C for 3 h then concentrated *in vacuo.* Purification by column chromatography (4 g cartridge, 0-10% MeOH/DCM) gave the title compound (64 mg, 94 µmol, 45% yield, 96% purity) as a yellow solid.

UPLC/MS (Method 2): m/z 654 (M+H)⁺, R_{T} 1.99 min.

### Step 4:

### 3-cyclopropyl-N⁵-(piperidin-4-ylmethyl)-N⁷-(4-(pyridin-2-yl)benzyl)pyrazolo[1,5-a]pyrimidine-5,7-diamine

Hydrogen chloride (4 M in dioxane) (0.23 mL, 0.92 mmol) was added to a solution of tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)piperidine-1-carboxylate (60 mg, 92 µmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (16 mg, 35 µmol, 38% yield, 99% purity) as a white solid.

UPLC/MS (Method 4): m/z 454 (M+H)⁺, RT 0.91 min.

¹H NMR (400 MHz, DMSO-d₆) δ 8.68 - 8.61 (m, 1H), 8.05 (d, *J* = 8.3 Hz, 2H), 7.96 - 7.90 (m, 1H), 7.86 (td, *J* = 7.7, 1.9 Hz, 1H), 7.84 - 7.76 (m, 1H), 7.54 (s, 1H), 7.45 (d, *J* = 8.2 Hz, 2H), 7.33 (ddd, *J =* 7.3, 4.8, 1.2 Hz, 1H), 6.61 (t, *J* = 5.6 Hz, 1H), 5.13 (s, 1H), 4.50 (d, *J =* 6.3 Hz, 2H), 3.07 (t, *J* = 5.5 Hz, 2H), 2.93 - 2.86 (m, 2H), 2.43 - 2.32 (m, 2H), 1.82 - 1.69 (m, 1H), 1.58 (d, *J =* 12.8 Hz, 3H), 1.06 - 0.94 (m, 2H), 0.75 (d, *J =* 8.1 Hz, 4H). 1H under water.

### Synthesis 056

### 3-cyclopropyl-N⁵-((3,3-difluoropiperidin-4-yl)methyl)-N⁷-(4-(pyridin-2-yl)benzyl)pyrazolo[1,5-a]pyrimidine-5,7-diamine

### (PPA-041)

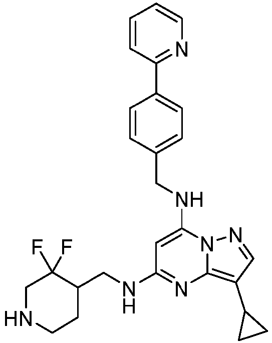

### Step 3:

### tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-3,3-difluoropiperidine-1-carboxylate

A solution tert-butyl (5-chloro-3-cyclopropylpyrazolo[1,5-a]pyrimidin-7-yl)(4-(pyridin-2-yl)benzyl)carbamate (175 mg, 368 µmol) (prepared as described herein), tert-butyl 4-(aminomethyl)-3,3-difluoropiperidine-1-carboxylate (147 mg, 588 µmol) in THF (2.0 mL) was degassed in N₂ for 5 min before adding tBuBrettPhos Pd G3 (47.1 mg, 55.1 µmol) and LiHMDS (1M in THF) (386 µL, 386 µmol). The reaction was degassed for another 5 min before being heated to 60 °C for 3 h then concentrated *in vacuo.* Purification by column chromatography (4 g cartridge, 0-10% MeOH (containing 0.7 M NH₃)/DCM) followed by further purification on RP Flash C18 (12 g cartridge, 15-75% MeCN/10 mM ammonium bicarbonate) gave the title compound (135 mg, 0.18 mmol, 48% yield, 90% purity) as a colourless oil.

UPLC/MS (Method 3): m/z 690 (M+H)⁺, R_{T} 1.99 min.

### Step 4:

### 3-cyclopropyl-N⁵-((3,3-difluoropiperidin-4-yl)methyl)-N⁷-(4-(pyridin-2-yl)benzyl)pyrazolo[1,5-a]pyrimidine-5,7-diamine

Hydrogen chloride (4 M in dioxane) (0.62 mL, 2.47 mmol) was added to a solution of tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-3,3-difluoropiperidine-1-carboxylate (126 mg, 164 µmol) in dioxane (3.0 mL). The reaction mixture was stirred at 35 °C for 2 h and concentrated *in vacuo.* Purification on RP Flash C18 (12 g cartridge, 20-80% MeCN/10 mM ammonium bicarbonate) gave the title compound (20 mg, 39 µmol, 24% yield, 95% purity) as an off-white solid.

LCMS (Method 3): m/z 490 (M+H)⁺, RT 1.49 min.

¹H NMR (400 MHz, DMSO-d₆) δ 8.67 - 8.62 (m, 1H), 8.05 (d, *J =* 8.4 Hz, 2H), 7.93 (dt, *J* = 8.0, 1.2 Hz, 1H), 7.89 - 7.83 (m, 2H), 7.57 (s, 1H), 7.44 (d, *J =* 8.2 Hz, 2H), 7.33 (ddd, J *=* 7.4, 4.8, 1.2 Hz, 1H), 6.68 (t, *J* = 5.8 Hz, 1H), 5.11 (s, 1H), 4.50 (d, *J* = 6.4 Hz, 2H), 3.68 - 3.58 (m, 1H), 3.18 - 3.08 (m, 1H), 3.05 - 2.95 (m, 1H), 2.89 - 2.79 (m, 1H), 2.73 - 2.61 (m, 1H), 2.43 - 2.33 (m, 1H), 2.25 - 2.08 (m, 1H), 1.82 - 1.70 (m, 2H), 1.33 - 1.20 (m, 1H), 0.84 - 0.70 (m, 4H). 1H under water.

### Synthesis 057

### 3-cyclopropyl-N-(4-(pyridin-2-yl)benzyl)-5-(2,7-diazaspiro[3.5]nonan-2-yl)pyrazolo[1,5-a]pyrimidin-7-amine

### (PPA-042)

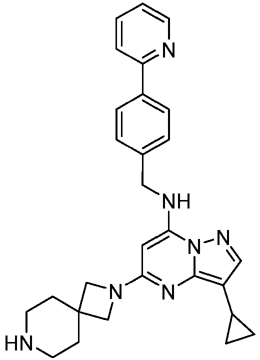

### Step 3:

### tert-butyl 2-(7-((tert-butoxycarbonyl)(4-(pyridin-2-yl)benzyl)amino)-3-cyclopropylpyrazolo[1,5-a]pyrimidin-5-yl)-2,7-diazaspiro[3.5]nonane-7-carboxylate

A solution tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 2,7-diazaspiro[3.5]nonane-7-carboxylate (71 mg, 315 µmol) in dioxane (2.0 mL) was degassed in N₂ for 5 min before adding tBuBrettPhos Pd G3 (18.0 mg, 21.0 µmol) and LiHMDS (1M in THF) (221 µL, 221 µmol). The reaction was degassed for another 5 min before being heated to 90 °C for 3 h then concentrated *in vacuo.* Purification by column chromatography (12 g cartridge, 0-10% MeOH (containing 0.7 M NH₃)/DCM) gave the title compound (68 mg, 98 µmol, 47% yield, 98% purity) as a yellow oil.

UPLC/MS (Method 2): m/z 666 (M+H)⁺, R_{T} 2.51 min.

### Step 4:

### 3-cyclopropyl-N-(4-(pyridin-2-yl)benzyl)-5-(2,7-diazaspiro[3.5]nonan-2-yl)pyrazolo[1,5-a]pyrimidin-7-amine

Hydrogen chloride (4 M in dioxane) (0.38 mL, 1.5 mmol) was added to a solution of tert-butyl 2-(7-((tert-butoxycarbonyl)(4-(pyridin-2-yl)benzyl)amino)-3-cyclopropylpyrazolo[1,5-a]pyrimidin-5-yl)-2,7-diazaspiro[3.5]nonane-7-carboxylate (68 mg, 0.10 mmol) in dioxane (3.0 mL). The reaction mixture was stirred at 35 °C for 2 h and concentrated *in vacuo.* The solid was loaded onto a column of SCX. The column was washed with MeOH (20 mL) and the product was eluted with 0.7 M NH₃ in MeOH (20 mL). The ammoniacal methanol solution was concentrated *in vacuo* to give the title compound (44 mg, 90 µmol, 88% yield, 95% purity) as an off-white solid.

LCMS (Method 5): m/z 466 (M+H)⁺, RT 1.54 min.

¹H NMR (400 MHz, DMSO-d₆) δ 8.66 - 8.62 (m, 1H), 8.05 (d, *J* = 8.3 Hz, 2H), 8.00 (t, *J* = 6.5 Hz, 1H), 7.95 - 7.91 (m, 1H), 7.86 (td, *J =* 7.7, 1.9 Hz, 1H), 7.57 (s, 1H), 7.48 (d, *J =* 8.3 Hz, 2H), 7.33 (ddd, *J* = 7.4, 4.8, 1.2 Hz, 1H), 5.05 (s, 1H), 4.57 (d, *J* = 6.5 Hz, 2H), 3.59 (s, 4H), 2.65 - 2.57 (m, 4H), 1.81 - 1.71 (m, 1H), 1.62 - 1.54 (m, 4H), 0.81 - 0.72 (m, 2H), 0.71 - 0.66 (m, 2H). 1H under water.

### Synthesis 058

### (R)-3-cyclopropyl-N⁷-(4-(pyridin-2-yl)benzyl)-N⁵-(pyrrolidin-3-yl)pyrazolo[1,5-a]pyrimidine-5,7-diamine

### (PPA-043)

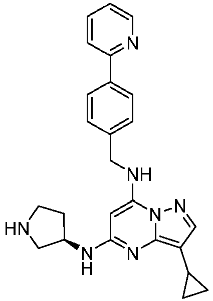

### Step 3:

### tert-butyl (R)-3-((7-((tert-butoxycarbonyl)(4-(pyridin-2-yl)benzyl)amino)-3-cyclopropylpyrazolo[1,5-a]pyrimidin-5-yl)amino)pyrrolidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl (R)-3-aminopyrrolidine-1-carboxylate (117 mg, 630 µmol) and cesium carbonate (205 mg, 630 µmol) in dioxane (4.0 mL) was degassed with N₂ for 5 min. Pd-171 (4.2 mg, 6.30 µmol) and Ruphos (3.4 mg, 7.35 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (91 mg, 0.11 mmol, 53% yield, 78% purity) as a yellow glass solid.

UPLC/MS (Method 2): m/z 627 (M+H)⁺, RT 2.39 min.

### Step 4:

### (R)-3-cyclopropyl-N⁷-(4-(pyridin-2-yl)benzyl)-N⁵-(pyrrolidin-3-yl)pyrazolo[1,5-a]pyrimidine-5,7-diamine

Hydrogen chloride (4 M in dioxane) (0.32 mL, 1.3 mmol) was added to a solution of tert-butyl (R)-3-((7-((tert-butoxycarbonyl)(4-(pyridin-2-yl)benzyl)amino)-3-cyclopropylpyrazolo[1,5-a]pyrimidin-5-yl)amino)pyrrolidine-1-carboxylate (80 mg, 128 mmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (11 mg, 26 µmol, 20% yield, 99% purity) as a white solid.

LCMS (Method 1): m/z 426 (M+H)⁺, RT 0.80 min.

¹H NMR (400 MHz, DMSO-d₆) δ 8.68 - 8.61 (m, 1H), 8.08 - 8.01 (m, 2H), 7.92 (d, *J* = 8.0 Hz, 1H), 7.90 - 7.80 (m, 2H), 7.55 (s, 1H), 7.44 (d, *J* = 8.2 Hz, 2H), 7.37 - 7.29 (m, 1H), 6.63 (d, *J* = 6.5 Hz, 1H), 5.09 (s, 1H), 4.50 (d, *J* = 6.3 Hz, 2H), 4.20 - 4.11 (m, 1H), 2.97 (dd, *J =* 11.0, 6.4 Hz, 1H), 2.86 - 2.75 (m, 1H), 2.75 - 2.67 (m, 1H), 2.00 - 1.86 (m, 1H), 1.81 - 1.70 (m, 1H), 1.54 - 1.43 (m, 1H), 0.80 - 0.72 (m, 4H). 2H under water.

### Synthesis 059

### (S)-3-cyclopropyl-N⁵-(piperidin-3-yl)-N⁷-(4-(pyridin-2-yl)benzyl)pyrazolo[1,5-a]pyrimidine-5,7-diamine

### (PPA-044)

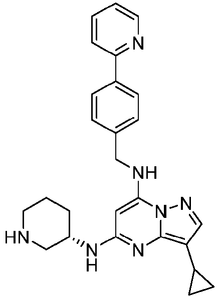

### Step 3:

### tert-butyl (S)-3-((7-((tert-butoxycarbonyl)(4-(pyridin-2-yl)benzyl)amino)-3-cyclopropylpyrazolo[1,5-a]pyrimidin-5-yl)amino)piperidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl (S)-3-aminopiperidine-1-carboxylate (126 mg, 630 µmol) and cesium carbonate (205 mg, 630 µmol) in dioxane (4.0 mL) was degassed with N₂ for 5 min. Pd-171 (4.2 mg, 6.30 µmol) and Ruphos (3.4 mg, 7.35 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated in vacuo. Purification by column chromatography (12 g cartridge, 0-10% MeOH (containing 0.7 M NH₃)/DCM) gave the title compound (90 mg, 0.13 mmol, 60% yield, 90% purity) as a yellow glass solid.

UPLC/MS (Method 2): m/z 641 (M+H)⁺, RT 2.44 min.

### Step 4:

### (S)-3-cyclopropyl-N⁵-(piperidin-3-yl)-N⁷-(4-(pyridin-2-yl)benzyl)pyrazolo[1,5-a]pyrimidine-5,7-diamine

Hydrogen chloride (4 M in dioxane) (0.35 mL, 1.4 mmol) was added to a solution of tert-butyl (S)-3-((7-((tert-butoxycarbonyl)(4-(pyridin-2-yl)benzyl)amino)-3-cyclopropylpyrazolo[1,5-a]pyrimidin-5-yl)amino)piperidine-1-carboxylate (90 mg, 0.14 mmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (28 mg, 61 µmol, 43% yield, 95% purity) as a white solid.

LCMS (Method 2): m/z 426 (M+H)⁺, RT 0.68 min.

¹H NMR (400 MHz, DMSO-d₆) δ 8.67 - 8.61 (m, 1H), 8.05 (d, *J* = 8.3 Hz, 2H), 7.93 (d, *J* = 8.0 Hz, 1H), 7.90 - 7.77 (m, 2H), 7.53 (s, 1H), 7.45 (d, *J* = 8.1 Hz, 2H), 7.37 - 7.29 (m, 1H), 6.41 (d, *J =* 7.7 Hz, 1H), 5.13 (s, 1H), 4.49 (d, *J* = 6.2 Hz, 2H), 3.77 - 3.68 (m, 1H), 3.04 - 2.97 (m, 1H), 2.77 - 2.69 (m, 1H), 2.44 - 2.34 (m, 1H), 2.29 - 2.19 (m, 1H), 1.86 - 1.69 (m, 2H), 1.61 - 1.51 (m, 1H), 1.43 - 1.21 (m, 2H), 0.79 - 0.69 (m, 4H). 1H under water.

### Synthesis 060

### (R)-3-cyclopropyl-N⁵-(piperidin-3-yl)-N⁷-(4-(pyridin-2-yl)benzyl)pyrazolo[1,5-a]pyrimidine-5,7-diamine

### (PPA-045)

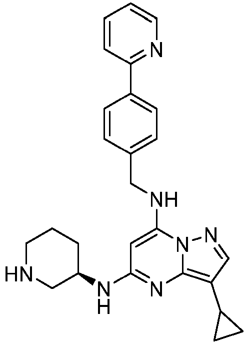

### Step 3:

### tert-butyl (R)-3-((7-((tert-butoxycarbonyl)(4-(pyridin-2-yl)benzyl)amino)-3-cyclopropylpyrazolo[1,5-a]pyrimidin-5-yl)amino)piperidine-1-carboxylate

A solution of tert-butyl (5-chloro-3-cyclopropylpyrazolo[1,5-a]pyrimidin-7-yl)(4-(pyridin-2-yl)benzyl)carbamate (76 mg, 135 µmol) (prepared as described herein), tert-butyl (R)-3-aminopiperidine-1-carboxylate (51 mg, 252 µmol) in THF (2.0 mL) was degassed in N2 for 5 min before adding tBuBrettPhos Pd G3 (18 mg, 21.0 µmol) and LiHMDS (1M in THF) (273 µL, 273 µmol). The reaction was degassed for another 5 min before being heated to 60 °C for 20 h. The reaction mixture was recharged with more tert-butyl (R)-3-aminopiperidine-1-carboxylate (101 mg, 504 µmol), ^{t}BuBrettPhos Pd G3 (36 mg, 42.0 µmol) and LiHMDS (1M in THF) (0.55 mL, 0.55 mmol). The reaction was degassed for another 5 min before being heated to 60 °C for 3 h. The reaction was concentrated *in vacuo.* Purification by column chromatography (12 g cartridge, 0-100% EtOAc/isohexane) gave the title compound (122 mg, 0.17 mmol, 82% yield, 90% purity) as a brown oil.

UPLC/MS (Method 2): m/z 640 (M+H)⁺, R_{T} 2.45 min.

### Step 4:

### (R)-3-cyclopropyl-N⁵-(piperidin-3-yl)-N⁷-(4-(pyridin-2-yl)benzyl)pyrazolo[1,5-a]pyrimidine-5,7-diamine

TFA (0.5 mL) was added to a solution of tert-butyl (R)-3-((7-((tert-butoxycarbonyl)(4-(pyridin-2-yl)benzyl)amino)-3-cyclopropylpyrazolo[1,5-a]pyrimidin-5-yl)amino)piperidine-1-carboxylate (120 mg, 178 µmol) in DCM (1.5 mL). The reaction mixture was stirred at RT for 4 h and *concentrated in vacuo.* The solid was loaded onto a column of SCX. The column was washed with MeOH (20 mL) and the product was eluted with 0.7 M NH₃ in MeOH (20 mL). The ammoniacal methanol solution was concentrated *in vacuo* to give the title compound (45 mg, 98 µmol, 55% yield, 96% purity) as a tan solid.

UPLC/MS (Method 3): m/z 440 (M+H)⁺, RT 1.51 min.

¹H NMR (500 MHz, DMSO-d₆) δ 8.64 (ddd, *J* = 4.8, 1.8, 1.0 Hz, 1H), 8.05 (d, *J* = 8.3 Hz, 2H), 7.93 (dt, *J* = 8.1, 1.1 Hz, 1H), 7.86 (td, *J* = 7.7, 1.8 Hz, 1H), 7.81 (t, *J =* 6.5 Hz, 1H), 7.53 (s, 1H), 7.45 (d, *J* = 8.2 Hz, 2H), 7.33 (ddd, J = 7.4, 4.8, 1.2 Hz, 1H), 6.42 (d, *J* = 7.7 Hz, 1H), 5.13 (s, 1H), 4.49 (d, *J* = 6.4 Hz, 2H), 3.79 - 3.69 (m, 1H), 3.05 - 2.98 (m, 1H), 2.78 - 2.71 (m, 1H), 2.45 - 2.37 (m, 1H), 2.29 - 2.22 (m, 1H), 1.86 - 1.79 (m, 1H), 1.78 - 1.71 (m, 1H), 1.61 - 1.54 (m, 1H), 1.43 - 1.33 (m, 1H), 1.33 = 1.23 (m, 1H), 0.79 - 0.71 (m, 4H). 1H under water.

### Synthesis 061

### 1-(4-(((3-cyclopropyl-7-((4-(pyridin-2-yl)benzyl)amino)pyrazolo[1,5-a]pyrimidin-5-yl)amino)methyl)piperidin-1-yl)ethan-1-one

### (PPA-046)

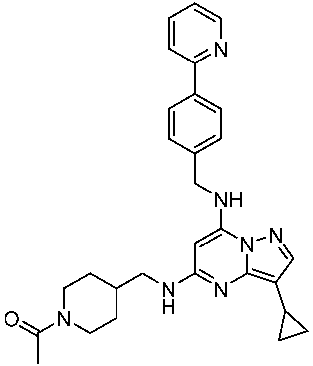

### Step 3:

### tert-butyl (5-(((1-acetylpiperidin-4-yl)methyl)amino)-3-cyclopropylpyrazolo[1,5-a]pyrimidin-7-yl)(4-(pyridin-2-yl)benzyl)carbamate

A solution of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), 1-(4-(aminomethyl)piperidin-1-yl)ethan-1-one (39 mg, 252 µmol) in THF (2.0 mL) was degassed in N₂ for 5 min before adding ^{t}BuBrettPhos Pd G3 (18 mg, 21.0 µmol) and LiHMDS (1M in THF) (273 µL, 273 µmol). The reaction mixture was degassed for another 5 min before being heated to 60 °C for 4 h. The reaction mixture was concentrated *in vacuo.* Purification by column chromatography (12 g cartridge, 0-100% (EtOH in EtOAc 25:75)/isohexane) gave the title compound (81 mg, 0.11 mmol, 83% yield, 82% purity) as a brown oil.

¹H NMR (400 MHz, DMSO-d₆) δ 8.69 - 8.62 (m, 1H), 8.04 (d, *J* = 8.3 Hz, 2H), 7.94 (dt, *J* = 8.0, 1.1 Hz, 1H), 7.87 (td, *J* = 7.7, 1.8 Hz, 1H), 7.62 (s, 1H), 7.37 (d, *J* = 8.4 Hz, 2H), 7.34 (ddd, *J* = 7.4, 4.8, 1.2 Hz, 1H), 7.24 (t, *J* = 5.5 Hz, 1H), 6.02 (s, 1H), 4.94 - 4.80 (m, 2H), 4.40 - 4.29 (m, 1H), 3.82 - 3.73 (m, 1H), 3.24 - 3.15 (m, 2H), 2.99 - 2.89 (m, 1H), 2.48 - 2.41 (m, 1H), 1.95 (s, 3H), 1.84 - 1.61 (m, 3H), 1.32 (s, 9H), 1.15 - 1.08 (m, 1H), 1.03 - 0.92 (m, 1H), 0.83 - 0.73 (m, 4H). ¹H under water.

### Step 4:

### 1-(4-(((3-cyclopropyl-7-((4-(pyridin-2-yl)benzyl)amino)pyrazolo[1,5-a]pyrimidin-5-yl)amino)methyl)piperidin-1-yl)ethan-1-one

TFA (0.25 mL) was added to a solution of tert-butyl (5-(((1-acetylpiperidin-4-yl)methyl)amino)-3-cyclopropylpyrazolo[1,5-a]pyrimidin-7-yl)(4-(pyridin-2-yl)benzyl)carbamate (81 mg, 0.11 mmol) in DCM (0.75 mL). The reaction mixture was stirred at RT for 4 h and concentrated *in vacuo.* The solid was loaded onto a column of SCX. The column was washed with MeOH (30 mL) and the product was eluted with 0.7 M NH₃ in MeOH (20 mL). The ammoniacal methanol solution was concentrated *in vacuo* and the solid was triturated in Et₂O (3 x 1 mL) to give the title compound (48 mg, 92 µmol, 83% yield, 95% purity) as a tan solid.

UPLC/MS (Method 2): m/z 496 (M+H)⁺, RT 1.18 min.

¹H NMR (400 MHz, DMSO-d₆) δ 8.64 (ddd, *J* = 4.8, 1.8, 0.9 Hz, 1H), 8.05 (d, *J* = 8.4 Hz, 2H), 7.93 (dt, *J* = 8.0, 1.2 Hz, 1H), 7.89 - 7.79 (m, 2H), 7.54 (s, 1H), 7.45 (d, *J* = 8.3 Hz, 2H), 7.33 (ddd, *J* = 7.4, 4.8, 1.2 Hz, 1H), 6.66 (t, *J* = 5.7 Hz, 1H), 5.12 (s, 1H), 4.51 (d, *J* = 6.3 Hz, 2H), 4.37 - 4.26 (m, 1H), 3.11 (t, *J* = 5.9 Hz, 2H), 2.97 - 2.84 (m, 1H), 2.47 - 2.38 (m, 1H), 1.93 (s, 3H), 1.80 - 1.58 (m, 4H), 1.11 - 1.03 (m, 1H), 1.01 - 0.88 (m, 1H), 0.75 (d, *J* = 6.9 Hz, 4H). 1H under water.

### Synthesis 062

### N⁵-(azetidin-3-yl)-3-cyclopropyl-N⁷-(4-(pyridin-2-yl)benzyl)pyrazolo[1,5-a]pyrimidine-5,7-diamine

### (PPA-047)

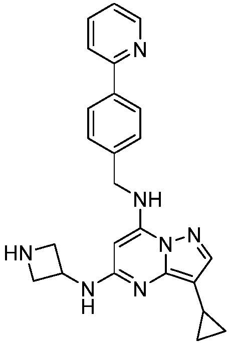

### Step 3:

### tert-butyl 3-((7-((tert-butoxycarbonyl)(4-(pyridine-2-yl)benzyl)amino)-3-cyclopropylpyrazolo[1,5-a]pyrimidin-5-yl)amino)azetidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 3-aminoazetidine-1-carboxylate (109 mg, 630 µmol) and cesium carbonate (205 mg, 630 µmol) in dioxane (4.0 mL) was degassed with N₂ for 5 min. Pd-171 (4.2 mg, 6.3 µmol) and Ruphos (3.4 mg, 7.4 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (90 mg, 0.13 mmol, 63% yield, 90% purity) as a yellow glass solid.

UPLC/MS (Method 2): m/z 612 (M+H)⁺, RT 2.36 min.

### Step 4:

### N⁵-(Pyridine-3-yl)-3-cyclopropyl-N⁷-(4-(pyridine-2-yl)benzyl)pyrazolo[1,5-a]pyrimidine-5,7-diamine

Hydrogen chloride (4 M in dioxane) (0.41 mL, 1.63 mmol) was added to a solution of tert-butyl 3-((7-((tert-butoxycarbonyl)(4-(pyridine-2-yl)benzyl)amino)-3-cyclopropylpyrazolo[1,5-a]pyrimidin-5-yl)amino)azetidine-1-carboxylate (100 mg, 0.16 mmol) in dioxane (2.0 mL). The reaction mixture was stirred at 30 °C for 3 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (12 mg, 28 µmol, 17% yield, 95% purity) as a white solid.

UPLC/MS (Method 2): m/z 412 (M+H)⁺, RT 1.02 min.

¹H NMR (400 MHz, DMSO-d₆) δ 8.67 - 8.63 (m, 1H), 8.07 - 8.00 (m, 3H), 7.96 - 7.82 (m, 3H), 7.56 (s, 1H), 7.46 - 7.41 (m, 2H), 7.33 (ddd, *J* = 7.4, 4.7, 1.2 Hz, 1H), 7.06 (d, *J* = 6.9 Hz, 1H), 5.07 (s, 1H), 4.62 - 4.53 (m, 1H), 4.51 (d, *J* = 6.4 Hz, 2H), 3.55 (t, *J* = 7.4 Hz, 2H), 1.81 - 1.69 (m, 1H), 0.76 (d, *J* = 6.9 Hz, 4H). 2H under water.

### Synthesis 063

### 3-cyclopropyl-N⁵-(piperidin-4-yl)-N⁷-(4-(pyridin-2-yl)benzyl)pyrazolo[1,5-a]pyrimidine-5,7-diamine

### (PPA-048)

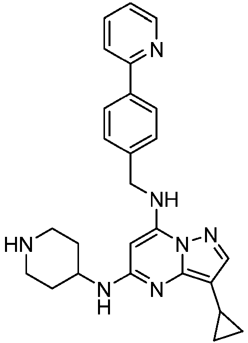

### Step 3:

### tert-butyl 4-((7-((tert-butoxycarbonyl)(4-(pyridin-2-yl)benzyl)amino)-3-cyclopropylpyrazolo[1,5-a]pyrimidin-5-yl)amino)piperidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 4-aminopiperidine-1-carboxylate (126 mg, 630 µmol) and cesium carbonate (205 mg, 630 µmol) in dioxane (4.0 mL) was degassed with N₂ for 5 min. Pd-171 (4.2 mg, 6.3 µmol) and Ruphos (3.4 mg, 7.4 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (100 mg, 0.14 mmol, 67% yield, 90% purity) as a yellow glass solid.

UPLC/MS (Method 3): m/z 640 (M+H)⁺, RT 2.48 min.

### Step 4:

### 3-cyclopropyl-N⁵-(piperidin-4-yl)-N⁷-(4-(pyridin-2-yl)benzyl)pyrazolo[1,5-a]pyrimidine-5,7-diamine

Hydrogen chloride (4 M in dioxane) (0.39 mL, 1.56 mmol) was added to a solution of tert-butyl 4-((7-((tert-butoxycarbonyl)(4-(pyridin-2-yl)benzyl)amino)-3-cyclopropylpyrazolo[1,5-a]pyrimidin-5-yl)amino)piperidine-1-carboxylate (100 mg, 0.156 mmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (24 mg, 54 µmol, 34% yield, 98% purity) as a white solid.

UPLC/MS (Method 2): m/z 440 (M+H)⁺, RT 0.94 min.

¹H NMR (400 MHz, DMSO-d₆) δ 8.67 - 8.61 (m, 1H), 8.05 (d, *J* = 8.2 Hz, 2H), 7.93 (d, *J* = 8.1 Hz, 1H), 7.90 - 7.77 (m, 2H), 7.53 (s, 1H), 7.45 (d, *J =* 8.1 Hz, 2H), 7.37 - 7.29 (m, 1H), 6.47 (d, *J= 7.4* Hz, 1H), 5.09 (s, 1H), 4.49 (d, *J= 6.2* Hz, 2H), 3.75 - 3.65 (m, 1H), 2.92 - 2.84 (m, 2H), 2.49 - 2.41 (m, 1H), 1.81 - 1.75 (m, 1H), 1.78 - 1.69 (m, 2H), 1.24 - 1.11 (m, 2H), 0.78 - 0.72 (m, 4H). 2H under water.

### Synthesis 064

### 5-(azetidin-3-yloxy)-3-cyclopropyl-N-(4-(pyridin-2-yl)benzyl)pyrazolo[1,5-a]pyrimidin-7-amine

### (PPA-049)

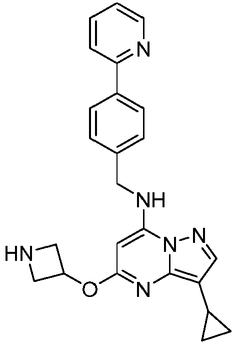

### Step 3:

### tert-butyl 3-((7-((tert-butoxycarbonyl)(4-(pyridin-2-yl)benzyl)amino)-3-cyclopropylpyrazolo[1,5-a]pyrimidin-5-yl)oxy)azetidine-1-carboxylate

A suspension of tert-butyl (5-chloro-3-cyclopropylpyrazolo[1,5-a]pyrimidin-7-yl)(4-(pyridin-2-yl)benzyl)carbamate (200 mg, 420 µmol) (prepared as described herein), tert-butyl-3-hydroxyazetidine-1-Carboxylate (291 mg, 1.68 mmol) and cesium carbonate (548 mg, 1.68 mmol) in dioxane (5.0 mL) was degassed with N₂ for 5 min. Pd-171 (28 mg, 42 µmol) and Ruphos (20 mg, 42 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-100% (EtOH in EtOAc 25:75)/isohexane) gave the title compound (180 mg, 0.27 mmol, 65% yield, 93% purity) as a yellow oil.

UPLC/MS (Method 5): m/z 613 (M+H)⁺, RT 2.20 min.

### Step 4:

### 5-(azetidin-3-yloxy)-3-cyclopropyl-N-(4-(pyridin-2-yl)benzyl)pyrazolo[1,5-a]pyrimidin-7-amine

TFA (0.5 mL) was added to a solution of tert-butyl 3-((7-((tert-butoxycarbonyl)(4-(pyridin-2-yl)benzyl)amino)-3-cyclopropylpyrazolo[1,5-a]pyrimidin-5-yl)oxy)azetidine-1-carboxylate (180 mg, 294 µmol) in DCM (1.5 mL). The reaction mixture was stirred at RT for 4 h and concentrated *in vacuo.* The solid was loaded onto a column of SCX. The column was washed with MeOH (50 mL) and the product was eluted with 0.7 M NH₃ in MeOH (80 mL). The ammoniacal methanol solution was concentrated *in vacuo* and the solid was triturated in Et₂O (2 x 1 mL) to give the title compound (70 mg, 160 µmol, 55% yield, 96% purity) as a white solid.

UPLC/MS (Method 4): m/z 413 (M+H)⁺, RT 1.44 min.

¹H NMR (400 MHz, DMSO-d₆) δ 8.67 - 8.61 (m, 1H), 8.41 (t, *J =* 6.4 Hz, 1H), 8.08 - 8.01 (m, 2H), 7.93 (d, *J =* 8.0 Hz, 1H), 7.86 (td, *J =* 7.7, 1.8 Hz, 1H), 7.79 (s, 1H), 7.48 (d, *J =* 8.2 Hz, 2H), 7.37 - 7.29 (m, 1H), 5.38 (s, 1H), 5.30 - 5.22 (m, 1H), 4.63 - 4.57 (m, 2H), 4.36 - 4.28 (m, 1H), 3.67 (dd, *J =* 9.0, 6.8 Hz, 2H), 3.50 - 3.38 (m, 2H), 1.85 - 1.76 (m, 1H), 0.84 - 0.77 (m, 4H).

### Synthesis 065

### 1-(3-cyclopropyl-7-((4-(pyridin-2-yl)benzyl)amino)pyrazolo[1,5-a]pyrimidin-5-yl)piperidin-4-ol

### (PPA-050)

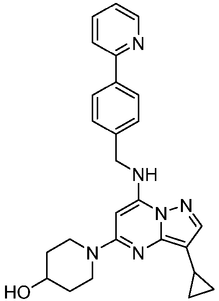

### Step 3:

### tert-butyl (3-cyclopropyl-5-(4-hydroxypiperidin-1-yl)pyrazolo[1,5-a]pyrimidin-7-yl)(4-(pyridin-2-yl)benzyl)carbamate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), piperidin-4-ol (64 mg, 630 µmol) and cesium carbonate (205 mg, 630 µmol) in dioxane (4.0 mL) was degassed with N₂ for 5 min. Pd-171 (4.2 mg, 6.3 µmol) and Ruphos (3.4 mg, 7.4 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH/DCM) gave the title compound (105 mg, 0.19 mmol, 91% yield, 98% purity) as a yellow solid.

UPLC/MS (Method 3): m/z 541 (M+H)⁺, RT 1.90 min.

### Step 4:

### 1-(3-cyclopropyl-7-((4-(pyridin-2-yl)benzyl)amino)pyrazolo[1,5-a]pyrimidin-5-yl)piperidin-4-ol

Hydrogen chloride (4 M in dioxane) (0.49 mL, 1.94 mmol) was added to a solution of tert-butyl (3-cyclopropyl-5-(4-hydroxypiperidin-1-yl)pyrazolo[1,5-a]pyrimidin-7-yl)(4-(pyridin-2-yl)benzyl)carbamate (105 mg, 194 µmol) in dioxane (2.0 mL). The reaction mixture was stirred at 30 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (35 mg, 78 µmol, 40% yield, 98% purity) as a white solid.

UPLC/MS (Method 3): m/z 441 (M+H)⁺, RT 1.14 min.

¹H NMR (400 MHz, DMSO-d₆) δ 8.67 - 8.61 (m, 1H), 8.04 (d, *J =* 8.3 Hz, 2H), 7.96 - 7.89 (m, 2H), 7.86 (td, J = 7.7, 1.8 Hz, 1H), 7.59 (s, 1H), 7.50 (d, *J* = 8.1 Hz, 2H), 7.37 - 7.29 (m, 1H), 5.54 (s, 1H), 4.63 (d, *J* = 4.3 Hz, 1H), 4.61 (d, *J* = 6.5 Hz, 2H), 4.03 - 3.95 (m, 2H), 3.69 - 3.59 (m, 1H), 3.11 - 2.99 (m, 2H), 1.85 - 1.63 (m, 3H), 1.33 - 1.21 (m, 2H), 0.81 - 0.71 (m, 4H).

### Synthesis 066

### 4-(((3-cyclopropyl-7-((4-(pyridin-2-yl)benzyl)amino)pyrazolo[1,5-a]pyrimidin-5-yl)amino)methyl)piperidin-2-one

### (PPA-051)

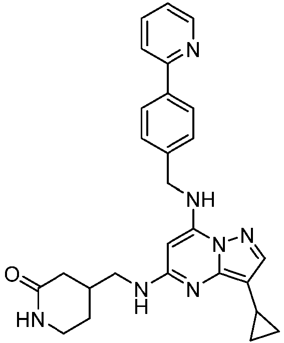

### Step 3:

### tert-butyl (3-cyclopropyl-5-(((2-oxopiperidin-4-yl)methyl)amino)pyrazolo[1,5-a]pyrimidin-7-yl)(4-(pyridin-2-yl)benzyl)carbamate

A solution of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), 4-(aminomethyl)piperidin-2-one, HCl (42 mg, 252 µmol) in THF (2.0 mL) was degassed in N₂ for 5 min before adding ^{t}BuBrettPhos Pd G3 (18 mg, 21.0 µmol) and LiHMDS (1M in THF) (273 µL, 273 µmol). The reaction mixture was degassed for another 5 min before being heated to 60 °C overnight then concentrated *in vacuo.* Purification by column chromatography (12 g cartridge, 0-10% MeOH/DCM) gave the title compound (50 mg, 85 µmol, 41% yield, 97% purity) as a brown oil.

UPLC/MS (Method 2): m/z 568 (M+H)⁺, RT 1.76 min.

### Step 4:

### 4-(((3-cyclopropyl-7-((4-(pyridin-2-yl)benzyl)amino)pyrazolo[1,5-a]pyrimidin-5-yl)amino)methyl)piperidin-2-one

TFA (0.25 mL) was added to a solution of tert-butyl (3-cyclopropyl-5-(((2-oxopiperidin-4-yl)methyl)amino)pyrazolo[1,5-a]pyrimidin-7-yl)(4-(pyridin-2-yl)benzyl)carbamate (50 mg, 88 µmol) in DCM (0.75 mL). The reaction mixture was stirred at RT for 4 h and concentrated *in vacuo.* The solid was loaded onto a column of SCX. The column was washed with MeOH (20 mL) and the product was eluted with 0.7 M NH₃ in MeOH (20 mL). The ammoniacal methanol solution was concentrated *in vacuo.* Purification by RP preparative HPLC (25-100% MeCN/0.3% NH₃ in water) gave the title compound (11 mg, 22 µmol, 25% yield, 95% purity) as a white solid.

UPLC/MS (Method 3): m/z 468 (M+H)⁺, RT 1.28 min.

¹H NMR (400 MHz, DMSO-d₆) δ 8.67 - 8.62 (m, 1H), 8.05 (d, *J* = 8.3 Hz, 2H), 7.96 - 7.90 (m, 1H), 7.89 - 7.80 (m, 2H), 7.55 (s, 1H), 7.45 (d, *J* = 8.1 Hz, 2H), 7.41 - 7.36 (m, 1H), 7.33 (ddd, *J* = 7.3, 4.8, 1.2 Hz, 1H), 6.70 (t, *J =* 5.7 Hz, 1H), 5.13 (s, 1H), 4.51 (d, *J =* 6.0 Hz, 2H), 3.23 - 3.11 (m, 3H), 3.04 (td, *J =* 11.2, 4.3 Hz, 1H), 2.21 (dd, *J =* 17.0, 5.1 Hz, 1H), 2.05 - 1.92 (m, 1H), 1.87 - 1.71 (m, 3H), 1.38 - 1.25 (m, 1H), 0.80 - 0.71 (m, 4H).

### Synthesis 067

### 1-(3-cyclopropyl-7-((4-(pyridin-2-yl)benzyl)amino)pyrazolo[1,5-a]pyrimidin-5-yl)azetidin-3-ol

### (PPA-052)

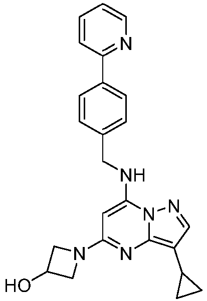

### Step 3:

### tert-butyl (3-cyclopropyl-5-(3-hydroxyazetidin-1-yl)pyrazolo[1,5-a]pyrimidin-7-yl)(4-(pyridin-2-yl)benzyl)carbamate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), azetidin-3-ol, HCl (69 mg, 630 µmol) and cesium carbonate (411 mg, 1.26 mmol) in dioxane (4.0 mL) was degassed with N₂ for 5 min. Pd-171 (4.2 mg, 6.3 µmol) and Ruphos (3.4 mg, 7.4 µmol) were added and N₂ was bubbled through the reaction mixture for another 5 min. The reaction mixture was stirred at 90 °C for 5 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (85 mg, 0.17 mmol, 79% yield, 99% purity) as a yellow glass solid.

UPLC/MS (Method 3): m/z 513 (M+H)⁺, RT 1.74 min.

### Step 4:

### 1-(3-cyclopropyl-7-((4-(pyridin-2-yl)benzyl)amino)pyrazolo[1,5-a]pyrimidin-5-yl)azetidin-3-ol

At 0 °C, hydrogen chloride (4 M in dioxane) (0.41 mL, 1.7 mmol) was added to a solution of tert-butyl (3-cyclopropyl-5-(3-hydroxyazetidin-1-yl)pyrazolo[1,5-a]pyrimidin-7-yl)(4-(pyridin-2-yl)benzyl)carbamate (85 mg, 0.17 mmol) in dioxane (2.0 mL). The reaction mixture was stirred at RT for 1.5 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo.* Purification by RP preparative HPLC (25-100% MeCN/0.3% NH₃ in water) gave the title compound (22 mg, 53 µmol, 32% yield, 99% purity) as a white solid.

UPLC/MS (Method 2): m/z 413 (M+H)⁺, RT 1.04 min.

¹H NMR (400 MHz, DMSO-d₆) δ 8.64 (d, *J* = 4.9 Hz, 1H), 8.08 - 7.98 (m, 3H), 7.93 (d, *J* = 8.0 Hz, 1H), 7.90 - 7.82 (m, 1H), 7.58 (s, 1H), 7.48 (d, *J* = 8.1 Hz, 2H), 7.37 - 7.29 (m, 1H), 5.60 - 5.56 (m, 1H), 5.06 (s, 1H), 4.60 - 4.54 (m, 2H), 4.50 - 4.46 (m, 1H), 4.13 - 4.05 (m, 2H), 3.62 (dd, J = 8.8, 4.6 Hz, 2H), 1.82 - 1.70 (m, 1H), 0.81 - 0.73 (m, 2H), 0.73 - 0.64 (m, 2H).

### Synthesis 068

### (3R,4R)-4-(((3-cyclopropyl-7-((4-(pyridin-2-yl)benzyl)amino)pyrazolo[1,5-a]pyrimidin-5-yl)(methyl)amino)methyl)piperidin-3-ol

### (PPA-053)

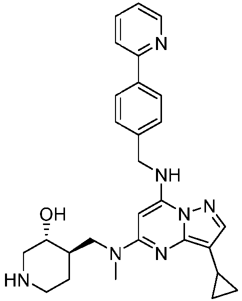

### Step A:

### tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-((tertbutyldimethylsilyl)oxy)piperidine-1-carboxylate

Tert-butylchlorodimethylsilane (47 mg, 311 µmol) was added to a solution of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-hydroxypiperidine-1-carboxylate (130 mg, 194 µmol) (prepared as described herein) and 1H-imidazole (26 mg, 388 µmol) in DMF (2.0 mL). The reaction mixture was stirred at RT overnight then recharged with 1H-imidazole (26 mg, 388 µmol) and tert-butylchlorodimethylsilane (47 mg, 311 µmol) and stirred at RT for 24 h. The reaction mixture was diluted with brine (20 mL) and extracted with TBME (3 x 20 mL), dried over Na₂SO₄, filtered and concentrated *in vacuo.* Purification by column chromatography (12 g cartridge, 0-10% MeOH/DCM) gave the title compound (130 mg, 0.16 mmol, 82% yield, 96% purity) as a colourless oil.

UPLC/MS (Method 2): m/z 785 (M+H)⁺, RT 2.50 min.

### Step B:

### tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)(methyl)amino)methyl)-3-((tertbutyldimethylsilyl)oxy)piperidine-1-carboxylate

To an ice cooled solution of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-((tertbutyldimethylsilyl)oxy)piperidine-1-carboxylate (130 mg, 166 µmol) in THF (3.00 mL) was added LiHMDS (1M in THF) (199 µL, 199 µmol). The reaction mixture was stirred for 10 min then iodomethane (12 µL, 199 µmol) was added and the reaction mixture was stirred at RT for 1 h then poured onto ice water (30 mL) and extracted into ethyl acetate (2 x 20 mL). The organics were dried over Na₂SO₄, filtered and concentrated *in vacuo.* Purification by column chromatography (24 g cartridge, 0-10% MeOH (containing 0.7 M NH₃)/DCM) gave the title compound (130 mg, 0.15 mmol, 88% yield, 90% purity) as a yellow solid.

UPLC/MS (Method 2): m/z 799 (M+H)⁺, RT 1.77 min.

### Step 4:

### (3R,4R)-4-(((3-cyclopropyl-7-((4-(pyridin-2-yl)benzyl)amino)pyrazolo[1,5-a]pyrimidin-5-yl)(methyl)amino)methyl)piperidin-3-ol

Hydrogen chloride (4 M in dioxane) (0.41 mL, 1.63 mmol) was added to a solution of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)(methyl)amino)methyl)-3-((tertbutyldimethylsilyl)oxy)piperidine-1-carboxylate (130 mg, 0.163 mmol) in dioxane (2.0 mL). The reaction mixture was stirred at 35 °C for 4 h and concentrated *in vacuo.* The solid was loaded onto a column of SCX. The column was washed with MeOH (40 mL) and the product was eluted with 0.7 M NH₃ in MeOH (10 mL). The ammoniacal methanol solution was concentrated *in vacuo* to give the title compound (28 mg, 55 µmol, 34% yield, 95% purity) as a white solid.

UPLC/MS (Method 2): m/z 484 (M+H)⁺, RT 0.99 min.

¹H NMR (400 MHz, DMSO-d₆) δ 8.67 - 8.61 (m, 1H), 8.04 (d, *J =* 8.1 Hz, 2H), 7.98 (t, *J =* 6.5 Hz, 1H), 7.94 - 7.89 (m, 1H), 7.89 - 7.81 (m, 1H), 7.57 (s, 1H), 7.53 (d, *J* = 8.1 Hz, 2H), 7.37 - 7.29 (m, 1H), 5.36 (s, 1H), 5.05 - 5.01 (m, 1H), 4.57 (d, *J* = 6.4 Hz, 2H), 3.60 - 3.46 (m, 2H), 3.09 - 2.98 (m, 1H), 2.97 (s, 3H), 2.97 - 2.89 (m, 1H), 2.70 - 2.62 (m, 1H), 2.15 (q, *J* = 9.9 Hz, 2H), 1.99 - 1.84 (m, 1H), 1.81 - 1.68 (m, 1H), 1.49 - 1.42 (m, 2H), 1.04 - 0.94 (m, 1H), 0.79 - 0.66 (m, 4H).

### Synthesis 071

### 5-(azetidin-3-ylmethoxy)-3-cyclopropyl-N-(4-(pyridin-2-yl)benzyl)pyrazolo[1,5-a]pyrimidin-7-amine

### (PPA-055)

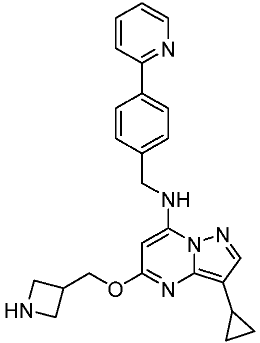

### Step 3:

### tert-butyl 3-(((7-((tert-butoxycarbonyl)(4-(pyridin-2-yl)benzyl)amino)-3-cyclopropylpyrazolo[1,5-a]pyrimidin-5-yl)oxy)methyl)azetidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 3-(hydroxymethyl)azetidine-1-carboxylate (175 mg, 840 µmol) and cesium carbonate (274 mg, 840 µmol) in dioxane (2.6 mL) was degassed with N₂ for 5 min. Pd-171 (13.9 mg, 21.0 µmol) and Ruphos (9.8 mg, 21.0 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-100% (EtOH in EtOAc 25:75)/ isohexane) gave the title compound (117 mg, 0.18 mmol, 84% yield, 95% purity) as a yellow oil.

UPLC/MS (Method 3): m/z 627 (M+H)⁺, RT 2.23 min.

### Step 4:

### 5-(azetidin-3-ylmethoxy)-3-cyclopropyl-N-(4-(pyridin-2-yl)benzyl)pyrazolo[1,5-a]pyrimidin-7-amine

At 0 °C, TFA (1.0 mL) was added to a solution of tert-butyl 3-(((7-((tert-butoxycarbonyl)(4-(pyridin-2-yl)benzyl)amino)-3-cyclopropylpyrazolo[1,5-a]pyrimidin-5-yl)oxy)methyl)azetidine-1-carboxylate (69 mg, 106 µmol) in DCM (2.0 mL). The reaction mixture was stirred at 0 °C for 3 h then RT for 1 h and concentrated *in vacuo.* The solid was loaded onto a column of SCX. The column was washed with MeOH (80 mL) and the product was eluted with 0.7 M NH₃ in MeOH (100 mL). The ammoniacal methanol solution was concentrated *in vacuo* and the solid was triturated in Et₂O (3 x 1 mL) to give the title compound (33 mg, 75 µmol, 71% yield, 97% purity) as a beige solid.

UPLC/MS (Method 3): m/z 427 (M+H)⁺, RT 2.00 min.

¹H NMR (400 MHz, DMSO-d₆) δ 8.67 - 8.62 (m, 1H), 8.36 (t, *J =* 6.5 Hz, 1H), 8.04 (d, *J =* 8.3 Hz, 2H), 7.92 (d, *J* = 8.0 Hz, 1H), 7.86 (td, *J* = 7.6, 1.8 Hz, 1H), 7.76 (s, 1H), 7.47 (d, *J* = 8.2 Hz, 2H), 7.33 (ddd, *J =* 7.3, 4.7, 1.2 Hz, 1H), 5.36 (s, 1H), 4.59 (d, *J* = 6.2 Hz, 2H), 4.36 (d, *J* = 7.1 Hz, 2H), 3.50 (t, *J* = 7.7 Hz, 2H), 3.27 (t, *J* = 6.8 Hz, 2H), 2.99 - 2.88 (m, 1H), 1.89 - 1.80 (m, 1H), 0.89 - 0.80 (m, 2H), 0.80 - 0.73 (m, 2H). ¹H under water.

### Synthesis 072

### 3-cyclopropyl-N⁵-((1-(methylsulfonyl)piperidin-4-yl)methyl)-N⁷-(4-(pyridin-2-yl)benzyl)pyrazolo[1,5-a]pyrimidine-5,7-diamine

### (PPA-056)

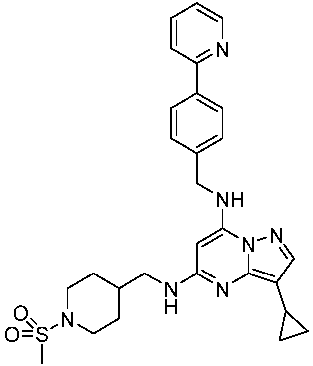

### Step 3:

### tert-butyl (3-cyclopropyl-5-(((1-(methylsulfonyl)piperidin-4-yl)methyl)amino)pyrazolo[1,5-a]pyrimidin-7-yl)(4-(pyridin-2-yl)benzyl)carbamate

A solution of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), (1-(methylsulfonyl)piperidin-4-yl)methanamine, HCl (58 mg, 252 µmol) in THF (2.0 mL) was degassed in N₂ for 5 min before adding ^{t}BuBrettPhos Pd G3 (18 mg, 21.0 µmol) and LiHMDS (1M in THF) (546 µL, 546 µmol). The reaction mixture was degassed for another 5 min before being heated to 60 °C for 1 h. The reaction mixture was recharged with more LiHMDS (1M in THF) (546 µL, 546 µmol). The reaction was degassed for another 5 min before being heated to 60 °C overnight. The reaction mixture was concentrated *in vacuo.* Purification by column chromatography (12 g cartridge, 0-100% EtOAc/isohexane) gave the title compound (87 mg, 0.13 mmol, 60% yield, 92% purity) as a tan oil.

UPLC/MS (Method 2): m/z 632 (M+H)⁺, RT 2.03 min.

### Step 4:

### 3-cyclopropyl-N⁵-((1-(methylsulfonyl)piperidin-4-yl)methyl)-N⁷-(4-(pyridin-2-yl)benzyl)pyrazolo[1,5-a]pyrimidine-5,7-diamine

TFA (0.5 mL) was added to a solution of tert-butyl (3-cyclopropyl-5-(((1-(methylsulfonyl)piperidin-4-yl)methyl)amino)pyrazolo[1,5-a]pyrimidin-7-yl)(4-(pyridin-2-yl)benzyl)carbamate (87 mg, 0.13 mmol) in DCM (1.5 mL). The reaction mixture was stirred at RT for 4 h and concentrated *in vacuo.* The solid was loaded onto a column of SCX. The column was washed with MeOH (80 mL) and the product was eluted with 0.7 M NH₃ in MeOH (100 mL). The ammoniacal methanol solution was concentrated *in vacuo* and the solid was triturated in Et₂O (3 x 1 mL) to give the title compound (69 mg, 0.13 mol, 91% yield, 97% purity) as a beige solid.

UPLC/MS (Method 3): m/z 532 (M+H)⁺, RT 1.55 min.

¹H NMR (400 MHz, DMSO-d₆) δ 8.68 - 8.62 (m, 1H), 8.05 (d, *J =* 8.4 Hz, 2H), 7.93 (dt, *J* = 8.0, 1.2 Hz, 1H), 7.89 - 7.81 (m, 2H), 7.55 (s, 1H), 7.45 (d, *J =* 8.2 Hz, 2H), 7.33 (ddd, *J =* 7.4, 4.8, 1.2 Hz, 1H), 6.69 (t, *J =* 5.8 Hz, 1H), 5.13 (s, 1H), 4.51 (d, *J =* 6.4 Hz, 2H), 3.58 - 3.47 (m, 2H), 3.13 (t, *J =* 6.1 Hz, 2H), 2.78 (s, 3H), 2.59 (t, *J =* 11.7 Hz, 2H), 1.81 - 1.70 (m, 3H), 1.66 - 1.51 (m, 1H), 1.25 - 1.12 (m, 2H), 0.75 (d, *J =* 7.4 Hz, 4H).

### Synthesis 073

### 5-(3-aminoazetidin-1-yl)-3-cyclopropyl-N-(4-(pyridin-2-yl)benzyl)pyrazolo[1,5-a]pyrimidin-7-amine

### (PPA-057)

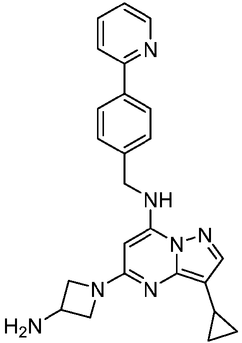

### Step 3:

### tert-butyl (5-(3-aminoazetidin-1-yl)-3-cyclopropylpyrazolo[1,5-a]pyrimidin-7-yl)(4-(pyridin-2-yl)benzyl)carbamate

A suspension of tert-butyl (5-chloro-3-cyclopropylpyrazolo[1,5-a]pyrimidin-7-yl)(4-(pyridin-2-yl)benzyl)carbamate (120 mg, 252 µmol) (prepared as described herein), tert-butyl azetidin-3-ylcarbamate (174 mg, 1.01 mmol), and cesium carbonate (329 mg, 1.01 mmol) in dioxane (3.2 mL) was degassed with N₂ for 5 min. Pd-171 (16.7 mg, 25.2 µmol) and Ruphos (11.8 mg, 25.2 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH/DCM) gave the title compound (122 mg, 0.19 mmol, 76% yield, 87% purity) as an orange oil.

UPLC/MS (Method 5): m/z 512 (M+H)⁺, RT 1.74 min.

### Step 4:

### 5-(3-aminoazetidin-1-yl)-3-cyclopropyl-N-(4-(pyridin-2-yl)benzyl)pyrazolo[1,5-a]pyrimidin-7-amine

At 0 °C, TFA (0.5 mL) was added to a solution of tert-butyl (5-(3-aminoazetidin-1-yl)-3-cyclopropylpyrazolo[1,5-a]pyrimidin-7-yl)(4-(pyridin-2-yl)benzyl)carbamate (122 mg, 0.19 mmol) in DCM (1.5 mL). The reaction mixture was stirred at 0 °C for 3 h then at RT for 1 h and concentrated in vacuo. The solid was loaded onto a column of SCX. The column was washed with MeOH (80 mL) and the product was eluted with 0.7 M NH₃ in MeOH (100 mL). The ammoniacal methanol solution was concentrated *in vacuo.* Purification by column chromatography (4 g cartridge, 0-10% MeOH (containing 0.7 M NH₃)/DCM) gave the title compound (42 mg, 98 µmol, 44% yield, 97% purity) as an orange solid.

UPLC/MS (Method 3): m/z 412 (M+H)⁺, RT 1.30 min.

¹H NMR (400 MHz, DMSO-d₆) δ 8.67 - 8.60 (m, 1H), 8.04 (d, *J =* 8.3 Hz, 2H), 7.99 (t, *J =* 6.6 Hz, 1H), 7.93 (dt, *J =* 8.0, 1.2 Hz, 1H), 7.86 (td, *J =* 7.7, 1.9 Hz, 1H), 7.57 (s, 1H), 7.48 (d, *J* = 8.3 Hz, 2H), 7.33 (ddd, *J* = 7.4, 4.8, 1.2 Hz, 1H), 5.03 (s, 1H), 4.56 (d, *J* = 6.5 Hz, 2H), 4.04 (t, *J* = 7.7 Hz, 2H), 3.75 - 3.65 (m, 1H), 3.52 - 3.43 (m, 2H), 2.13 - 1.94 (m, 2H), 1.81 - 1.70 (m, 1H), 0.82 - 0.72 (m, 2H), 0.71 - 0.64 (m, 2H).

### Synthesis 074

### 5-(azetidin-3-yl)-3-cyclopropyl-N-(4-(pyridin-2-yl)benzyl)pyrazolo[1,5-a]pyrimidin-7-amine

### (PPA-058)

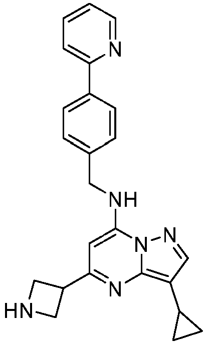

### Step 3:

### tert-butyl 3-(7-((tert-butoxycarbonyl)(4-(pyridin-2-yl)benzyl)amino)-3-cyclopropylpyrazolo[1,5-a]pyrimidin-5-yl)azetidine-1-carboxylate / 5-(azetidin-3-yl)-3-cyclopropyl-N-(4-(pyridin-2-yl)benzyl)pyrazolo[1,5-a]pyrimidin-7-amine (1/0.94)

A mixture of tert-butyl (5-chloro-3-cyclopropylpyrazolo[1,5-a]pyrimidin-7-yl)(4-(pyridin-2-yl)benzyl)carbamate (200 mg, 420 µmol) (prepared as described herein), (1-(tert-butoxycarbonyl)azetidin-3-yl)zinc(II) iodide (0.5M in THF) (2.10 mL, 1.05 mmol), Pd(dppf)Cl₂·DCM (34 mg, 42.0 µmol) in THF (2.0 mL) was sparged with N₂ for 15 min before being heated to 50 °C for 72 h. The reaction mixture was concentrated *in vacuo.* Purification by column chromatography (24 g cartridge, 0-100% EtOAc/isohexane) gave a mixture of tert-butyl 3-(7-((tert-butoxycarbonyl)(4-(pyridin-2-yl)benzyl)amino)-3-cyclopropylpyrazolo[1,5-a]pyrimidin-5-yl)azetidine-1-carboxylate / 5-(azetidin-3-yl)-3-cyclopropyl-N-(4-(pyridin-2-yl)benzyl)pyrazolo[1,5-a]pyrimidin-7-amine (1/0.94) (140 mg, 175 µmol, 41% yield, 56% purity) as a brown oil.

UPLC/MS (Method 2): m/z 497 (M+H)⁺, RT 1.76 min; m/z 397 (M+H)⁺, RT 1.03 min

### Step 4:

### 5-(azetidin-3-yl)-3-cyclopropyl-N-(4-(pyridin-2-yl)benzyl)pyrazolo[1,5-a]pyrimidin-7-amine

At 0 °C, TFA (0.5 mL) was added to a mixture of tert-butyl 3-(7-((tert-butoxycarbonyl)(4-(pyridin-2-yl)benzyl)amino)-3-cyclopropylpyrazolo[1,5-a]pyrimidin-5-yl)azetidine-1-carboxylate / 5-(azetidin-3-yl)-3-cyclopropyl-N-(4-(pyridin-2-yl)benzyl)pyrazolo[1,5-a]pyrimidin-7-amine (1/0.94) (140 mg, 175 µmol) in DCM (1.5 mL). The reaction mixture was stirred at RT for 3 h and concentrated *in vacuo.* The solid was loaded onto a column of SCX. The column was washed with MeOH (80 mL) and the product was eluted with 0.7 M NH₃ in MeOH (100 mL). The ammoniacal methanol solution was concentrated *in vacuo.* Further purification by RP preparative HPLC (30-100% MeCN/0.3% NH₃ in water) gave solid which was dissolved in MeOH (0.50 mL). The solution was treated with a 2M aq. solution of sodium hydroxide (59 µL, 117 µmol) and the reaction mixture was stirred at RT for 30 min. Sat. aq. NH₄Cl (0.5 mL) was added and the solution was loaded onto a column of SCX. The column was washed with MeOH (50 mL) and the product was eluted with 0.7 M NH₃ in MeOH (60 mL). The ammoniacal methanol solution was concentrated *in vacuo* and the solid was triturated in Et₂O (3 x 1 mL) to give the title compound (8 mg, 20 µmol, 20% yield, 97% purity) as a beige solid.

UPLC/MS (Method 2): m/z 397 (M+H)⁺, RT 1.03 min.

¹H NMR (400 MHz, DMSO-d₆) δ 8.66 - 8.63 (m, 1H), 8.44 - 8.40 (m, 1H), 8.04 (d, *J =* 8.1 Hz, 3H), 7.92 (d, *J* = 8.0 Hz, 1H), 7.90 - 7.81 (m, 2H), 7.49 (d, *J =* 8.1 Hz, 2H), 7.37 - 7.29 (m, 1H), 6.01 (s, 1H), 4.66 (d, *J =* 6.4 Hz, 2H), 3.83 - 3.70 (m, 2H), 3.63 - 3.55 (m, 2H), 1.99 - 1.90 (m, 1H), 0.90 - 0.76 (m, 4H). ¹H under water.

### Synthesis 076

### (R)-3-cyclopropyl-N-(4-(pyridin-2-yl)benzyl)-5-(pyrrolidin-3-yloxy)pyrazolo[1,5-a]pyrimidin-7-amine

### (PPA-060)

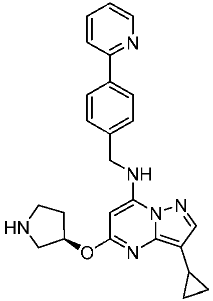

### Step 3:

### tert-butyl (R)-3-((7-((tert-butoxycarbonyl)(4-(pyridin-2-yl)benzyl)amino)-3-cyclopropylpyrazolo[1,5-a]pyrimidin-5-yl)oxy)pyrrolidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl (R)-3-hydroxypyrrolidine-1-carboxylate (162 mg, 840 µmol) and cesium carbonate (274 mg, 840 µmol) in dioxane (2.6 mL) was degassed with N₂ for 5 min. Pd-171 (13.9 mg, 21.0 µmol) and Ruphos (9.8 mg, 21.0 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-100% (EtOH in EtOAc 25:75)/isohexane) gave the title compound (86 mg, 0.13 mmol, 64% yield, 98% purity) as a yellow oil.

UPLC/MS (Method 3): m/z 627 (M+H)⁺, RT 2.26 min.

### Step 4:

### (R)-3-cyclopropyl-N-(4-(pyridin-2-yl)benzyl)-5-(pyrrolidin-3-yloxy)pyrazolo[1,5-a]pyrimidin-7-amine

Hydrogen chloride (4 M in dioxane) (0.68 mL, 2.7 mmol) was added to a solution of tert-butyl (R)-3-((7-((tert-butoxycarbonyl)(4-(pyridin-2-yl)benzyl)amino)-3-cyclopropylpyrazolo[1,5-a]pyrimidin-5-yl)oxy)pyrrolidine-1-carboxylate (85 mg, 0.14 mmol) in dioxane (2.0 mL). The reaction mixture was stirred at 40 °C for 2 h and concentrated *in vacuo.* Hydrogen chloride **(1.25 M in MeOH) (2.0 mL, 2.50 mmol)** was added and the resulting reaction mixture was stirred at 40 °C for 1.5 h then concentrated *in vacuo.* The solid was triturated in MeCN/Et₂O 1:1 (3 mL) and the solid was loaded onto a column of SCX. The column was washed with MeOH (80 mL) and the product was eluted with 0.7 M NH₃ in MeOH (100 mL). The ammoniacal methanol solution was concentrated *in vacuo* to give the title compound (44 mg, 100 µmol, 75% yield, 98% purity) as a yellow solid.

UPLC/MS (Method 3): m/z 427 (M+H)⁺, RT 1.61 min.

¹H NMR (400 MHz, DMSO-d₆) δ 8.66 - 8.62 (m, 1H), 8.35 (t, *J =* 6.5 Hz, 1H), 8.04 (d, *J =* 8.3 Hz, 2H), 7.92 (d, *J =* 8.0 Hz, 1H), 7.86 (td, *J =* 7.7, 1.9 Hz, 1H), 7.78 (s, 1H), 7.47 (d, *J =* 8.1 Hz, 2H), 7.33 (ddd, *J* = 7.4, 4.8, 1.2 Hz, 1H), 5.34 - 5.28 (m, 2H), 4.59 (d, *J* = 6.3 Hz, 2H), 3.08 (dd, *J* = 12.3, 5.7 Hz, 1H), 2.89 - 2.70 (m, 3H), 2.06 - 1.94 (m, 1H), 1.87 - 1.79 (m, 1H), 1.76 - 1.67 (m, 1H), 0.89 - 0.76 (m, 4H). ¹H under water.

### Synthesis 077

### (R)-3-cyclopropyl-5-(piperidin-3-yloxy)-N-(4-(pyridin-2-yl)benzyl)pyrazolo[1,5-a]pyrimidin-7-amine

### (PPA-061)

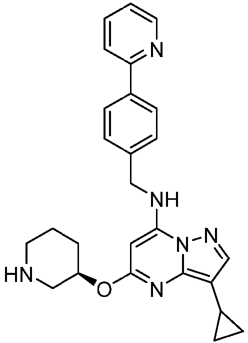

### Step 3:

### tert-butyl (R)-3-((7-((tert-butoxycarbonyl)(4-(pyridin-2-yl)benzyl)amino)-3-cyclopropylpyrazolo[1,5-a]pyrimidin-5-yl)oxy)piperidine-1-carboxylate

A suspension of tert-butyl (5-chloro-3-cyclopropylpyrazolo[1,5-a]pyrimidin-7-yl)(4-(pyridin-2-yl)benzyl)carbamate (103 mg, 216 µmol) (prepared as described herein), tert-butyl (R)-3-hydroxypiperidine-1-carboxylate (183 mg, 866 µmol) and cesium carbonate (282 mg, 866 µmol) in dioxane (2.6 mL) was degassed with N₂ for 5 min. Pd-171 (14.4 mg, 21.6 µmol) and Ruphos (10.1 mg, 21.6 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-100% (EtOH in EtOAc 25:75)/ isohexane) gave the title compound (202 mg, 88 µmol, 41% yield, 28% purity) as a yellow oil.

UPLC/MS (Method 2): m/z 641 (M+H)⁺, RT 2.56 min.

### Step 4:

### (R)-3-cyclopropyl-5-(piperidin-3-yloxy)-N-(4-(pyridin-2-yl)benzyl)pyrazolo[1,5-a]pyrimidin-7-amine

Hydrogen chloride (4 M in dioxane) (0.88 mL, 3.5 mmol) was added to a solution of tert-butyl (R)-3-((7-((tert-butoxycarbonyl)(4-(pyridin-2-yl)benzyl)amino)-3-cyclopropylpyrazolo[1,5-a]pyrimidin-5-yl)oxy)piperidine-1-carboxylate (202 mg, 88 µmol) in dioxane (2.0 mL). The reaction mixture was stirred at 40 °C for 2 h and concentrated *in vacuo.* Hydrogen chloride (1.25 M in MeOH) (2.0 mL, 2.50 mmol) was added and the resulting reaction mixture was stirred at 40 °C for 1 h then concentrated *in vacuo.* The solid was triturated in MeCN/Et₂O 1:1 (3 mL) and the solid was loaded onto a column of SCX. The column was washed with MeOH (80 mL) and the product was eluted with 0.7 M NH₃ in MeOH (100 mL). The ammoniacal methanol solution was concentrated *in vacuo* to give the title compound (37 mg, 82 µmol, 93% yield, 99% purity) as a white solid.

UPLC/MS (Method 3): m/z 441 (M+H)⁺, RT 1.68 min.

¹H NMR (400 MHz, DMSO-d₆) δ 8.66 - 8.62 (m, 1H), 8.34 (t, *J =* 6.4 Hz, 1H), 8.04 (d, *J* = 8.3 Hz, 2H), 7.95 - 7.91 (m, 1H), 7.86 (td, *J =* 7.7, 1.9 Hz, 1H), 7.76 (s, 1H), 7.48 (d, *J =* 8.1 Hz, 2H), 7.33 (ddd, *J* = 7.3, 4.8, 1.2 Hz, 1H), 5.31 (s, 1H), 4.94 - 4.86 (m, 1H), 4.59 (d, *J* = 6.5 Hz, 2H), 3.10 (dd, *J =* 11.9, 3.9 Hz, 1H), 2.79 - 2.71 (m, 1H), 2.48 - 2.39 (m, 1H), 2.06 - 1.98 (m, 1H), 1.88 - 1.78 (m, 1H), 1.67 - 1.58 (m, 1H), 1.51 - 1.37 (m, 2H), 0.87 - 0.76 (m, 4H). 2H under water.

### Synthesis 078

### 3-cyclopropyl-N⁵-((4-fluoropiperidin-4-yl)methyl)-N⁷-(4-(pyridin-2-yl)benzyl)pyrazolo[1,5-a]pyrimidine-5,7-diamine

### (PPA-062)

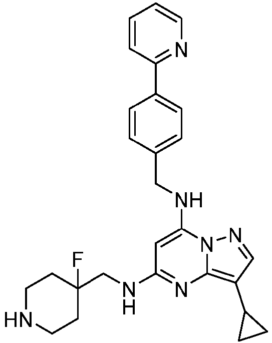

### Step 3:

### tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-4-fluoropiperidine-1-carboxylate

A solution of tert-butyl (5-chloro-3-cyclopropylpyrazolo[1,5-a]pyrimidin-7-yl)(4-(pyridin-2-yl)benzyl)carbamate (101 mg, 200 µmol) (prepared as described herein), tert-butyl 4-(aminomethyl)-4-fluoropiperidine-1-carboxylate (56 mg, 240 µmol) in THF (2.0 mL) was degassed in N₂ for 5 min before adding ^{t}BuBrettPhos Pd G3 (17 mg, 20.0 µmol) and LiHMDS (1M in THF) (240 µL, 240 µmol). The reaction mixture was degassed for another 5 min before being heated to 60 °C for 6 h. At RT, the reaction mixture was diluted with EtOAc (10 mL) and filtered through celite, rinsing with EtOAc (15 mL). The filtrate was concentrated *in vacuo.* Purification by column chromatography (12 g cartridge, 0-100% (EtOH in EtOAc 25:75)/isohexane) gave the title compound (92 mg, 0.13 mmol, 65% yield, 95% purity) as a yellow oil.

UPLC/MS (Method 3): m/z 672 (M+H)⁺, RT 2.13 min.

### Step 4:

### 3-cyclopropyl-N⁵-((4-fluoropiperidin-4-yl)methyl)-N⁷-(4-(pyridin-2-yl)benzyl)pyrazolo[1,5-a]pyrimidine-5,7-diamine

Hydrogen chloride (4 M in dioxane) (0.64 mL, 2.6 mmol) was added to a solution of tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-4-fluoropiperidine-1-carboxylate (91 mg, 0.13 mmol) in dioxane (2.0 mL). The reaction mixture was stirred at 40 °C for 2 h and concentrated *in vacuo.* Hydrogen chloride (1.25 M in MeOH) (2.0 mL, 2.50 mmol) was added and the resulting reaction mixture was stirred at 40 °C for 1.5 h then concentrated *in vacuo.* The solid was triturated in MeCN (3 mL) and the solid was loaded onto a column of SCX. The column was washed with MeOH (80 mL) and the product was eluted with 0.7 M NH₃ in MeOH (100 mL). The ammoniacal methanol solution was concentrated *in vacuo* and the solid was triturated in Et₂O (3 x 1 mL) to give the title compound (47 mg, 96 µmol, 75% yield, 97% purity) as an off-white solid.

UPLC/MS (Method 3): m/z 472 (M+H)⁺, RT 1.48 min.

¹H NMR (400 MHz, DMSO-d₆) δ 8.67 - 8.62 (m, 1H), 8.05 (d, *J =* 8.3 Hz, 2H), 7.93 (dt, *J* = 8.0, 1.2 Hz, 1H), 7.89 - 7.83 (m, 2H), 7.55 (s, 1H), 7.46 (d, *J =* 8.1 Hz, 2H), 7.33 (ddd, *J =* 7.4, 4.8, 1.2 Hz, 1H), 6.76 (t, *J =* 6.1 Hz, 1H), 5.29 (s, 1H), 4.49 (d, *J* = 6.4 Hz, 2H), 3.51 (dd, *J =* 21.1, 6.1 Hz, 2H), 2.78 - 2.60 (m, 4H), 1.81 - 1.70 (m, 1H), 1.67 - 1.45 (m, 4H), 0.80 - 0.70 (m, 4H). ¹H under water.

### Synthesis 079

### 3-cyclopropyl-N⁵-((4-methylpiperidin-4-yl)methyl)-N⁷-(4-(pyridin-2-yl)benzyl)pyrazolo[1,5-a]pyrimidine-5,7-diamine

### (PPA-063)

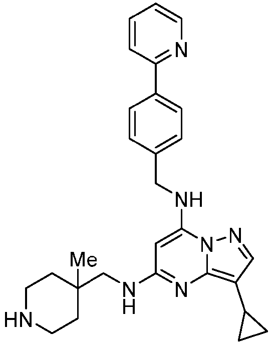

### Step 3:

### tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-4-methylpiperidine-1-carboxylate

A solution of tert-butyl (5-chloro-3-cyclopropylpyrazolo[1,5-a]pyrimidin-7-yl)(4-(pyridin-2-yl)benzyl)carbamate (101 mg, 200 µmol) (prepared as described herein), tert-butyl 4-(aminomethyl)-4-methylpiperidine-1-carboxylate (57 mg, 240 µmol) in THF (2.0 mL) was degassed in N₂ for 5 min before adding ^{t}BuBrettPhos Pd G3 (17.1 mg, 20.0 µmol) and LiHMDS (1M in THF) (240 µL, 240 µmol). The reaction was degassed for another 5 min before being heated to 60 °C for 6 h. The reaction mixture was recharged with more tert-butyl 4-(aminomethyl)-4-methylpiperidine-1-carboxylate (23 mg, 100 µmol), ^{t}BuBrettPhos Pd G3 (17.1 mg, 20.0 µmol) and LiHMDS (1M in THF) (100 µL, 100 µmol). The reaction was degassed for another 5 min before being heated to 60 °C for 2.5 h. At RT, the reaction mixture was diluted with EtOAc (10 mL) and filtered through celite, rinsing with EtOAc (15 mL). The filtrate was concentrated *in vacuo.* Purification by column chromatography (12 g cartridge, 0-100% (EtOH in EtOAc 25:75)/isohexane) gave the title compound (78 mg, 0.11 mmol, 56% yield, 97% purity) as a tan oil.

UPLC/MS (Method 3): m/z 668 (M+H)⁺, R_{T} 2.22 min.

### Step 4:

### 3-cyclopropyl-N⁵-((4-methylpiperidin-4-yl)methyl)-N⁷-(4-(pyridin-2-yl)benzyl)pyrazolo[1,5-a]pyrimidine-5,7-diamine

Hydrogen chloride (4 M in dioxane) (0.56 mL, 2.2 mmol) was added to a solution of tert-butyl 4-(((7-((tert-butoxycarbonyl)(4-(pyridin-2-yl)benzyl)amino)-3-cyclopropylpyrazolo[1,5-a]pyrimidin-5-yl)amino)methyl)-4-methylpiperidine-1-carboxylate (77 mg, 0.11 mmol) in dioxane (2.0 mL). The reaction mixture was stirred at 40 °C for 1.5 h and concentrated *in vacuo.* Hydrogen chloride (1.25 M in MeOH) (2.0 mL, 2.50 mmol) was added and the resulting reaction mixture was stirred at 40 °C for 30 min then concentrated *in vacuo.* The solid was triturated in MeCN (3 mL) and the solid was loaded onto a column of SCX. The column was washed with DCM/MeOH 30/70 (80 mL) and the product was eluted with 0.7 M NH₃ in MeOH (100 mL). The ammoniacal methanol solution was concentrated *in vacuo* and the solid was triturated in Et₂O (3 x 1 mL) to give the title compound (42 mg, 85 µmol, 76% yield, 96% purity) as a yellow solid.

UPLC/MS (Method 3): m/z 468 (M+H)⁺, RT 1.95 min.

¹H NMR (400 MHz, DMSO-d₆) δ 8.67 - 8.60 (m, 1H), 8.05 (d, *J =* 8.1 Hz, 2H), 7.93 (d, *J* = 8.0 Hz, 1H), 7.89 - 7.83 (m, 1H), 7.77 (t, *J* = 6.6 Hz, 1H), 7.53 (s, 1H), 7.45 (d, *J* = 8.0 Hz, 2H), 7.33 (dd, *J* = 7.2, 4.9 Hz, 1H), 6.51 (t, *J* = 6.1 Hz, 1H), 5.24 (s, 1H), 4.50 (d, *J* = 6.4 Hz, 2H), 3.18 (d, *J* = 5.7 Hz, 2H), 2.80 - 2.65 (m, 2H), 2.63 - 2.54 (m, 2H), 1.81 - 1.71 (m, 1H), 1.40 - 1.28 (m, 2H), 1.18 - 1.09 (m, 2H), 0.86 (s, 3H), 0.80 - 0.71 (m, 4H). 1H under water.

### Synthesis 080

### (S)-3-cyclopropyl-5-(piperidin-3-yloxy)-N-(4-(pyridin-2-yl)benzyl)pyrazolo[1,5-a]pyrimidin-7-amine

### (PPA-064)

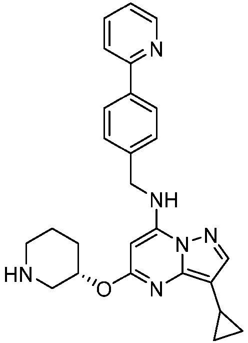

### Step 3:

### tert-butyl (S)-3-((7-((tert-butoxycarbonyl)(4-(pyridin-2-yl)benzyl)amino)-3-cyclopropylpyrazolo[1,5-a]pyrimidin-5-yl)oxy)piperidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 216 µmol) (prepared as described herein), tert-butyl (S)-3-hydroxypiperidine-1-carboxylate (178 mg, 841 µmol) and cesium carbonate (274 mg, 841 µmol) in dioxane (2.6 mL) was degassed with N₂ for 5 min. Pd-171 (13.9 mg, 21.0 µmol) and Ruphos **(9.8 mg, 21.0 µmol)** were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (110 mg, 140 µmol, 66% yield, 81% purity) as a yellow oil.

UPLC/MS (Method 2): m/z 641 (M+H)⁺, RT 2.56 min.

### Step 4:

### (S)-3-cyclopropyl-5-(piperidin-3-yloxy)-N-(4-(pyridin-2-yl)benzyl)pyrazolo[1,5-a]pyrimidin-7-amine

Hydrogen chloride (4 M in dioxane) (1.72 mL, 6.9 mmol) was added to a solution of tert-butyl (S)-3-((7-((tert-butoxycarbonyl)(4-(pyridin-2-yl)benzyl)amino)-3-cyclopropylpyrazolo[1,5-a]pyrimidin-5-yl)oxy)piperidine-1-carboxylate (110 mg, 172 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* to give the title compound (53 mg, 120 µmol, 68% yield, 97% purity) as a white solid.

UPLC/MS (Method 3): m/z 441 (M+H)⁺, RT 1.21 min.

¹H NMR (400 MHz, DMSO-d₆) δ 8.67 - 8.61 (m, 1H), 8.34 (t, *J =* 6.5 Hz, 1H), 8.04 (d, *J =* 8.1 Hz, 2H), 7.93 (d, *J* = 8.0 Hz, 1H), 7.86 (td, *J =* 7.7, 1.9 Hz, 1H), 7.76 (s, 1H), 7.48 (d, *J* = 8.1 Hz, 2H), 7.37 - 7.29 (m, 1H), 5.31 (s, 1H), 4.93 - 4.84 (m, 1H), 4.59 (d, *J =* 6.5 Hz, 2H), 3.13 - 3.05 (m, 1H), 2.77 - 2.68 (m, 1H), 2.43 (ddd, *J* = 16.4, 12.0, 8.9 Hz, 1H), 2.24 - 2.13 (m, 1H), 2.03 - 1.99 (m, 1H), 1.88 - 1.77 (m, 1H), 1.66 - 1.58 (m, 1H), 1.51 - 1.37 (m, 2H), 0.87 - 0.75 (m, 4H). ¹H under water.

### Synthesis 081

### (S)-3-cyclopropyl-N-(4-(pyridin-2-yl)benzyl)-5-(pyrrolidin-3-yloxy)pyrazolo[1,5-a]pyrimidin-7-amine

### (PPA-065)

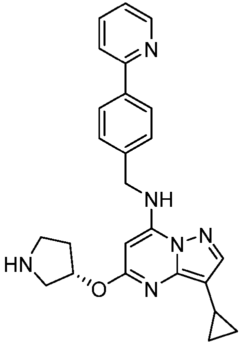

### Step 3:

### tert-butyl (S)-3-((7-((tert-butoxycarbonyl)(4-(pyridin-2-yl)benzyl)amino)-3-cyclopropylpyrazolo[1,5-a]pyrimidin-5-yl)oxy)pyrrolidine-1-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl (S)-3-hydroxypyrrolidine-1-carboxylate (157 mg, 840 µmol) and cesium carbonate (274 mg, 840 µmol) in dioxane (3.0 mL) was degassed with N₂ for 5 min. Pd-171 (13.9 mg, 21.0 µmol) and Ruphos (9.8 mg, 21.0 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (120 mg, 160 µmol, 74% yield, 81% purity) as a yellow oil.

UPLC/MS (Method 4): m/z 627 (M+H)⁺, RT 2.49 min.

### Step 4:

### (S)-3-cyclopropyl-N-(4-(pyridin-2-yl)benzyl)-5-(pyrrolidin-3-yloxy)pyrazolo[1,5-a]pyrimidin-7-amine

Hydrogen chloride (4 M in dioxane) (1.91 mL, 7.7 mmol) was added to a solution of tert-butyl (S)-3-((7-((tert-butoxycarbonyl)(4-(pyridin-2-yl)benzyl)amino)-3-cyclopropylpyrazolo[1,5-a]pyrimidin-5-yl)oxy)pyrrolidine-1-carboxylate (120 mg, 191 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (30 mL) and the product was eluted with 0.7 M NH₃ in MeOH (30 mL). The ammoniacal methanol solution was concentrated *in vacuo* to give the title compound (58 mg, 130 µmol, 69% yield, 97% purity) as an off-white solid.

UPLC/MS (Method 2): m/z 427 (M+H)⁺, RT 1.17 min.

¹H NMR (400 MHz, DMSO-d₆) δ 8.67 - 8.61 (m, 1H), 8.35 (t, *J =* 6.5 Hz, 1H), 8.04 (d, *J =* 8.1 Hz, 2H), 7.92 (d, *J* = 8.0 Hz, 1H), 7.86 (td, *J =* 7.7, 1.8 Hz, 1H), 7.78 (s, 1H), 7.47 (d, *J* = 8.2 Hz, 2H), 7.37 - 7.29 (m, 1H), 5.33 - 5.28 (m, 2H), 4.59 (d, *J* = 6.4 Hz, 2H), 3.07 (dd, *J =* 12.3, 5.8 Hz, 1H), 2.88 - 2.78 (m, 1H), 2.78 - 2.68 (m, 2H), 2.06 - 1.92 (m, 1H), 1.89 - 1.78 (m, 1H), 1.75 - 1.66 (m, 1H), 0.86 - 0.79 (m, 4H). 1H under water.

### Synthesis 089

### (S)-3-cyclopropyl-N⁷-(2-fluoro-4-(pyridin-2-yl)benzyl)-N⁵-(pyrrolidin-3-yl)pyrazolo[1,5-a]pyrimidine-5,7-diamine

### (PPA-072)

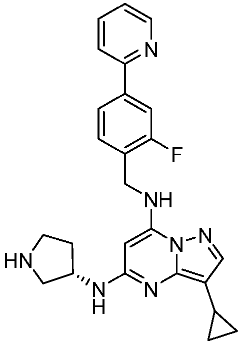

### Step 3:

### tert-butyl (S)-3-((7-((tert-butoxycarbonyl)(2-fluoro-4-(pyridin-2-yl)benzyl)amino)-3-cyclopropylpyrazolo[1,5-a]pyrimidin-5-yl)amino)pyrrolidine-1-carboxylate

A suspension of tert-butyl (5-chloro-3-cyclopropylpyrazolo[1,5-a]pyrimidin-7-yl)(2-fluoro-4-(pyridin-2-yl)benzyl)carbamate (100 mg, 202 µmol) (prepared as described herein), tert-butyl (S)-3-aminopyrrolidine-1-carboxylate (155 mg, 810 µmol) and cesium carbonate (264 mg, 810 µmol) in dioxane (2.6 mL) was degassed with N₂ for 5 min. Pd-171 (13.4 mg, 20.2 µmol) and Ruphos (9.5 mg, 20.2 µmol) were added and N₂ was bubbled through the reaction mixture for another 5 min. The reaction mixture was stirred at 90 °C for 6 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-10% MeOH (containing 0.7 M NH₃)/DCM) gave the title compound (90 mg, 0.13 mmol, 63% yield, 91% purity) as a yellow oil.

UPLC/MS (Method 4): m/z 644 (M+H)⁺, RT 2.49 min.

### Step 4:

### (S)-3-cyclopropyl-N⁷-(2-fluoro-4-(pyridin-2-yl)benzyl)-N⁵-(pyrrolidin-3-yl)pyrazolo[1,5-a]pyrimidine-5,7-diamine

Hydrogen chloride (4 M in dioxane) (1.4 mL, 5.6 mmol) was added to a solution of tert-butyl (S)-3-((7-((tert-butoxycarbonyl)(2-fluoro-4-(pyridin-2-yl)benzyl)amino)-3-cyclopropylpyrazolo[1,5-a]pyrimidin-5-yl)amino)pyrrolidine-1-carboxylate (90 mg, 0.14 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (18 mg, 39 µmol, 28% yield, 97% purity) as a white solid.

UPLC/MS (Method 4): m/z 444 (M+H)⁺, RT 1.10 min.

¹H NMR (400 MHz, DMSO-d₆) δ 8.69 - 8.63 (m, 1H), 7.99 (d, *J =* 8.0 Hz, 1H), 7.95 - 7.86 (m, 3H), 7.84 (t, *J =* 6.3 Hz, 1H), 7.58 (s, 1H), 7.45 - 7.34 (m, 2H), 6.77 (d, *J* = 5.9 Hz, 1H), 5.09 (s, 1H), 4.55 (d, *J* = 6.3 Hz, 2H), 4.25 - 4.20 (m, 1H), 3.16 - 3.07 (m, 1H), 2.98 - 2.91 (m, 1H), 2.91 - 2.83 (m, 1H), 2.73 - 2.65 (m, 1H), 2.06 - 1.96 (m, 1H), 1.83 - 1.71 (m, 1H), 1.66 - 1.54 (m, 1H), 0.83 - 0.71 (m, 4H). ¹H under water.

### Synthesis 090

### 3-cyclopropyl-5-(((3R,4R)-4-fluoropyrrolidin-3-yl)oxy)-N-(4-(pyridin-2-yl)benzyl)pyrazolo[1,5-a]pyrimidin-7-amine /

### 3-cyclopropyl-5-(((3S,4S)-4-fluoropyrrolidin-3-yl)oxy)-N-(4-(pyridin-2-yl)benzyl)pyrazolo[1,5-a]pyrimidin-7-amine (1:1)

### (PPA-073)

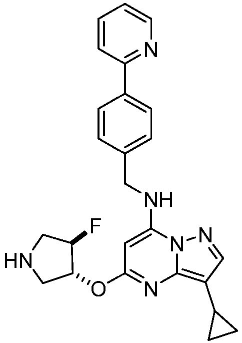

### Step 3:

### tert-butyl (3R,4R)-3-((7-((tert-butoxycarbonyl)(4-(pyridin-2-yl)benzyl)amino)-3-cyclopropylpyrazolo[1,5-a]pyrimidin-5-yl)oxy)-4-fluoropyrrolidine-1-carboxylate / tert-butyl (3S,4S)-3-((7-((tert-butoxycarbonyl)(4-(pyridin-2-yl)benzyl)amino)-3-cyclopropylpyrazolo[1,5-a]pyrimidin-5-yl)oxy)-4-fluoropyrrolidine-1-carboxylate (1:1)

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), racemic trans-tert-butyl 3-fluoro-4-hydroxypyrrolidine-1-carboxylate (178 mg, 841 µmol) and cesium carbonate (274 mg, 840 µmol) in dioxane (2.6 mL) was degassed with N₂ for 5 min. Pd-171 (13.9 mg, 21.0 µmol) and Ruphos (9.8 mg, 21.0 µmol) were added and N₂ was bubbled through the reaction mixture for another 5 min. The reaction mixture was stirred at 90 °C overnight then concentrated in vacuo. Purification by column chromatography (12 g cartridge, 0-10% MeOH (containing 0.7 M NH₃)/DCM) gave the title compound (120 mg, 0.15 mmol, 73% yield, 82% purity) as a yellow oil.

UPLC/MS (Method 2): m/z 645 (M+H)⁺, RT 2.52 min.

### Step 4:

### 3-cyclopropyl-5-(((3R,4R)-4-fluoropyrrolidin-3-yl)oxy)-N-(4-(pyridin-2-yl)benzyl)pyrazolo[1,5-a]pyrimidin-7-amine / 3-cyclopropyl-5-(((3S,4S)-4-fluoropyrrolidin-3-yl)oxy)-N-(4-(pyridin-2-yl)benzyl)pyrazolo[1,5-a]pyrimidin-7-amine (1:1)

Hydrogen chloride (4 M in dioxane) (1.9 mL, 7.4 mmol) was added to a solution of tert-butyl (3R,4R)-3-((7-((tert-butoxycarbonyl)(4-(pyridin-2-yl)benzyl)amino)-3-cyclopropylpyrazolo[1,5-a]pyrimidin-5-yl)oxy)-4-fluoropyrrolidine-1-carboxylate / tert-butyl (3S,4S)-3-((7-((tert-butoxycarbonyl)(4-(pyridin-2-yl)benzyl)amino)-3-cyclopropylpyrazolo[1,5-a]pyrimidin-5-yl)oxy)-4-fluoropyrrolidine-1-carboxylate (1:1) (120 mg, 186 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (78 mg, 0.17 mmol, 92% yield, 98% purity) as a white solid.

UPLC/MS (Method 4): m/z 445 (M+H)⁺, RT 1.20 min.

¹H NMR (400 MHz, DMSO-d₆) δ 8.64 (d, *J* = 4.8 Hz, 1H), 8.46 (t, *J* = 6.5 Hz, 1H), 8.04 (d, *J* = 8.1 Hz, 2H), 7.92 (d, *J =* 8.0 Hz, 1H), 7.86 (td, *J* = 7.7, 1.9 Hz, 1H), 7.82 (s, 1H), 7.47 (d, *J* = 8.0 Hz, 2H), 7.37 - 7.29 (m, 1H), 5.36 (s, 1H), 5.34 - 5.26 (m, 1H), 5.19 - 5.02 (m, 1H), 4.60 (d, *J =* 6.5 Hz, 2H), 3.36 (dd, *J* = 12.6, 6.0 Hz, 1H), 3.00 (d, *J* = 3.8 Hz, 1H), 2.92 (d, *J* = 3.1 Hz, 1H), 2.70 (dd, *J =* 12.7, 3.7 Hz, 1H), 1.90 - 1.79 (m, 1H), 0.92 - 0.78 (m, 4H). 1H under water.

### Synthesis 092

### (S)-4-((3-cyclopropyl-7-((4-(pyridin-2-yl)benzyl)amino)pyrazolo[1,5-a]pyrimidin-5-yl)amino)pyrrolidin-2-one

### (PPA-075)

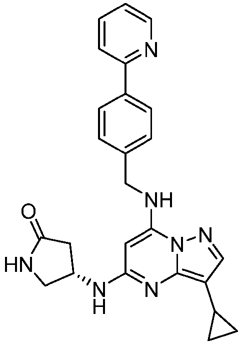

### Step 3:

### tert-butyl (S)-(3-cyclopropyl-5-((5-oxopyrrolidin-3-yl)amino)pyrazolo[1,5-a]pyrimidin-7-yl)(4-(pyridin-2-yl)benzyl)carbamate

A solution of tert-butyl (5-chloro-3-cyclopropylpyrazolo[1,5-a]pyrimidin-7-yl)(4-(pyridin-2-yl)benzyl)carbamate (120 mg, 252 µmol) (prepared as described herein), (S)-4-aminopyrrolidin-2-one (114 mg, 1.13 mmol) in THF (2.0 mL) was degassed in N₂ for 5 min before adding ^{t}BuBrettPhos Pd G3 (21.5 mg, 25.2 µmol) and LiHMDS (1M in THF) (378 µL, 378 µmol). The reaction was degassed for another 5 min before being heated to 60 °C for 5 h. The reaction mixture was concentrated *in vacuo.* Purification by column chromatography (12 g cartridge, 0-10% MeOH (containing 0.7 M NH₃)/DCM) gave the title compound (90 mg, 0.16 mmol, 65% yield, 98% purity) as a yellow solid.

UPLC/MS (Method 2): m/z 540 (M+H)⁺, R_{T} 1.68 min.

### Step 4:

### (S)-4-((3-cyclopropyl-7-((4-(pyridin-2-yl)benzyl)amino)pyrazolo[1,5-a]pyrimidin-5-yl)amino)pyrrolidin-2-one

Hydrogen chloride (4 M in dioxane) (1.70 mL, 6.7 mmol) was added to a solution of tert-butyl (S)-(3-cyclopropyl-5-((5-oxopyrrolidin-3-yl)amino)pyrazolo[1,5-a]pyrimidin-7-yl)(4-(pyridin-2-yl)benzyl)carbamate (90 mg, 0.17 mmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (26 mg, 58 µmol, 35% yield, 98% purity) as a white solid.

UPLC/MS (Method 2): m/z 440 (M+H)⁺, RT 1.07 min.

¹H NMR (400 MHz, DMSO-d₆) δ 8.67 - 8.61 (m, 1H), 8.05 (d, *J* = 8.1 Hz, 2H), 7.96 - 7.89 (m, 2H), 7.86 (td, *J* = 7.7, 1.9 Hz, 1H), 7.59 (s, 1H), 7.56 (s, 1H), 7.44 (d, *J* = 8.1 Hz, 2H), 7.33 (dd, *J* = 7.3, 4.8 Hz, 1H), 7.00 (d, *J* = 5.9 Hz, 1H), 5.11 (s, 1H), 4.50 (d, *J* = 6.4 Hz, 2H), 4.48 - 4.40 (m, 1H), 3.56 (dd, *J* = 9.9, 6.8 Hz, 1H), 3.04 (dd, *J* = 9.9, 4.5 Hz, 1H), 2.04 (dd, *J* = 16.7, 5.6 Hz, 1H), 1.82 - 1.71 (m, 1H), 0.85 - 0.72 (m, 4H). 1H under water.

### Synthesis 095

### 3-cyclopropyl-N-(4-(pyridin-2-yl)benzyl)-5-(4,7-diazaspiro[2.5]octan-7-yl)pyrazolo[1,5-a]pyrimidin-7-amine

### (PPA-077)

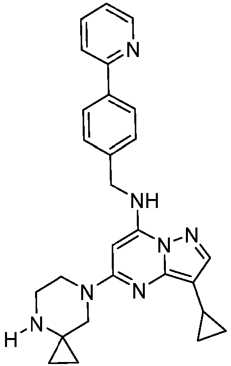

### Step 3:

### tert-butyl 7-(7-((tert-butoxycarbonyl)(4-(pyridin-2-yl)benzyl)amino)-3-cyclopropylpyrazolo[1,5-a]pyrimidin-5-yl)-4,7-diazaspiro[2.5]octane-4-carboxylate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), tert-butyl 4-carbamoylpiperidine-1-carboxylate (48 mg, 210 µmol) and cesium carbonate (274 mg, 840 µmol) in dioxane (2.6 mL) was degassed with N₂ for 5 min. Pd-171 (13.9 mg, 21.0 µmol) and Ruphos (9.8 mg, 21.0 µmol) were added and N₂ was bubbled through the reaction mixture for another 5 min. The reaction mixture was concentrated *in vacuo.* Purification by column chromatography (12 g cartridge, 0-10% MeOH (containing 0.7 M NH₃)/DCM) gave the title compound (60 mg, 88 µmol, 42% yield, 98% purity) as a yellow solid.

UPLC/MS (Method 2): m/z 668 (M+H)⁺, RT 2.36 min.

### Step 4:

### 3-cyclopropyl-N-(4-(pyridin-2-yl)benzyl)-5-(4,7-diazaspiro[2.5]octan-7-yl)pyrazolo[1,5-a]pyrimidin-7-amine

Hydrogen chloride (4 M in dioxane) (1.20 mL, 4.9 mmol) was added to a solution of tert-butyl 7-(7-((tert-butoxycarbonyl)(4-(pyridin-2-yl)benzyl)amino)-3-cyclopropylpyrazolo[1,5-a]pyrimidin-5-yl)-4,7-diazaspiro[2.5]octane-4-carboxylate (60 mg, 92 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo.* Purification by column chromatography (4 g cartridge, 0-10% MeOH (containing 0.7 M NH₃)/DCM) gave the title compound (17 mg, 37 µmol, 40% yield, 97% purity) as a yellow solid.

UPLC/MS (Method 2): m/z 452 (M+H)⁺, RT 1.17 min.

¹H NMR (400 MHz, DMSO-d₆) δ 8.67 - 8.61 (m, 1H), 8.03 (d, *J =* 8.3 Hz, 2H), 7.95 - 7.87 (m, 2H), 7.86 (td, *J =* 7.6, 1.8 Hz, 1H), 7.58 (s, 1H), 7.50 (d, *J =* 8.2 Hz, 2H), 7.37 - 7.29 (m, 1H), 5.45 (s, 1H), 4.59 (d, *J =* 6.4 Hz, 2H), 3.51 - 3.44 (m, 2H), 2.77 - 2.71 (m, 2H), 1.76 (tt, *J =* 8.1, 5.4 Hz, 1H), 0.80 - 0.68 (m, 4H), 0.42 - 0.32 (m, 4H). 3H under water.

### Synthesis 096

### (S)-4-amino-1-(3-cyclopropyl-7-((4-(pyridin-2-yl)benzyl)amino)pyrazolo[1,5-a]pyrimidin-5-yl)pyrrolidin-2-one

### (PPA-078)

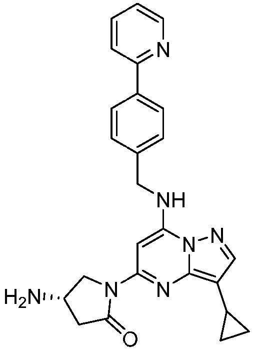

### Step 3:

### tert-butyl (S)-(5-(4-amino-2-oxopyrrolidin-1-yl)-3-cyclopropylpyrazolo[1,5-a]pyrimidin-7-yl)(4-(pyridin-2-yl)benzyl)carbamate

A suspension of tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein), (S)-4-aminopyrrolidin-2-one (84 mg, 840 µmol) and cesium carbonate (274 mg, 840 µmol) in dioxane (2.6 mL) was degassed with N₂ for 5 min. Pd-171 (13.6 mg, 21.0 µmol) and Ruphos (9.8 mg, 21.0 µmol) were added and N₂ was bubbled through the reaction mixture for another 5 min. The reaction mixture was stirred at 90 °C for 6 h. The reaction mixture was concentrated *in vacuo.* Purification by column chromatography (12 g cartridge, 0-10% MeOH (containing 0.7 M NH₃)/DCM) gave the title compound (40 mg, 73 µmol, 35% yield, 98% purity) as a yellow solid.

UPLC/MS (Method 2): m/z 540 (M+H)⁺, RT 1.36 min.

### Step 4:

### (S)-4-amino-1-(3-cyclopropyl-7-((4-(pyridin-2-yl)benzyl)amino)pyrazolo[1,5-a]pyrimidin-5-yl)pyrrolidin-2-one

Hydrogen chloride (4 M in dioxane) (0.74 mL, 3.0 mmol) was added to a solution of tert-butyl (S)-(5-(4-amino-2-oxopyrrolidin-1-yl)-3-cyclopropylpyrazolo[1,5-a]pyrimidin-7-yl)(4-(pyridin-2-yl)benzyl)carbamate (40 mg, 74 µmol) in dioxane (2.0 mL). The reaction mixture was stirred at 35 °C for 4 h and concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (18 mg, 41 µmol, 55% yield, 99% purity) as a white solid.

UPLC/MS (Method 2): m/z 440 (M+H)⁺, RT 1.07 min.

¹H NMR (400 MHz, DMSO-d₆) δ 8.66 - 8.62 (m, 1H), 8.52 (t, *J =* 6.4 Hz, 1H), 8.04 (d, *J =* 8.3 Hz, 2H), 7.93 (d, *J =* 8.0 Hz, 1H), 7.87 (dd, *J =* 7.6, 1.9 Hz, 1H), 7.83 (s, 1H), 7.47 (d, *J =* 8.3 Hz, 2H), 7.36 - 7.30 (m, 1H), 7.20 (s, 1H), 4.58 (d, *J =* 6.3 Hz, 2H), 3.99 (dd, *J =* 11.4, 6.1 Hz, 1H), 3.71 (dd, *J =* 11.3, 3.4 Hz, 1H), 3.59 - 3.52 (m, 1H), 2.75 (dd, *J =* 16.8, 6.9 Hz, 1H), 2.21 (dd, *J =* 16.8, 3.9 Hz, 1H), 2.09 - 1.93 (m, 2H), 1.92 - 1.83 (m, 1H), 0.84 (d, *J =* 8.5 Hz, 4H).

### Synthesis 097

### 3-cyclopropyl-5-(1H-pyrazol-5-yl)-N-(4-(pyridin-2-yl)benzyl)pyrazolo[1,5-a]pyrimidin-7-amine

### (PPA-079)

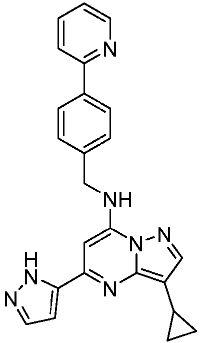

### Step 3:

### tert-butyl (3-cyclopropyl-5-(1H-pyrazol-5-yl)pyrazolo[1,5-a]pyrimidin-7-yl)(4-(pyridin-2-yl)benzyl)carbamate

A mixture of 3-(tetramethyl-1,3,2-dioxaborolan-2-yl)-1H-pyrazole (44 mg, 227 µmol), tert-butyl (5-chloro-3-cyclopropylpyrazolo[1,5-a]pyrimidin-7-yl)(4-(pyridin-2-yl)benzyl)carbamate (90 mg, 189 µmol) (prepared as described herein) and Pd(dppf)Cl₂ (27.7 mg, 37.8 µmol) in dioxane (3.0 mL) was sparged with N₂ for 5 min. A solution of Potassium phosphate, tribasic (120 mg, 567 µmol) in water (0.75 mL) was added and the reaction mixture was sparged with N₂ for 5 min. The mixture was heated to 90 °C for 6 h. At RT, the reaction mixture was diluted with EtOAc (10 mL) and filtered through celite, rinsing with EtOAc (15 mL). The filtrate was concentrated *in vacuo.* Purification by column chromatography (12 g cartridge, 0-100% (EtOH in EtOAc 25:75)/isohexane) gave the title compound (102 mg, 0.19 mmol, 76% yield, 96% purity) as a yellow oil.

UPLC/MS (Method 3): m/z 508 (M+H)⁺, R_{T} 1.79 min.

### Step 4:

### 3-cyclopropyl-5-(1H-pyrazol-5-yl)-N-(4-(pyridin-2-yl)benzyl)pyrazolo[1,5-a]pyrimidin-7-amine

Hydrogen chloride (4 M in dioxane) (0.96 mL, 3.8 mmol) was added to a solution of tert-butyl (3-cyclopropyl-5-(1H-pyrazol-5-yl)pyrazolo[1,5-a]pyrimidin-7-yl)(4-(pyridin-2-yl)benzyl)carbamate (101 mg, 191 µmol) in dioxane (2.0 mL). The reaction mixture was stirred at 40 °C for 1.5 h then hydrogen chloride (125 M in MeOH) (2.0 mL, 2.5 mmol) was added and the reaction mixture was stirred at 40 °C for 2 h then concentrated *in vacuo.* The solid was triturated in MeCN (3 mL) and the solid was loaded onto a column of SCX. The column was washed with MeOH (20 mL) and the product was eluted with 0.7 M NH₃ in MeOH (20 mL). The ammoniacal methanol solution was concentrated *in vacuo* and the solid was triturated in Et₂O (2 mL) to give the title compound (63 mg, 151 µmol, 80% yield, 99% purity) as a tan solid.

UPLC/MS (Method 3): m/z 408 (M+H)⁺, RT 1.42 min.

¹H NMR (400 MHz, DMSO-d₆) - 0.80/0.20 mixture of tautomers - δ 13.43 (s, 0.2H), 12.99 (s, 0.8H), 8.69 - 8.59 (m, 1.2H), 8.58 - 8.45 (m, 0.8H), 8.09 - 8.01 (m, 2H), 7.96 - 7.81 (m, 3.2H), 7.78 (s, 0.8H), 7.61 - 7.45 (m, 2H), 7.32 (ddd, *J* = 7.3, 4.8, 1.2 Hz, 1H), 6.91 - 6.74 (m, 1H), 6.57 (s, 1H), 4.72 (d, *J* = 6.4 Hz, 2H), 2.09 - 1.93 (m, 1H), 0.97 - 0.79 (m, 4H).

¹H NMR (400 MHz, DMSO-d₆) - VT at 90 °C - δ 13.40 - 12.40 (m, 1H), 8.69 - 8.59 (m, 1H), 8.15 - 8.00 (m, 3H), 7.90 - 7.79 (m, 3H), 7.75 - 7.61 (m, 1H), 7.54 (d, *J* = 8.0 Hz, 2H), 7.30 (ddd, *J* = 7.3, 4.8, 1.4 Hz, 1H), 6.82 (d, *J =* 2.1 Hz, 1H), 6.61 (s, 1H), 4.75 (d, *J =* 6.4 Hz, 2H), 2.09 - 1.98 (m, 1H), 0.97 - 0.85 (m, 4H).

### Synthesis 098

### 5-(3,6-diazabicyclo[3.1.1]heptan-3-yl)-3-cyclopropyl-N-(4-(pyridin-2-yl)benzyl)pyrazolo[1,5-a]pyrimidin-7-amine

### (PPA-080)

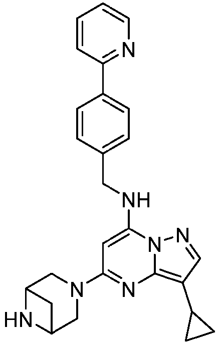

### Step 3:

### tert-butyl 3-(7-((tert-butoxycarbonyl)(4-(pyridin-2-yl)benzyl)amino)-3-cyclopropylpyrazolo[1,5-a]pyrimidin-5-yl)-3,6-diazabicyclo[3.1.1]heptane-6-carboxylate

A suspension of tert-butyl (5-chloro-3-cyclopropylpyrazolo[1,5-a]pyrimidin-7-yl)(4-(pyridin-2-yl)benzyl)carbamate (106 mg, 209 µmol) (prepared as described herein), 6-(tert-Butyloxycarbonyl)-3,6-diazabicyclo[3.1.1]heptane (87.38 mg, 419 µmol) and cesium carbonate (136 mg, 419 µmol) in dioxane (2.7 mL) was degassed with N₂ for 5 min. Pd-171 (20.8 mg, 31.4 µmol) and Ruphos (14.7 mg, 31.4 µmol) were added and N₂ was bubbled through the reaction mixture for another 5 min. The reaction mixture was stirred at 90 °C for 8 h. At RT, the reaction mixture was diluted with EtOAc (10 mL) and filtered through celite, rinsing with EtOAc (20 mL). The filtrate was concentrated *in vacuo.* Purification by column chromatography (12 g cartridge, 0-100% (EtOH in EtOAc 25:75)/isohexane) gave the title compound (72 mg, 0.11 mmol, 50% yield, 94% purity) as a yellow oil.

UPLC/MS (Method 3): m/z 638 (M+H)⁺, RT 2.11 min.

### Step 4:

### 5-(3,6-diazabicyclo[3.1.1]heptan-3-yl)-3-cyclopropyl-N-(4-(pyridin-2-yl)benzyl)pyrazolo[1,5-a]pyrimidin-7-amine

Hydrogen chloride (4 M in dioxane) (0.56 mL, 2.2 mmol) was added to a solution of tert-butyl 3-(7-((tert-butoxycarbonyl)(4-(pyridin-2-yl)benzyl)amino)-3-cyclopropylpyrazolo[1,5-a]pyrimidin-5-yl)-3,6-diazabicyclo[3.1.1]heptane-6-carboxylate (71 mg, 0.11

mmol) in dioxane (2.0 mL). The reaction mixture was stirred at 40 °C for 1.5 h then hydrogen chloride (1.25 M in MeOH) (2.0 mL, 2.5 mmol). The reaction mixture was stirred at 40 °C for 1 h then concentrated *in vacuo.* The solid was triturated in MeCN (3 mL) and the solid was loaded onto a column of SCX. The column was washed with MeOH (80 mL) and the product was eluted with 0.7 M NH₃ in MeOH (100 mL). The ammoniacal methanol solution was concentrated *in vacuo.* Purification by RP preparative HPLC (30-100% MeCN/0.3% NH₃ in water) gave the title compound (17 mg, 39 µmol, 35% yield, 98% purity) as an off-white solid.

UPLC/MS (Method 5): m/z 438 (M+H)⁺, RT 1.22 min.

¹H NMR (400 MHz, DMSO-d₆) δ 8.64 (ddd, *J =* 4.8, 1.8, 0.9 Hz, 1H), 8.04 (d, *J =* 8.3 Hz, 2H), 7.99 (t, *J* = 6.6 Hz, 1H), 7.92 (dt, *J* = 8.1, 1.2 Hz, 1H), 7.86 (td, *J* = 7.7, 1.9 Hz, 1H), 7.60 (s, 1H), 7.51 (d, *J* = 8.3 Hz, 2H), 7.33 (ddd, *J* = 7.4, 4.8, 1.2 Hz, 1H), 5.31 (s, 1H), 4.61 (d, *J =* 6.4 Hz, 2H), 3.62 (d, *J* = 6.0 Hz, 2H), 3.59 - 3.48 (m, 4H), 2.47 - 2.40 (m, 1H), 1.83 - 1.73 (m, 1H), 1.67 - 1.58 (m, 1H), 1.38 (d, *J =* 8.5 Hz, 1H), 0.81 - 0.71 (m, 4H).

### Synthesis 102

### 3-cyclopropyl-N-(4-(pyridin-2-yl)benzyl)-5-(2,6-diazaspiro[3.3]heptan-2-yl)pyrazolo[1,5-a]pyrimidin-7-amine

### (PPA-084)

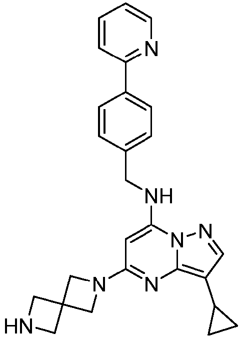

### Step 3:

### tert-butyl (3-cyclopropyl-5-(2,6-diazaspiro[3.3]heptan-2-yl)pyrazolo[1,5-a]pyrimidin-7-yl)(4-(pyridin-2-yl)benzyl)carbamate / tert-butyl 6-(7-((tert-butoxycarbonyl)(4-(pyridin-2-yl)benzyl)amino)-3-cyclopropylpyrazolo[1,5-a]pyrimidin-5-yl)-2,6-diazaspiro[3.3]heptane-2-carboxylate (1:0.24)

A suspension of tert-butyl (5-chloro-3-cyclopropylpyrazolo[1,5-a]pyrimidin-7-yl)(4-(pyridin-2-yl)benzyl)carbamate (80 mg, 168 µmol) (prepared as described herein), tert-butyl 2,6-diazaspiro[3.3]heptane-2-carboxylate, 0.5Oxalic acid (164 mg, 672 µmol) and cesium carbonate (438 mg, 1.34 mmol) in dioxane (2.6 mL) was degassed with N₂ for 5 min. Pd-171 (11.2 mg, 16.8 µmol) and Ruphos (7.8 mg, 16.8 µmol) were added and N₂ was bubbled through the reaction mixture for another 5 min. The reaction mixture was stirred at 90 °C for 6 h. The reaction mixture was concentrated *in vacuo.* Purification by column chromatography (12 g cartridge, 0-10% MeOH (containing 0.7 M NH₃)/DCM) gave a mixture of tert-butyl (3-cyclopropyl-5-(2,6-diazaspiro[3.3]heptan-2-yl)pyrazolo[1,5-a]pyrimidin-7-yl)(4-(pyridin-2-yl)benzyl)carbamate / tert-butyl 6-(7-((tert-butoxycarbonyl)(4-(pyridin-2-yl)benzyl)amino)-3-cyclopropylpyrazolo[1,5-a]pyrimidin-5-yl)-2,6-diazaspiro[3.3]heptane-2-carboxylate (1:0.24) (80 mg, 136 µmol, 84% yield, 98% purity) as a yellow solid.

UPLC/MS (Method 2): m/z 638 (M+H)⁺, RT 2.32 min; m/z 538 (M+H)+, RT 1.51 min.

### Step 4:

### 3-cyclopropyl-N-(4-(pyridin-2-yl)benzyl)-5-(2,6-diazaspiro[3.3]heptan-2-yl)pyrazolo[1,5-a]pyrimidin-7-amine

TFA (0.5 mL) was added to a mixture of tert-butyl (3-cyclopropyl-5-(2,6-diazaspiro[3.3]heptan-2-yl)pyrazolo[1,5-a]pyrimidin-7-yl)(4-(pyridin-2-yl)benzyl)carbamate / tert-butyl 6-(7-((tert-butoxycarbonyl)(4-(pyridin-2-yl)benzyl)amino)-3-cyclopropylpyrazolo[1,5-a]pyrimidin-5-yl)-2,6-diazaspiro[3.3]heptane-2-carboxylate (1:0.24) (80 mg, 136 µmol) in DCM (1.5 mL). The reaction mixture was stirred at RT for 4 h and concentrated *in vacuo.* The solid was loaded onto a column of SCX. The column was washed with MeOH (20 mL) and the product was eluted with 0.7 M NH₃ in MeOH (40 mL). The ammoniacal methanol solution was concentrated *in vacuo* and the solid was triturated in Et₂O (3 x 1 mL) to give the title compound (30 mg, 68 µmol, 54% yield, 99% purity) as a white solid.

UPLC/MS (Method 4): m/z 438 (M+H)⁺, RT 0.88 min.

¹H NMR (400 MHz, DMSO-d₆) δ 8.67 - 8.62 (m, 1H), 8.07 - 8.02 (m, 3H), 7.93 (d, *J =* 8.0 Hz, 1H), 7.86 (td, *J =* 7.6, 1.8 Hz, 1H), 7.58 (s, 1H), 7.48 (d, *J =* 8.2 Hz, 2H), 7.33 (ddd, *J =* 7.3, 4.8, 1.2 Hz, 1H), 5.05 (s, 1H), 4.59 - 4.55 (m, 2H), 3.97 (s, 4H), 3.67 (s, 4H), 1.80 - 1.71 (m, 1H), 0.80 - 0.73 (m, 2H), 0.72 - 0.66 (m, 2H). ¹H under water.

### Synthesis 105

### 3-cyclopropyl-N⁵-(((3R,4R)-3-methoxypiperidin-4-yl) methyl)-N'-(4-(pyridin-2-yl)benzyl)pyrazolo[1,5-a]pyrimidine-5,7-diamine

### (PPA-087)

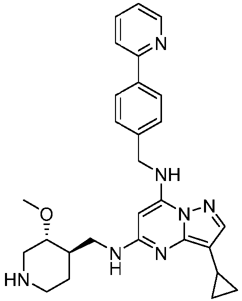

### Step A:

### tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-methoxypiperidine-1-carboxylate

To a mixture of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-hydroxypiperidine-1-carboxylate (300 mg, 448 µmol) (prepared as described herein) and tetrabutylammonium bromide (14.4 mg, 44.8 µmol) in toluene (3.0 mL) was added an aq. solution of sodium hydroxide (12.5 M) (322 µL, 4.03 mmol) and iodomethane (252, 4.03 mmol). The reaction mixture was stirred at 50 °C for 3 h. At RT, water (30 mL) and EtOAc (25 mL) were added. The layers were separated and aq. layer was extracted with EtOAc (2 x 25 mL). The combined organic layers were dried over Na₂SO₄, filtered and concentrated *in vacuo.* Purification by column chromatography (24 g cartridge, 0-10% MeOH (containing 0.7 M NH₃)/DCM) gave the title compound (130 mg, 0.19 mmol, 42% yield, 98% purity) as a yellow solid.

UPLC/MS (Method 5): m/z 684 (M+H)⁺, RT 2.11 min.

### Step 4:

### 3-cyclopropyl-N⁵-(((3R,4R)-3-methoxypiperidin-4-yl)methyl)-N⁷-(4-(pyridin-2-yl)benzyl)pyrazolo[1,5-a]pyrimidine-5,7-diamine

Hydrogen chloride (4 M in dioxane) (0.48 mL, 1.9 mmol) was added to a solution of tert-butyl (3R,4R)-4-(((7-((tert-butoxycarbonyl)(4-(pyridin-2-yl)benzyl)amino)-3-cyclopropylpyrazolo[1,5-a]pyrimidin-5-yl)amino)methyl)-3-methoxypiperidine-1-carboxylate (130 mg, 0.19 mmol) in dioxane (2.0 mL). The reaction mixture was stirred at RT for 4 h then concentrated *in vacuo.* The solid was triturated in MeCN (3 mL) and the solid was loaded onto a column of SCX. The column was washed with MeOH (40 mL) and the product was eluted with 0.7 M NH₃ in MeOH (40 mL). The ammoniacal methanol solution was concentrated *in vacuo* to give the title compound (38 mg, 77 µmol, 41% yield, 98% purity) as a white solid.

UPLC/MS (Method 5): m/z 484 (M+H)⁺, RT 1.36 min.

¹H NMR (400 MHz, DMSO-d₆) δ 8.68 - 8.61 (m, 1H), 8.05 (d, *J* = 8.3 Hz, 2H), 7.92 (dt, *J* = 8.2, 1.2 Hz, 1H), 7.86 (td, *J =* 7.6, 1.8 Hz, 1H), 7.81 (t, *J* = 6.4 Hz, 1H), 7.53 (s, 1H), 7.45 (d, *J =* 8.3 Hz, 2H), 7.33 (ddd, *J* = 7.4, 4.8, 1.2 Hz, 1H), 6.51 (t, *J =* 5.9 Hz, 1H), 5.17 (s, 1H), 4.53 - 4.47 (m, 2H), 3.56 - 3.47 (m, 1H), 3.27 (s, 3H), 3.25 - 3.20 (m, 1H), 3.19 - 3.11 (m, 1H), 2.90 (td, *J =* 9.2, 4.2 Hz, 1H), 2.84 - 2.77 (m, 1H), 2.39 - 2.28 (m, 1H), 2.19 (dd, *J =* 11.7, 9.2 Hz, 1H), 1.81 - 1.64 (m, 2H), 1.59 - 1.48 (m, 1H), 1.19 - 1.06 (m, 1H), 0.80 - 0.70 (m, 4H). ¹H under water.

### Synthesis 106

### 3-cyclopropyl-N-(4-(pyridin-2-yl)benzyl)-5-(1,2,5,6-tetrahydropyridin-3-yl)pyrazolo[1,5-a]pyrimidin-7-amine

### (PPA-088)

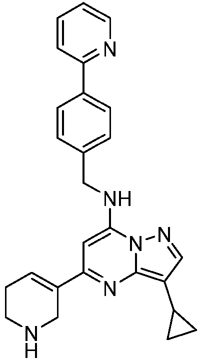

### Step 3:

### tert-butyl 5-(7-((tert-butoxycarbonyl)(4-(pyridin-2-yl)benzyl)amino)-3-cyclopropylpyrazolo[1,5-a]pyrimidin-5-yl)-3,6-dihydropyridine-1(2H)-carboxylate

A mixture of tert-butyl 5-(4,4,5,5-tetramethyl-1,3,2-dioxaborolan-2-yl)-3,6-dihydro-1(2H)-pyridinecarboxylate (78 mg, 252 µmol), tert-butyl (5-chloro-3-cyclopropylpyrazolo[1,5-a]pyrimidin-7-yl)(4-(pyridin-2-yl)benzyl)carbamate (100 mg, 210 µmol) (prepared as described herein) and Pd(dppf)Cl₂ (30.8 mg, 39.4 µmol) in dioxane (2.0 mL) was sparged with N₂ for 5 min. A solution of potassium phosphate, tribasic (134 mg, 630 µmol) in water (0.50 mL) was added and the reaction mixture was sparged with N₂ for 5 min. The mixture was heated to 90 °C for 6 h then concentrated *in vacuo.* Purification by column chromatography (12 g cartridge, 0-100% EtOAc/isohexane) gave the title compound (137 mg, 0.21 mmol, 99% yield, 95% purity) as a yellow glass solid.

UPLC/MS (Method 4): m/z 623 (M+H)⁺, R_{T} 2.60 min.

### Step 4:

### 3-cyclopropyl-N-(4-(pyridin-2-yl)benzyl)-5-(1,2,5,6-tetrahydropyridin-3-yl)pyrazolo[1,5-a]pyrimidin-7-amine

TFA (0.20 mL) was added to a mixture of tert-butyl 5-(7-((tert-butoxycarbonyl)(4-(pyridin-2-yl)benzyl)amino)-3-cyclopropylpyrazolo[1,5-a]pyrimidin-5-yl)-3,6-dihydropyridine-1(2H)-carboxylate (27 mg, 43 µmol) in DCM (0.60 mL). The reaction mixture was stirred at RT for 3 h then concentrated *in vacuo.* The solid was loaded onto a column of SCX. The column was washed with MeOH (20 mL) and the product was eluted with 0.7 M NH₃ in MeOH (10 mL). The ammoniacal methanol solution was concentrated *in vacuo* and the solid was triturated in Et₂O (2 x 1 mL) to give the title compound (14 mg, 31 µmol, 73% yield, 95% purity) as a yellow solid.

UPLC/MS (Method 4): m/z 423 (M+H)⁺, RT 1.12 min.

¹H NMR (400 MHz, DMSO-d₆) δ 8.67 - 8.61 (m, 1H), 8.35 (t, *J =* 6.6 Hz, 1H), 8.04 (d, *J* = 8.3 Hz, 2H), 7.92 (dt, *J =* 8.2, 1.2 Hz, 1H), 7.89 - 7.82 (m, 2H), 7.51 (d, *J =* 8.3 Hz, 2H), 7.33 (ddd, *J* = 7.4, 4.8, 1.2 Hz, 1H), 6.65 (t, *J =* 4.1 Hz, 1H), 6.16 (s, 1H), 4.70 (d, *J =* 6.4 Hz, 2H), 3.63 - 3.59 (m, 2H), 2.77 (t, *J =* 5.7 Hz, 2H), 2.20 - 2.09 (m, 2H), 1.93 (p, *J =* 6.8 Hz, 1H), 0.90 - 0.80 (m, 4H). ¹H under water.

### Synthesis 113

### 3-cyclopropyl-5-(3-methyl-1H-pyrazol-4-yl)-N-(4-(pyridin-2-yl)benzyl)pyrazolo[1,5-a]pyrimidin-7-amine

### (PPA-095)

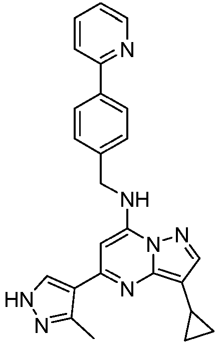

### Step 3:

### tert-butyl (3-cyclopropyl-5-(3-methyl-1H-pyrazol-4-yl)pyrazolo[1,5-a]pyrimidin-7-yl)(4-(pyridin-2-yl)benzyl)carbamate

A mixture of 3-methyl-4-(4,4,5,5-tetramethyl-1,3,2-dioxaborolan-2-yl)-1H-pyrazole (66 mg, 315 µmol), tert-butyl (5-chloro-3-cyclopropylpyrazolo[1,5-a]pyrimidin-7-yl)(4-(pyridin-2-yl)benzyl)carbamate (125 mg, 263 µmol) (prepared as described herein) and Pd(dppf)Cl₂ (28.8 mg, 39.4 µmol) in dioxane (3.0 mL) was sparged with N₂ for 5 min. A solution of potassium phosphate, tribasic (167 mg, 788 µmol) in water (0.75 mL) was added and the reaction mixture was sparged with N₂ for 5 min. The mixture was heated to 90 °C for 6 h. At RT, the reaction mixture was diluted with EtOAc (10 mL) and filtered through celite, rinsing with EtOAc (15 mL). The filtrate was concentrated *in vacuo.* Purification by column chromatography (12 g cartridge, 0-100% (EtOH in EtOAc 25:75)/isohexane) gave the title compound (130 mg, 0.20 mmol, 79% yield, 80% purity) as a yellow oil.

UPLC/MS (Method 5): m/z 522 (M+H)⁺, R_{T} 1.76 min.

### Step 4:

### 3-cyclopropyl-5-(3-methyl-1H-pyrazol-4-yl)-N-(4-(pyridin-2-yl)benzyl)pyrazolo[1,5-a]pyrimidin-7-amine

TFA (1.0 mL) was added to a mixture of tert-butyl (3-cyclopropyl-5-(3-methyl-1H-pyrazol-4-yl)pyrazolo[1,5-a]pyrimidin-7-yl)(4-(pyridin-2-yl)benzyl)carbamate (130 mg, 249 µmol) in DCM (4.0 mL). The reaction mixture was stirred at RT overnight and concentrated *in vacuo.* Purification by column chromatography (12 g cartridge, 0-20% MeOH (containing 0.7 M NH₃)/DCM) gave the title compound (49 mg, 0.11 mmol, 44% yield, 95% purity) as a white solid.

UPLC/MS (Method 5): m/z 422 (M+H)⁺, RT 1.27 min.

¹H NMR (400 MHz, DMSO-d₆) - 0.65/0.35 mixture of tautomers - δ 12.76 (s, 0.65H), 12.67 (s, 0.35H), 8.66 - 8.60 (m, 1H), 8.41 - 8.29 (m, 1H), 8.25 (s, 0.35H), 8.08 - 8.02 (m, 2H), 7.94 - 7.89 (m, 1.65), 7.88 - 7.81 (m, 2H), 7.60 - 7.46 (m, 2H), 7.32 (ddd, *J =* 7.4, 4.8, 1.2 Hz, 1H), 6.29 (s, 0.35H), 6.25 (s, 0.65H), 4.76 - 4.66 (m, 2H), 2.54 (s, 2H), 2.47 (s, 1H), 2.00 - 1.89 (m, 1H), 0.98 - 0.89 (m, 2H), 0.88 - 0.82 (m, 2H).

### Synthesis 114

### 3-cyclopropyl-5-(2-methylpyridin-3-yl)-N-(4-(pyridin-2-yl)benzyl)pyrazolo[1,5-a]pyrimidin-7-amine

### (PPA-096)

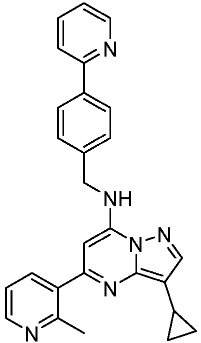

### Step 3:

### tert-butyl (3-cyclopropyl-5-(2-methylpyridin-3-yl)pyrazolo[1,5-a]pyrimidin-7-yl)(4-(pyridin-2-yl)benzyl)carbamate

A mixture of 2-methyl-3-(4,4,5,5-tetramethyl-1,3,2-dioxaborolan-2-yl)pyridine (69.0 mg, 315 µmol), tert-butyl (5-chloro-3-cyclopropylpyrazolo[1,5-a]pyrimidin-7-yl)(4-(pyridin-2-yl)benzyl)carbamate (125 mg, 263 µmol) (prepared as described herein) and Pd(dppf)Cl₂ (28.8 mg, 39.4 µmol) in dioxane (3.0 mL) was sparged with N₂ for 5 min. A solution of potassium phosphate, tribasic (167 mg, 788 µmol) in water (0.75 mL) was added and the reaction mixture was sparged with N₂ for 5 min. The mixture was heated to 90 °C for 6 h. At RT, the reaction mixture was diluted with EtOAc (10 mL) and filtered through celite, rinsing with EtOAc (15 mL). The filtrate was concentrated *in vacuo.* Purification by column chromatography (12 g cartridge, 0-10% MeOH (containing 0.7 M NH₃)/DCM) gave the title compound (130 mg, 0.23 mmol, 92% yield, 95% purity) as a yellow oil.

UPLC/MS (Method 5): m/z 533 (M+H)⁺, R_{T} 1.88 min.

### Step 4:

### 3-cyclopropyl-5-(2-methylpyridin-3-yl)-N-(4-(pyridin-2-yl)benzyl)pyrazolo[1,5-a]pyrimidin-7-amine

TFA (1.0 mL) was added to a mixture of tert-butyl (3-cyclopropyl-5-(2-methylpyridin-3-yl)pyrazolo[1,5-a]pyrimidin-7-yl)(4-(pyridin-2-yl)benzyl)carbamate (130 mg, 244 µmol) in DCM (4.0 mL). The reaction mixture was stirred at RT overnight and concentrated *in vacuo.* Purification by column chromatography (12 g cartridge, 0-20% MeOH (containing 0.7 M NH₃)/DCM) followed by further purification on RP Flash C18 (15 g cartridge, 30-100% MeCN/10 mM ammonium bicarbonate) gave the title compound (59 mg, 0.13 mmol, 54% yield, 97% purity) as an off-white solid.

UPLC/MS (Method 5): m/z 422 (M+H)⁺, RT 1.27 min.

¹H NMR (400 MHz, DMSO-d₆) δ 8.69 (t, *J =* 6.6 Hz, 1H), 8.66 - 8.62 (m, 1H), 8.48 (dd, *J* = 4.8, 1.8 Hz, 1H), 8.05 (d, *J =* 8.4 Hz, 2H), 7.96 (s, 1H), 7.92 (dt, *J =* 8.1, 1.2 Hz, 1H), 7.85 (td, *J =* 7.7, 1.9 Hz, 1H), 7.77 (dd, *J* = 7.8, 1.8 Hz, 1H), 7.53 (d, *J =* 8.3 Hz, 2H), 7.35 - 7.28 (m, 2H), 6.22 (s, 1H), 4.72 (d, *J =* 6.4 Hz, 2H), 2.43 (s, 3H), 2.02 - 1.92 (m, 1H), 0.92 - 0.81 (m, 4H).

### Synthesis 118

### 3-cyclopropyl-5-(piperidin-4-yl)-N-(4-(pyridin-2-yl)benzyl)pyrazolo[1,5-a]pyrimidin-7-amine

### (PPA-100)

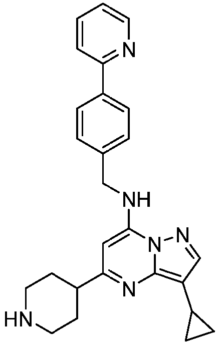

### Step 3:

### tert-butyl 4-(7-((tert-butoxycarbonyl)(4-(pyridin-2-yl)benzyl)amino)-3-cyclopropylpyrazolo[1,5-a]pyrimidin-5-yl)piperidine-1-carboxylate

A mixture of tert-butyl (5-chloro-3-cyclopropylpyrazolo[1,5-a]pyrimidin-7-yl)(4-(pyridin-2-yl)benzyl)carbamate (200 mg, 420 µmol) (prepared as described herein), 1-(tert-butoxycarbonyl)piperidin-4-yl)zinc(II) bromide (0.74M in THF) (2.84 mL, 2.11 mmol), Pd(dppf)Cl₂ (22.2 mg, 30.3 µmol) in THF (2.0 mL) was sparged with N₂ for 15 min before being heated to 50 °C for 2 h. At RT, the reaction mixture was quenched with MeOH (1 mL) then concentrated *in vacuo.* Purification by column chromatography (24 g cartridge, 0-100% EtOAc/isohexane) gave the title compound (250 mg, 0.39 mmol, 92% yield, 98% purity) as a yellow solid.

UPLC/MS (Method 4): m/z 525 (M-Boc+H)⁺, RT 2.53 min.

### Step 4:

### 3-cyclopropyl-5-(piperidin-4-yl)-N-(4-(pyridin-2-yl)benzyl)pyrazolo[1,5-a]pyrimidin-7-amine

TFA (1.0 mL) was added to a mixture of tert-butyl 4-(7-((tert-butoxycarbonyl)(4-(pyridin-2-yl)benzyl)amino)-3-cyclopropylpyrazolo[1,5-a]pyrimidin-5-yl)piperidine-1-carboxylate (250 mg, 400 µmol) in DCM (3.0 mL). The reaction mixture was stirred at RT overnight then concentrated *in vacuo.* The solid was loaded onto a column of SCX. The column was washed with MeOH (40 mL) and the product was eluted with 0.7 M NH₃ in MeOH (40 mL). The ammoniacal methanol solution was concentrated in vacuo. Further purification on RP Flash C18 (24 g cartridge, 20-100% MeCN/10 mM ammonium bicarbonate) gave the title compound (80 mg, 0.18 mmol, 46% yield, 98% purity) as a white solid.

UPLC/MS (Method 4): m/z 425 (M+H)⁺, RT 1.01 min.

¹H NMR (400 MHz, DMSO-d₆) δ 8.66 - 8.61 (m, 1H), 8.35 (t, *J =* 6.6 Hz, 1H), 8.08 - 8.01 (m, 2H), 7.93 (d, *J* = 8.0 Hz, 1H), 7.86 (td, *J* = 7.7, 1.9 Hz, 1H), 7.81 (s, 1H), 7.50 (d, *J =* 8.2 Hz, 2H), 7.33 (dd, *J* = 7.4, 4.8 Hz, 1H), 5.94 (s, 1H), 4.65 (d, *J =* 6.3 Hz, 2H), 3.01 - 2.91 (m, 2H), 2.63 - 2.53 (m, 2H), 1.96 - 1.88 (m, 1H), 1.72 - 1.62 (m, 2H), 1.55 (qd, *J =* 12.0, 3.9 Hz, 2H), 0.88 - 0.76 (m, 4H). 2H under water.

### Biological Methods

### IC₅₀ Assay

### Materials and solutions:

- HEPES-NaOH (Sigma, H-3375)
- Sodium orthovanadate (Sigma, S-6508)
- DTT (Sigma, D-0632)
- MgCl₂ (M-3634)
- MnCl₂ (VWR, 1.05927.1000)
- PEG-20000 (SERVA, 33138)
- ATP (Sigma, A-7699)
- [γ-33P]-ATP (Hartmann Analytic, FF301T)
- H₃PO₄ (VWR, 1.00563.1000)
- NaCl (Merck, 1.06404)
- Human CDK12 wt/CycK (ProQinase, 1483-1484-1 - Lot 2)
- RBER-IRStide (ProQinase, 0863-0000-1 - Lot 036)
- 96-well FlashPlates^{™} (PerkinElmer, SMP200)

Additionally for the CDK12 assay:
- Human CDK12 wt/CycK (ProQinase, 1483-1484-1 - Lot 2)
- RBER-IRStide (ProQinase, 0863-0000-1 - Lot 036)

Additionally for the CDK7 assay:
- Human CDK7/CycH/MAT1 (ProQinase, 0366-0360-4 - Lot 2).
- RBER-CHKtide (ProQinase, 0581-0000-5 - Lot 106).

### Assay procedure:

A radiometric protein kinase assay (³³PanQinase^{®} Activity Assay) was used for measuring the kinase activity of the two protein kinases (CDK12 / CDK7). All kinase assays were performed in 96-well FlashPlates^{™} from PerkinElmer (Boston, MA, USA) in a 50 µL reaction volume.

The reaction cocktail was pipetted in four steps in the following order:
- 20 µL of assay buffer (standard buffer)
- 5 µL of ATP solution (in H2O)
- 5 µL of test compound (in 10 % DMSO)
- 10 µL of substrate / 10 µL of enzyme solution (premixed)

For the CDK7 assay, a reaction mixture (50 µL) containing the following components was prepared:
- 70 mM HEPES-NaOH (pH 7.5);
- sodium orthovanadate (3 µM);
- PEG-20000 (50 µg/mL);
- DTT (1.2 mM);
- MgCl₂ (3 mM);
- MnCl₂ (3 mM);
- purified human CDK7/CycH/MAT1 (3.3 nM - Lot 02);
- RBER-CHKtide substrate (40 µg/mL - Lot 106);
- ATP (3 µM);
- [y-33P]-ATP (approx. 8 x 10⁵ cpm per well); and
- test compound at the appropriate concentration such that the final concentration of DMSO was 10% w/w.

For the CDK7 assay, a reaction mixture (50 µL) containing the following components was prepared:
- 70 mM HEPES-NaOH (pH 7.5);
- sodium orthovanadate (3 µM);
- PEG-20000 (50 µg/mL);
- DTT (1.2 mM);
- MgCl₂ (3 mM);
- MnCl₂ (3 mM);
- purified human CDK12 wt/CycK (14.7 nM - Lot 02);
- RBER-IRStide substrate (40 µg/mL - Lot 036);
- ATP (0.3 µM);
- [y-33P]-ATP (approx. 8 x 10⁵ cpm per well); and
- test compound at the appropriate concentration such that the final concentration of DMSO was 10% w/w.

The reaction mixture was incubated at 30°C for 60 min and then stopped by the addition of 2 % (v/v) H₃PO₄. Plates were aspirated and washed two times with 200 µL 0.9 % (w/v) NaCl. Incorporation of ³³Pi was determined with a microplate scintillation count (Microbeta, Wallac).

The residual activities for each concentration and the compound IC₅₀ values were calculated using *Quattro Workflow V3.1.1* (Quattro Research GmbH, Munich, Germany). The fitting model for the IC₅₀ determinations was "Sigmoidal response (variable slope)" with parameters "top" fixed at 100 % and "bottom" at 0 %. The fitting method used was a least-squares fit.

### Western Blotting Analysis of Cyclin K Depletion in Cell Culture

A673 cells were incubated for 2 hours with of 1 µM compound in presence or in absence of 10 µM of the NAE (NEDD8 activating enzyme) inhibitor MLN4924 (Cell Signalling Technologies). Cells were washed 2x with cold Phosphate Buffered Saline (PBS, Sigma Aldrich), followed by the addition of lysis buffer for 15 minutes on ice before harvesting and centrifugation.

Protein content in the cell lysate was assessed by BCA assay (Thermo Fisher). Samples were then prepared with 4x Laemmlli Buffer (Bio-Rad) and heated to 95°C for 5 minutes. Each sample was loaded onto a Mini-PROTEAN TGX Stain Free Gel (Bio-Rad) at 50 µg protein/well. SDS-PAGE was performed and then protein was transferred onto a PVDF membrane using a Trans-Blot^{®} Turbo^{™} Midi PVDF Transfer Pack (Bio-Rad. Membranes were blocked in TBS-T (Invitrogen), 5% (w/v) milk powder (Marvel) overnight at 4°C.

Membranes were incubated with anti-CCNK antibody (rabbit) primary antibody (Abcam) and anti-GAPDH antibody (rabbit) in TBS-T for 2 hours. Membranes were then washed three times with TBS-T, before incubation secondary goat anti-rabbit HRP antibody (Cell Signalling Technologies) followed by 3x washing in TBS-T. Blots were imaged using a Chemidoc imager (Bio-Rad). Cyclin K depletion was measured as the observable drop in band intensity in compound treated samples relative to proteasome-inhibitor treated samples.

### Cytotoxicity in A673 Cells

Inhibition of cell viability by test compounds was assessed in A673 cells (ATCC CRL-1598) and the compound mode of action was attributed to a "molecular-glue"/cyclin K-degradation mechanism by probing for a loss of compound potency in the presence of the NEDD8-activating enzyme inhibitor, MLN4924.

Cells were plated in 96-well plates at a density of 1.2 x 10⁵ viable cells per well in 200 µL fully supplemented growth medium (DMEM + 10% heat inactivated foetal bovine serum) and incubated for 24 hours in standard tissue culture conditions. Compounds, including MLN4924, were solubilised in DMSO and then diluted in fully supplemented growth medium at 2X their final assay concentration such that the final DMSO concentration was 0.1%. Growth medium in the assay plates was replaced with 50 µL of growth medium containing 2X MLN4924 (final assay concentration: 30 nM, where tested) or vehicle only and 50 µL of growth medium containing a 2X compound concentration series or vehicle only such that each compound was at 1X final assay concentration in 100 µL.

Cells were then incubated for 72 hours in standard tissue culture conditions before viability was assessed using 50 µL Cell Titer Glo reagent (Promega) per well and luminescence measured on an EnVision plate reader. Data were normalised to vehicle control (100 %) and cells treated with 0.1% Triton x-100 for 1 hour before viability measurement (0%; full cell kill). IC₅₀ values were determined using a semi-logistic curve fit with parameters "top" fixed at 100% and "bottom" at or below 0%. Viability curves generated in the presence of MLN4924 were normalised and fixed to controls treated with MLN4924.

### Biological Data

The PPA compounds were assessed using the biological methods described above.

The following reference compound, CR8, was also assessed, for comparison purposes. CR8 acts as a molecular glue degrader that depletes cyclin K. See, e.g., Slabicki *et al.,* 2020.

| | |
|---|---|
| REF-001 | 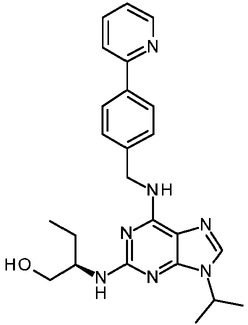 |
| (CR8) Slabicki *et al.,* 2020 | |

The resulting data are summarized in the following table. A ratio of CDK7 / CDK12 of greater than 1 indicated selectivity for CDK12.

| Table 1 | | | | |
|---|---|---|---|---|
| Biochemical Assay Data | | | | |
| Compound | A673 IC₅₀ (nM) | CDK12 IC₅₀ (nM) | CDK7 IC₅₀ (nM) | CDK7 / CDK12 |
| REF-001 (CR8) | 84.5 | 302 ^{‡} | 1769 ^{†} | 5.9 |
| | | | | |
| PPA-005(*) | 1.1 | 34 | 112 | 3.3 |
| PPA-006 | 3.5 | 51 | 312 | 6.1 |
| PPA-015 | 179.4 | 235 | 445 | 1.9 |
| PPA-019 | 52.4 | 70 | 224 | 3.2 |
| PPA-020 | 3.3 | 99 | 311 | 3.1 |
| PPA-022 | 1.5 | 72 | 130 | 1.8 |
| PPA-027 | 2.2 | 163 | 300 | 1.8 |
| PPA-028 | 9.7 | 242 | 468 | 1.9 |
| PPA-030 | 6.2 | 218 | 370 | 1.7 |
| PPA-032 | 51.5 | 1160 | 2760 | 2.4 |
| PPA-033 | 53 | 1050 | 6650 | 6.3 |
| PPA-036 | 312.5 | 245 | 388 | 1.6 |
| PPA-037 | 0.7 | 28 | 250 | 8.9 |
| PPA-038 | 6.2 | 115 | 3020 | 26.3 |
| PPA-039 | 2.8 | 169 | 2090 | 12.4 |
| PPA-040 | 20.2 | 743 | 6920 | 9.3 |
| PPA-041 | 17.3 | 189 | 1600 | 8.5 |
| PPA-042 | 232.4 | 2020 | 10300 | 5.1 |
| PPA-043 | 13.7 | 254 | 6080 | 23.9 |
| PPA-044 | 2.7 | 51 | 338 | 6.6 |
| PPA-045 | 33.2 | 374 | 9610 | 25.7 |
| PPA-046 | 305.3 | 3780 | > 30000 | > 8 |
| PPA-047 | 17.3 | 31 | 892 | 28.8 |
| PPA-048 | 6.8 | 82 | 1290 | 15.7 |
| PPA-049 | 6.9 | 32 | 982 | 30.7 |
| PPA-050 | 44.8 | 423 | 10500 | 24.8 |
| PPA-051 | 28.9 | 664 | 11200 | 16.9 |
| PPA-052 | > 100 | 6630 | > 30000 | > 4.5 |
| PPA-053 | 2.5 | 53 | 169 | 3.2 |
| PPA-055 | 24.9 | 134 | 2390 | 17.8 |
| PPA-056 | > 100 | | | |
| PPA-057 | 50.3 | 1240 | 13300 | 10.7 |
| PPA-058 | 6.9 | 204 | 5260 | 25.8 |
| PPA-060 | 2.9 | 77 | 2870 | 37.3 |
| PPA-061 | 5.3 | 97 | 1260 | 13.0 |
| PPA-062 | 13.4 | 459 | 3640 | 7.9 |
| PPA-063 | 5.9 | | | |
| PPA-064 | 1.2 | 18 | 268 | 14.9 |
| PPA-065 | 3.2 | 29 | 284 | 9.8 |
| PPA-072 | 0.8 | 34 | 1000 | 29.4 |
| PPA-073 | 19.2 | 33 | 529 | 16.0 |
| PPA-075 | 5.7 | 145 | 4720 | 32.6 |
| PPA-077 | 7.5 | 41 | 1390 | 33.9 |
| PPA-078 | 30.2 | 593 | 6240 | 10.5 |
| PPA-079 | > 100 | 1190 | > 30000 | |
| PPA-080 | 12.6 | 152 | 1210 | 8.0 |
| PPA-087 | 8.7 | 503 | 1630 | 3.2 |
| PPA-088 | 2.5 | 60 | 762 | 12.7 |
| PPA-095 | 4.7 | | | |
| PPA-096 | 7.6 | | | |
| PPA-100 | 43.5 | | | |

| | | | | |
|---|---|---|---|---|
| (*) Reference compound. ‡ K_{D} reported in Delehouzé *et al.,* 2014. † Pubchem Bioassay ID 1587605, Pubchem SID 103586761 (see https://pubchem.ncbi.nlm.nih.gov/substance/103586761#section=BioAssay-Results). | | | | |

The data demonstrated that the PPA compounds are highly potent inhibitors of CDK12, and, in addition, some also have substantial selectivity for CDK12 as compared to CDK7.

### Cyclin K Degradation

Many of the compounds described herein are characterised not only by the ability to inhibit CDK12 but also to cause Cyclin K degradation.

Cyclin K degradation was evaluated by Western blotting for cyclin K in A673 cells exposed to 1 µM test compound for 2 hours in the absence and presence of 10 µM MLN4924 (to inhibit proteosome activity).

Figure 1 is shows the effect of THZ-531, CR8, reference example PPA-005 and example PPA-006 on cyclin K degradation in A673 cells, as assessed by Western blotting, as well as the effect of MLN4924 on cytotoxic potency in A673 cells.

As reported by Slabicki *et al.,* 2020, the known CDK12 / 13 inhibitor THZ-531 showed no protesomal dependent Cyclin K degradation, whereas the known CDK1 / 2 / 5 / 9 / 12 inhibitor CR8 did cause proteosomal dependent degradation of Cyclin K. Furthermore, evaluation of cytotoxicity to THZ-531 and CR8 in A673 cells incubated for 72 hours in the presence and absence of 30 nM MLN4924 showed a decrease in potency for CR8, but not for THZ-531 demonstrating Cyclin K degradation is a factor in the cytotoxic potency for CR8 but not THZ-531.

As shown in Figure 1, reference example PPA-005 and example PPA-006 were also found to degrade Cyclin K in A673 cells by western blot and a significant decrease in cytotoxic potency in the presence of MLN4924.

Compounds that are cyclin K degraders, such as CR8, were also found to *more potent* in cellular assays than biochemical assays. In contrast, THZ-531 and Dinaciclib, which are known not to degrade cyclin K despite being potent biochemical inhibitors of cyclin K, have *similar potency* in cellular assays and biochemical assays.

Data summarising biochemical and cellular potency as well as cyclin K degrader activity as evaluated by a decrease in cytoxic potency in A673 cells in the presence of MLN4924 are shown in the following table.

| Table 2 | | | | |
|---|---|---|---|---|
| Cyclin K Degradation and its Relationship to CDK12 Kinase Activity and Cellular Potency | | | | |
| Compound | CKD12 Biochemical Potency IC₅₀ (nM) | Cellular Potency IC₅₀ (nM) (in A673 cells) | Ratio of Biochemical Potency : Cellular Potency | Cyclin K Degrader Activity (A673 potency shift +MLN4924) |
| Dinaciclib | 4.1 | 4.9 | 0.83 | |
| THZ-531 | 160 | 210 | 0.74 | - |
| CR8 | 302 ^{‡} | 84.5 | 3.57 | ++ |
| | | | | |
| PPA-005(*) | 34 | 1.1 | 30.91 | ++ |
| PPA-006 | 51 | 3.5 | 14.57 | +++ |
| PPA-015 | 235 | 179.4 | 1.31 | |
| PPA-019 | 70 | 52.4 | 1.34 | |
| PPA-020 | 99 | 3.3 | 30.00 | +++ |
| PPA-022 | 72 | 1.5 | 48.00 | |
| PPA-027 | 163 | 2.2 | 74.09 | |
| PPA-028 | 242 | 9.7 | 24.95 | |
| PPA-030 | 218 | 6.2 | 35.16 | |
| PPA-032 | 1160 | 51.5 | 22.52 | |
| PPA-033 | 1050 | 53 | 19.81 | |
| PPA-036 | 245 | 312.5 | 0.78 | |
| PPA-037 | 28 | 0.7 | 40.00 | +++ |
| PPA-038 | 115 | 6.2 | 18.55 | |
| PPA-039 | 169 | 2.8 | 60.36 | + |
| PPA-040 | 743 | 20.2 | 36.78 | +++ |
| PPA-041 | 189 | 17.3 | 10.92 | |
| PPA-042 | 2020 | 232.4 | 8.69 | |
| PPA-043 | 254 | 13.7 | 18.54 | |
| PPA-044 | 51 | 2.7 | 18.89 | +++ |
| PPA-045 | 374 | 33.2 | 11.27 | |
| PPA-046 | 3780 | 305.3 | 12.38 | |
| PPA-047 | 31 | 17.3 | 1.79 | ++ |
| PPA-048 | 82 | 6.8 | 12.06 | |
| PPA-049 | 32 | 6.9 | 4.64 | +++ |
| PPA-050 | 423 | 44.8 | 9.44 | |
| PPA-051 | 664 | 28.9 | 22.98 | |
| PPA-052 | 6630 | > 100 | < 66.3 | |
| PPA-053 | 53 | 2.5 | 21.20 | |
| PPA-055 | 134 | 24.9 | 5.38 | ++ |
| PPA-056 | | > 100 | | |
| PPA-057 | 1240 | 50.3 | 24.65 | |
| PPA-058 | 204 | 6.9 | 29.57 | +++ |
| PPA-060 | 77 | 2.9 | 26.55 | +++ |
| PPA-061 | 97 | 5.3 | 18.30 | |
| PPA-062 | 459 | 13.4 | 34.25 | |
| PPA-063 | n/d | 5.9 | | |
| PPA-064 | 18 | 1.2 | 15.00 | +++ |
| PPA-065 | 29 | 3.2 | 9.06 | +++ |
| PPA-072 | 34 | 0.8 | 42.50 | +++ |
| PPA-073 | 33 | 19.2 | 1.72 | |
| PPA-075 | 145 | 5.7 | 25.44 | |
| PPA-077 | 41 | 7.5 | 5.47 | |
| PPA-078 | 593 | 30.2 | 19.64 | |
| PPA-079 | 1190 | > 100 | < 11.9 | |
| PPA-080 | 152 | 12.6 | 12.06 | +++ |
| PPA-084 | 1120 | 73.3 | 15.28 | |
| PPA-087 | 503 | 8.7 | 57.82 | +++ |
| PPA-088 | 60 | 2.5 | 24.00 | +++ |
| PPA-095 | | 43.5 | | + |
| PPA-096 | | 14.9 | | +++ |
| PPA-100 | | 1.5 | | +++ |

| | | | | |
|---|---|---|---|---|
| (*) Reference compound. ‡ K_{D} reported in Delehouzé *et al.,* 2014. | | | | |

### Legend:

(-): ≤ 1 fold change.
(+): > 1 and ≤ 2 fold change.
(++): > 2 and ≤ 5 fold change.
(+++): > 5 fold change.

The data show that many of the compounds described herein show enhanced cellular potency compared to biochemical potency, and that their potency is dependent on proteosome activity. Therefore, they act both as direct inhibitors of CDK12 and as inducers of Cyclin K degradation, so as to elicit a cytotoxic effect in cancer cells.

The foregoing has described the principles, preferred embodiments, and modes of operation of the present invention. However, the invention should not be construed as limited to the particular embodiments discussed. Instead, the above-described embodiments should be regarded as illustrative rather than restrictive.

### REFERENCES

A number of publications are cited herein in order to more fully describe and disclose the invention and the state of the art to which the invention pertains. Full citations for these references are provided below.
Bahl et al., 2019, International Patent Application Publication No. WO 2019/057825 A1, published 28 March 2019.
Blazek et al., 2011, "The Cyclin K/Cdk12 complex maintains genomic stability via regulation of expression of DNA damage response genes", Genes Dev, Vol. 25, No. 20, pp. 2158-2172.
Choi et al., 2019, "CDK12 drives breast tumor initiation and trastuzumab resistance via WNT and IRS1-ErbB-PI3K signalling", EMBO Rep, Vol. 20, No. 10, e48058.
Choi et al., 2020, "Gene expression regulation by CDK12: a versatile kinase in cancer with functions beyond CTD phosphorylation", Experimental & Molecular Medicine, Vol. 52, pp. 762-771.
Delehouzé et al., 2014 "CDK/CK1 inhibitors roscovitine and CR8 downregulate amplified MYCN in neuroblastoma cells", Oncogene, Vol. 33, pp. 5675-5687.
Greifenberg et al., 2016, "Structural and Functional Analysis of the CDK13/Cyclin K Complex," Cell Rep., Vol. 14, No. 2, pp. 320-331.
Guzi et al., 2004a, International Patent Application Publication No. WO 2004/022561 A1, published 18 March 2004.
Guzi et al., 2004b, US Patent Application Publication No. US 2004/0209878 A1, published 21 October 2004.
Guzi et al., 2006, US Patent Application Publication No. US 2006/0128725 A1, published 15 June 2006.
Guzi et al., 2007, US Patent Application Publication No. US 2007/0281951 A1, published 06 December 2007.
Guzi et al., 2008a, US Patent Application Publication No. US 2008/0050384 A1, published 28 February 2008.
Guzi et al., 2008b, International Patent Application Publication No. WO 2008/130569 A1, published 30 October 2008.
Guzi et al., 2008c, International Patent Application Publication No. WO 2008/130570 A1, published 30 October 2008.
Gyl et al., 2018, "CDK12: an emerging therapeutic target for cancer", J Clin Pathol, Vol. 71, No. 11, pp. 957-962.
Iniguez et al., 2018, "EWS/FLI Confers Tumor Cell Synthetic Lethality to CDK12 Inhibition in Ewing Sarcoma", Cancer Cell, Vol. 33, No. 2, pp. 202-216.
Johnson et al., 2016, "CDK12 Inhibition Reverses De Novo and Acquired PARP Inhibitor Resistance in BRCA Wild-Type and Mutated Models of Triple-Negative Breast Cancer", Cell Rep, Vol. 17, No. 9, pp. 2367-2381.
Kwiatkowski et al., 2019, International Patent Application Publication No. WO 2019/035866 A1, published 21 February 2019.
Lei et al., 2018, "Cyclin K regulates prereplicative complex assembly to promote mammalian cell proliferation", Nature Communications, Vol. 9, Article 1876.
Li et al., 2016, "CDK12 is a gene-selective RNA polymerase II kinase that regulates a subset of the transcriptome, including Nrf2 target genes", Sci Rep, Vol. 6, 21455.
Lord et al., 2016, "BRCAness revisited", Nat Rev Cancer, Vol. 16, No. 2, pp. 110-120.
Lu Lv et al., 2020, "Discovery of a molecular glue promoting CDK12-DDB1 interaction to trigger cyclin K degradation", eLife 2020; 9:e59994.
Malumbres et al., 2009, "Cyclin-dependent kinases: a family portrait", Nature Cell Biology, Vol. 11, No. 11, pp. 1275-1276.
Morgan, 1995, "Principles of CDK regulation", Nature, Vol. 374, pp. 131-134.
Nam et al., 2019, International Patent Application Publication No. WO 2019/197549 A1, published 17 October 2019.
Parratt et al., 2019, International Patent Application Publication No. WO 2004/087707 A1, published 14 October 2004.
Pines, 1995, "Cyclins and cyclin-dependent kinases: a biochemical view", Biochem. J., Vol. 308, No. 3, pp. 697-711.
Samajdar et al., 2016, International Patent Application Publication No. WO 2016/142855 A2, published 15 September 2016.
Slabicki et al., 2020, "The CDK inhibitor CR8 acts as a molecular glue degrader that depletes cyclin K", Nature, Vol. 585, pp. 293-297.
Vankayalapati et al., 2020, International Patent Application Publication No. WO 2020/186196 A1, published 17 September 2020.

## Claims

1. A compound of the following formula: 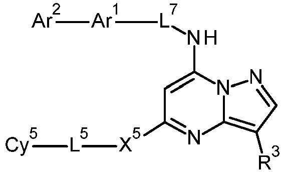
or a pharmaceutically acceptable salt or solvate thereof;
wherein:
-L⁷- is independently -CH₂-, -CH(R^{L7})-, or -C(R^{L7})₂-;
each -R^{L7} is independently linear or branched saturated C₁₋₄alkyl;
-Ar¹- is 1,4-phenylene;
and is optionally substituted with one or more groups -R^{AR1C};
-Ar² is pyridin-2-yl;
and is optionally substituted *on carbon* with one or more groups -R^{AR2C};
-X⁵- is independently -NH-, -NR^{X5}-, -O-, or a single bond;
-R^{X5} is linear or branched saturated C₁₋₄alkyl;
-L⁵- is independently -CH₂-, -CH(R^{L5})-, -C(R^{L5})₂-, or a single bond;
each -R^{L5} is independently linear or branched saturated C₁₋₄alkyl;
-Cy⁵ is independently -Cy^{5A} or -Cy^{5B};
-Cy^{5A} is non-aromatic C₄₋₁₀heterocyclyl having at least one nitrogen ring atom;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN};
-Cy^{5B} is C₅₋₆heteroaryl having at least one nitrogen ring atom;
and is:
optionally substituted *on carbon* with one or more groups -R^{Cy5BC}; and
optionally substituted *on secondary nitrogen, if present,* with a group -R^{Cy5BN};
-R³ is -R^{3B};
-R^{3B} is cyclopropyl;
and wherein:
each -R^{AR1C} and each -R^{AR2C} is independently selected from:
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{TM},
-C(=O)OH, -C(=O)OR^{TT},
-OC(=O)R^{TT},
-C(=O)NH₂, -C(=O)NHR^{TT}, -C(=O)NR^{TT}₂, -C(=O)R^{TM},
-NHC(=O)R^{TT}, -NR^{TN}C(=O)R^{TT},
-NHC(=O)NH₂, -NHC(=O)NHR^{TT}, -NHC(=O)NR^{TT}₂, -NHC(=O)R^{TM},
-NR^{TN}C(=O)NH₂, -NR^{TN}C(=O)NHR^{TT}, -NR^{TN}C(=O)NR^{TT}₂, -NR^{TN}C(=O)R^{TM},
-NHC(=O)OR^{TT}, -NR^{TN}C(=O)OR^{TT},
-OC(=O)NH₂, -OC(=O)NHR^{TT}, -OC(=O)NR^{TT}₂, -OC(=O)R^{TM},
-C(=O)R^{TT},
-SR^{TT}, -S(=O)R^{TT}, -S(=O)₂R^{TT},
-S(=O)NH₂, -S(=O)NHR^{TT}, -S(=O)NR^{TT}₂, -S(=O)R^{TM},
-S(=O)₂NH₂, -S(=O)₂NHR^{TT}, -S(=O)₂NR^{TT}₂, -S(=O)₂R^{TM},
-NHS(=O)₂R^{TT}, -NR^{TN}S(=O)₂R^{TT},
-CN, and -NO₂;
wherein:
each -L^{T}- is independently linear or branched saturated C₁₋₄alkylene;
each -R^{TT} is independently -R^{TT1}, -R^{TT2}, -L^{TT}-R^{TT2}, -R^{TT3}, or -L^{TT}-R^{TT3};
each -R^{TT1} is independently linear or branched saturated C₁₋₆alkyl, and is optionally substituted with one or more groups selected from -F, -OH, and -OR^{TTT};
each -R^{TT2} is saturated C₃₋₆cycloalkyl, and is optionally substituted with one or more groups selected from -F, -R^{TTT}, -OH, and -OR^{TTT};
each -R^{TT3} is independently phenyl or naphthyl, and is optionally substituted with one or more groups selected from -F, -Cl, -Br, -I, -R^{TTT}, OH, -OR^{TTT}, -OCF₃, -NH₂, -NHR^{TTT}, and -NR^{TTT}₂;
each -L^{TT}- is independently linear or branched saturated C₁₋₄alkylene;
each -R^{TN} is linear or branched saturated C₁₋₄alkyl, phenyl, or benzyl;
each -R^{TM} is independently azetidino, pyrrolidino, piperidino, piperazino, morpholino, azepano, or diazepano, and is:
optionally substituted *on carbon* with one or more groups selected from: -R^{TMM}, -C(=O)R^{TMM}, -S(=O)₂R^{TMM}, -F, -NH₂, -NHR^{TMM}, -NR^{TMM}₂, -OH, and -OR^{TMM}; and
optionally substituted *on secondary nitrogen, if present,* with a group selected from: -R^{TMM}, -C(=O)R^{TMM}, -C(=O)OR^{TMM}, and -S(=O)₂R^{TMM};
each -R^{TTT} is independently linear or branched saturated C₁₋₄alkyl, phenyl, or benzyl; and
each -R^{TMM} is independently linear or branched saturated C₁₋₄alkyl, phenyl, or benzyl;
and wherein:
each -R^{Cy5AC} and each -R^{Cy5BC} is independently selected from:
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-C(=O)OH, -C(=O)OR^{JJ},
-OC(=O)R^{JJ},
-C(=O)NH₂, -C(=O)NHR^{JJ}, -C(=O)NR^{JJ}₂, -C(=O)R^{JM},
-NHC(=O)R^{JJ}, -NR^{JN}C(=O)R^{JJ},
-NHC(=O)NH₂, -NHC(=O)NHR^{JJ}, -NHC(=O)NR^{JJ}₂, -NHC(=O)R^{JM},
-NR^{JN}C(=O)NH₂, -NR^{JN}C(=O)NHR^{JJ}, -NR^{JN}C(=O)NR^{JJ}₂, -NR^{JN}C(=O)R^{JM},
-NHC(=O)OR^{JJ}, -NR^{JN}C(=O)OR^{JJ},
-OC(=O)NH₂, -OC(=O)NHR^{JJ}, -OC(=O)NR^{JJ}₂, -OC(=O)R^{JM},
-C(=O)R^{JJ},
-SR^{JJ}, -S(=O)R^{JJ}, -S(=O)₂R^{JJ},
-S(=O)NH₂, -S(=O)NHR^{JJ}, -S(=O)NR^{JJ}₂, -S(=O)R^{JM},
-S(=O)₂NH₂, -S(=O)₂NHR^{JJ}, -S(=O)₂NR^{JJ}₂, -S(=O)₂R^{JM},
-NHS(=O)₂R^{JJ}, -NR^{JN}S(=O)₂R^{JJ},
-CN, and -NO₂;
each -R^{Cy5AN} and each -R^{Cy5BN} is independently selected from:
-R^{JJ},
-L^{J}-OH, -L^{J}-OR^{JJ},
-L^{J}-NH₂, -L^{J}-NHR^{JJ}, -L^{J}-NR^{JJ}₂, -L^{J}-R^{JM},
-C(=O)R^{JJ},
-C(=O)OR^{JJ},
-C(=O)NH₂, -C(=O)NHR^{JJ}, -C(=O)NR^{JJ}₂, -C(=O)R^{JM}, and
-S(=O)₂R^{JJ};
wherein:
each -L^{J}- is independently linear or branched saturated C₁₋₄alkylene;
each -R^{JJ} is independently -R^{JJ1}, -R^{JJ2}, -L^{JJ}-R^{JJ2}, -R^{JJ3}, or -L^{JJ}-R^{JJ3};
each -R^{JJ1} is independently linear or branched saturated C₁₋₆alkyl, and is optionally substituted with one or more groups selected from -F, -OH, and -OR^{JJJ};
each -R^{JJ2} is saturated C₃₋₆cycloalkyl, and is optionally substituted with one or more groups selected from -F, -R^{JJJ}, -OH, and -OR^{JJJ};
each -R^{JJ3} is independently phenyl or naphthyl, and is optionally substituted with one or more groups selected from -F, -Cl, -Br, -I, -R^{JJJ}, OH, -OR^{JJJ}, -OCF₃, -NH₂, -NHR^{JJJ}, and -NR^{JJJ}₂;
each -L^{JJ}- is independently linear or branched saturated C₁₋₄alkylene;
each -R^{JN} is linear or branched saturated C₁₋₄alkyl, phenyl, or benzyl;
each -R^{JM} is independently azetidino, pyrrolidino, piperidino, piperazino, morpholino, azepano, or diazepano, and is:
optionally substituted *on carbon* with one or more groups selected from: -R^{JMM}, -C(=O)R^{JMM}, -S(=O)₂R^{JMM}, -F, -NH₂, -NHR^{JMM}, -NR^{JMM}₂, -OH, and -OR^{JMM}; and
optionally substituted *on secondary nitrogen, if present,* with a group selected from: -R^{JMM}, -C(=O)R^{JMM}, -C(=O)OR^{JMM}, and -S(=O)₂R^{JMM};
each -R^{JJJ} is independently linear or branched saturated C₁₋₄alkyl, phenyl, or benzyl; and
each -R^{JMM} is independently linear or branched saturated C₁₋₄alkyl, phenyl, or benzyl.

2. The compound of claim 1, wherein:
-L⁷- is -CH₂-.

3. The compound of claim 1 or 2, wherein:
-X⁵- is independently -NH-, -NR^{X5}-, or a single bond.

4. The compound of claim 1 or 2, wherein:
-X⁵- is -NH-.

5. The compound of any one of claims 1-4, wherein:
-L⁵- is independently -CH₂- or a single bond.

6. The compound of any one of claims 1-4, wherein:
-L⁵- is -CH₂-.

7. The compound of any one of claims 1-6, wherein:
-Ar¹- is 1,4-phenylene.

8. The compound of any one of claims 1-7, wherein:
-Ar² is pyridin-2-yl.

9. The compound of any one of claims 1-6, wherein:
-Ar¹- is 1,4-phenylene; and
-Ar² is pyridin-2-yl.

10. The compound of any one of claims 1-8, wherein:
each -R^{AR1C}, if present, and each -R^{AR2C}, if present, is independently selected from: -F, -Me, -OMe, -CF₃, and -OCF₃.

11. The compound of any one of claims 1-10, wherein:
-Cy⁵ is -Cy^{5A}; and
-Cy^{5A} is independently azetidinyl, pyrrolidinyl, piperidinyl, piperazinyl, or morpholinyl;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN}.

12. The compound of any one of claims 1-11, wherein:
(a) -Cy^{5A}, if present, is piperidinyl;
and is:
optionally substituted *on carbon* with one or more groups -R^{Cy5AC}; and
optionally substituted *on carbon* with =O; and
optionally substituted *on secondary nitrogen, if present,* with a group -R^{Cy5AN};
(b) -Cy^{5A}, if present, is piperidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}; or
(c) -Cy^{5A}, if present, is (3S)-piperidin-3-yl;
and is:
optionally substituted *on carbon* with one or more groups -R^{Cy5AC}; and
optionally substituted *on carbon* with =O; and
optionally substituted *on secondary nitrogen* with a group -R^{Cy5AN}.

13. The compound of any one of claims 1-12, wherein:
each -R^{Cy5AC}, if present, and each -R^{Cy5BC}, if present, is independently selected from: -F, -Me, -OH, -OMe, and -NH₂;
each -R^{Cy5AN}, if present, and each -R^{Cy5BN}, if present, is independently selected from: -R^{JJ}, -C(=O)R^{JJ}, and -C(=O)OR^{JJ};
each -R^{JJ} is -R^{JJ1}; and
each -R^{JJ1} is independently linear or branched saturated C₁₋₄alkyl.

14. The compound of any one of claims 1-11, wherein:
-Cy^{5A}, if present, is piperidin-3-yl.

15. The compound of any one of claims 1-11, wherein:
-Cy^{5A}, if present, is (3S)-piperidin-3-yl.

16. The compound of any one of claims 1-11, wherein:
-Cy^{5A}, if present, is (3R,4R)-3-hydroxy-piperidin-4-yl.

17. The compound of claim 1, selected from compounds of the following formulae and pharmaceutically acceptable salts and solvates thereof:

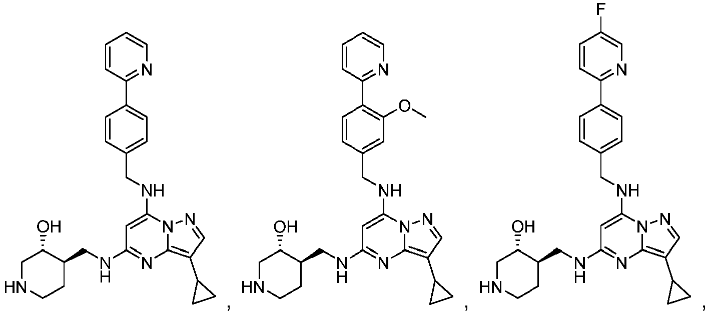

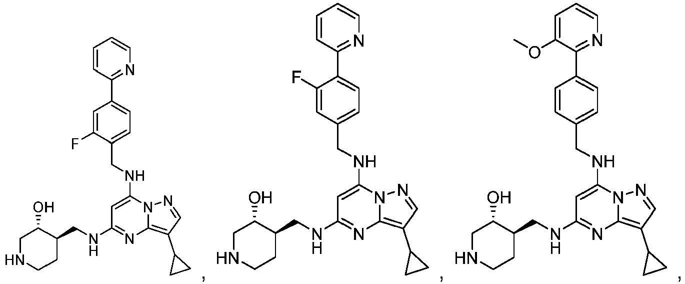

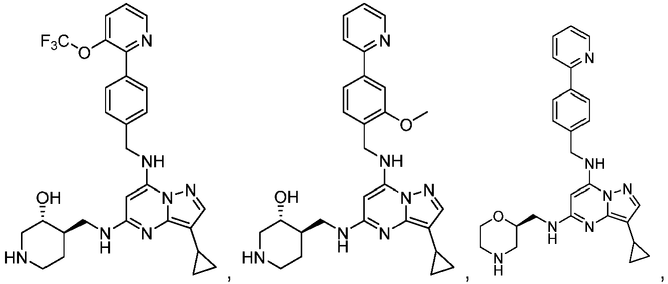

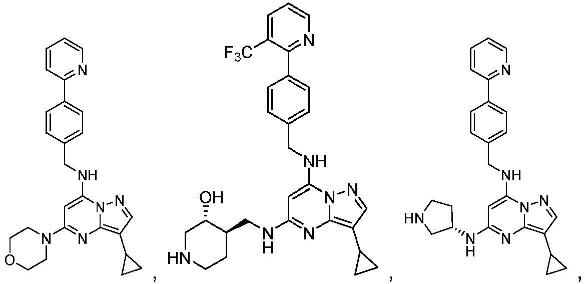

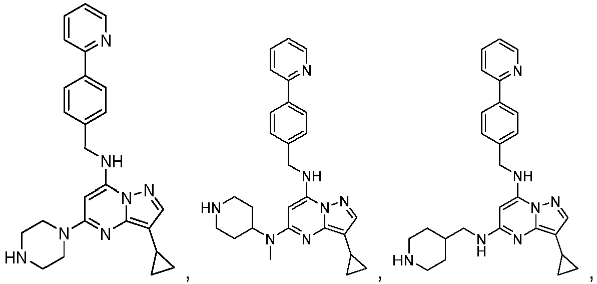

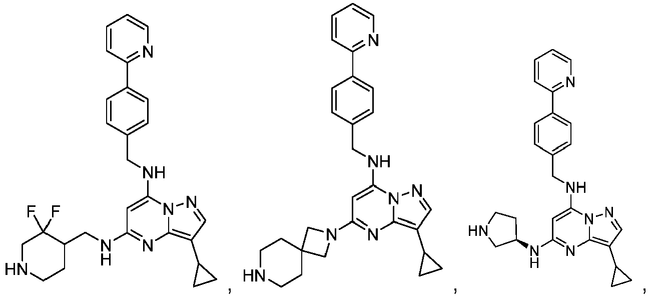

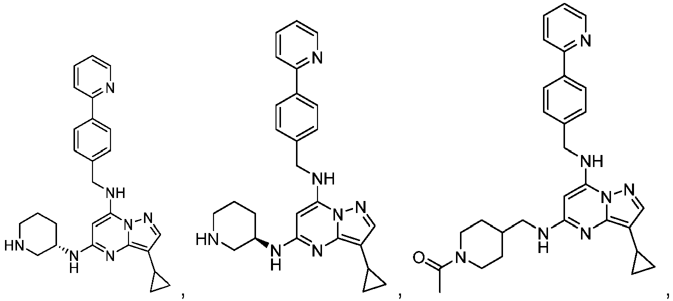

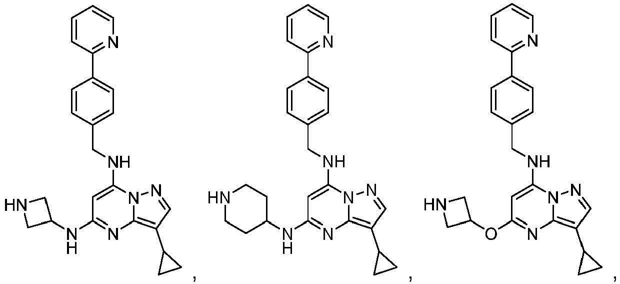

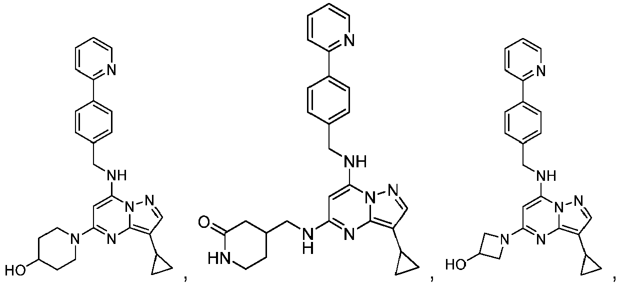

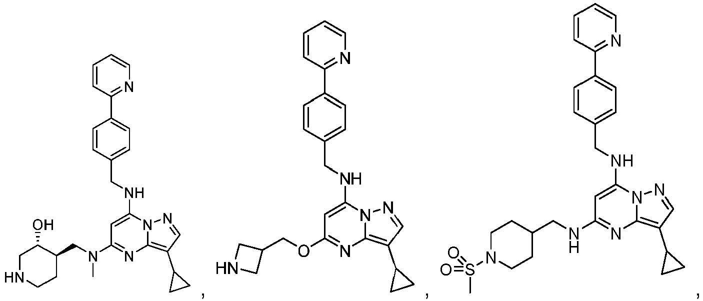

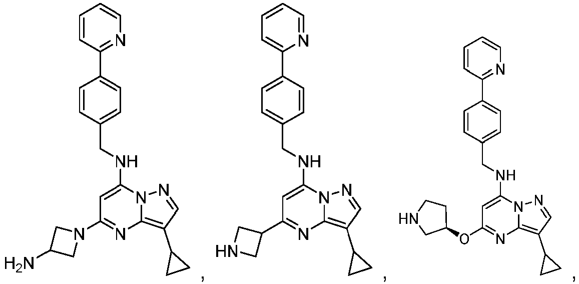

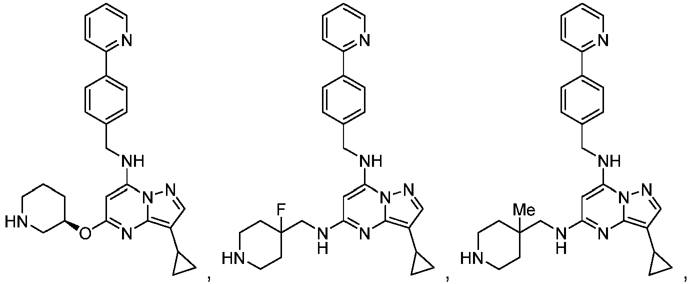

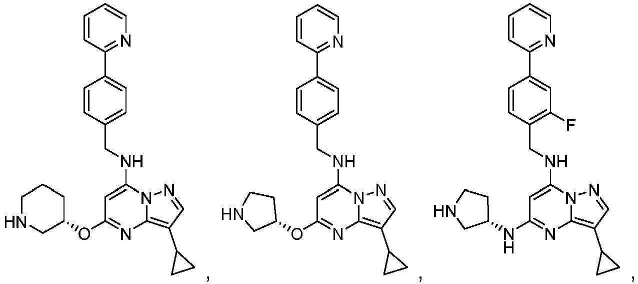

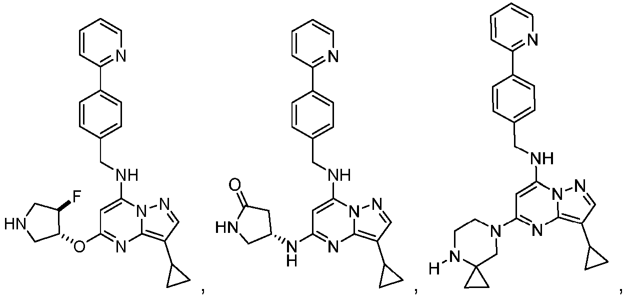

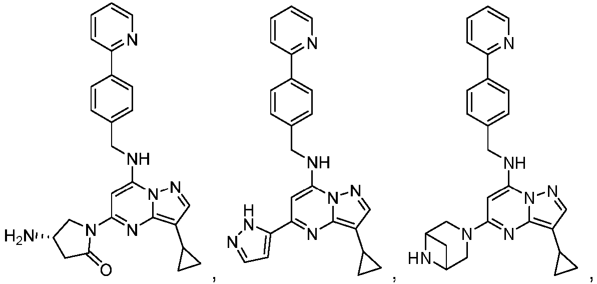

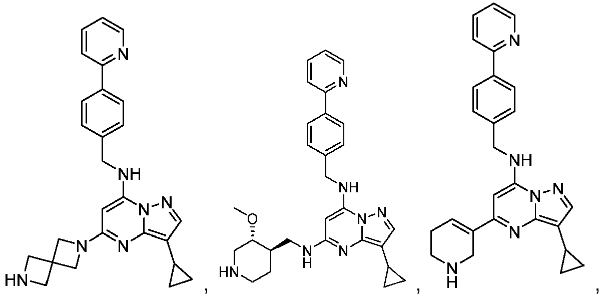

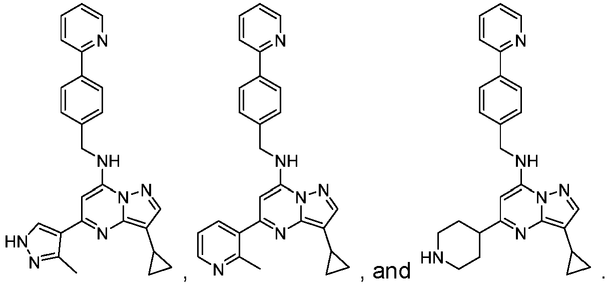

18. A composition comprising a compound according to any one of claims 1-17, and a pharmaceutically acceptable carrier or diluent.

19. A method of preparing a composition comprising the step of mixing a compound according to any one of claims 1-17, and a pharmaceutically acceptable carrier or diluent.

20. A method of inhibiting cell proliferation, inhibiting cell cycle progression, promoting apoptosis, or a combination of one or more these, *in vitro,* comprising contacting a cell with an effective amount of a compound according to any one of claims 1-17.

21. A compound according to any one of claims 1-17 for use in a method of treatment of the human or animal body by therapy.

22. A compound for use according to claim 21, wherein the disorder is: a proliferative disorder; cancer; a viral infection (e.g., HIV); a neurodegenerative disorder (e.g., Alzheimer's disease, Parkinson's disease); ischaemia; a renal disease; a cardiovascular disorder (e.g., atherosclerosis); an autoimmune disorder (e.g., rheumatoid arthritis, systemic lupus erythematosus, psoriasis, Sjogren's syndrome); or a disorder caused by dysfunction of translation in cells (e.g., muscular dystrophy, myotonic dystrophy, amyotrophic lateral sclerosis, spinal muscular atrophy, Fragile X syndrome).

23. A compound for use according to claim 21, wherein the disorder is: a proliferative disorder.

24. A compound for use according to claim 21, wherein the disorder is: cancer.

25. A compound for use according to any one of claims 21-24, wherein the treatment further comprises treatment with:
a DNA repair inhibitor;
an immune checkpoint inhibitor;
an agent stimulating the immune system;
a cell cycle checkpoint inhibitor;
a further active agent which is a Her2 blocker;
a transcriptional inhibitor; or
a further cytotoxic chemotherapeutic agent.

## Patentansprüche

1. Verbindung der folgenden Formel: 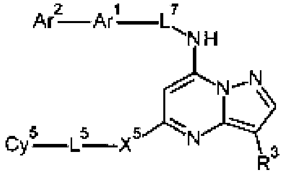
oder ein pharmazeutisch annehmbares Salz oder Solvat davon;
worin:
-L⁷- unabhängig -CH₂-, -CH(R^{L7})- oder -C(R^{L7})₂- ist;
die -R^{L7} jeweils unabhängig unverzweigtes oder verzweigtes gesättigtes C₁₋₄-Alkyl sind;
-Ar¹ 1,4-Phenylen ist;
und gegebenenfalls mit einer oder mehreren Gruppen -R^{AR1C} substituiert ist;
-Ar² Pyridin-2-yl ist;
und gegebenenfalls auf einem Kohlenstoff mit einer oder mehreren Gruppen -R^{AR2C} substituiert ist;
-X⁵- unabhängig -NH-, -NR^{X5}-, -O- oder eine Einfachbindung ist;
-R^{X5} unverzweigtes oder verzweigtes gesättigtes C₁₋₄-Alkyl ist;
-L⁵- unabhängig -CH₂-, -CH(R^{L5})-, -C(R^{L5})₂- oder eine Einfachbindung ist;
die -R^{L5} jeweils unabhängig unverzweigtes oder verzweigtes gesättigtes C₁₋₄-Alkyl sind;
-Cy⁵ unabhängig -Cy^{5A} oder -Cy^{5B} ist;
-Cy^{5A} nichtaromatisches C₄₋₁₀-Heterocyclyl mit zumindest einem Stickstoff-Ringatom ist;
und Folgendes ist:
gegebenenfalls auf einem Kohlenstoff mit einer oder mehreren Gruppen -R^{Cy5AC} substituiert und
gegebenenfalls auf einem Kohlenstoff mit =O substituiert und
gegebenenfalls auf einem sekundären Stickstoff, sofern vorhanden, mit einer Gruppe -R^{Cy5AN} substituiert;
-Cy^{5B} C₅₋₆-Heteroaryl mit zumindest einem Stickstoff-Ringatom ist;
und Folgendes ist:
gegebenenfalls auf einem Kohlenstoff mit einer oder mehreren Gruppen -R^{Cy5BC} substituiert und
gegebenenfalls auf einem sekundären Stickstoff, sofern vorhanden, mit einer Gruppe -R^{Cy5BN} substituiert;
-R³ -R^{3B} ist;
-R^{3B} Cyclopropyl ist;
und wobei:
die -R^{ARC1C} und die -R^{ARC2C} jeweils unabhängig aus Folgendem ausgewählt sind:
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{TM},
-C(=O)OH, -C(=O)OR^{TT},
-OC(=O)R^{TT},
-C(=O)NH₂, -C(=O)NHR^{TT}, -C(=O)NR^{TT}₂, -C(=O)R^{TM},
-NHC(=O)R^{TT}, -NR^{TN}C(=O)R^{TT}.
-NHC(=O)NH₂, -NHC(=O)NHR^{TT}, -NHC(=O)NR^{TT}₂, -NHC(=O)R^{TM},
-NR^{TN}C(=O)NH₂, -NR^{TN}C(=O)NHR^{TT}, -NR^{TN}C(=O)NR^{TT}₂, -NR^{TN}C(=O)R^{TM},
-NHC(=O)OR^{TT}, -NR^{TN}C(=O)OR^{TT},
-OC(=O)NH₂, -OC(=O)NHR^{TT}, -CC(=O)NR^{TT}₂, -OC(=O)R^{TM},
-C(=O)R^{TT},
-SR^{TT}, -S(=O)R^{TT}, -S(=O)₂R^{TT},
-S(=O)NH₂, -S(=O)NHR^{TT}, -S(=O)NR^{TT}₂, -S(=O)R^{TM},
-S(=O)₂NH₂, -S(=O)₂NHR^{TT}, -S(=O)₂NR^{TT}₂, -S(=O)₂R^{TM},
-NHS(=O)₂R^{TT}, -NR^{TN}S(=O)₂R^{TT},
-CN und -NO₂⁻,
worin:
die -L^{T}- jeweils unabhängig unverzweigtes oder verzweigtes gesättigtes C₁₋₄-Alkylen sind;
die -R^{TT} jeweils unabhängig -R^{TT1}, -R^{TT2}, -L^{TT}-R^{TT2}, -R^{TT3} oder -L^{TT}-R^{TT3} sind;
die -R^{TT1} jeweils unabhängig unverzweigtes oder verzweigtes gesättigtes C₁₋₆-Alkyl sind und gegebenenfalls mit einer oder mehreren Gruppen substituiert sind, die aus -F, -OH und -OR^{TTT} ausgewählt sind;
die -R^{TT2} jeweils gesättigtes C₃₋₆-Cycloalkyl sind und gegebenenfalls mit einer oder mehreren Gruppen substituiert sind, die aus -F, -R^{TTT}, -OH und -OR^{TTT} ausgewählt sind;
die -R^{TT3} jeweils unabhängig Phenyl oder Naphthyl sind und gegebenenfalls mit einer oder mehreren Gruppen substituiert sind, die aus -F, -Cl, -Br, -I, -R^{TTT}, OH, -OR^{TTT}, -OCF₃, -NH₂, -NHR^{TTT} und -NR^{TTT}₂ ausgewählt sind;
die -L^{TT}- jeweils unabhängig unverzweigtes oder verzweigtes gesättigtes C₁₋₄-Alkylen sind;
die -R^{TN} jeweils unverzweigtes oder verzweigtes gesättigtes C₁₋₄-Alkyl, Phenyl oder Benzyl sind;
die -R^{TM} jeweils unabhängig Azetidino, Pyrrolidino, Piperidino, Piperazino, Morpholino, Azepano oder Diazepano sind und Folgendes sind:
gegebenenfalls auf einem Kohlenstoff mit einer oder mehreren Gruppen substituiert, die aus den folgenden ausgewählt sind: -R^{TMM}, -C(=O)R^{TMM}, -S(=O)₂R^{TMM}, -F, -NH₂, -NHR^{TMM}, -NR^{TMM}₂, -OH und -OR^{TMM}; und
gegebenenfalls auf einem sekundären Stickstoff, sofern vorhanden, mit einer Gruppe substituiert, die aus den Folgenden ausgewählt ist: -R^{TMM}, -C(=O)R^{TMM}, -C(=O)OR^{TMM} und -S(=O)₂R^{TMM};
die -R^{TTT} jeweils unabhängig unverzweigtes oder verzweigtes gesättigtes C₁₋₄-Alkyl, Phenyl oder Benzyl sind; und
die -R^{TMM} jeweils unabhängig unverzweigtes oder verzweigtes gesättigtes C₁₋₄-Alkyl, Phenyl oder Benzyl sind;
und worin:
die -R^{Cy5AC} und die -R^{Cy5BC} jeweils unabhängig aus Folgendem ausgewählt sind:
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-C(=O)OH, -C(=O)OR^{JJ},
-OC(=O)R^{JJ},
-C(=O)NH₂, -C(=O)NHR^{JJ}, -C(=O)NR^{JJ}₂, -C(=O)R^{JM},
-NHC(=O)R^{JJ}, -NR^{JN}C(=O)R^{JJ},
-NHC(=O)NH₂, -NHC(=O)NHR^{JJ}, -NHC(=O)NR^{JJ}₂, -NHC(=O)R^{JM},
-NR^{JN}C(=O)NH₂, -NR^{JN}C(=O)NHR^{JJ}, -NR^{JN}C(=O)NR^{JJ}₂, -NR^{JN}C(=O)R^{JM},
-NHC(=O)OR^{JJ}, -NR^{JN}C(=O)OR^{JJ},
-OC(=O)NH₂, -OC(=O)NHR^{JJ}, -OC(=O)NR^{JJ}₂, -OC(=O)R^{JM},
-C(=O)R^{JJ},
-SR^{JJ}, -S(=O)R^{JJ}, -S(=O)₂R^{JJ},
-S(=O)NH₂, -S(=O)NHR^{JJ}, -S(=O)NR^{JJ}₂, -S(=O)R^{JM},
-S(=O)₂NH₂, -S(=O)₂NHR^{JJ}, -S(=O)₂NR^{JJ}₂, -S(=O)₂R^{JM},
-NHS(=O)₂R^{JJ}, -NR^{JN}S(=O)₂R^{JJ},
-CN und -NO₂;
die -R^{Cy5AN} und die -R^{Cy5BN} jeweils unabhängig aus Folgendem ausgewählt sind:
-R^{JJ},
-L^{J}-OH, -L^{J}-OR^{JJ},
-L^{J}-NH₂, -L^{J}-NHR^{JJ}, -L^{J}-NR^{JJ}₂, -L^{J}-R^{JM},
-C(=O)R^{JJ},
-C(=O)OR^{JJ},
-C(=O)NH₂, -C(=O)NHR^{JJ}, -C(=O)NR^{JJ}₂, -C(=O)R^{JM} und
-S(=O)₂R^{JJ};
worin:
die -L^{J}- jeweils unabhängig unverzweigtes oder verzweigtes gesättigtes C₁₋₄-Alken sind;
die -R^{JJ} jeweils unabhängig -R^{JJ1}, -R^{JJ2}, -L^{JJ}-R^{JJ2}, -R^{JJ3} oder -L^{JJ}-R^{JJ3} sind;
die -R^{JJ1} jeweils unabhängig unverzweigtes oder verzweigtes gesättigtes C₁₋₆-Alkyl sind und gegebenenfalls mit einer oder mehreren Gruppen substituiert sind, die aus -F, -OH und -OR^{JJJ} ausgewählt sind;
die -R^{JJ2} jeweils gesättigtes C₃₋₆-Cycloalkyl sind und gegebenenfalls mit einer oder mehreren Gruppen substituiert sind, die aus -F, -R^{JJJ}, -OH und -OR^{JJJ} ausgewählt sind;
die -R^{JJJ3} jeweils unabhängig Phenyl oder Naphthyl sind und gegebenenfalls mit einer oder mehreren Gruppen substituiert sind, die aus -F, -Cl, -Br, -I, -R^{JJJ}, OH, -OR^{JJJ}, -OCF₃, -NH₂, -NHR^{JJJ} und -NR^{JJJ}₂ ausgewählt sind;
die -L^{JJ}- jeweils unabhängig unverzweiges oder verzweigtes gesättigtes C₁₋₄-Alkylen sind;
die -R^{JN} jeweils unverzweigtes oder verzweigtes gesättigtes C₁₋₄-Alkyl, Phenyl oder Benzyl sind;
die -R^{JM} jeweils unabhängig Azetidino, Pyrrolidino, Piperidino, Piperazino, Morpholino, Azepano oder Diazepano sind und Folgendes sind:
gegebenenfalls auf einem Kohlenstoff mit einer oder mehreren Gruppen substituiert, die aus den folgenden ausgewählt sind: -R^{JMM}, -C(=O)R^{JMM}, -S(=O)₂R^{JMM}, -F, -NH₂, -NHR^{JMM}, -NRJ^{MM}₂, -OH und -OR^{JMM}; und
gegebenenfalls auf einem sekundären Stickstoff, sofern vorhanden, mit einer Gruppe substituiert, die aus den folgenden ausgewählt ist: -R^{JMM}, -C(=O)R^{JMM}, -C(=O)OR^{JMM} und -S(=O)₂R^{JMM};
die -R^{JJJ} jeweils unabhängig unverzweigtes oder verzweigtes gesättigtes C₁₋₄-Alkyl, Phenyl oder Benzyl sind; und
die -R^{JMM} jeweils unabhängig unverzweigtes oder verzweigtes gesättigtes C₁₋₄-Alkyl, Phenyl oder Benzyl sind.

2. Verbindung nach Anspruch 1, worin:
-L⁷- -CH₂- ist.

3. Verbindung nach Anspruch 1 oder 2, worin:
-X⁵- unabhängig -NH-, -NR^{X5}- oder eine Einfachbindung ist.

4. Verbindung nach Anspruch 1 oder 2, worin:
-X⁵- -NH- ist.

5. Verbindung nach einem der Ansprüche 1 bis 4, worin:
-L⁵- unabhängig -CH₂- oder eine Einfachbindung ist.

6. Verbindung nach einem der Ansprüche 1 bis 4, worin:
-L⁵- -CH₂- ist.

7. Verbindung nach einem der Ansprüche 1 bis 6, worin:
-Ar¹- 1,4-Phenylen ist.

8. Verbindung nach einem der Ansprüche 1 bis 7, worin:
-Ar² Pyridin-2-yl ist.

9. Verbindung nach einem der Ansprüche 1 bis 6, worin:
-Ar¹- 1,4-Phenylen ist; und
-Ar² Pyridin-2-yl ist.

10. Verbindung nach einem der Ansprüche 1 bis 8, worin:
die -R^{AR1C}, sofern vorhanden, und die -R^{AR2C}, sofern vorhanden, jeweils unabhängig aus Folgendem ausgewählt sind: -F, -Me, -Ome, -CF₃ und -OCF₃.

11. Verbindung nach einem der Ansprüche 1 bis 10, worin:
-Cy⁵ -Cy^{5A} ist; und
-Cy^{5A} unabhängig Azetidinyl, Pyrrolidinyl, Piperidinyl, Piperazinyl oder Morpholinyl ist; und Folgendes ist:
gegebenenfalls auf einem Kohlenstoff mit einer oder mehreren Gruppen -R^{Cy5AC} substituiert und
gegebenenfalls auf einem Kohlenstoff mit =O substituiert und
gegebenenfalls auf einem sekundären Stickstoff, sofern vorhanden, mit einer Gruppe -R^{Cy5AN} substituiert.

12. Verbindung nach einem der Ansprüche 1 bis 11, worin:
(a) -Cy^{5A}, sofern vorhanden, Piperidinyl ist;
und Folgendes ist:
gegebenenfalls auf einem Kohlenstoff mit einer oder mehreren Gruppen -R^{Cy5AC} substituiert und
gegebenenfalls auf einem Kohlenstoff mit =O substituiert und
gegebenenfalls auf einem sekundären Stickstoff, sofern vorhanden, mit einer Gruppe -R^{cy5AN} substituiert;
(b) -Cy^{5A}, sofern vorhanden, Piperidin-3-yl ist;
und Folgendes ist:
gegebenenfalls auf einem Kohlenstoff mit einer oder mehreren Gruppen -R^{Cy5AC} substituiert und
gegebenenfalls auf einem Kohlenstoff mit =O substituiert und
gegebenenfalls auf einem sekundären Stickstoff, sofern vorhanden, mit einer Gruppe -R^{cy5AN} substituiert;
(c) -Cy^{5A}, sofern vorhanden, (3S)-Piperidin-3-yl ist;
und Folgendes ist:
gegebenenfalls auf einem Kohlenstoff mit einer oder mehreren Gruppen -R^{Cy5AC} substituiert und
gegebenenfalls auf einem Kohlenstoff mit =O substituiert und
gegebenenfalls auf einem sekundären Stickstoff, sofern vorhanden, mit einer Gruppe -R^{cy5AN} substituiert.

13. Verbindung nach einem der Ansprüche 1 bis 12, worin:
die -R^{Cy5AC}, sofern vorhanden, und die -R^{Cy5BC}, sofern vorhanden, jeweils unabhängig aus Folgendem ausgewählt sind: -F, -Me, -OH, -Ome und -NH₂;
die -R^{Cy5AN}, sofern vorhanden, und die -R^{Cy5BN}, sofern vorhanden, jeweils unabhängig aus Folgendem ausgewählt sind: -R^{JJ}, -C(=O)R^{JJ} und -C(=O)OR^{JJ};
die -R^{JJ} jeweils -R^{JJ1} sind; und
die -R^{JJ1} jeweils unabhängig unverzweigtes oder verzweigtes gesättigtes C₁₋₄-Alkyl sind.

14. Verbindung nach einem der Ansprüche 1 bis 11, worin:
-Cy^{5A}, sofern vorhanden, Piperidin-3-yl ist.

15. Verbindung nach einem der Ansprüche 1 bis 11, worin:
-Cy^{5A}, sofern vorhanden, (3S)-Piperidin-3yl ist.

16. Verbindung nach einem der Ansprüche 1 bis 11, worin:
-Cy^{5A}, sofern vorhanden, (3R,4R)-3-Hydroxypiperidin-4-yl ist.

17. Verbindung nach Anspruch 1, die aus Verbindungen der folgenden Formeln und pharmazeutisch annehmbaren Salzen und Solvaten davon ausgewählt ist:

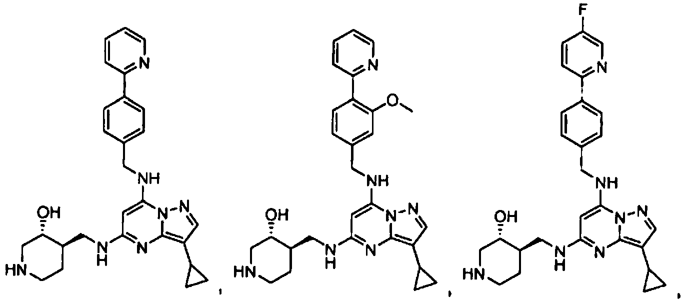

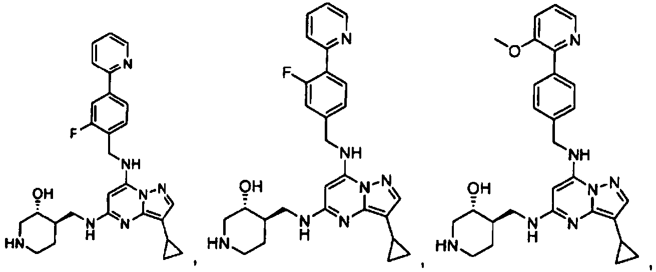

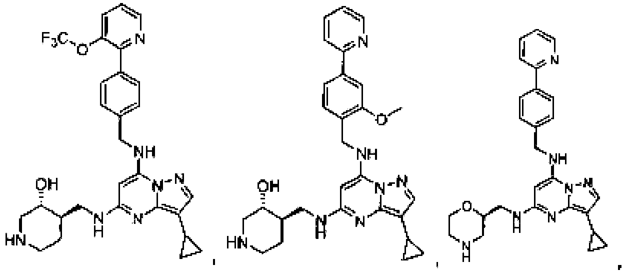

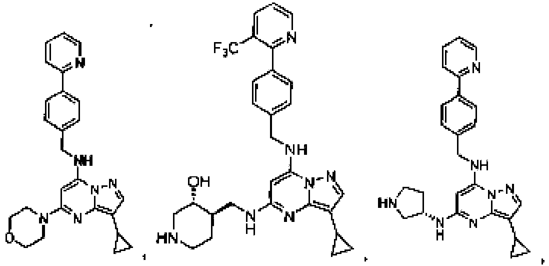

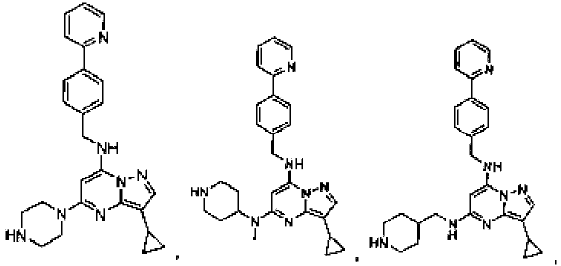

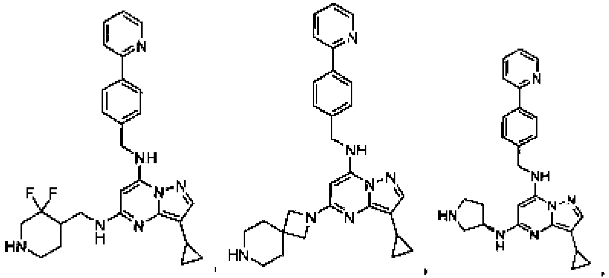

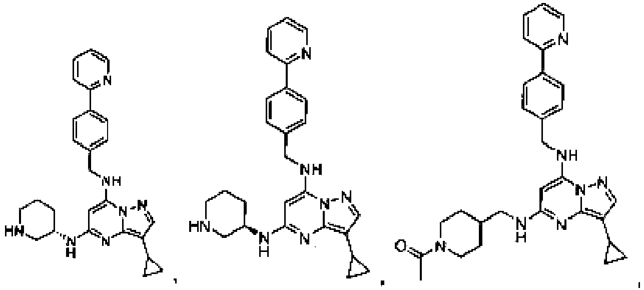

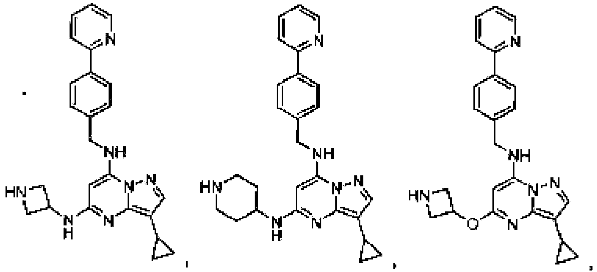

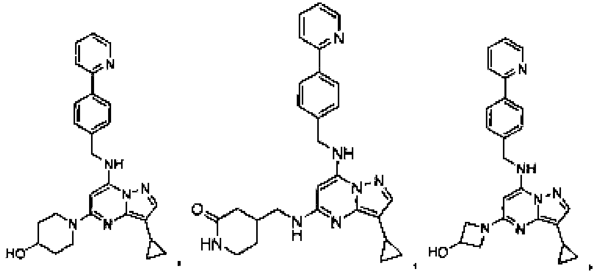

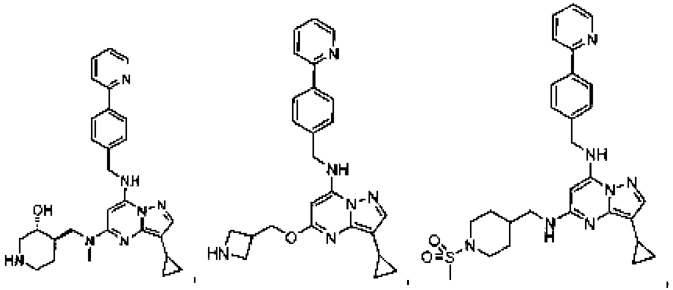

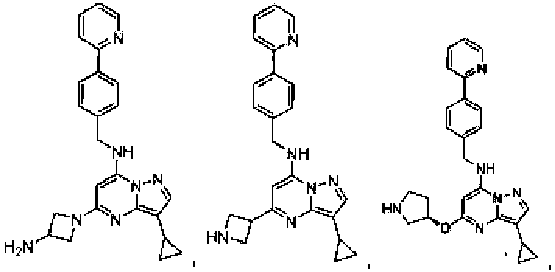

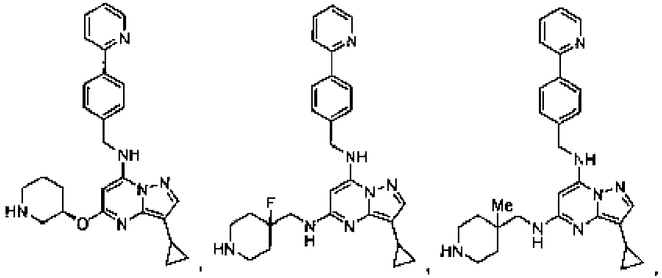

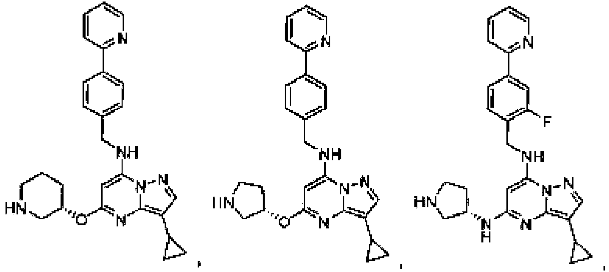

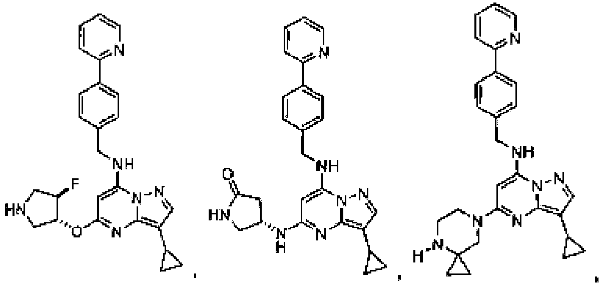

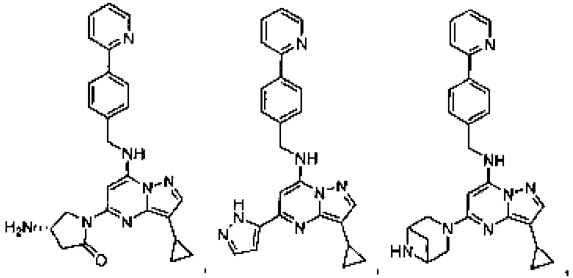

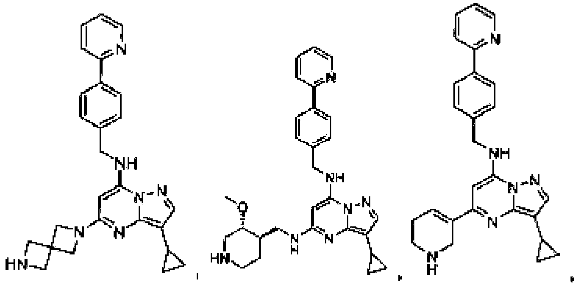

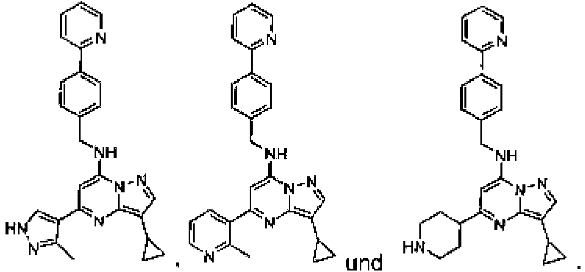

18. Zusammensetzung, die eine Verbindung nach einem der Ansprüche 1 bis 17 und einen pharmazeutisch annehmbaren Träger oder Verdünner umfasst.

19. Verfahren zur Herstellung einer Zusammensetzung, das den Schritt des Vermischens einer Verbindung nach einem der Ansprüche 1 bis 17 und eines pharmazeutisch annehmbaren Trägers oder Verdünners umfasst.

20. Verfahren zum Hemmen der Zellproliferation, zum umfasst der Zellzyklus-Progression, zum Fördern der Apoptose oder für eine Kombination eines oder mehrerer davon, *in vitro,* welches das Kontaktieren einer Zelle mit einer wirksamen Menge einer Verbindung nach einem der Ansprüche 1 bis 17 umfasst.

21. Verbindung nach einem der Ansprüche 1 bis 17 zur Verwendung in einem Verfahren zur Behandlung des menschlichen oder tierischen Körpers durch Therapie.

22. Verbindung zur Verwendung nach Anspruch 21, wobei die Störung Folgendes ist: eine Proliferationsstörung; Krebs; eine Virusinfektion (z. B. HIV); eine neurodegenerative Störung; eine kardiovaskuläre Störung (z. B. Atherosklerose); eine Autoimmunstörung (z. B. rheumatoide Arthritis, systemischer Lupus erythematodes, Psoriasis, Sjögren-Syndrom); oder eine Störung, die durch eine Dysfunktion der Translation in Zellen verursacht wird (z. B. muskuläre Dystrophie, myotonische Dystrophie, amyotrophe Lateralsklerose, spinale muskuläre Atrophie, Fragile-X-Syndrom).

23. Verbindung zur Verwendung nach Anspruch 21, wobei die Störung Folgendes ist: eine Proliferationsstörung.

24. Verbindung zur Verwendung nach Anspruch 21, wobei die Störung Folgendes ist: Krebs.

25. Verbindung zur Verwendung nach einem der Ansprüche 21 bis 24, wobei die Behandlung weiters eine Behandlung mit Folgendem umfasst:
einem DNA-Reparatur-Inhibitor;
einem Immun-Checkpoint-Inhibitor;
einem das Immunsystem stimulierenden Mittel;
einem Zellzyklus-Checkpoint-Inhibitor;
einem weiteren Wirkstoff, der ein Her2-Blocker ist;
einem Transkriptions-Inhibitor; oder
einem weiteren zytotoxischen Chemotherapeutikum.

## Revendications

1. Composé de la formule suivante : 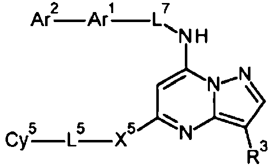 ou un sel ou un solvate pharmaceutiquement acceptable de celui-ci ;
dans laquelle :
-L⁷- est indépendamment -CH₂-, -CH(R^{L7})- ou -C(R^{L7})₂- ;
chaque -R^{L7} est indépendamment alkyle en C₁₋₄ saturé linéaire ou ramifié ;
-Ar¹- est 1,4-phénylène et est éventuellement substitué par un ou plusieurs groupes -R^{AR1C} ;
-Ar² est pyridin-2-yle et est éventuellement substitué *sur le carbone* par un ou plusieurs groupes -R^{AR2C} ;
-X⁵- est indépendamment -NH-, -NR^{X5}-, -O- ou une liaison simple ;
-R^{X5} est un alkyle en C₁₋₄ saturé linéaire ou ramifié ;
-L⁵- est indépendamment -CH₂-, -CH(R^{L5})-, -C(R^{L5})₂- ou une liaison simple ;
chaque R^{L5} est indépendamment alkyle en C₁₋₄ saturé linéaire ou ramifié ;
-Cy⁵ est indépendamment -Cy^{5A} ou -Cy^{5B} ;
-Cy^{5A} est hétérocyclyle en C₄₋₁₀ non aromatique ayant au moins un atome d'azote dans le noyau et est :
éventuellement substitué *sur le carbone* par un ou plusieurs groupes -R^{Cy5AC} ; et
éventuellement substitué *sur le carbone* par =O ; et
éventuellement substitué *sur l'azote secondaire, s'il est présent,* par un groupe -R^{Cy5AN} ;
-Cy^{5B} est hétéroaryle en C₅₋₆ ayant au moins un atome d'azote dans le noyau et est :
éventuellement substitué *sur le carbone* par un ou plusieurs groupes -R^{Cy5BC} ; et
éventuellement substitué *sur l'azote secondaire, s'il est présent,* par un groupe -R^{Cy5BN} ;
-R³ est -R^{3B} ;
-R^{3B} est cyclopropyle ;
et dans laquelle :
chaque -R^{AR1C} et chaque -R^{AR2C} est choisi indépendamment parmi :
-R^{TT},
-F, -Cl, -Br, -I,
-OH, -OR^{TT},
-L^{T}-OH, -L^{T}-OR^{TT},
-CF₃, -CHF₂, -OCF₃, -OCHF₂,
-NH₂, -NHR^{TT}, -NR^{TT}₂, -R^{TM},
-L^{T}-NH₂, -L^{T}-NHR^{TT}, -L^{T}-NR^{TT}₂, -L^{T}-R^{TM},
-C(=O)OH, -C(=O)OR^{TT},
-OC(=O)R^{TT},
-C(=O)NH₂, -C(=O)NHR^{TT}, -C(=O)NR^{TT}₂, -C(=O)R^{TM},
-NHC(=O)R^{TT}, -NR^{TN}C(=O)R^{TT},
-NHC (=O)NH₂, -NHC (=O)NHR^{TT}, -NHC (=O)NR^{TT}₂, -NHC (=O)R^{TM},
-NR^{TN}C(=O)NH₂, -NR^{TN}C(=O)NHR^{TT}, -NR^{TN}C(=O) NR^{TT}₂, -NR^{TN}C-(=O)R^{TM},
-NHC (=O)OR^{TT}, -NR^{TN}C(=O)OR^{TT},
-OC(=O)NH₂, -OC(=O)NHR^{TT}, -OC (=O)NR^{TT}₂, -OC (=O)R^{TM},
-C(=O)R^{TT},
-SR^{TT}, -S(=O)R^{TT}, -S(=O)₂R^{TT},
-S(=O)NH₂, -S(=O)NHR^{TT}, -S(=O)NR^{TT}₂, -S(=O)R^{TM},
-S(=O)₂NH₂, -S(=O)₂NHR^{TT}, -S(=O)₂NR^{TT}₂, -S (=O)₂R^{TM},
-NHS (=O)₂R^{TT}, -NR^{TN}S(=O)₂R^{TT},
-CN et -NO₂ ;
où :
chaque -L^{T}- est indépendamment alkylène en C₁₋₄ saturé linéaire ou ramifié ;
chaque -R^{TT} est indépendamment -R^{TT1}, -R^{TT2}, -L^{TT}-R^{TT2}, -R^{TT3} ou -L^{TT}-R^{TT3} ;
chaque -R^{TT1} est indépendamment alkyle en C₁₋₆ saturé linéaire ou ramifié et est éventuellement substitué par un ou plusieurs groupes choisis parmi -F, -OH, et -OR^{TTT} ;
chaque -R^{TT2} est cycloalkyle en C₃₋₆ saturé et est éventuellement substitué par un ou plusieurs groupes choisis parmi -F, -R^{TTT}, -OH et -OR^{TTT} ;
chaque -R^{TT3} est indépendamment phényle ou naphtyle et est éventuellement substitué par un ou plusieurs groupes choisis parmi -F, -Cl, -Br, -I, -R^{TTT}, -OH, -OR^{TTT}, -OCF₃, -NH₂, -NHR^{TTT} et -NR^{TTT}₂ ;
chaque -L^{TT}- est indépendamment alkylène en C₁₋₄ saturé linéaire ou ramifié ;
chaque -R^{TN} est alkyle en C₁₋₄ saturé linéaire ou ramifié, phényle ou benzyle ;
chaque -R^{TM} est indépendamment azétidino, pyrrolidino, pipéridino, pipérazino, morpholino, azépano ou diazépano et est :
éventuellement substitué *sur le carbone* par un ou plusieurs groupes choisis parmi : -R^{TMM} -C(=O)R^{TMM}, -S(=O)₂R^{TMM}, -F, -NH₂, -NHR^{TMM}, -NR^{TMM}₂, -OH et -OR^{TMM} ; et
éventuellement substitué *sur l'azote secondaire, s'il est présent,* par un groupe choisi parmi -R^{TMM} -C(=O)R^{TM}, -C(=O)OR^{TMM} et -S(=O)₂R^{TMM} ;
chaque -R^{TTT} est indépendamment alkyle en C₁₋₄ saturé linéaire ou ramifié, phényle ou benzyle ; et
chaque -R^{TMM} est indépendamment alkyle en C₁₋₄ saturé linéaire ou ramifié, phényle ou benzyle ;
et dans laquelle :
chaque -R^{Cy5AC} et chaque -R^{Cy5BC} est choisi indépendamment parmi :
-R^{JJ},
-F, -Cl, -Br, -I,
-OH, -OR^{JJ},
-L^{J}-OH, -L^{J}-OR^{JJ},
-CF₃, -CHF₂, -OCF₃, -OCHF₂,
-NH₂, -NHR^{JJ}, -NR^{JJ}₂, -R^{JM},
-L^{J}-NH₂, -L^{J}-NHR^{JJ}, -L^{J}-NR^{JJ}₂, -L^{J}-R^{JM},
-C(=O)OH, -C(=O)OR^{JJ},
-OC (=O) R^{JJ},
-C(=O)NH₂, -C (=O) NHR^{JJ}, -C(=O)NR^{JJ}₂, -C(=O)R^{JM},
-NHC(=O)R^{JJ}, -NR^{JN}C(=O)R^{JJ},
-NHC (=O)NH₂, -NHC (=O)NHR^{JJ}, -NHC (=O)NR^{JJ}₂, -NHC (=O)R^{JM},
-NR^{JJ}NC(=O)NH₂, -NR^{JJ}NC(=O)NHR^{JJ}, -NR^{JJ}NC(=O)NR^{JJ}₂, -NR^{JJ}NC(=O)R^{JM},
-NHC(=O)OR^{JJ}, -NR^{JN}C(=O)OR^{JJ},
-OC(=O)NH₂, -OC(=O)NHR^{JJ}, -OC(=O)NR^{JJ}₂, -OC (=O)R^{JM},
-C (=O)R^{JJ},
-SR^{JJ}, -S (=O)R^{JJ}, -S (=O)₂R^{JJ},
-S(=O)NH₂, -S(=O)NHR^{JJ}, -S(=O)NR^{JJ}₂, -S(=O)R^{JM},
-S (=O)₂NH₂, -S (=O)₂NHR^{JJ}, -S (=O)₂NR^{JJ}₂, -S (=O)₂R^{JM},
-NHS (=O)₂R^{JJ}, -NR^{JJ}NS(=O)₂R^{JJ},
-CN et -NO₂;
chaque -R^{Cy5AN} et chaque -R^{Cy5BN} est choisi indépendamment parmi :
-R^{JJ},
-L^{J}-OH, -L^{J}-OR^{JJ},
-L^{J}-NH₂, -L^{J}-NHR^{JJ}, -L^{J}-NR^{JJ}₂, -L^{J}-R^{JM},
-C (=O)R^{JJ},
-C (=O) OR^{JJ},
-C(=O)NH₂, -C (=O)NHR^{JJ}, -C(=O)NR^{JJ}₂, -C(=O)R^{JM} et
-S(=O)₂R^{JJ};
où :
chaque -L^{J}- est indépendamment alkylène en C₁₋₄ saturé linéaire ou ramifié ;
chaque -R^{JJ} est indépendamment -R^{JJ1}, -R^{JJ2}, -L^{JJ}-R^{JJ2}, -R^{JJ3} ou -L^{JJ}-R^{JJ3} ;
chaque ; -R^{JJ1} est indépendamment alkyle en C₁₋₆ saturé linéaire ou ramifié et est éventuellement substitué par un ou plusieurs groupes choisis parmi -F, -OH et -OR^{JJJ} ;
chaque -R^{JJ2} est cycloalkyle en C₃₋₆ saturé et est éventuellement substitué par un ou plusieurs groupes choisis parmi -F, -R^{JJJ}, -OH et -OR^{JJJ} ;
chaque -R^{JJ3} est indépendamment phényle ou naphtyle et est éventuellement substitué par un ou plusieurs groupes choisis parmi -F, -Cl, -Br, -I, -R^{JJJ}, -OH, -OR^{JJJ}, -OCF₃, -NH₂, -NHR^{JJJ} et -NR^{JJJ}2 ;
chaque -L^{JJ}- est indépendamment alkylène en C₁₋₄ saturé linéaire ou ramifié ;
chaque -R^{JN} est alkyle en C₁₋₄ saturé linéaire ou ramifié, phényle ou benzyle ;
chaque -R^{JM} est indépendamment azétidino, pyrrolidino, pipéridino, pipérazino, morpholino, azépano ou diazépano et est :
éventuellement substitué *sur le carbone* par un ou plusieurs groupes choisis parmi -R^{JMM} -C(=O)R^{JMM}, -S(=O)₂R^{JMM}, -F, -NH₂, -NHR^{JMM}, -NR^{JMM}₂, -OH et -OR^{JMM} ; et
éventuellement substitué *sur l'azote secondaire, s'il est présent,* par un groupe choisi parmi -R^{JMM}, -C (=O)R^{JMM}, -C(=O) OR^{JMM} et -S(=O)₂R^{JMM} ;
chaque -R^{JJJ} est indépendamment alkyle en C₁₋₄ saturé linéaire ou ramifié, phényle ou benzyle ; et
chaque -R^{JMM} est indépendamment alkyle en C₁₋₄ saturé linéaire ou ramifié, phényle ou benzyle.

2. Composé selon la revendication 1, dans lequel :
-L⁷- est -CH₂-.

3. Composé selon la revendication 1 ou la revendication 2, dans lequel -X⁵- est indépendamment -NH-, -NR^{X5}- ou une liaison simple.

4. Composé selon la revendication 1 ou la revendication 2, dans lequel
-X⁵- est -NH-.

5. Composé selon l'une quelconque des revendications 1 à 4, dans lequel :
-L⁵- est indépendamment -CH₂- ou une liaison simple.

6. Composé selon l'une quelconque des revendications 1 à 4, dans lequel :
-L⁵- est -CH₂-.

7. Composé selon l'une quelconque des revendications 1 à 6, dans lequel :
-Ar¹- est 1,4-phénylène.

8. Composé selon l'une quelconque des revendications 1 à 7, dans lequel :
-Ar² est pyridin-2-yle.

9. Composé selon l'une quelconque des revendications 1 à 6, dans lequel :
-Ar¹- est 1,4-phénylène ; et
-Ar² est pyridin-2-yle.

10. Composé selon l'une quelconque des revendications 1 à 8, dans lequel :
chaque -R^{AR1C}, s'il est présent, et chaque -R^{AR2C}, s'il est présent, est choisi indépendamment parmi -F, -Me, -OMe, -CF₃ et -OCF₃.

11. Composé selon l'une quelconque des revendications 1 à 10, dans lequel :
-Cy⁵ est -Cy^{5A} ; et
-C^{5A} est indépendamment azétidinyle, pyrrolidinyle, pipéridinyle, pipérazinyle ou morpholinyle et est :
éventuellement substitué *sur le carbone* par un ou plusieurs groupes -R^{Cy5AC} ; et
éventuellement substitué *sur le carbone* par =O ; et
éventuellement substitué *sur l'azote secondaire, s'il* est *présent,* par un groupe -R^{Cy5AN}.

12. Composé selon l'une quelconque des revendications 1 à 11, dans lequel :
(a) -Cy^{5A}, s'il est présent, est pipéridinyle et est :
éventuellement substitué *sur le carbone* par un ou plusieurs groupes -R^{Cy5AC} ; et
éventuellement substitué *sur le carbone* par =O ; et
éventuellement substitué *sur l'azote secondaire, s'il est présent,* par un groupe -R^{Cy5AN} ;
(b) -Cy^{5A}, s'il est présent, est pipéridin-3-yle et est :
éventuellement substitué *sur le carbone* par un ou plusieurs groupes -R^{Cy5AC} ; et
éventuellement substitué *sur le carbone* par =O ; et
éventuellement substitué *sur l'azote secondaire* par un groupe -R^{Cy5AN} ; ou
(c) -Cy^{5A}, s'il est présent, est (3S)-pipéridin-3-yle et est :
éventuellement substitué *sur le carbone* par un ou plusieurs groupes -R^{Cy5AC} ; et
éventuellement substitué *sur le carbone* par =O ; et
éventuellement substitué *sur l'azote secondaire* par un groupe -R^{Cy5AN} ;

13. Composé selon l'une quelconque des revendications 1 à 12, dans lequel :
chaque -R^{Cy5AC}, s'il est présent, et chaque -R^{Cy5BC}, s'il est présent, est choisi indépendamment parmi -F, -Me, -OH, -OMe et -NH₂ ;
chaque -R^{Cy5AN}, s'il est présent, et chaque -R^{Cy5BN}, s'il est présent, est choisi indépendamment parmi -R^{JJ}, -C(=O)^{JJ} et -C (=O) O^{JJ};
chaque -R^{JJ} est -R^{JJ1} ; et
chaque -R^{JJ1} est indépendamment alkyle en C₁₋₄ saturé linéaire ou ramifié.

14. Composé selon l'une quelconque des revendications 1 à 11, dans lequel :
-Cy^{5A}, s'il est présent, est pipéridin-3-yle.

15. Composé selon l'une quelconque des revendications 1 à 11, dans lequel :
-Cy^{5A}, s'il est présent, est (3S)-pipéridin-3-yle.

16. Composé selon l'une quelconque des revendications 1 à 11, dans lequel :
-Cy^{5A}, s'il est présent, est (3R,4R)-3-hydroxypipéridin-4-yle.

17. Composé selon la revendication 1, choisi parmi les composés des formules suivantes et des sels et solvates pharmaceutiquement acceptables de ceux-ci :

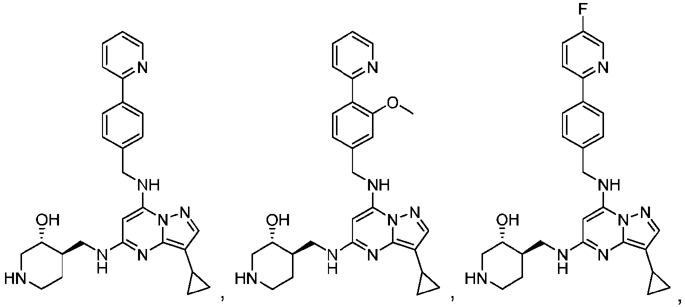

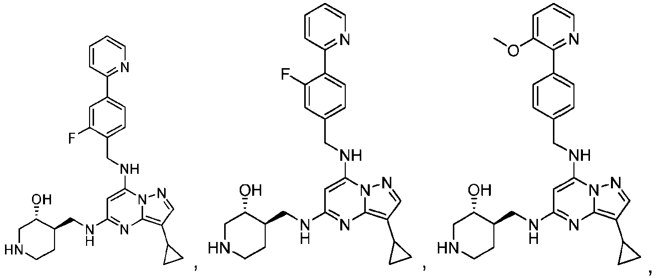

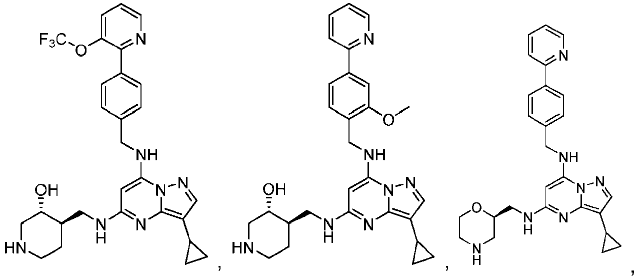

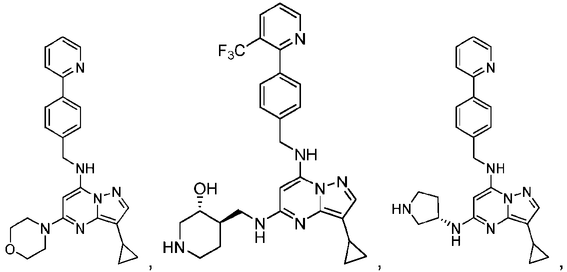

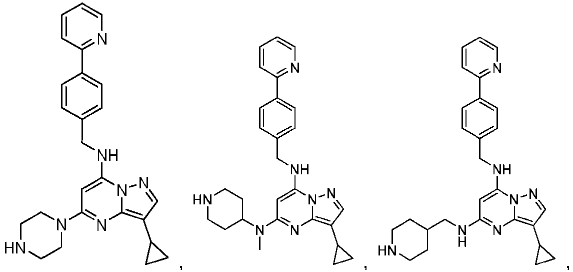

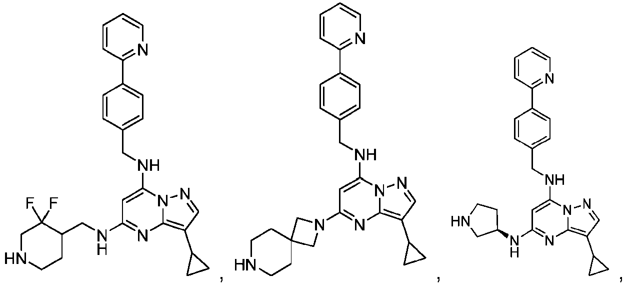

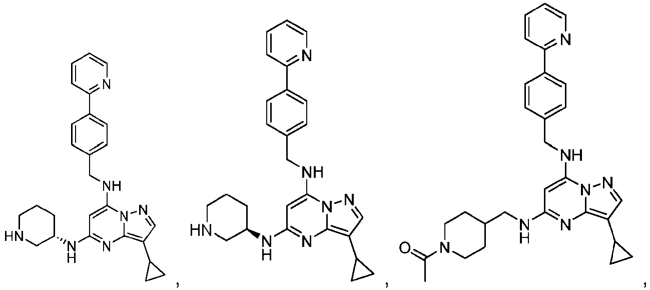

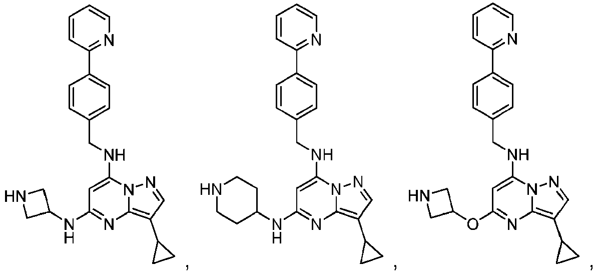

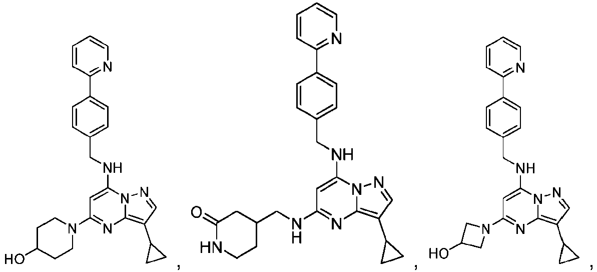

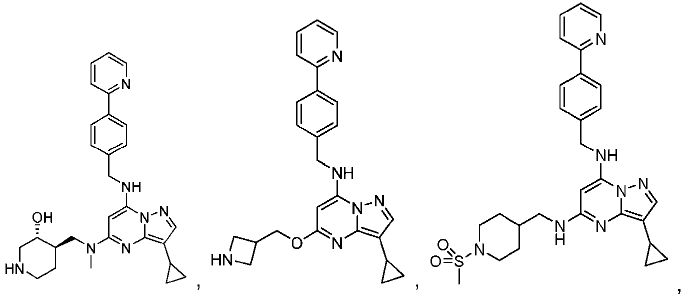

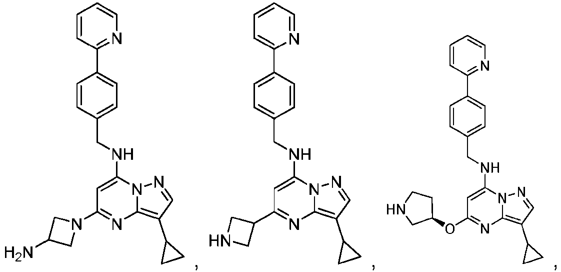

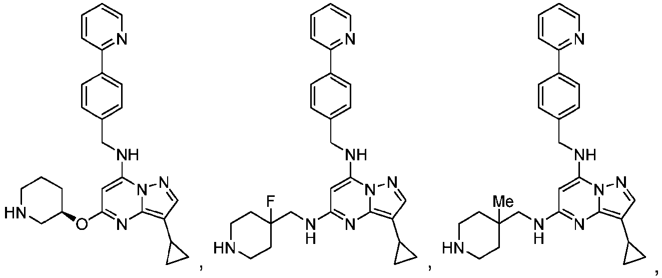

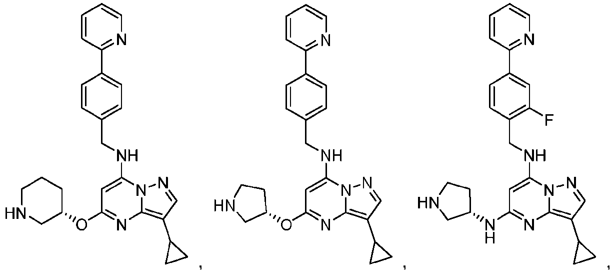

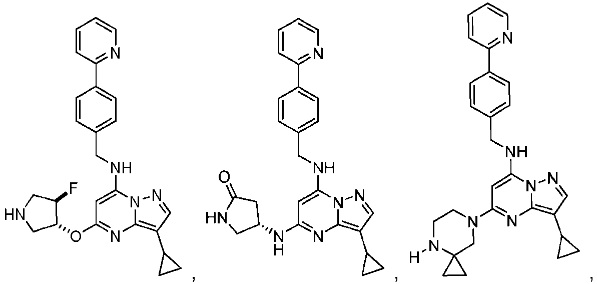

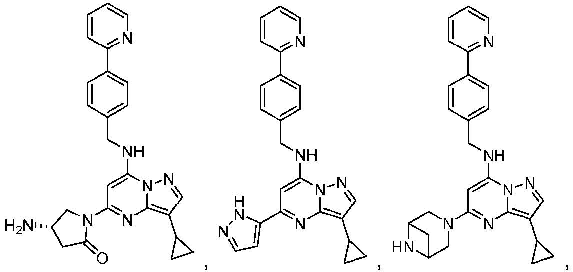

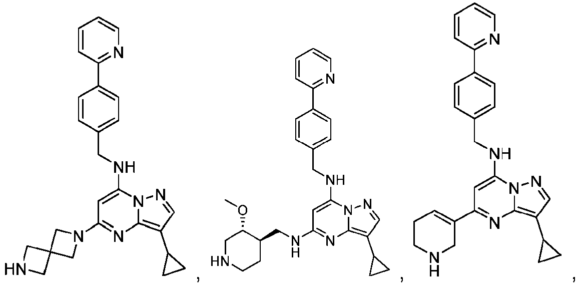

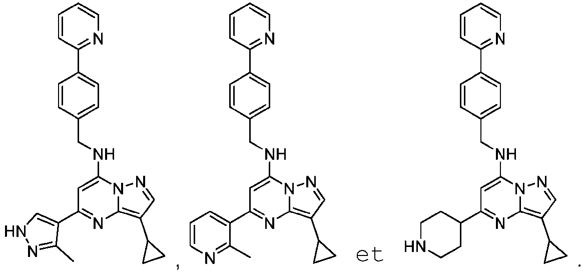

18. Composition comprenant un composé selon l'une quelconque des revendications 1 à 17 et un excipient ou diluant pharmaceutiquement acceptable.

19. Procédé de préparation d'une composition, comprenant l'étape consistant à mélanger un composé selon l'une quelconque des revendications 1 à 17 et un excipient ou diluant pharmaceutiquement acceptable.

20. Procédé d'inhibition de la prolifération cellulaire, d'inhibition de la progression du cycle cellulaire, de promotion de l'apoptose, ou d'une combinaison d'une ou plusieurs de celles-ci, *in vitro,* comprenant la mise en contact d'une cellule avec une quantité efficace d'un composé selon l'une quelconque des revendications 1 à 17.

21. Composé selon l'une quelconque des revendications 1 à 17, destiné à être utilisé dans un procédé de traitement du corps humain ou animal par thérapie.

22. Composé destiné à être utilisé selon la revendication 21, dans lequel le trouble est un trouble prolifératif ; un cancer ; une infection virale (par exemple le VIH) ; un trouble neurodégénératif (par exemple la maladie d'Alzheimer, la maladie de Parkinson) ; une ischémie ; une maladie rénale ; un trouble cardiovasculaire (par exemple l'athérosclérose) ; un trouble auto-immun (par exemple la polyarthrite rhumatoïde, le lupus érythémateux systémique, le psoriasis, le syndrome de Sjδgren) ; ou un trouble provoqué par un dysfonctionnement de la traduction dans les cellules (par exemple la dystrophie musculaire, la dystrophie myotonique, la sclérose latérale amyotrophique, l'atrophie musculaire spinale, le syndrome de l'X fragile).

23. Composé destiné à être utilisé selon la revendication 21, dans lequel le trouble est un trouble prolifératif.

24. Composé destiné à être utilisé selon la revendication 21, dans lequel le trouble est un cancer.

25. Composé destiné à être utilisé selon l'une quelconque des revendications 21 à 24, dans lequel le traitement comprend en outre un traitement avec :
un inhibiteur de la réparation de l'ADN ;
un inhibiteur de point de contrôle immunitaire ;
un agent stimulant le système immunitaire ;
un inhibiteur de point de contrôle du cycle cellulaire ;
un autre principe actif qui est un bloqueur de Her2 ;
un inhibiteur transcriptionnel ; ou
un autre agent chimiothérapeutique cytotoxique.
